# EUROPEAN PATENT SPECIFICATION

(11) **EP 3 507 306 B1**
(45) Date of publication and mention of the grant of the patent: **01.10.2025**
(21) Application number: 17765002.5
(22) Date of filing: 30.08.2017
(51) Int. Cl.: C07K 16/46, C07K 16/28, A61K 39/395, A61P 35/00

(54) **BISPECIFIC IMMUNOMODULATORY ANTIBODIES THAT BIND COSTIMULATORY AND CHECKPOINT RECEPTORS**
BISPEZIFISCHE IMMUNMODULATORISCHE ANTIKÖRPER, DIE AN KOSTIMULATORISCHE UND CHECKPOINT-REZEPTOREN BINDEN
ANTICORPS IMMUNOMODULATEURS BISPÉCIFIQUES QUI SE LIENT À DES RÉCEPTEURS DE COSTIMULATION ET DE POINTS DE CONTRÔLE

(30) Priority: 30.08.2016 US 201662381239 P; 07.02.2017 US 201762456033 P; 31.03.2017 US 201762479723 P; 14.06.2017 US 201715623314
(43) Date of publication of application: 10.07.2019
(73) Proprietor: Xencor, Inc., Pasadena, CA 91107 (US)
(72) Inventor: BERNETT, Matthew, Monrovia, CA 91016 (US); MOORE, Gregory, Monrovia, CA 91016 (US); DESJARLAIS, John, Monrovia, CA 91016 (US); HEDVAT, Michael, Monrovia, CA 91016 (US); BONZON, Christine, Monrovia, CA 91016 (US); MUCHHAL, Umesh, S, Monrovia, CA 91016 (US)
(74) Representative: HGF
(86) International application number: PCT/US2017/049497
(87) International publication number: WO 2018/045110

(56) References cited:
- WO-A1-2013/164694
- WO-A1-2014/145907
- WO-A1-2014/207064
- WO-A1-2016/110584
- WO-A1-2016/115274
- WO-A1-2016/120789
- WO-A2-2015/095423
- WO-A2-2016/086196
- US-A1- 2012 121 597
- US-A1- 2014 056 879
- ROSS STEWART ET AL: "The role of Fc gamma receptors in the activity of immunomodulatory antibodies for cancer", JOURNAL FOR IMMUNOTHERAPY OF CANCER, BIOMED CENTRAL, LONDON, UK, vol. 2, no. 1, 19 August 2014 (2014-08-19), pages 29, XP021193931, ISSN: 2051-1426, DOI: 10.1186/S40425-014-0029-X
- N. POIRIER ET AL: "CD28-Specific Immunomodulating Antibodies: What Can Be Learned From Experimental Models? : CD28-Specific Immunomodulating Antibodies", AMERICAN JOURNAL OF TRANSPLANTATION, vol. 12, no. 7, 1 July 2012 (2012-07-01), DK, pages 1682 - 1690, XP055590905, ISSN: 1600-6135, DOI: 10.1111/j.1600-6143.2012.04032.x
- VAN BILSEN K ET AL: "The neonatal Fc receptor is expressed by human lymphocytes", JOURNAL OF TRANSLATIONAL MEDICINE, BIOMED CENTRAL, vol. 8, no. Suppl 1, 25 November 2010 (2010-11-25), pages P1, XP021078948, ISSN: 1479-5876, DOI: 10.1186/1479-5876-8-S1-P1
- MARSH C B ET AL: "Monocyte IL-8 release is induced by two independent Fc gamma R- mediated pathways", THE JOURNAL OF IMMUNOLOGY,, vol. 157, no. 6, 15 September 1996 (1996-09-15), pages 2632 - 2637, XP002335639, ISSN: 0022-1767
- SPIESS CHRISTOPH ET AL: "Alternative molecular formats and therapeutic applications for bispecific antibodies", MOLECULAR IMMUNOLOGY, PERGAMON, GB, vol. 67, no. 2, 27 January 2015 (2015-01-27), pages 95 - 106, XP029246892, ISSN: 0161-5890, DOI: 10.1016/J.MOLIMM.2015.01.003
- CELINE MONNET ET AL: "Selection of IgG variants with increased FcRn binding using random and directed mutagenesis: impact on effector functions", FRONTIERS IN IMMUNOLOGY, vol. 6, no. 39, 4 February 2015 (2015-02-04), pages 1 - 14, XP055238838, DOI: 10.3389/fimmu.2015.00039
- SONDERMANN PETER ET AL: "Harnessing Fc receptor biology in the design of therapeutic antibodies", CURRENT OPINION IN IMMUNOLOGY, ELSEVIER, OXFORD, GB, vol. 40, 30 March 2016 (2016-03-30), pages 78 - 87, XP029551351, ISSN: 0952-7915, DOI: 10.1016/J.COI.2016.03.005
- MADRENAS JOAQUÍN ET AL: "Conversion of CTLA-4 from inhibitor to activator of T cells with a bispecific tandem single-chain Fv ligand.", JOURNAL OF IMMUNOLOGY, vol. 172, no. 10, 15 May 2004 (2004-05-15), (BALTIMORE, MD. : 1950), pages 5948 - 5956, XP002775234, ISSN: 0022-1767, DOI: 10.4049/jimmunol.172.10.5948
- YOKOSUKA TADASHI ET AL: "Spatiotemporal basis of CTLA-4 costimulatory molecule-mediated negative regulation of T cell activation.", IMMUNITY, vol. 33, no. 3, 24 September 2010 (2010-09-24), pages 326 - 339, XP002775235, ISSN: 1097-4180, DOI: 10.1016/j.immuni.2010.09.006
- CARPENTER ERICA L ET AL: "Activation of human B cells by the agonist CD40 antibody CP-870,893 and augmentation with simultaneous toll-like receptor 9 stimulation.", JOURNAL OF TRANSLATIONAL MEDICINE, vol. 7, 93, 11 November 2009 (2009-11-11), pages 1 - 10, XP021063609, ISSN: 1479-5876, DOI: 10.1186/1479-5876-7-93
- XIAOZHOU FAN ET AL: "Engagement of the ICOS pathway markedly enhances efficacy of CTLA-4 blockade in cancer immunotherapy", THE JOURNAL OF EXPERIMENTAL MEDICINE, vol. 211, no. 4, 31 March 2014 (2014-03-31), US, pages 715 - 725, XP055414943, ISSN: 0022-1007, DOI: 10.1084/jem.20130590
- GILBOA ELI ET AL: "Use of Oligonucleotide Aptamer Ligands to Modulate the Function of Immune Receptors", CLINICAL CANCER RESEARCH, vol. 19, no. 5, March 2013 (2013-03-01), pages 1054 - 1062, XP002775237

## Description

### BACKGROUND OF THE INVENTION

Tumor-reactive T cells lose their cytotoxic ability over time due to up-regulation of inhibitory immune checkpoints such as PD-1 and CTLA-4. Two parallel therapeutic strategies are being pursued for de-repressing tumor-reactive T cells so that they can continue to kill tumor cells.

The first approach is immune immunomodulatory blockade by treating with antagonistic monoclonal antibodies that bind to either the immunomodulatory itself (PD-1, CTLA-4, etc.) or its ligand (PD-L1, PD-L2, CD80, CD86, etc.), thus removing the inhibitory signals holding back tumor-reactive T cells from tumor cell killing. Immunomodulatory receptors such as CTLA-1, PD-1 (programmed cell death 1), TIM-3 (T cell immunoglobulin and mucin domain 3), LAG-3 (lymphocyte-activation gene 3), TIGIT (T cell immunoreceptor with Ig and ITIM domains), and others, inhibit the activation, proliferation, and/or effector activities of T cells and other cell types. Guided by the hypothesis that immunomodulatory receptors suppress the endogenous T cell response against tumor cells, preclinical and clinical studies of anti-CTLA4 and anti-PD1 antibodies, including nivolumab, pembrolizumab, ipilimumab, and tremelimumab, have indeed demonstrated that immunomodulatory blockade results in impressive anti-tumor responses, stimulating endogenous T cells to attack tumor cells, leading to long-term cancer remissions in a fraction of patients with a variety of malignancies. Unfortunately, only a subset of patients responds these therapies, with response rates generally ranging from 10 to 30% and sometimes higher for each monotherapy, depending on the indication and other factors.

The second approach for de-repressing tumor-reactive T cells is T cell costimulation by treating with agonistic antibodies that bind to costimulatory proteins such as ICOS, thus adding a positive signal to overcome the negative signaling of the immune checkpoints.

WO 20161/10584 discloses agonistic TNF receptor binding agents. Madrenas et al. (J Immunol (2004) 172 (10): 5948-5956.) discloses the conversion of CTLA-4 from inhibitor to activator of T Cells with a bispecific tandem single-chain Fv Ligand. Yokosuka et al., (Immunity. 2010 Sep 24;33(3):326-39) discloses the spatiotemporal basis of CTLA-4 costimulatory molecule-mediated negative regulation of T Cell activation. WO 2016/086196 discloses heterodimeric antibodies that bind CD3 and CD38. US 2012/121597 discloses fusion proteins for HIV therapy. US 2014/056879 discloses Fc variants that extend antibody half-life. Carpenter et al. (J Transl Med. 2009 Nov 11;7:93) discloses the activation of human B cells by the agonist CD40 antibody CP-870,893 and augmentation with simultaneous toll-like receptor 9 stimulation. WO 2016120789 discloses agonistic ICOS binding proteins. Fan et al. (J Exp Med. 2014 Apr 7;211(4):715-25) discloses that engagement of the ICOS pathway markedly enhances efficacy of CTLA-4 blockade in cancer immunotherapy. Gilboa et al. (Clin Cancer Res (2013) 19 (5): 1054-1062) discloses the use of oligonucleotide aptamer ligands to modulate the function of immune receptors. WO 2013/164694 discloses targeted/immunomodulatory fusion proteins and methods for making the same. WO 2014/207064 discloses bispecific molecules capable of specifically binding to both CTLA-4 and CD40. WO 2016/115274 discloses multispecific immunomodulatory antigen-binding constructs. WO 2015/095423 discloses a combination therapy comprising OX40 binding agonists and PD-1 axis binding antagonists. Stewart et al. (J Immunotherapy Cancer 2, 29 (2014) discloses the role of Fc gamma receptors in the activity of immunomodulatory antibodies for cancer. Poirier et al. Am J Transplant. 2012 Jul;12(7):1682-90) discloses a study on CD28-Specific immunomodulating antibodies: what can be learned from experimental models? Van Bilsen K. et al. (J Transl Med. 2010; 8(Suppl 1): P1) discloses that the neonatal Fc receptor is expressed by human lymphocytes. Marsh C. B. et al. ( J Immunol. 1996 Sep 15;157(6):2632-7) discloses that monocyte IL-8 release is induced by two independent Fc gamma R- mediated pathways. Spiess et al. ( Mol Immunol 2015 Oct;67(2 Pt A):95-106) discloses alternative molecular formats and therapeutic applications for bispecific antibodies. WO 2014/145907 discloses the targeting of t cells with heterodimeric proteins. Monnet et al. (Front Immunol. 2015 Feb 4;6:3) discloses a study on the selection of lgG variants with increased FcRn binding using random and directed mutagenesis: impact on effector functions. Sondermann et al. (Curr Opin Immunol. 2016 Jun;40:78-87) discloses the harnessing of Fc receptor biology in the design of therapeutic antibodies.

Accordingly, disclosed herein are bispecific antibodies that bind to costimulatory receptors (e.g. ICOS, GITR, OX40, 4-1BB) as well as checkpoint receptors (e.g. PD-1, PD-L1, CTLA-4, LAG-3, TIM-3, BTLA and TIGIT.

### SUMMARY OF THE INVENTION

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes. Any references in the description to methods of treatments refer to the compounds, pharmaceutical compositions and medicaments of the present invention for use in a method for treatment of the human (or animal) body by therapy (or for diagnosis).

The present invention relates to a heterodimeric antibody comprising a first monomer, a second monomer, and a light chain, wherein the first monomer consists of amino acid sequence of SEQ ID NO:26362; the second monomer consists of the amino acid sequence of SEQ ID NO:26367; and the light chain consists of the amino acid sequence of SEQ ID NO:26377.

Disclosed herein are bispecific antibodies that monovalently binds a human costimulatory receptor and monovalently binds a human checkpoint receptor for use in activating T cells for the treatment of cancer.

The costimulatory receptor may be selected from the group consisting of ICOS, GITR, OX40 and 4-1BB.

The checkpoint receptor may be selected from the group consisting of PD-1, PD-L1, CTLA-4, LAG-3, TIGIT and TIM-3.

The antibody may bind an antigen pair selected from: ICOS and PD-1, ICOS and CTLA-4, ICOS and LAG-3, ICOS and TIM-3, ICOS and PD-L1, ICOS and BTLA, ICOS and TIGIT, GITR and TIGIT, GITR and PD-1, GITR and CTLA-4, GITR and LAG-3, GITR and TIM-3, GITR and PD-L1, GITR and BTLA, OX40 and PD-1, OX40 and TIGIT, OX40 and CTLA-4, IC OX40 OS and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1, TIGIT and 4-1BB and 4-1BB and BTLA.

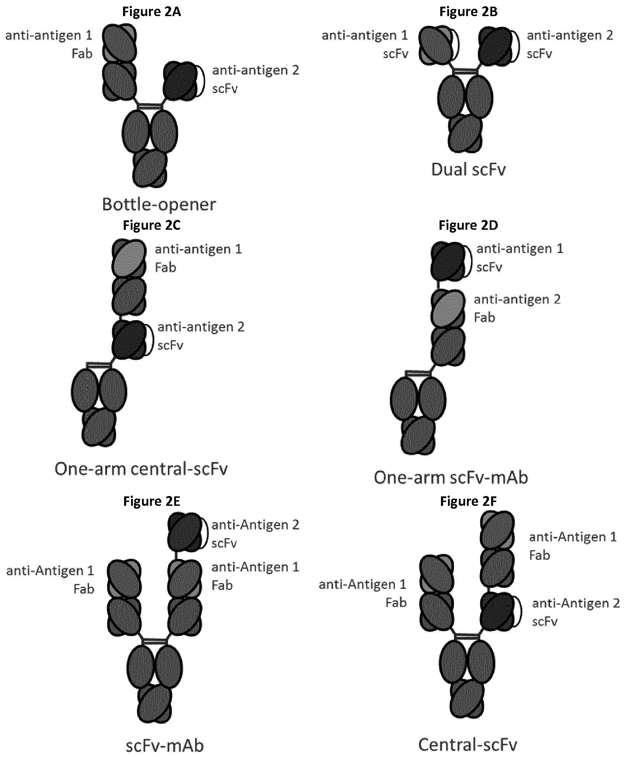

The bispecific antibody may have a format selected from those outlined in Figure 2A-2N.

The disclosure also provides heterodimeric antibodies comprising: a) a first heavy chain comprising a first Fc domain, an optional domain linker and a first antigen binding domain comprising an scFv that binds a first antigen; b) a second heavy chain comprising a heavy chain comprising a heavy chain constant domain comprising a second Fc domain, a hinge domain, a CH1 domain and a variable heavy domain; and c) a light chain comprising a variable light domain and a light chain constant domain; wherein said variable heavy domain and said variable light domain form a second antigen binding domain that binds a second antigen, wherein one of said first and second antigen binding domains binds human ICOS and the other binds human PD-1.

The disclosure also provides heterodimeric bispecific antibodies comprising: a) a first heavy chain comprising: i) a first variant Fc domain; and ii) a single chain Fv region (scFv) that binds a first antigen, wherein said scFv region comprises a first variable heavy chain, a variable light chain and a charged scFv linker, wherein said charged scFv linker covalently attaches said first variable heavy chain and said variable light chain; and b) a second heavy chain comprising a VH-CH1-hinge-CH2-CH3 monomer, wherein VH is a second variable heavy chain and CH2-CH3 is a second variant Fc domain; and c) a light chain; wherein said second variant Fc domain comprises amino acid substitutions N208D/Q295E/N384D/Q418E/N241D, wherein said first and second variant Fc domains each comprise amino acid substitutions E233P/L234V/L235A/G236del/S267K; and wherein said first variant Fc domain comprises amino acid substitutions S364K/E357Q and second variant Fc domain comprises amino acid substitutions L368D/K370S, wherein one of said first and second antigen binding domains binds human ICOS and the other binds human PD-1, and wherein numbering is according to the EU index as in Kabat.

The heterodimeric antibodies may have first and second variant Fc domains that each comprise M428L/N434S.

Further provided are heterodimeric antibodies comprising:a) a first heavy chain comprising: i) a first variant Fc domain; and ii) a single chain Fv region (scFv) that binds a first antigen, wherein said scFv region comprises a first variable heavy chain, a variable light chain and a charged scFv linker, wherein said charged scFv linker covalently attaches said first variable heavy chain and said variable light chain; and b) a second heavy chain comprising a VH-CH1-hinge-CH2-CH3 monomer, wherein VH is a second variable heavy chain and CH2-CH3 is a second variant Fc domain; and c) a light chain; wherein said first and second variant Fc domains comprises a set of heterodimerization variants selected from the group consisting of L368D/K370S : S364K/E357Q; L368D/K370S : S364K; L368E/K370S : S364K; T411E/K360E/Q362E : D401K; and T366S/L368A/Y407V : T366W, and wherein one of said first and second antigen binding domains binds human ICOS and the other binds human PD-1, and wherein numbering is according to the EU index as in Kabat.

Further provided are nucleic acid compositions comprising nucleic acids that encode the heterodimeric antibodies of the invention, expression vector compositions comprising the nucleic acids, and host cells comprising the expression vector compositions.

Also provided are heterodimeric antibodies for use in the activation of T cells for the treatment of cancer.

### BRIEF DESCRIPTION OF THE DRAWINGS

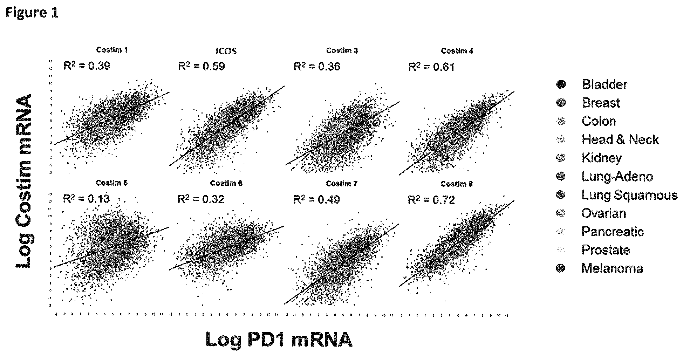

Figure 1 presents expression data (RNAseq V2 RSEM) of PD-1 and T cell costimulatory receptors for bladder, breast, colon, head & neck, kidney, lung-adeno, lung squamous, ovarian, pancreatic, prostate, and melanoma cancer compiled from The Cancer Genome Atlas (TCGA). The square of the Pearson correlation coefficient was calculated for PD-1 against T cell costimulatory receptors.

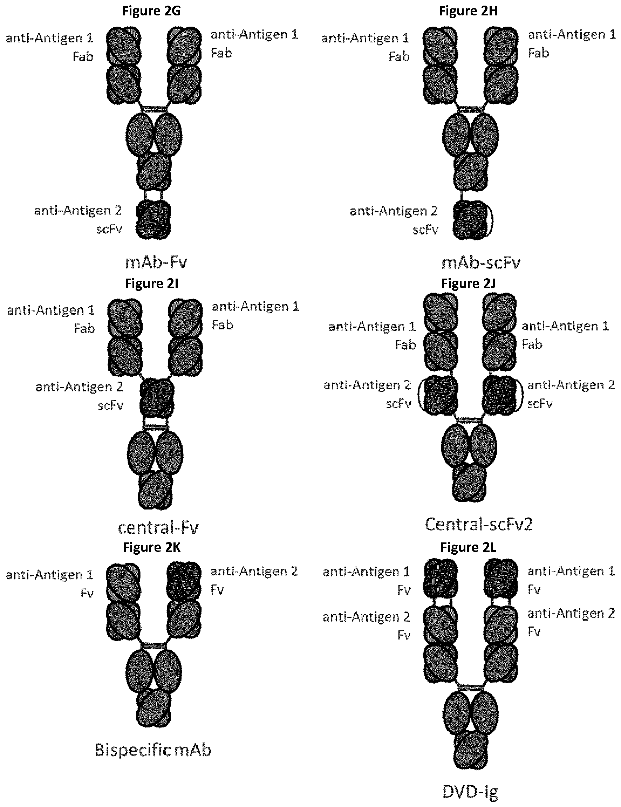

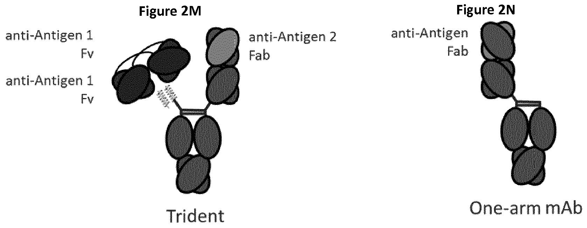

Figure 2A to 2N depict several formats for the bispecific antibodies disclosed herein. The first is the "bottle opener" format, with a first and a second anti-antigen binding domain. Additionally, mAb-Fv, mAb-scFv, Central-scFv, Central-Fv, one armed central-scFv, one scFv-mAb, scFv-mAb dual scFv format are all shown. Figure 2J depicts the "central-scFv2" format, with two Fab-scFv arms, wherein the Fabs bind a first antigen and the scFvs bind a second antigen. Figure 2K depicts the bispecific mAb format, with a first Fab arm binding a first antigen and a second Fab arm binding a second antigen. Figure 2L depicts the DVD-IgG format (see, e.g., U.S. Patent No. 7,612,181 as discussed below). Figure 2M depicts the Trident format (see, e.g., WO 2015/184203 as discussed below). For all of the scFv domains depicted, they can be either N- to C-terminus variable heavy-(optional linker)-variable light, or the opposite. In addition, for the one armed scFv-mAb, the scFv can be attached either to the N-terminus of a heavy chain monomer or to the N-terminus of the light chain.

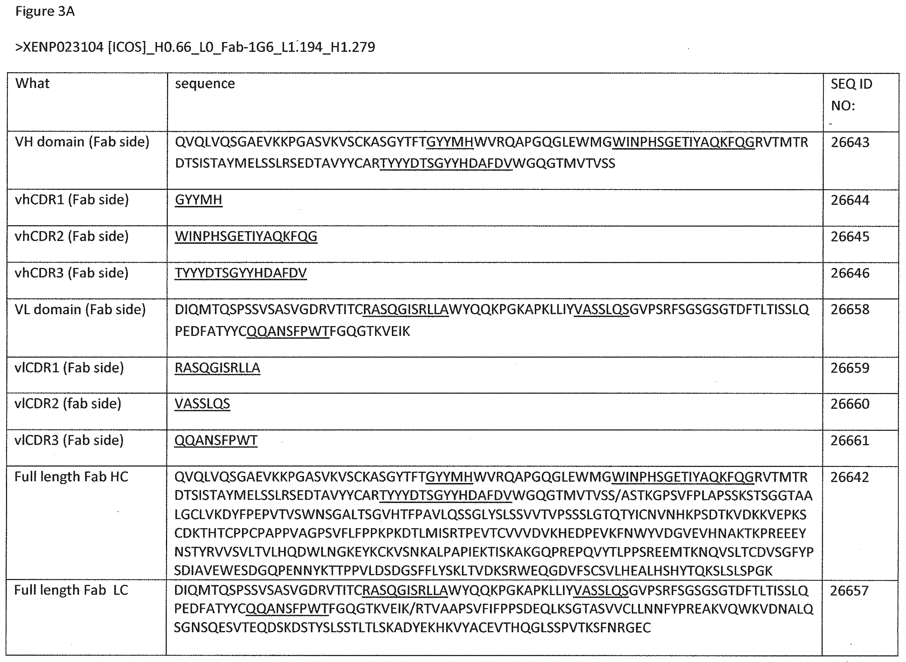

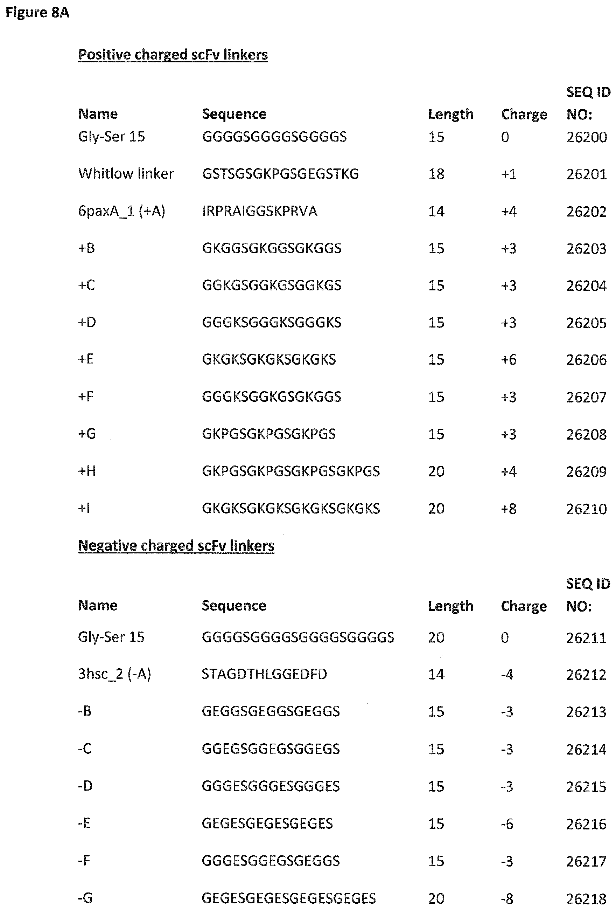

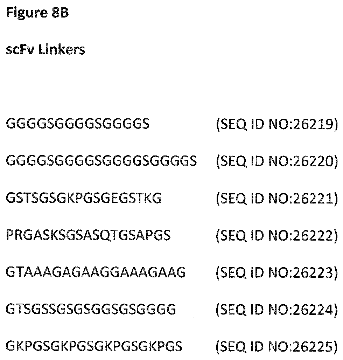

Figure 3A-3B depicts the sequences of XENP23104, a bottle opener format with the ICOS as the Fab side ([ICOS]_H0.66_L0) and the PD-1 as the scFv (1G6_L1.94_H1.279), and includes the M428L/434S variant to extend serum half life. The CDRs are underlined, the scFv linker is double underlined (in the sequences, the scFv linker is a positively charged scFv (GKPGS)₄ linker, although as will be appreciated by those in the art, this linker can be replaced by other linkers, including uncharged or negatively charged linkers, some of which are depicted in Figure 8), and the slashes indicate the border(s) of the variable domains. In addition, the naming convention illustrates the orientation of the scFv from N- to C-terminus; some of the sequences herein are oriented as V_{H}-scFv linker-V_{L} (from N- to C-terminus), while some are oriented as V_{L}-scFv linker-V_{H} (from N- to C-terminus), although as will be appreciated by those in the art, these sequences may also be used in the opposition orientation from their depiction herein. As noted herein and is true for every sequence herein containing CDRs, the exact identification of the CDR locations may be slightly different depending on the numbering used as is shown in Table 1, and thus included herein are not only the CDRs that are underlined but also CDRs included within the V_{H} and V_{L} domains using other numbering systems.

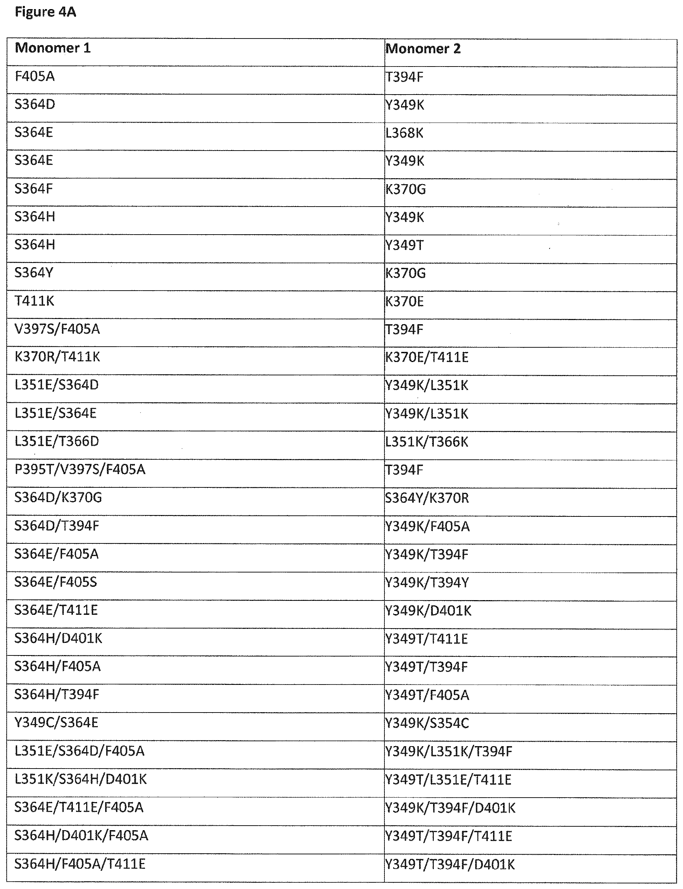

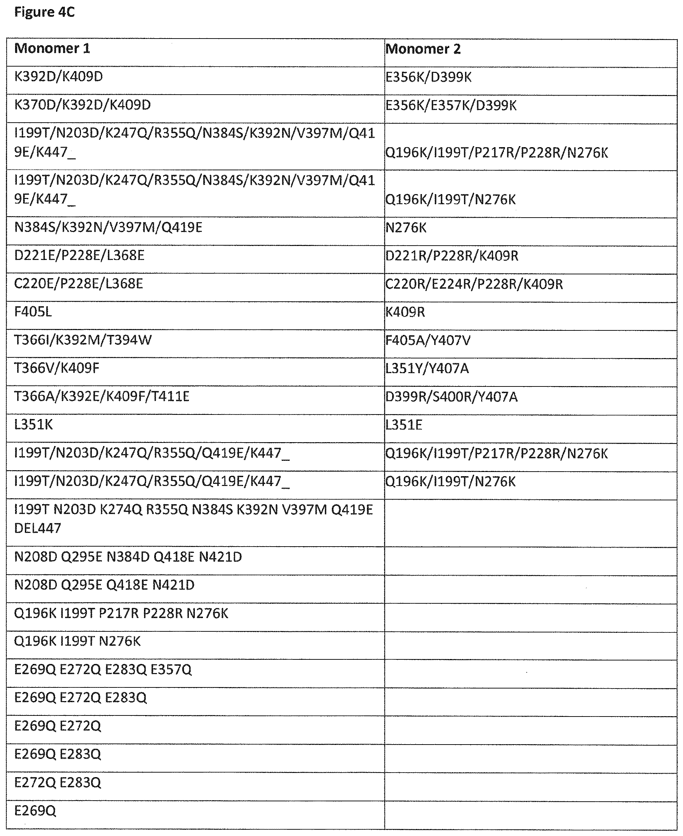

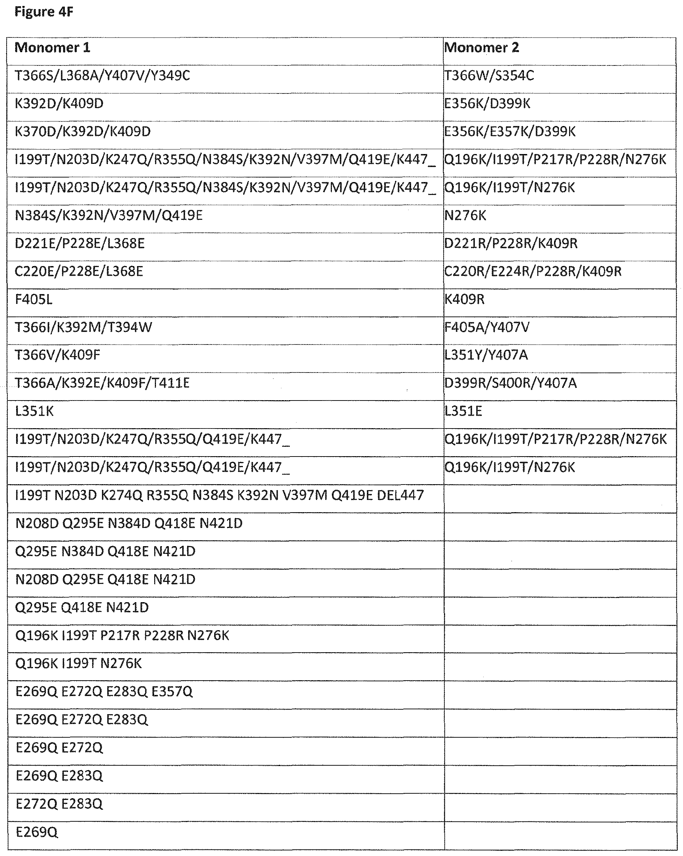

Figure 4A to 4F depict useful pairs of heterodimerization variant sets (including skew and pI variants). On Figure 4C and F, there are variants for which there are no corresponding "monomer 2" variants; these are pI variants which can be used alone on either monomer, or included on the Fab side of a bottle opener, for example, and an appropriate charged scFv linker can be used on the second monomer that utilizes a scFv as the second antigen binding domain. Suitable charged linkers are shown in Figure 8.

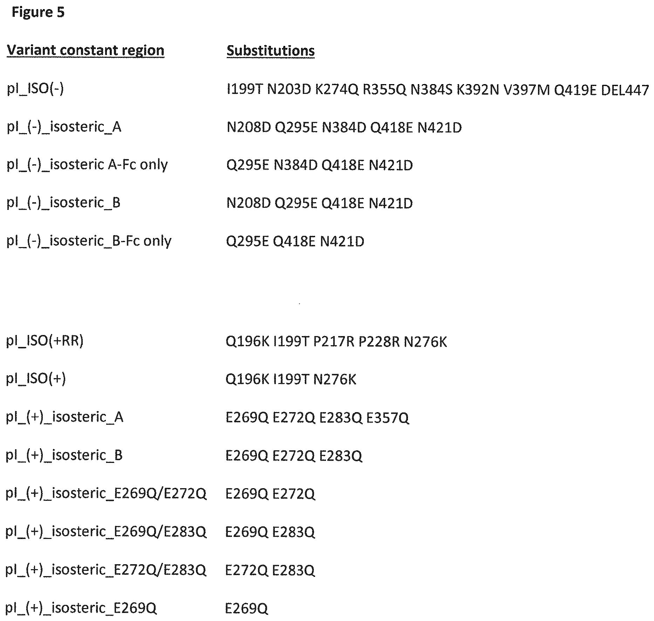

Figure 5 depict a list of isosteric variant antibody constant regions and their respective substitutions. pI_(-) indicates lower pI variants, while pI_(+) indicates higher pI variants. These can be optionally and independently combined with other heterodimerization variants disclosed herein (and other variant types as well, as outlined herein).

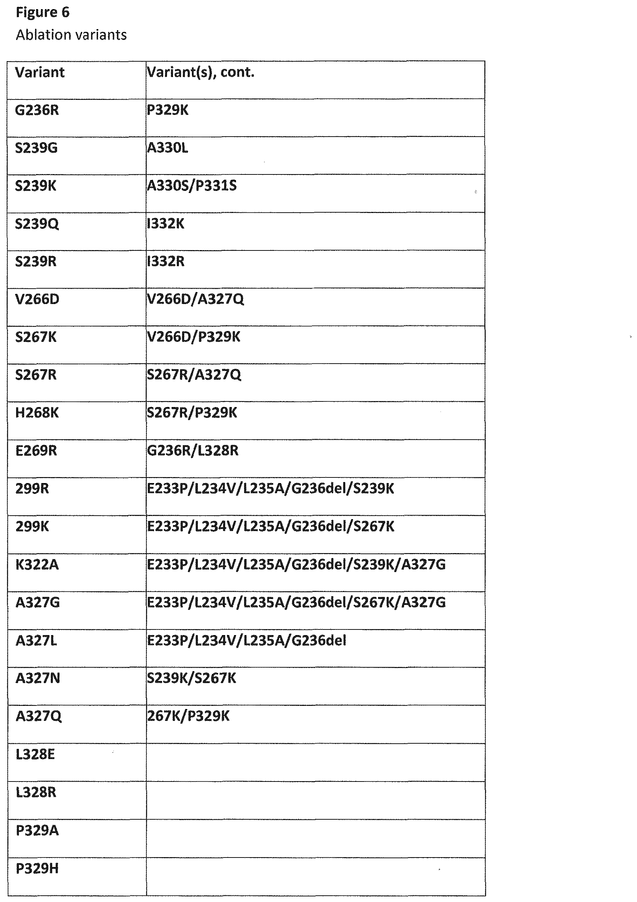

Figure 6 depict useful ablation variants that ablate FcγR binding (sometimes referred to as "knock outs" or "KO" variants). Generally, ablation variants are found on both monomers, although in some cases they may be on only one monomer.

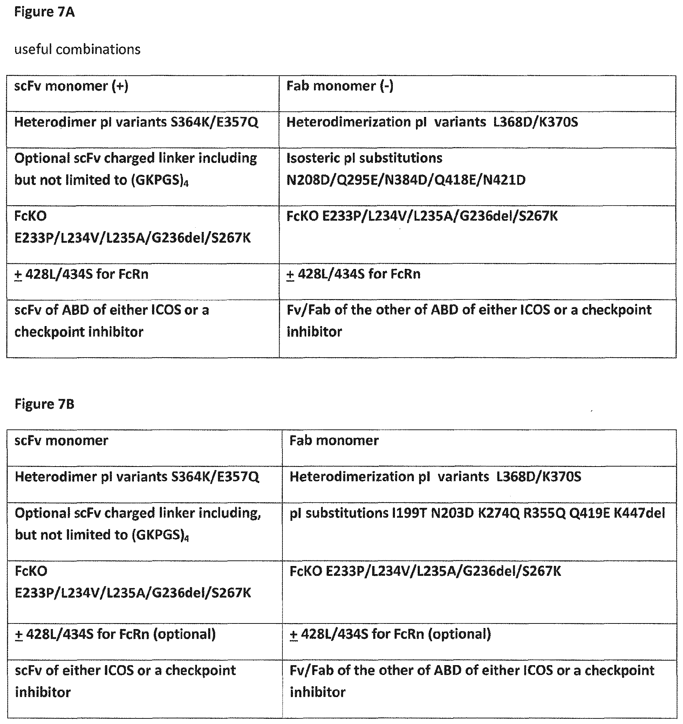

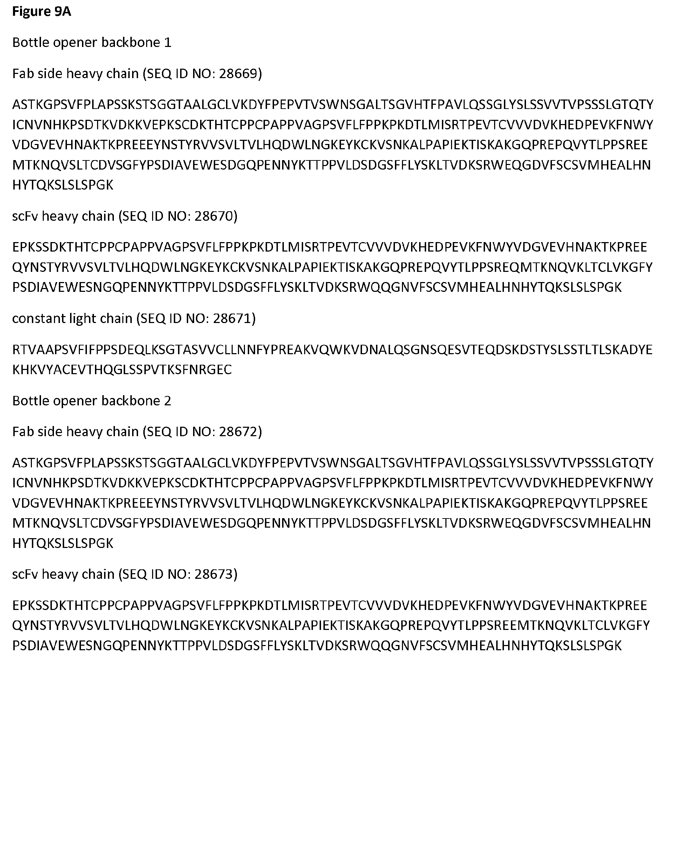

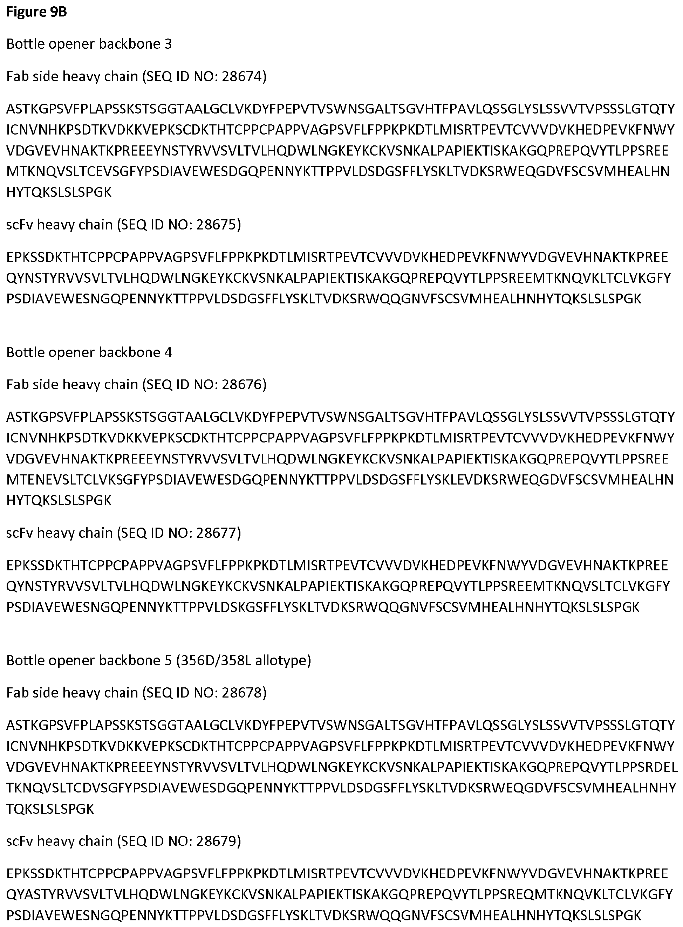

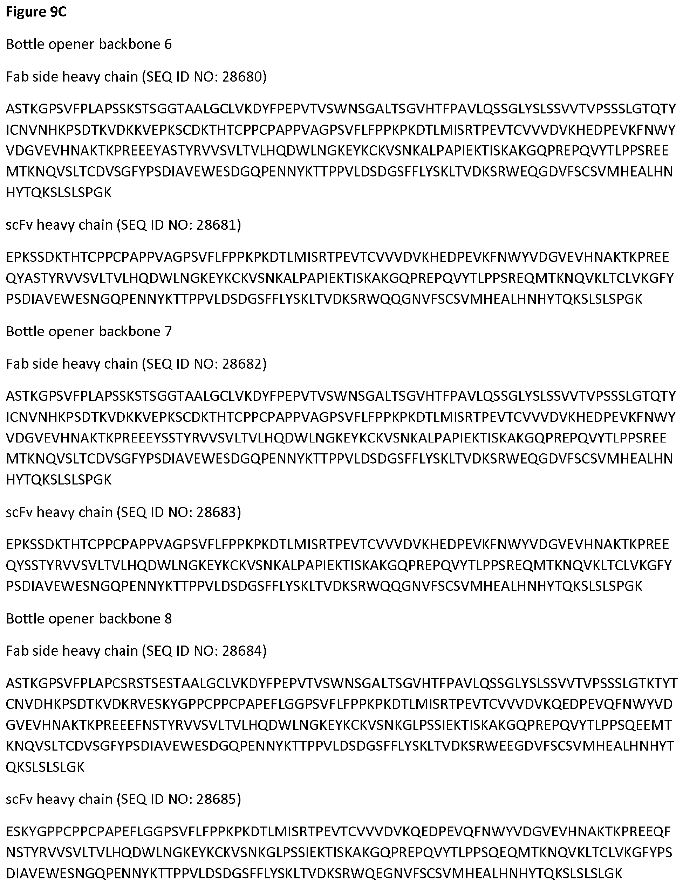

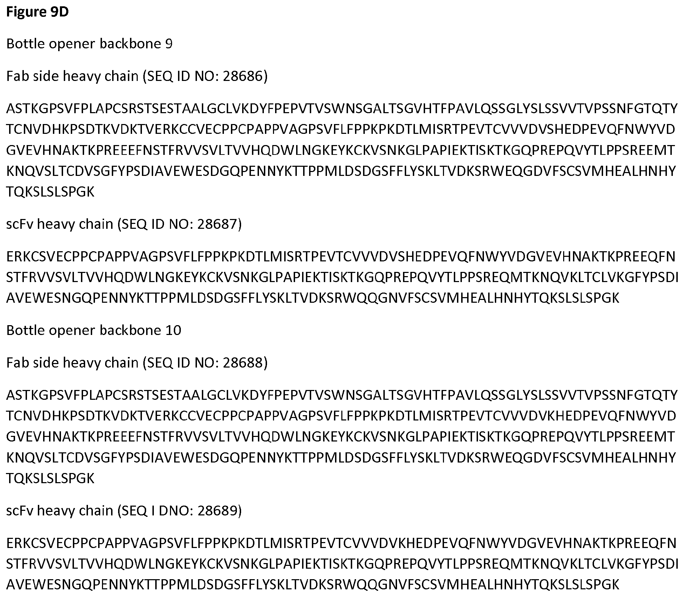

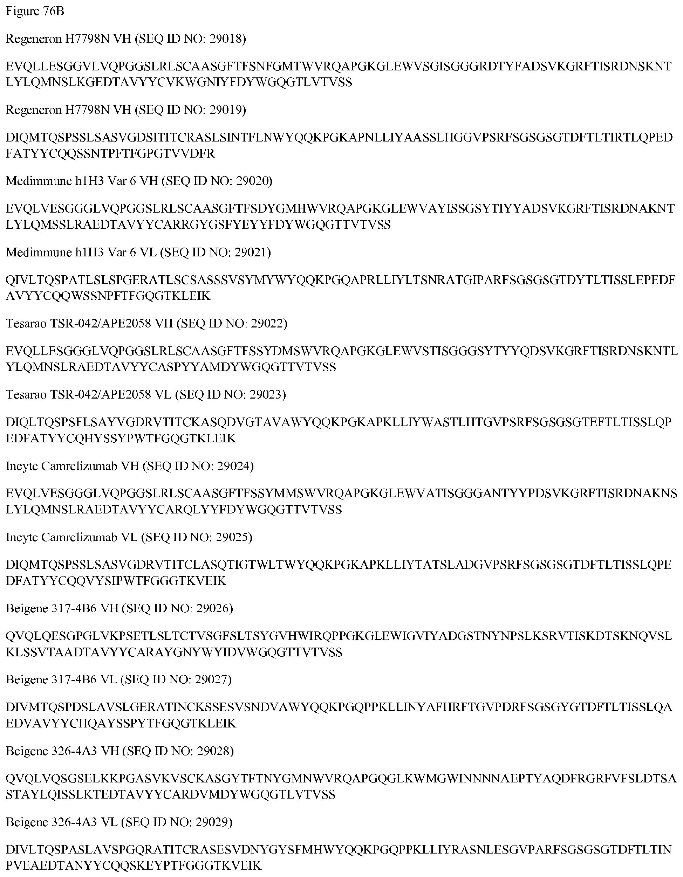

Figure 7A-7B show two particularly useful examples of the disclosure. The "non-Fv" components of this example is shown in Figure 9A, although the other formats of Figure 9 can be used as well.
Figure 8A and 8B depict a number of charged scFv linkers that find use in increasing or decreasing the pI of heterodimeric antibodies that utilize one or more scFv as a component. The (+H) positive linker finds particular use herein. A single prior art scFv linker with single charge is references as "Whitlow", from Whitlow et al., Protein Engineering 6(8):989-995 (1993). It should be noted that this linker was used for reducing aggregation and enhancing proteolytic stability in scFvs.
Figure 9A-9D shows the sequences of several useful bottle opener format backbones based on human IgG1, without the Fv sequences (e.g. the scFv and the vh and vl for the Fab side). Bottle opener backbone 1 is based on human IgG1 (356E/358M allotype), and includes the S364K/E357Q : L368D/K370S skew variants, the N208D/Q295E/N384D/Q418E/N421D pI variants on the Fab side and the E233P/L234V/L235A/G236del/S267K ablation variants on both chains. Bottle opener backbone 2 is based on human IgG1 (356E/358M allotype), and includes different skew variants, the N208D/Q295E/N384D/Q418E/N421D pI variants on the Fab side and the E233P/L234V/L235A/G236del/S267K ablation variants on both chains. Bottle opener backbone 3 is based on human IgG1 (356E/358M allotype), and includes different skew variants, the N208D/Q295E/N384D/Q418E/N421D pI variants on the Fab side and the E233P/L234V/L235A/G236del/S267K ablation variants on both chains. Bottle opener backbone 4 is based on human IgG1 (356E/358M allotype), and includes different skew variants, the N208D/Q295E/N384D/Q418E/N421D pI variants on the Fab side and the E233P/L234V/L235A/G236del/S267K ablation variants on both chains. Bottle opener backbone 5 is based on human IgG1 (356D/358L allotype), and includes the S364K/E357Q : L368D/K370S skew variants, the N208D/Q295E/N384D/Q418E/N421D pI variants on the Fab side and the E233P/L234V/L235A/G236del/S267K ablation variants on both chains. Bottle opener backbone 6 is based on human IgG1 (356E/358M allotype), and includes the S364K/E357Q : L368D/K370S skew variants, N208D/Q295E/N384D/Q418E/N421D pI variants on the Fab side and the E233P/L234V/L235A/G236del/S267K ablation variants on both chains, as well as an N297A variant on both chains. Bottle opener backbone 7 is identical to 6 except the mutation is N297S. Alternative formats for bottle opener backbones 6 and 7 can exclude the ablation variants E233P/L234V/L235A/G236del/S267K in both chains. Backbone 8 is based on human IgG4, and includes the S364K/E357Q : L368D/K370S skew variants, the N208D/Q295E/N384D/Q418E/N421D pI variants on the Fab side and the E233P/L234V/L235A/G236del/S267K ablation variants on both chains, as well as a S228P (EU numbering, this is S241P in Kabat) variant on both chains that ablates Fab arm exchange as is known in the art. Alternative formats for bottle opener backbone 8 can exclude the ablation variants E233P/L234V/L235A/G236del/S267K in both chains Backbone 9 is based on human IgG2, and includes the S364K/E357Q : L368D/K370S skew variants, the N208D/Q295E/N384D/Q418E/N421D pI variants on the Fab side. Backbone 10 is based on human IgG2, and includes the S364K/E357Q : L368D/K370S skew variants, the N208D/Q295E/N384D/Q418E/N421D pI variants on the Fab side as well as a S267K variant on both chains.

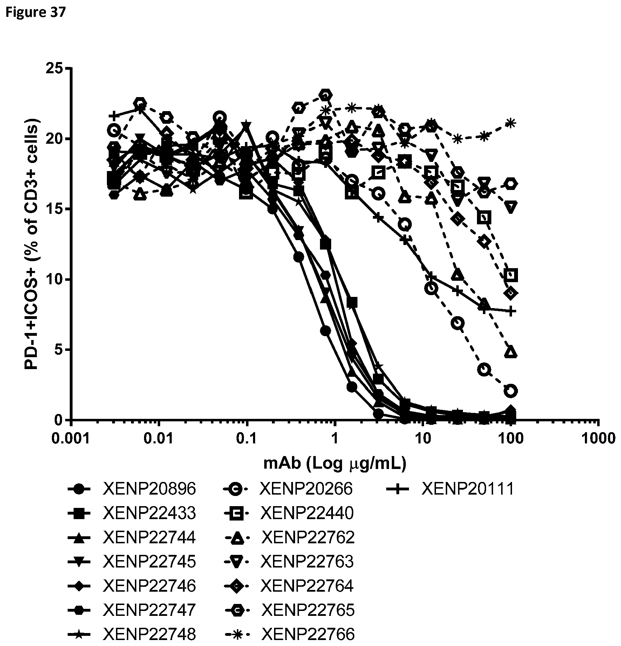

As will be appreciated by those in the art and outlined below, these sequences can be used with any vh and vl pairs outlined herein, with one monomer including a scFv (optionally including a charged scFv linker) and the other monomer including the Fab sequences (e.g. a vh attached to the "Fab side heavy chain" and a vl attached to the "constant light chain"). That is, any Fv sequences outlined herein for anti-CTLA-4, anti-PD-1, anti-LAG-3, anti-TIM-3, anti-TIGIT and anti-BTLA, whether as scFv (again, optionally with charged scFv linkers) or as Fabs, can be incorporated into these Figure 37 backbones in any combination. The constant light chain depicted in Figure 9A can be used for all of the constructs in the figure, although the kappa constant light chain can also be substituted.

It should be noted that these bottle opener backbones find use in the Central-scFv format of Figure 1, where an additional, second Fab (vh-CH1 and vl-constant light) with the same antigen binding as the first Fab is added to the N-terminus of the scFv on the "bottle opener side".

Included within each of these backbones are sequences that are 90, 95, 98 and 99% identical (as defined herein) to the recited sequences, and/or contain from 1, 2, 3, 4, 5, 6, 7, 8, 9 or 10 additional amino acid substitutions (as compared to the "parent" of the Figure, which, as will be appreciated by those in the art, already contain a number of amino acid modifications as compared to the parental human IgG1 (or IgG2 or IgG4, depending on the backbone). That is, the recited backbones may contain additional amino acid modifications (generally amino acid substitutions) in addition to the skew, pI and ablation variants contained within the backbones of this figure.

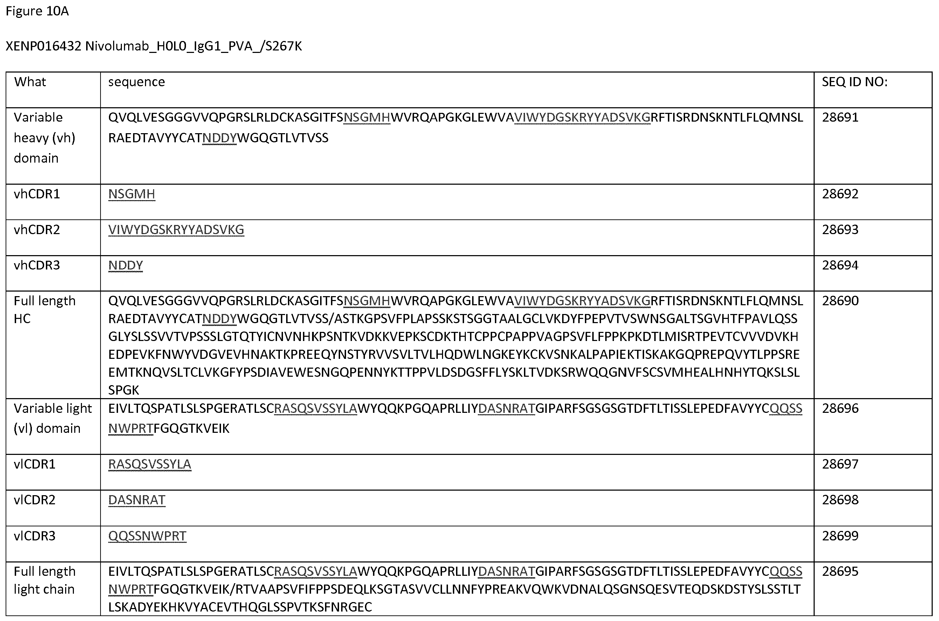

Figure 10A-10E depicts the sequences for a select number of anti-PD-1 antibodies. It is important to note that these sequences were generated based on human IgG1, with an ablation variant (E233P/L234V/L235A/G236del/S267K, "IgG1_PVA_/S267K") which is depicted in Figure 6A. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems.

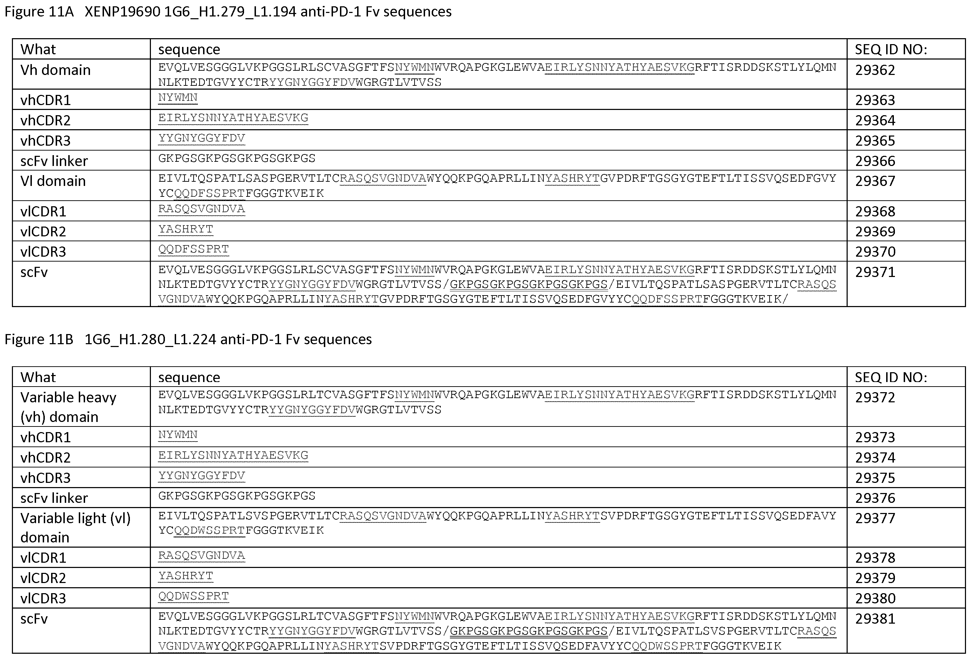

Figure 11A-11E depict a select number of PD-1 ABDs, with additional anti-PD-1 ABDs being listed as SEQ 1-2392, 3125-3144, 4697-7594 and 4697-21810. The CDRs are underlined, the scFv linker is double underlines (in the sequences, the scFv linker is a positively charged scFv (GKPGS)₄ linker although as will be appreciated by those in the art, this linker can be replaced by other linkers, including uncharged or negatively charged linkers, some of which are depicted in Figure 8), and the slashes indicate the border(s) of the variable domains. In addition, the naming convention illustrates the orientation of the scFv from N- to C-terminus; some of the sequences in this Figure are oriented as VH-scFv linker-VL (from N- to C-terminus), while some are oriented as VL-scFv linker-VH (from N- to C-terminus), although as will be appreciated by those in the art, these sequences may also be used in the opposition orientation from their depiction herein. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems. Furthermore, as for all the sequences in the Figures, these VH and VL sequences can be used either in a scFv format or in a Fab format.

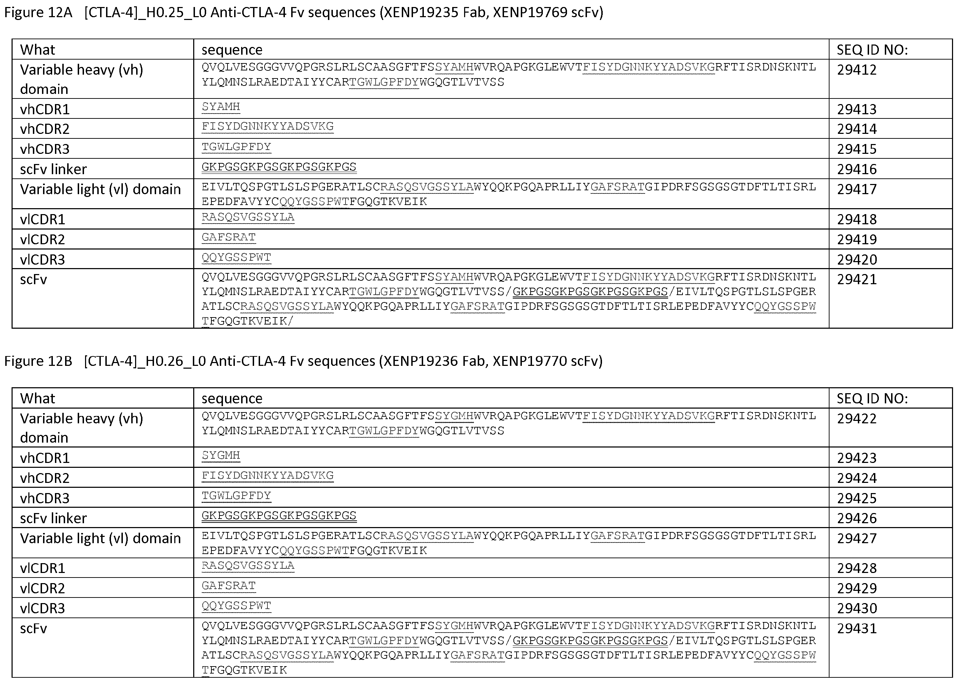

Figure 12A-12PP depict a select number of CTLA-4 ABDs, with additional anti-CTLA-4 ABDs being listed as SEQ ID NO:2393-2414 and 3737-3816. The CDRs are underlined, the scFv linker is double underlines (in the sequences, the scFv linker is a positively charged scFv (GKPGS)₄ linker although as will be appreciated by those in the art, this linker can be replaced by other linkers, including uncharged or negatively charged linkers, some of which are depicted in Figure 8), and the slashes indicate the border(s) of the variable domains. In addition, the naming convention illustrates the orientation of the scFv from N- to C-terminus; some of the sequences in this Figure are oriented as VH-scFv linker-VL (from N- to C-terminus), while some are oriented as VL-scFv linker-VH (from N- to C-terminus), although as will be appreciated by those in the art, these sequences may also be used in the opposition orientation from their depiction herein. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems. Furthermore, as for all the sequences in the Figures, these VH and VL sequences can be used either in a scFv format or in a Fab format.

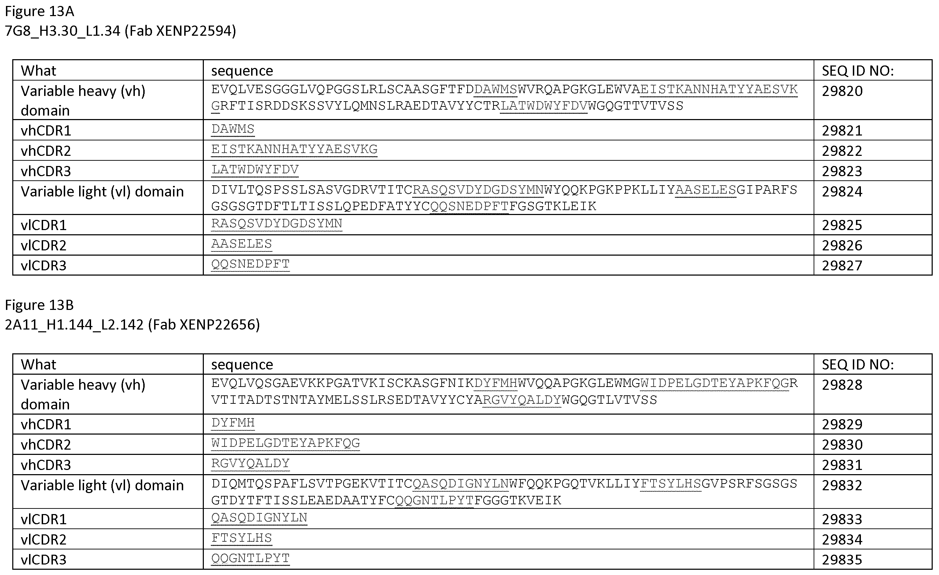

Figure 13A-13N depict a select number of LAG-3 ABDs, with additional anti-LAG-3 ABDs being listed as SEQ ID NO:2415-2604 and 3817-3960. The CDRs are underlined, the scFv linker is double underlines (in the sequences, the scFv linker is a positively charged scFv (GKPGS)₄ linker (SEQ ID NO: XXX), although as will be appreciated by those in the art, this linker can be replaced by other linkers, including uncharged or negatively charged linkers, some of which are depicted in Figure 8), and the slashes indicate the border(s) of the variable domains> In addition, the naming convention illustrates the orientation of the scFv from N- to C-terminus; some of the sequences in this Figure are oriented as VH-scFv linker-VL (from N- to C-terminus), while some are oriented as VL-scFv linker-VH (from N- to C-terminus), although as will be appreciated by those in the art, these sequences may also be used in the opposition orientation from their depiction herein. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems. Furthermore, as for all the sequences in the Figures, these VH and VL sequences can be used either in a scFv format or in a Fab format.

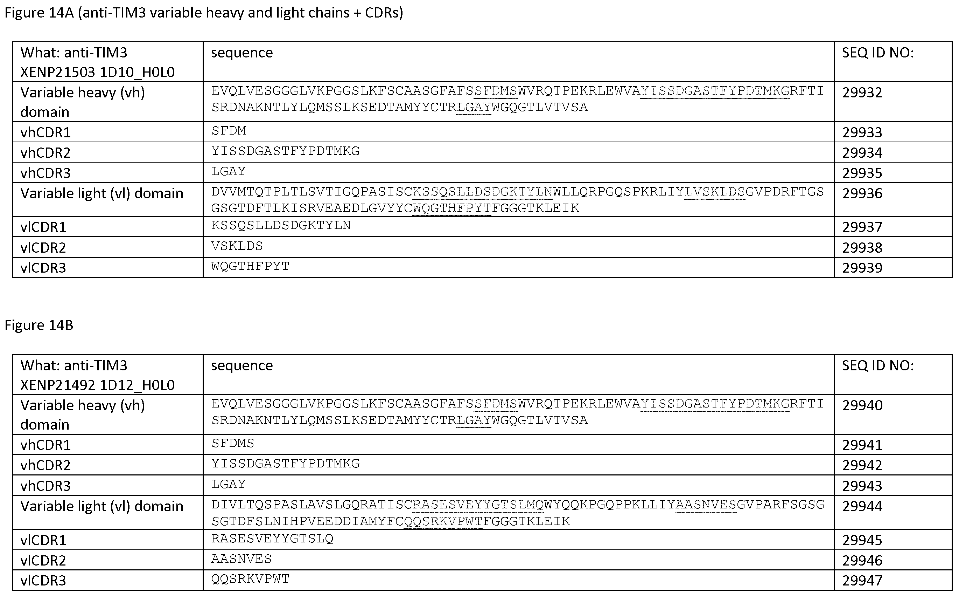

Figure 14A-14I depicts the sequences for a select number of anti-TIM-3 antibodies. It is important to note that these sequences were generated based on human IgG1 backbone, with an ablation variant (E233P/L234V/L235A/G236del/S267K, "IgG1_PVA_/S267K") although other formats can be used as well. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the V_{H} and V_{L} domains using other numbering systems.

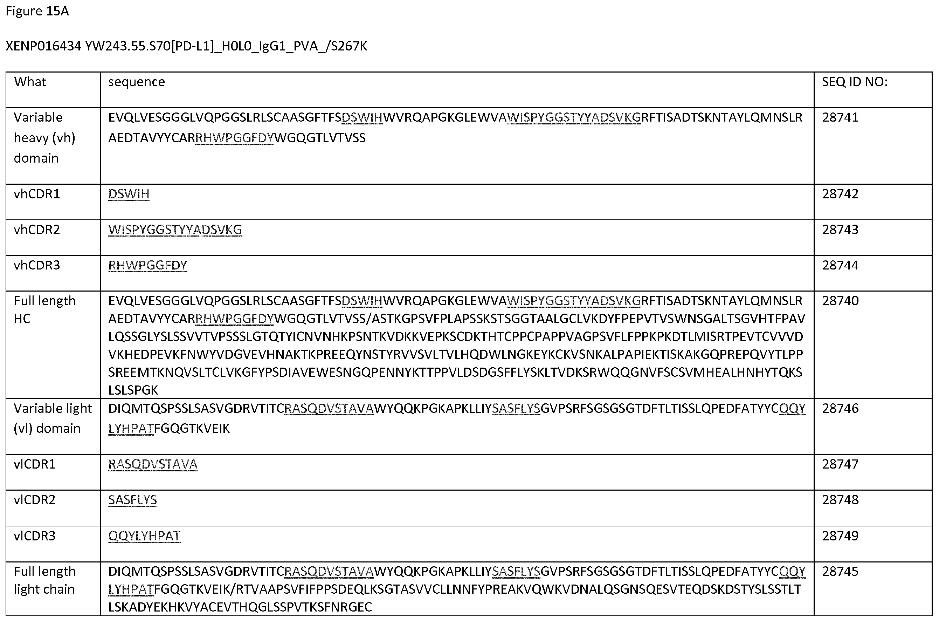

Figure 15A-15C depicts the sequences for a select number of anti-PD-L1 antibodies. It is important to note that these sequences were generated based on human IgG1 backbone, with an ablation variant (E233P/L234V/L235A/G236del/S267K, "IgG1_PVA_/S267K") as outlined herein, although other formats can be used as well. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems.

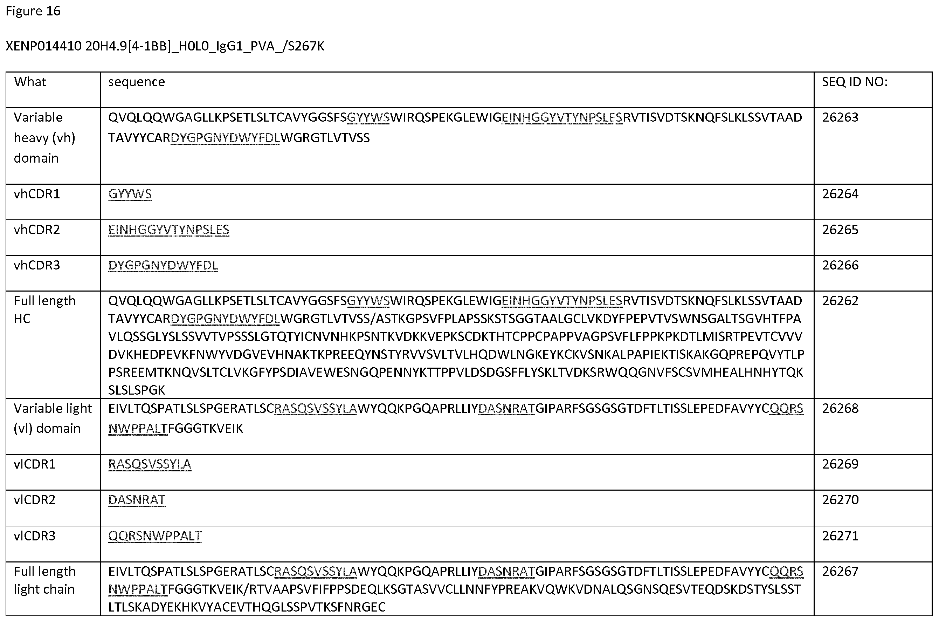

Figure 16 depicts the sequences for a prototype anti-4-1BB antibody. It is important to note that these sequences were generated based on human IgG1 backbone, with an ablation variant (E233P/L234V/L235A/G236del/S267K, "IgG1_PVA_/S267K"), although the other formats can be used as well. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems.

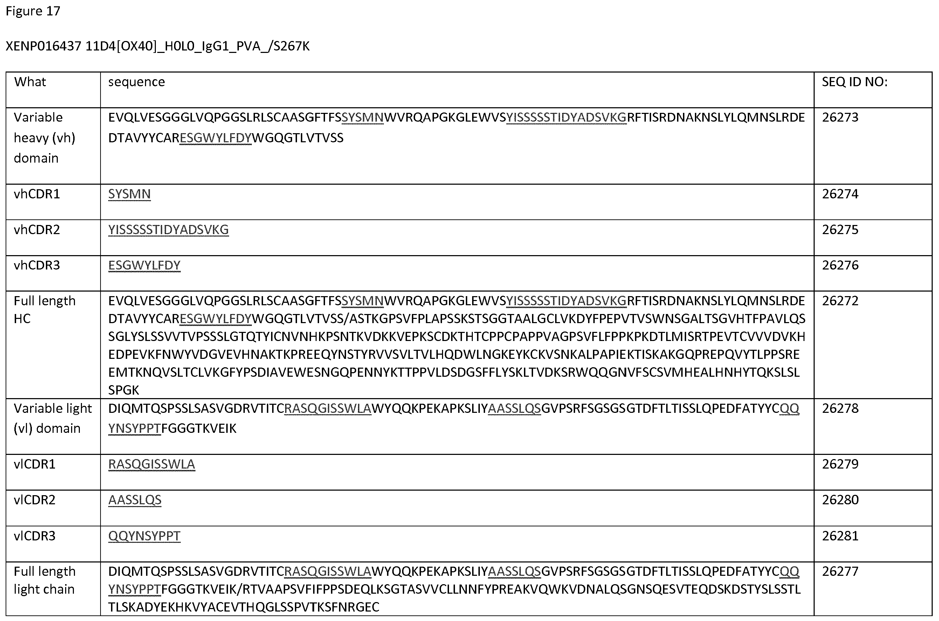

Figure 17 depicts the sequences for a prototype anti-OX40 antibody. It is important to note that these sequences were generated based on human IgG1 backbone, with an ablation variant (E233P/L234V/L235A/G236del/S267K, "IgG1_PVA_/S267K"), although other formats can be used as well. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems.

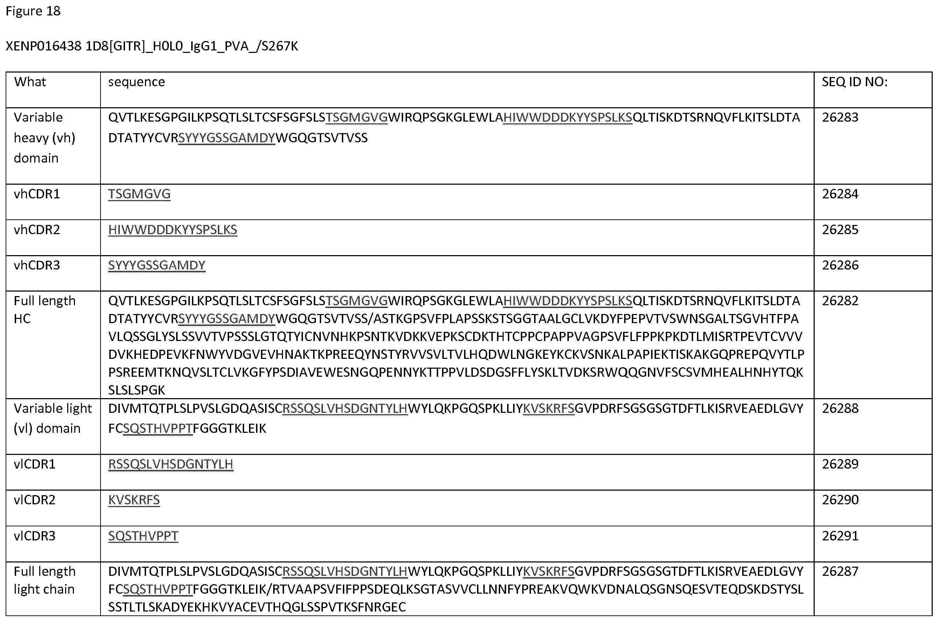

Figure 18 depicts the sequences for a prototype anti-GITR antibody. It is important to note that these sequences were generated based on human IgG1 backbone, with an ablation variant (E233P/L234V/L235A/G236del/S267K, "IgG1_PVA_/S267K"), although other formats can be used as well. The CDRs are underlined. As noted herein and is true for every sequence herein containing CDRs, the exact identification of the CDR locations may be slightly different depending on the numbering used as is shown in Table 1, and thus included herein are not only the CDRs that are underlined but also CDRs included within the VH and VL domains using other numbering systems.

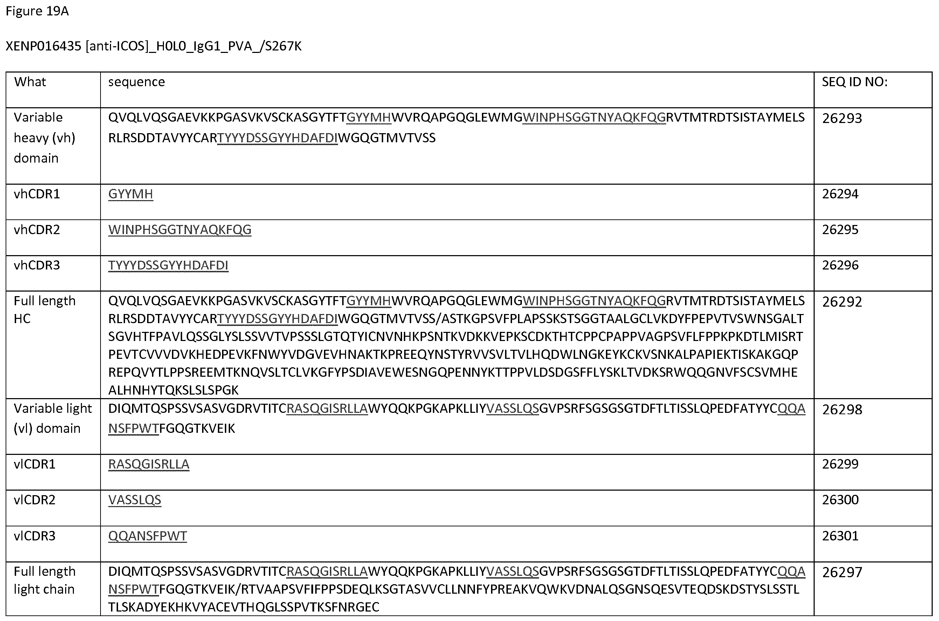

Figure 19A-19G depicts the sequences for prototype anti-ICOS antibodies. It is important to note that these sequences were generated based on human IgG1 backbone, with an ablation variant (E233P/L234V/L235A/G236del/S267K, "IgG1_PVA_/S267K"), although other formats can be used as well. The CDRs are underlined. As noted herein and is true for every sequence herein containing CDRs, the exact identification of the CDR locations may be slightly different depending on the numbering used as is shown in Table X, and thus included herein are not only the CDRs that are underlined but also CDRs included within the VH and VL domains using other numbering systems.

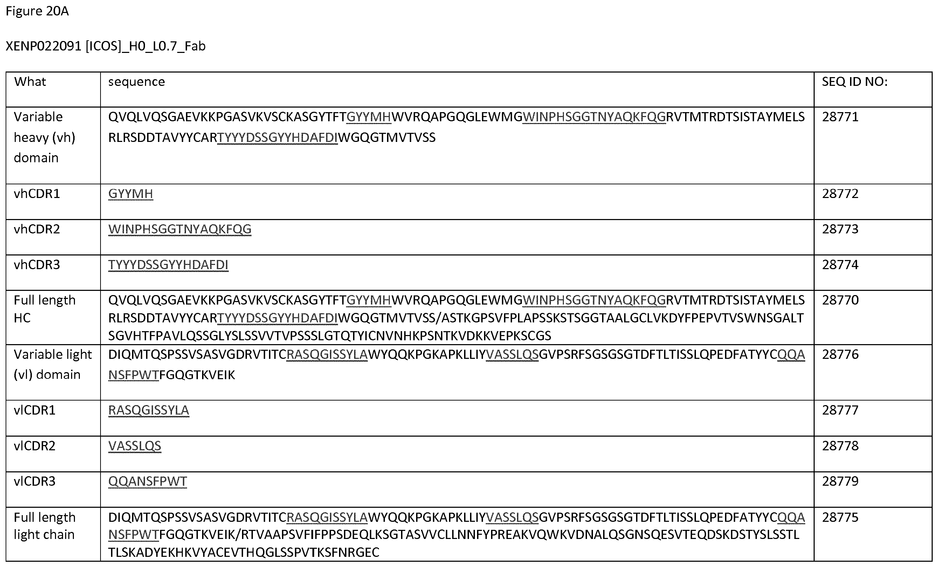

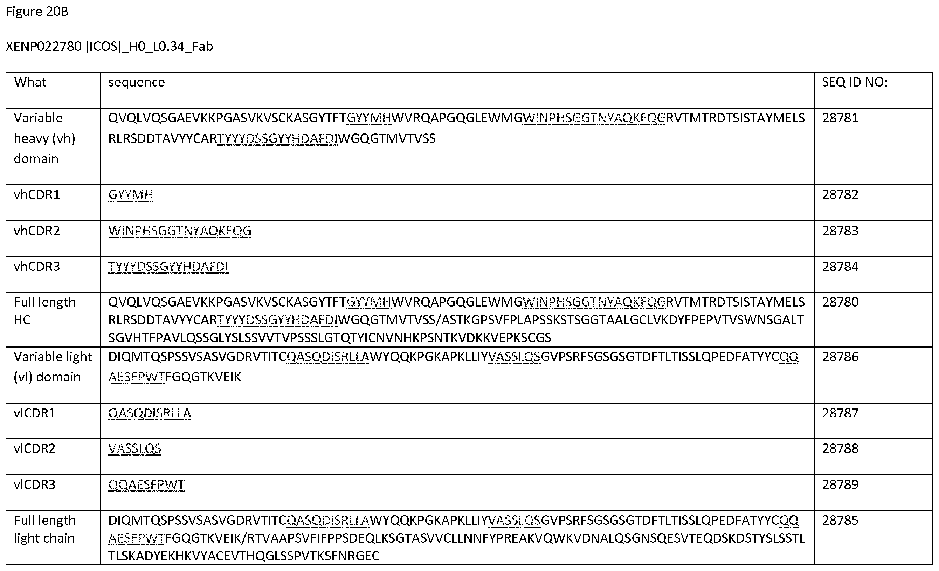

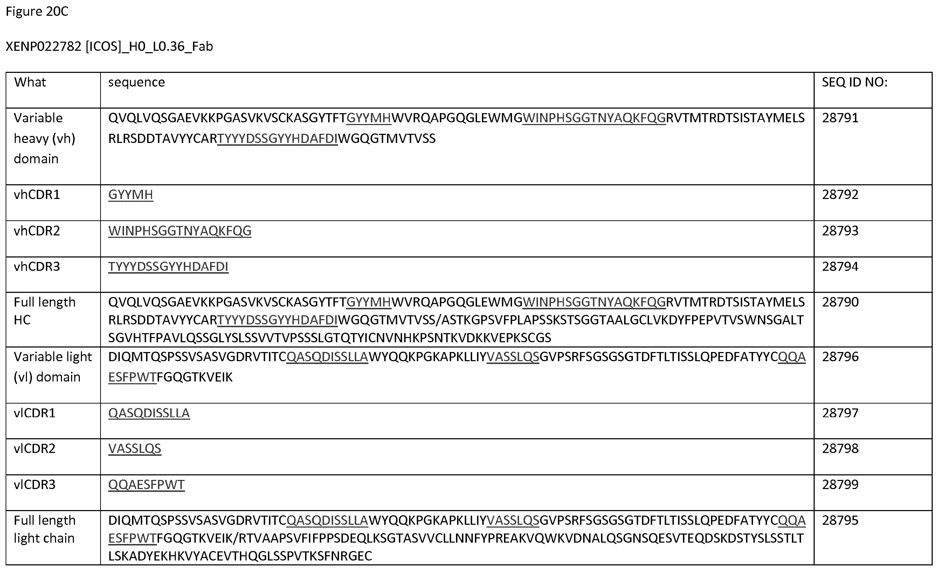

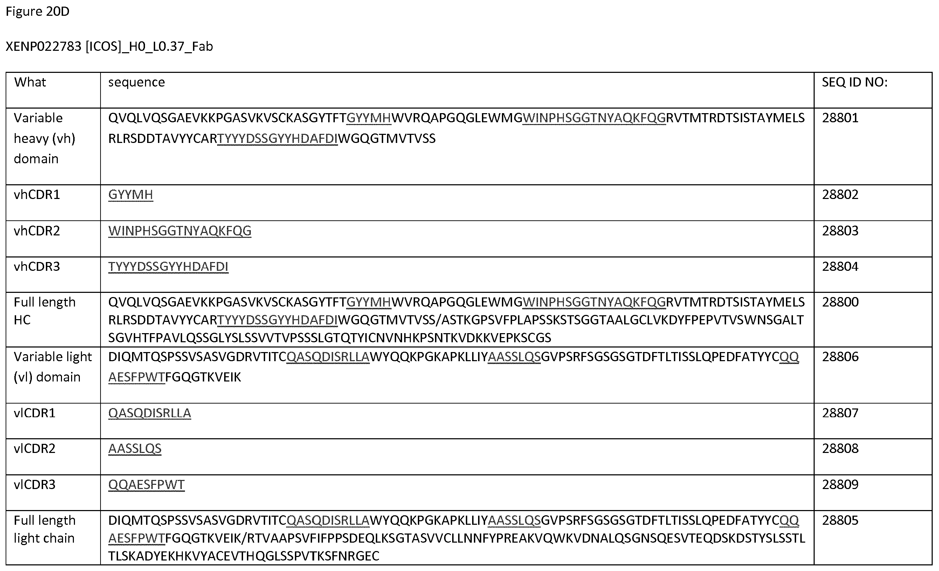

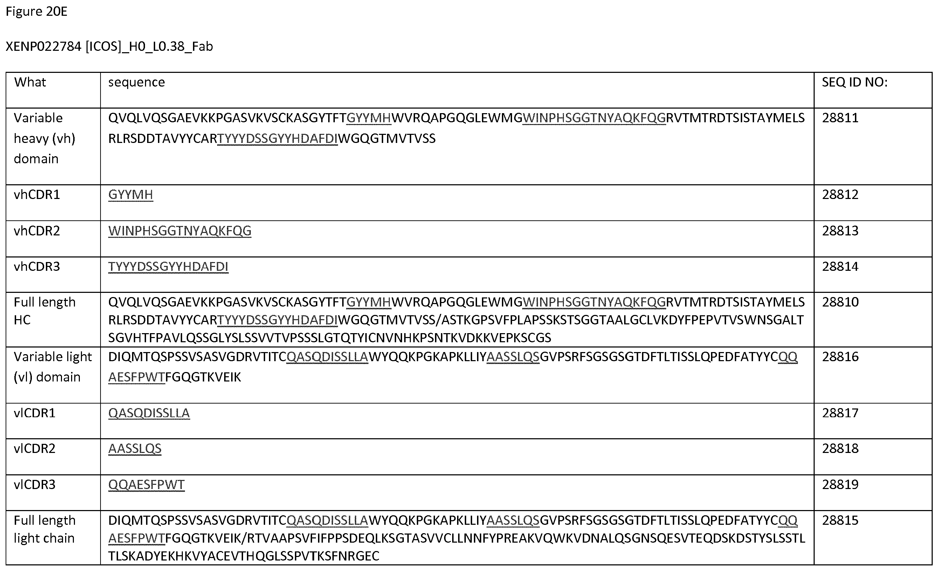

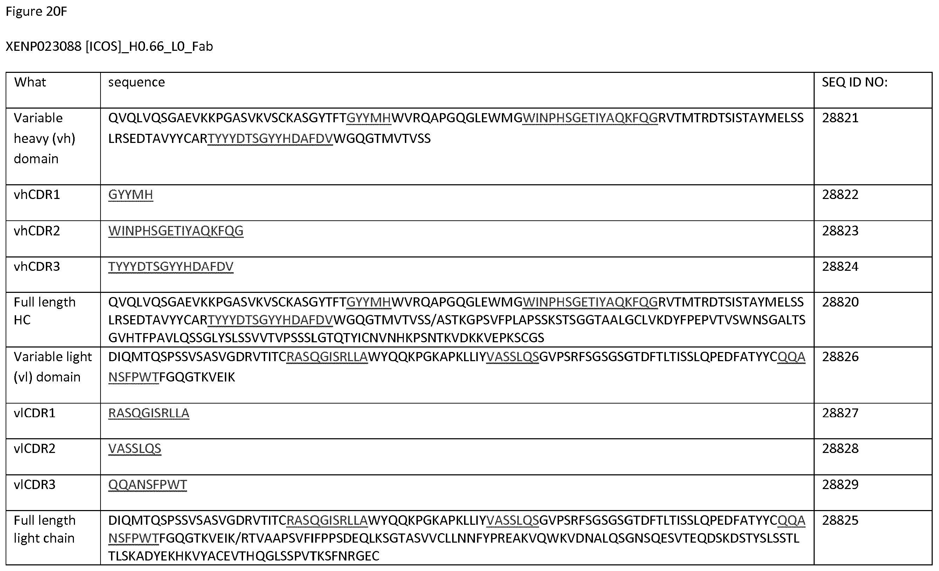

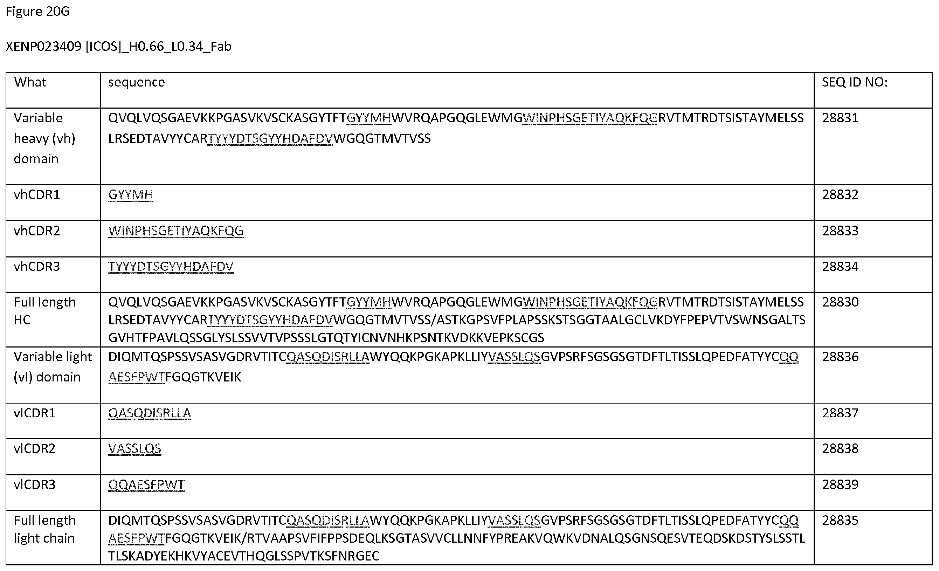

Figure 20A-20G depicts sequences for exemplary anti-ICOS Fabs. The CDRs are underlined and slashes (/) indicate the border(s) of the variable regions. As noted herein and is true for every sequence herein containing CDRs, the exact identification of the CDR locations may be slightly different depending on the numbering used as is shown in Table X, and thus included herein are not only the CDRs that are underlined but also CDRs included within the VH and VL domains using other numbering systems. Furthermore, as for all the sequences in the Figures, these VH and VL sequences can be used either in a scFv format or in a Fab format. It is important to note that these sequences were generated using six-histidine (His6 or HHHHHH) (SEQ ID NO: 28666) C-terminal tags at the C-terminus of the heavy chains, which have been removed.

Figure 21A-21B depicts melting temperatures (Tₘ) and changes in melting temperature from the parental Fab (XENP22050) as determined by DSF of variant anti-ICOS Fabs engineered for stability.

Figure 22A-22C depicts equilibrium dissociation constants (KD), association rates (ka), and dissociation rates (kd) of variant anti-ICOS Fabs for murine Fc fusions of human ICOS captured on AMC biosensors as determined by Octet.

Figure 23 depicts equilibrium dissociation constants (KD), association rates (ka), and dissociation rates (kd) of variant anti-ICOS Fabs for biotinylated IgG1 Fc fusions of human ICOS captured on SA biosensors as determined by Octet.

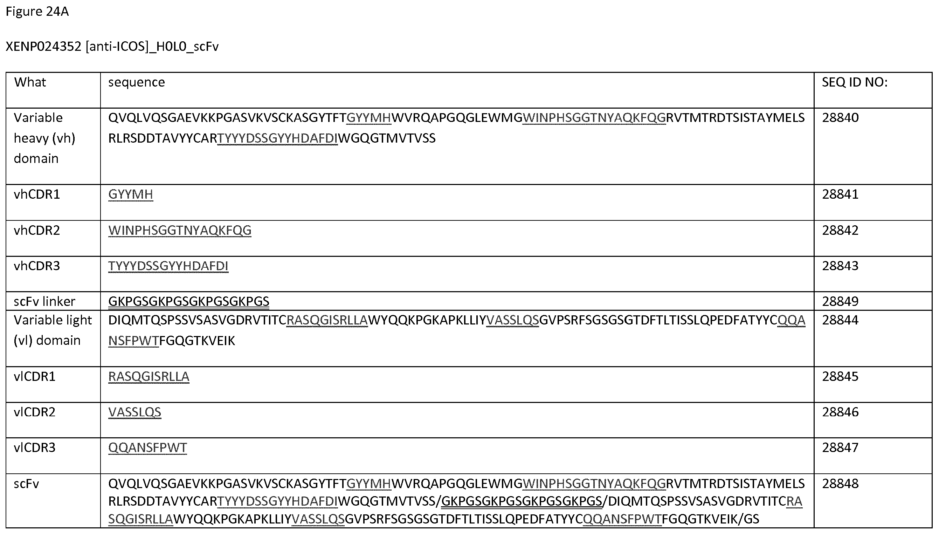

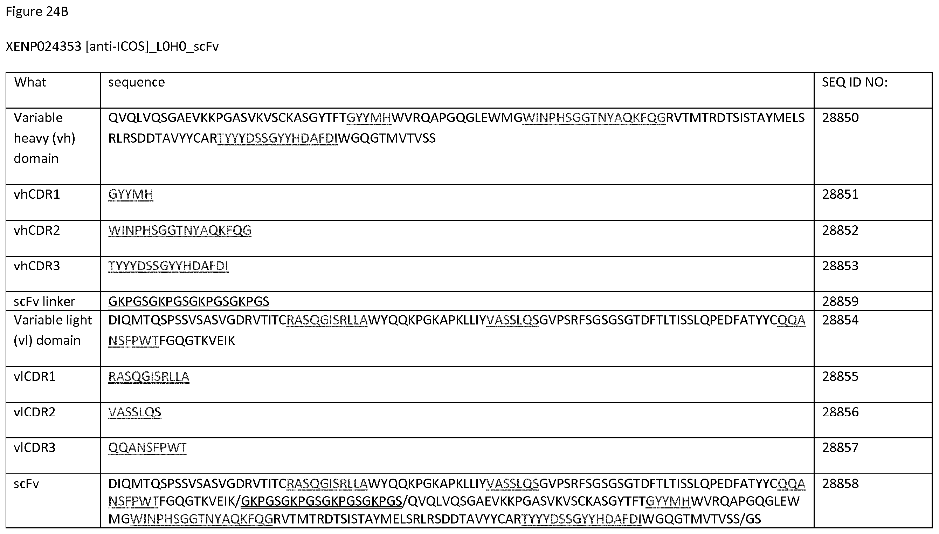

Figure 24A-24M depicts sequences for exemplary anti-ICOS scFvs. The CDRs are underlined, the scFv linker is double underline (in the sequences, the scFv linker is a positively charged scFv (GKPGS)4 linker (SEQ ID NO: 28849), although as will be appreciated by those in the art, this linker can be replaced by other linkers, including uncharged or negatively charged linkers, some of which are depicted in Figure 24A-24M), and slashes (/) indicate the border(s) of the variable regions. The naming convention illustrates the orientation of the scFv from N- to C-terminus; some of the sequences in this Figure are oriented as VH-scFv linker-VL (from N- to C-terminus, see Figure 24A-24M), while some are oriented as VL-scFv linker-VH (from N- to C-terminus, see Figure 24B), although as will be appreciated by those in the art, these sequences may also be used in the opposition orientation from their depiction herein. As noted herein and is true for every sequence herein containing CDRs, the exact identification of the CDR locations may be slightly different depending on the numbering used as is shown in Table X, and thus included herein are not only the CDRs that are underlined but also CDRs included within the VH and VL domains using other numbering systems. Furthermore, as for all the sequences in the Figures, these VH and VL sequences can be used either in a scFv format or in a Fab format. It is important to note that these sequences were generated using polyhistidine (His6 or HHHHHH) (SEQ ID NO: 28666) C-terminal tags at the C-terminus of the heavy chains, which have been removed.

Figure 25 depicts melting temperatures (Tm) and changes in melting temperature from the parental scFv (XENP24352; oriented as VH-scFv linker-VL from N- to C-terminus) as determined by DSF of variant anti-ICOS scFvs engineered for stability.

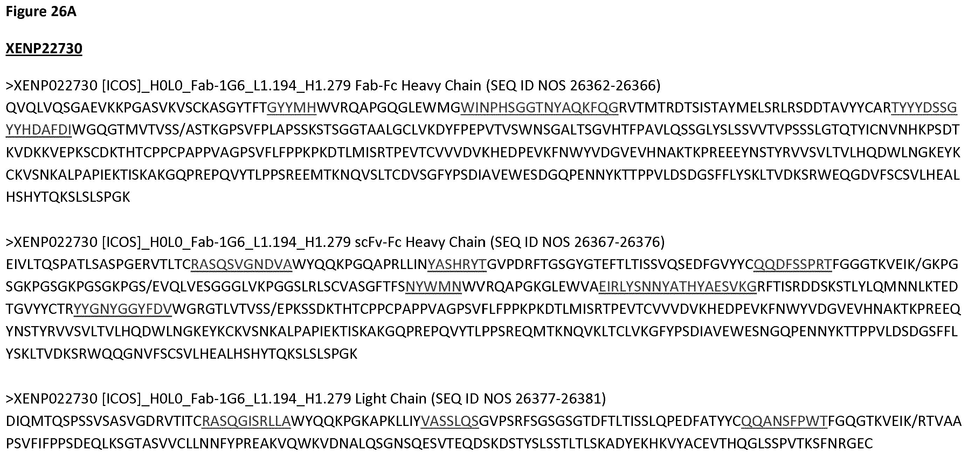

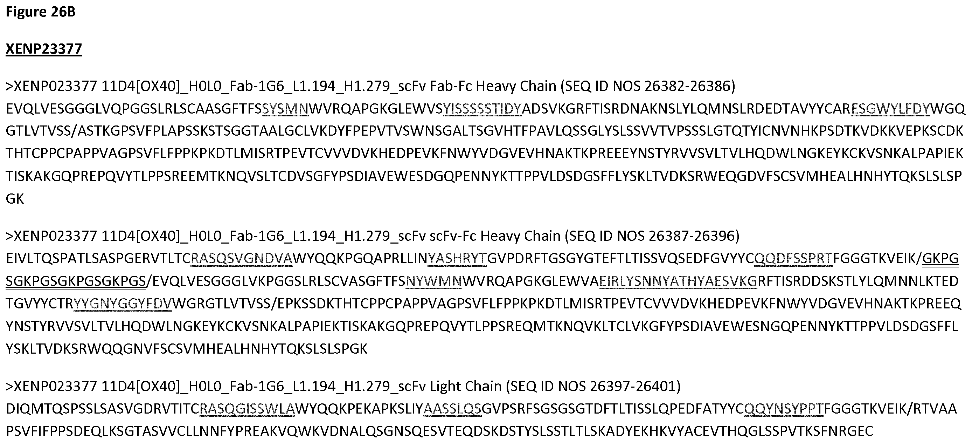

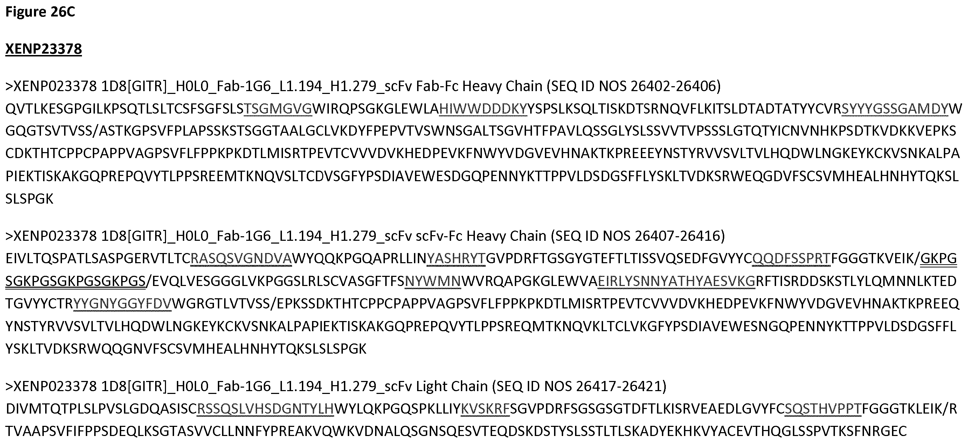

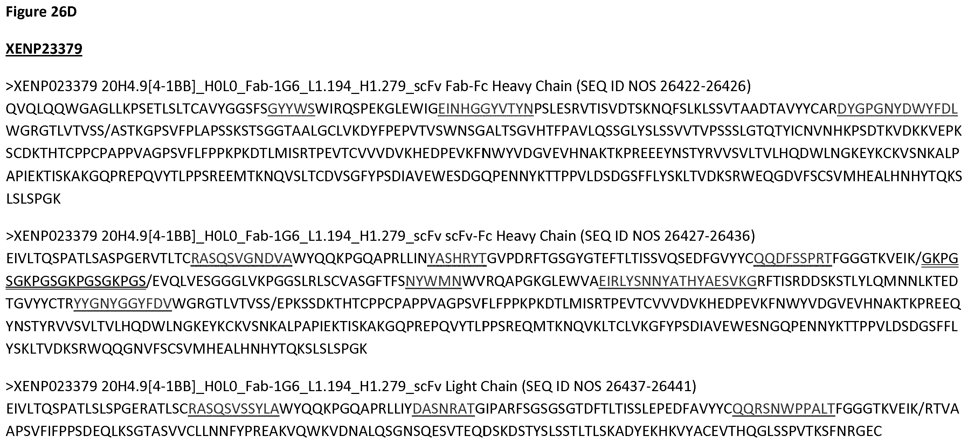

Figure 26A-26D depicts the amino acid sequences of prototype anti-costim x anticheckpoint antibodies in the bottle-opener format (Fab-scFv-Fc). The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either V_{H}-scFv linker-V_{L} or V_{L}-scFv linker-V_{H} as indicated, although this can be reversed. In addition, each sequence outlined herein can include or exclude the M428L/N434S variants in one or preferably both Fc domains, which results in longer half-life in serum.

Figure 27 depicts induction of cytokine secretion by prototype costim/checkpoint bottle-openers in an SEB-stimulated PBMC assay.

Figure 28 depicts induction of IL-2 secretion in naive (non-SEB stimulated) and SEB-stimulated PBMCs following treatment with the indicated test articles.

Figure 29 depicts a schematic associated with the benefit of a costim x checkpoint blockade bispecific antibody, showing that because tumor TILs co-express immune checkpoint receptors and costimulatory receptors, a bispecific antibody increases specificity, enhancing anti-tumor activity and avoiding peripheral toxicity.

Figure 30 depicts that double-positive cells are selectively occupied by exemplary anti-ICOS x anti-PD-1 antibody (XENP20896) as compared to one-arm anti-PD-1 antibody (XENP20111) and one-arm anti-ICOS antibody (XENP20266).

Figure 31A-31B shows the receptor occupancy of anti-ICOS x anti-PD-1 bispecific antibody (XENP20896), one-arm anti-ICOS antibody (XENP20266) and one-arm anti-PD-1 antibody (XENP20111) on A) PD-1 and ICOS double-positive T cells and B) PD-1 and ICOS double-negative T cells after SEB stimulation of human PBMCs.

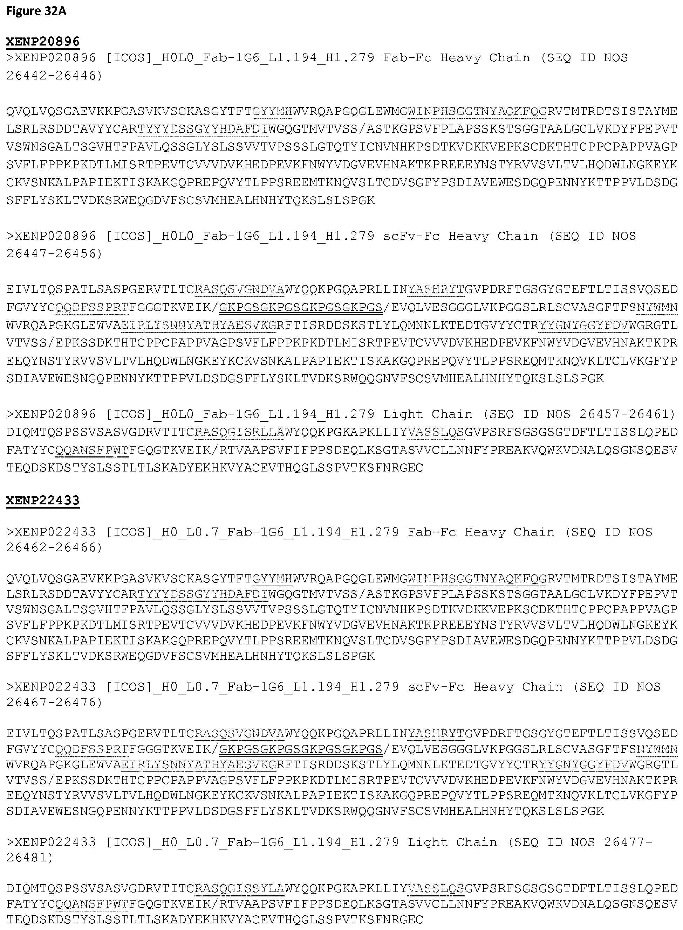

Figure 32A-32D depicts the amino acid sequences of exemplary anti-ICOS x anti-PD-1 antibodies in the bottle-opener format (Fab-scFv-Fc). The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either V_{H}-scFv linker-V_{L} or V_{L}-scFv linker-V_{H} as indicated, although this can be reversed. In addition, each sequence outlined herein can include or exclude the M428L/N434S variants in one or preferably both Fc domains, which results in longer half-life in serum.

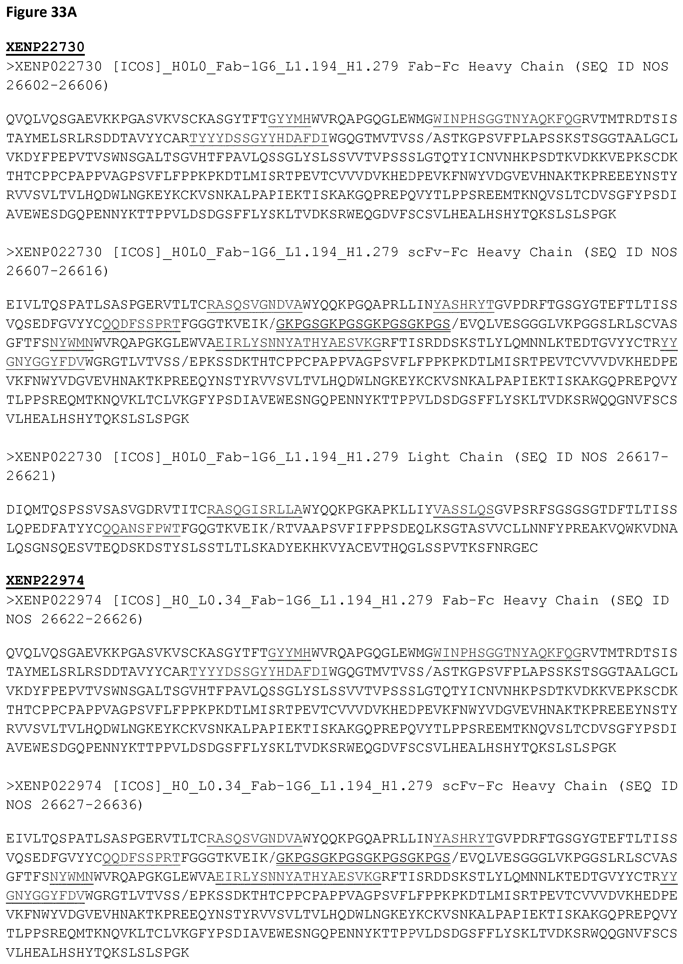

Figure 33A-33C depicts the amino acid sequences of exemplary anti-ICOS x anti-PD-1 antibodies in the bottle-opener format (Fab-scFv-Fc) which include the M428L/N434S variants in one or preferably both Fc domains, which results in longer half-life in serum. The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either V_{H}-scFv linker-V_{L} or V_{L}-scFv linker-V_{H} as indicated, although this can be reversed.

Figure 34A-34C depicts equilibrium dissociation constants (KD), association rates (ka), and dissociation rates (kd) of variant anti-ICOS x anti-PD-1 bispecific antibodies for murine Fc fusion of human ICOS captured on AMC biosensors as determined by Octet.

Figure 35 depicts equilibrium dissociation constants (KD), association rates (ka), and dissociation rates (kd) of variant anti-ICOS x anti-PD-1 bispecific antibodies for biotinylated IgG1 Fc fusions of human and ICOS captured on SA/SAX biosensors as determined by Octet.

Figure 36A-36B depicts cell surface binding of variant anti-ICOS x anti-PD-1 bispecific to human T cells in SEB-stimulated PBMC assays in two separate experiments depicted in A) and B).

Figure 37 shows the receptor occupancy of variant anti-ICOS x anti-PD-1 bispecific antibodies, one-arm anti-ICOS antibodies and one-arm anti-PD-1 antibody (XENP20111) on PD-1 and ICOS double-positive T cells after SEB stimulation of human PBMCs.

Figure 38A-38B show that variant anti-ICOS x anti-PD-1 bispecific antibodies promote A) IL-2 and B) IFNγ secretion from SEB stimulated PBMCs.

Figure 39A-39B show that variant anti-ICOS x anti-PD-1 bispecific antibodies promote A) IL-2 and B) IFNγ secretion from SEB-stimulated PBMCs.

Figure 40A-40B depicts the concentration of IFNγ in mice on Day A) 7 and B) 11 after engraftment with human PBMCs and treatment with the indicated test articles.

Figure 41A-41B depicts CD45+ cell counts in mice as determined by flow cytometry on Day A) 11 and B)14 after engraftment with human PBMCs and treatment with the indicated test articles.

Figure 42A-42B depicts A) CD8+ T cell and B) CD4+ T cell counts in mice as determined by flow cytometry on Day 14 after engraftment with human PBMCs and treatment with the indicated test articles (**p ≤ 0.01).

Figure 43 depicts the change in body weight in mice by Day 14 after engraftment with human PBMCs and treatment with the indicated test articles (**p ≤ 0.01).

Figure 44A-44B depicts the concentration of IFNγ in mice on Day A) 7 and B) 14 after engraftment with human PBMCs and treatment with the indicated test articles.

Figure 45 depicts CD45+ cell counts in mice as determined by flow cytometry on Day 14 after engraftment with human PBMCs and treatment with the indicated test articles.

Figure 46A-46C depicts A) CD8+ T cell and B) CD4+ T cell counts in mice as determined by flow cytometry on Day 14 after engraftment with human PBMCs and treatment with the indicated test articles."

Figure 47A-47B depicts the change in body weight in mice by Day 12 and 15 after engraftment with human PBMCs and treatment with the indicated test articles.

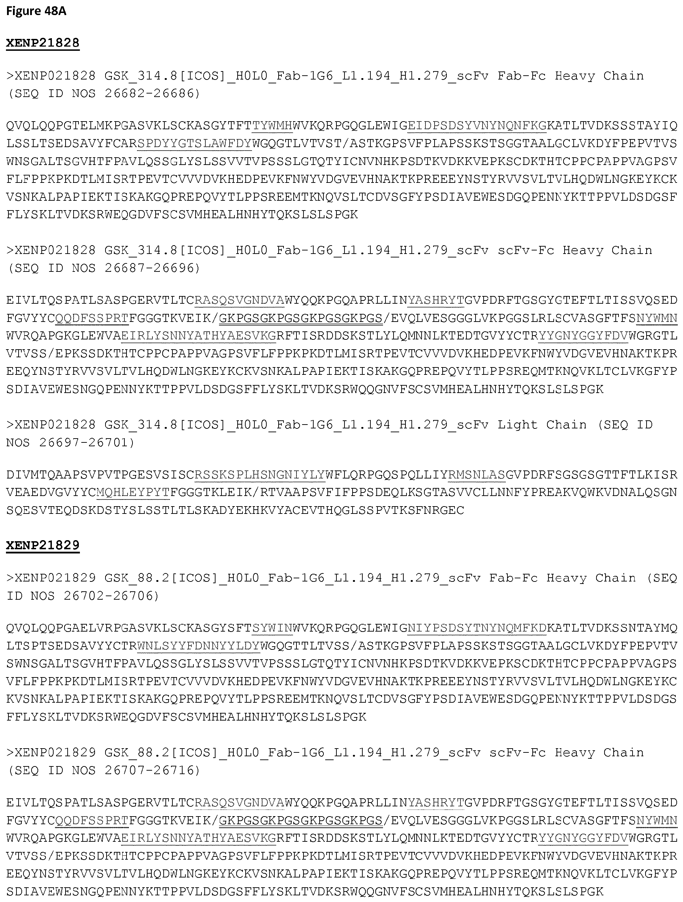

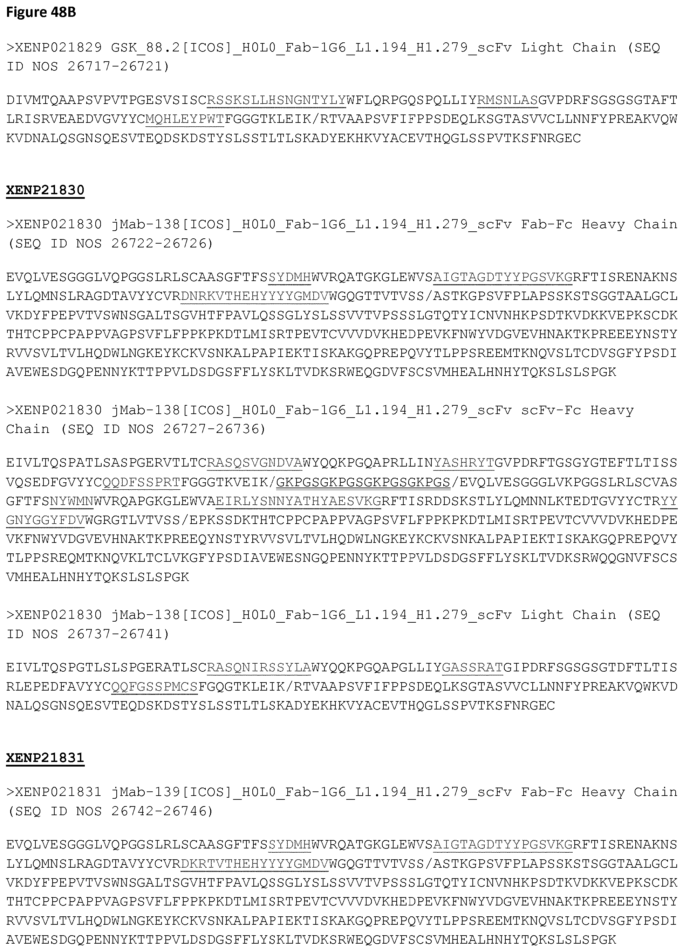

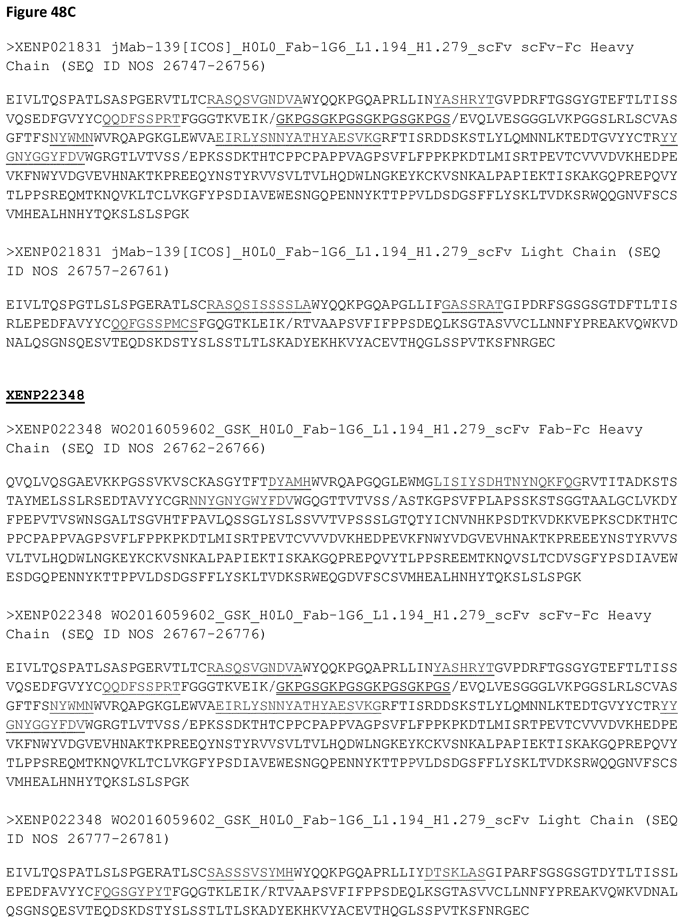

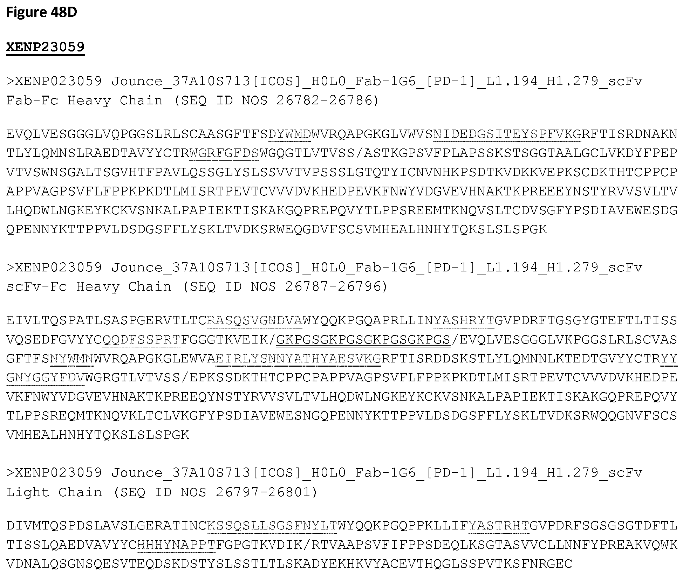

Figure 48A-48D depicts the amino acid sequences of exemplary anti-ICOS x anti-PD-1 antibodies in the bottle-opener format (Fab-scFv-Fc) with alternative ICOS ABDs. The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

Figure 49 depict cytokine release assay for IL-2 after SEB-stimulation of human PBMCs and treatment with alternative anti-ICOS x anti-PD-1 bispecific antibodies.

Figure 50 depict cytokine release assay for IL-2 (as fold induction over bivalent anti-RSV mAb) after SEB-stimulation of human PBMCs and treatment with alternative anti-ICOS x anti-PD-1 bispecific antibodies.

Figure 51 depicts AKT phosphorylation in SEB-stimulated purified CD3+ T cells after treatment with anti-ICOS x anti-PD-1 bispecific antibodies.

Figure 52 depicts fold induction of A) IL-17A, B) IL17F, C) IL-22, D) IL-10, E) IL-9, and F) IFNγ gene expression by the indicated test articles over induction by bivalent anti-RSV as determined by NanoString.

Figure 53A-53F depict mean fold induction in expression of selected immune response genes by indicated test articles over treatment with bivalent anti-RSV mAb as determined by NanoString. The shading intensity corresponds to the magnitude of the fold change.

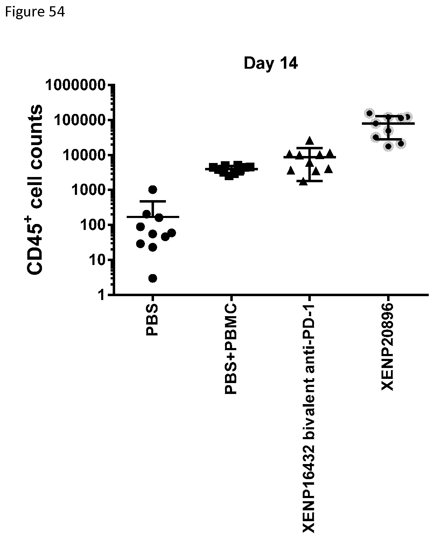

Figure 54 depicts CD45+ cell counts in mice as determined by flow cytometry on Day 14 after engraftment with human PBMCs and treatment with the indicated test articles.

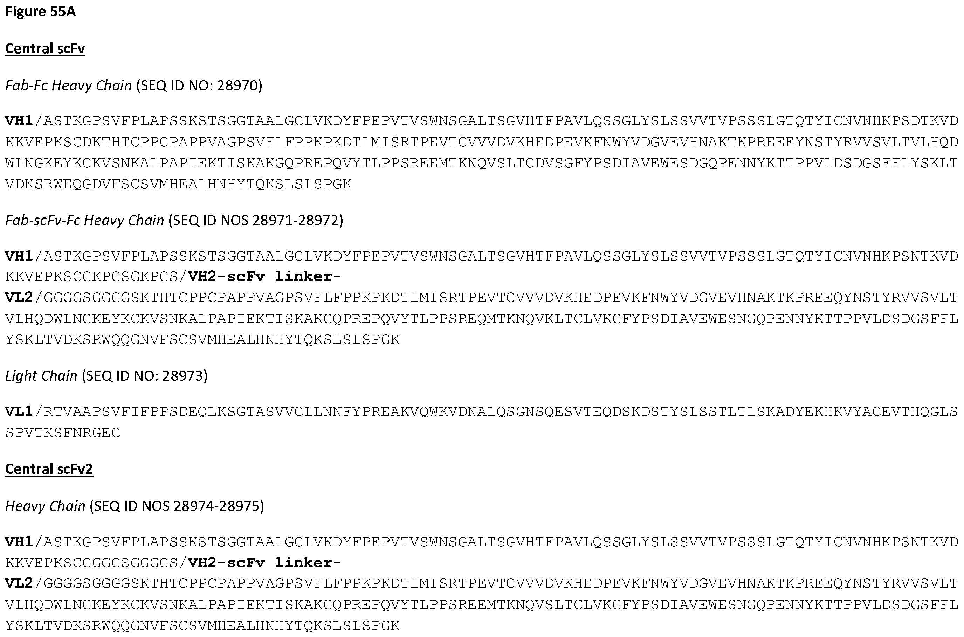

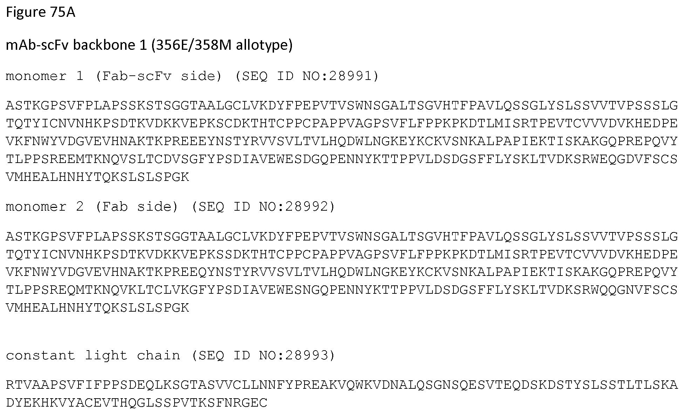

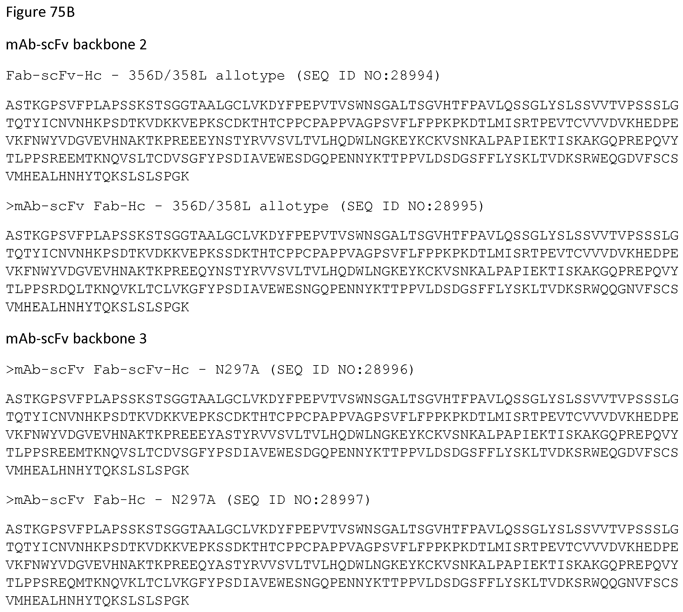

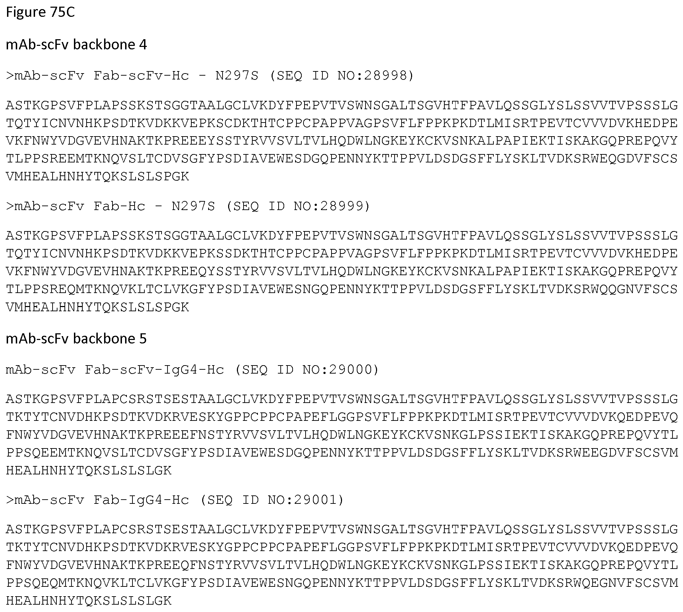

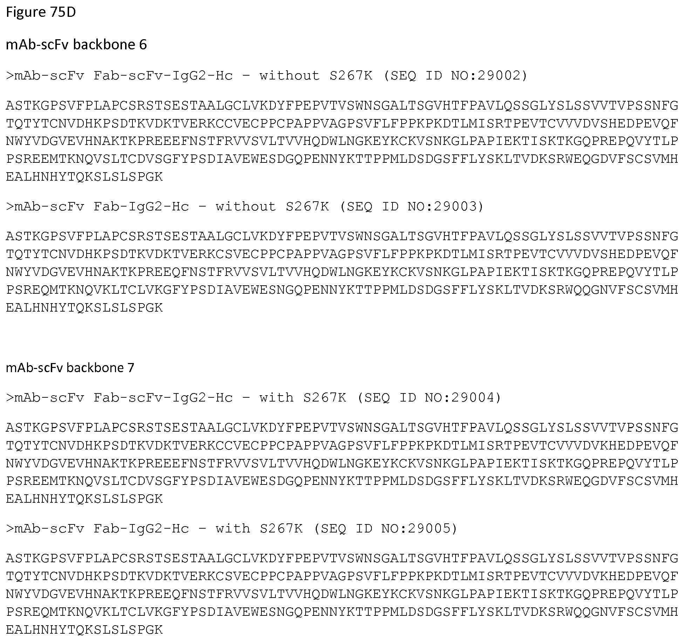

Figure 55A-55D, similar to Figure 9 and Figure 75, depicts the sequences of the "backbone" portion (e.g. without the Fvs) of a number of additional formats, including the Central scFv of Figure 2F, the Central-scFv2 format of Figure 2J, the bispecific mAb of Figure 2K, the DVD-Ig of Figure 2L and the Trident format of Figure 2M. In Figure 2L, the DVD-Ig^{®} linkers are shown with double underlining, with other linkers found in WO2007/024715, in particular for those sequences. In the Trident format, other Trident linkers and coil-coil sequences are shown in WO 2015/184203, in particular for those sequences. As will be appreciated by those in the art, bolded domains (e.g. "VH1", VH2-scFv linker-VL2", etc.) are separated with slashes "/", and may include optional domain linkers as needed. All of these backbones utilize the kappa constant region for the light chain, although the lambda chain can also be used. As for Figure 9 and Figure 75, these backbones can be combined with any vh and vl domains as outlined herein.

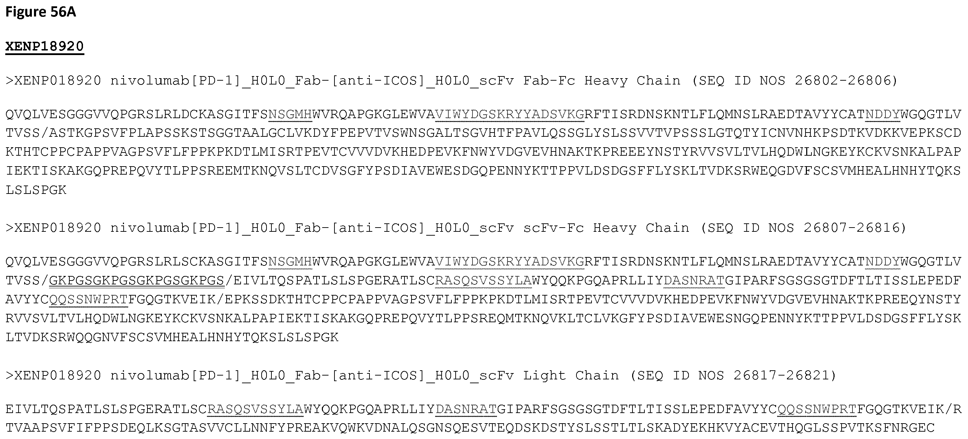

Figure 56A-56B depicts the amino acid sequence of illustrative anti-PD-1 x anti-ICOS antibodies in the bottle-opener format (Fab-scFv-Fc). The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

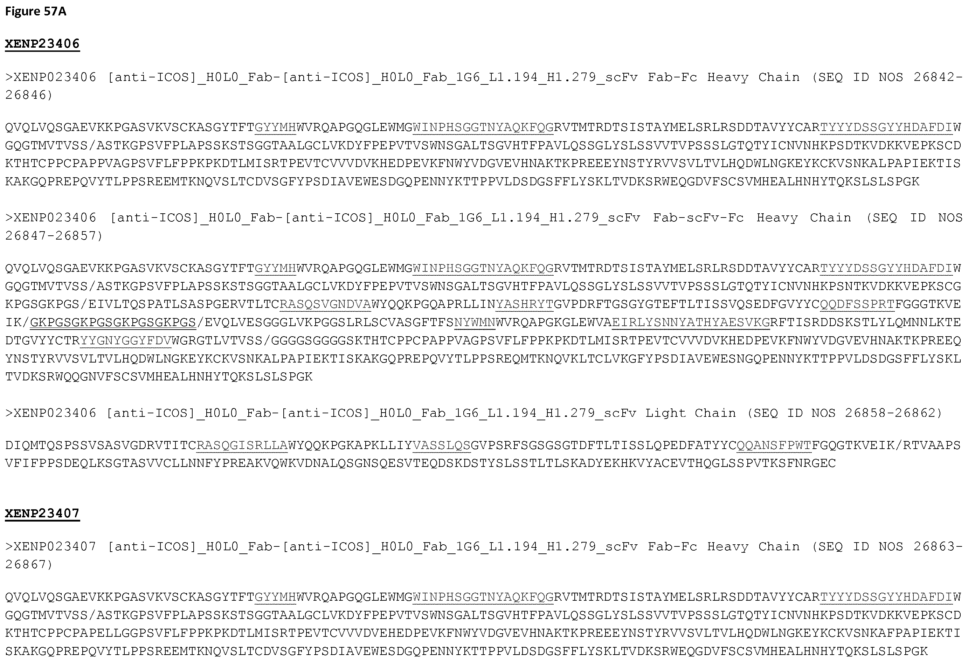

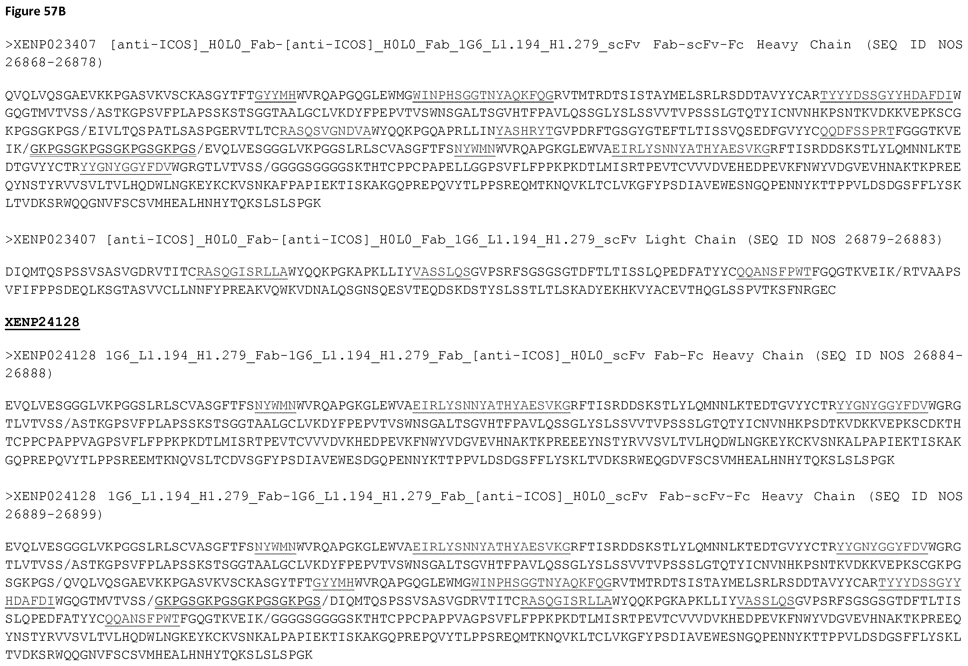

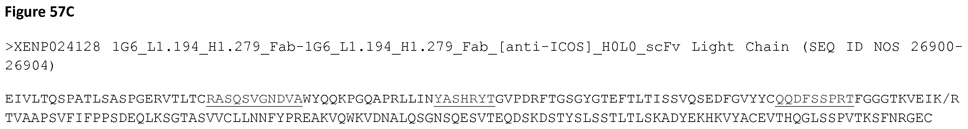

Figure 57A-57C depicts the amino acid sequence of illustrative anti-PD-1 x anti-ICOS antibodies in the central-scFv format. The antibodies are named using the first Fab-Fc variable region first and the Fab-scFv-Fc variable region second, separated by a dash, followed by the chain designation (Fab-Fc heavy chain, Fab-scFv-Fc heavy chain or light chain). CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

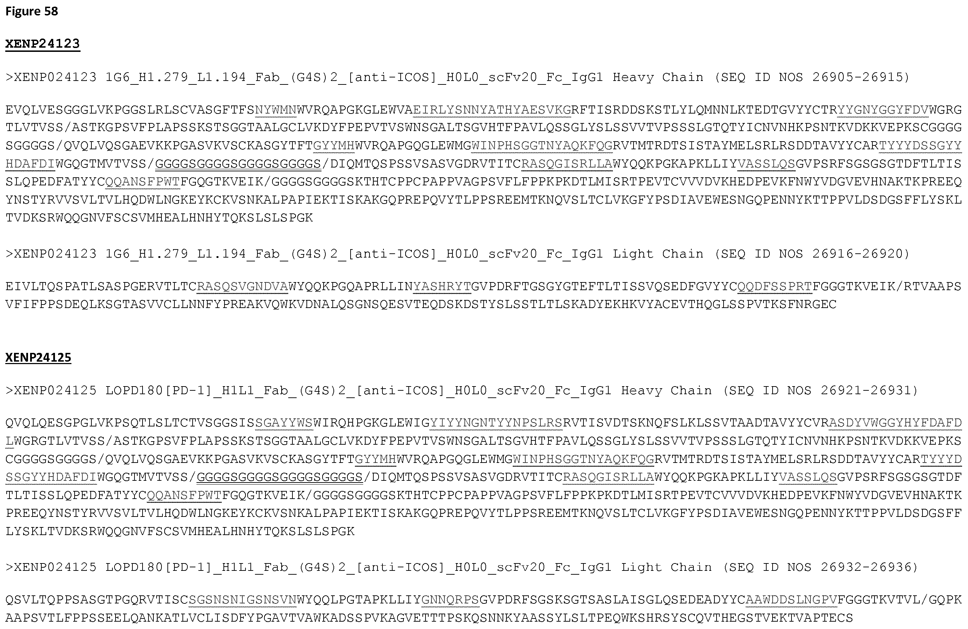

Figure 58 depicts the amino acid sequence of illustrative anti-PD-1 x anti-ICOS antibodies in the central-scFv2 format. The antibodies are named using the Fab variable region first and the scFv variable region second, followed by the chain designation (heavy chain or light chain). CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

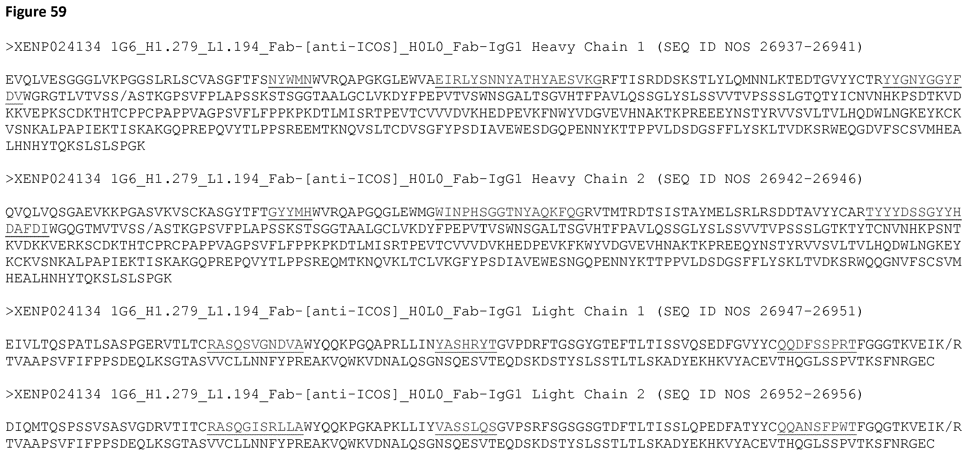

Figure 59 depicts the amino acid sequence of an illustrative anti-PD-1 x anti-ICOS antibody in the bispecific mAb format. The antibodies are named using the first Fab variable region for a first antigen and the second Fab variable region for a second antigen, separated by a dash, followed by the chain designation (Heavy Chain 1 or Light Chain 1 for the first antigen and Heavy Chain 2 or Light Chain 2 for the second antigen). CDRs are underlined and slashes indicate the border(s) of the variable regions. Each sequence outlined herein can include or exclude the M428L/N434S variants in one or preferably both Fc domains, which results in longer half-life in serum.

Figure 60 depicts the amino acid sequence of an illustrative anti-PD-1 x anti-ICOS antibody in the DVD-IgG format. The antibodies are named using the first variable region for a first antigen and the second Fab variable region for a second antigen, followed by the chain designation (Heavy Chain or Light Chain). CDRs are underlined and slashes indicate the border(s) of the variable regions. Each sequence outlined herein can include or exclude the M428L/N434S variants in one or preferably both Fc domains, which results in longer half-life in serum.

Figure 61A-61B depicts the amino acid sequence of an illustrative anti-PD-1 x anti-ICOS antibody in the Trident format. The antibodies are named using the VL and VH of a first antigen which comprises a DART and the Fab variable region for a second antigen, separated by a dash, followed by the chain designation (Heavy Chain or Light Chain). CDRs are underlined and slashes indicate the border(s) of the variable regions. Each sequence outlined herein can include or exclude the M428L/N434S variants in one or preferably both Fc domains, which results in longer half-life in serum.

Figure 62 depicts induction of cytokine secretion (IL-2) by alternative format costim x checkpoint blockade bispecific antibodies in an SEB-stimulated PBMC assay.

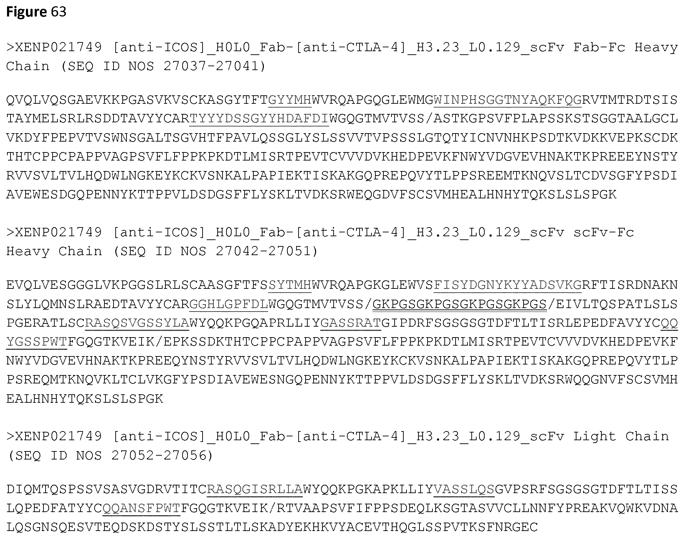

Figure 63 depicts the amino acid sequences of an illustrative anti-ICOS x anti-CTLA-4 antibody in the bottle-opener format (Fab-scFv-Fc). The antibodies are named using the Fab variable region first and the scFv variable region second, separated by a dash, followed by the chain designation (Fab-Fc heavy chain, scFv-Fc heavy chain or light chain). CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

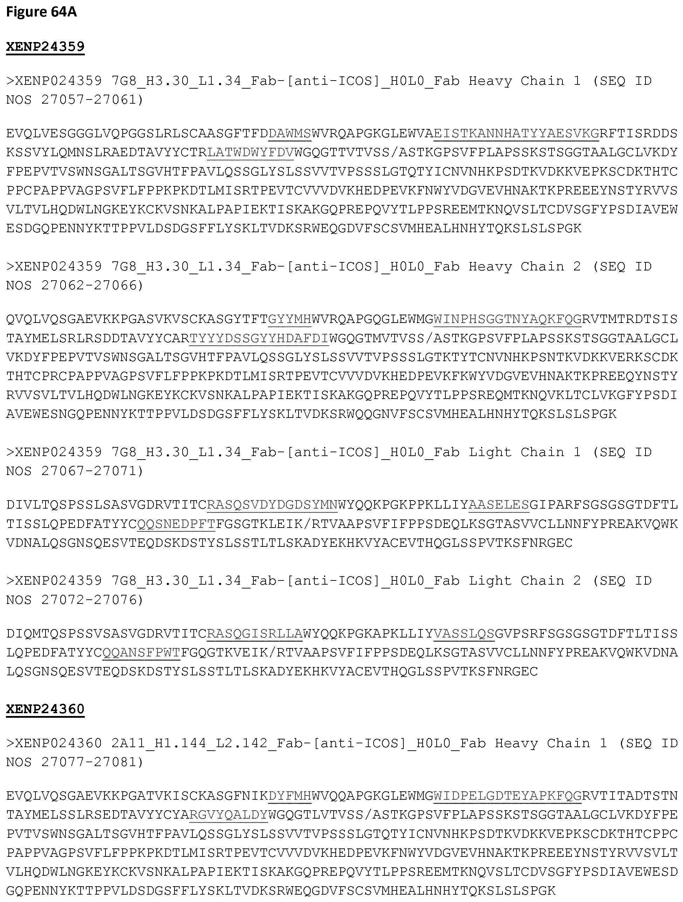

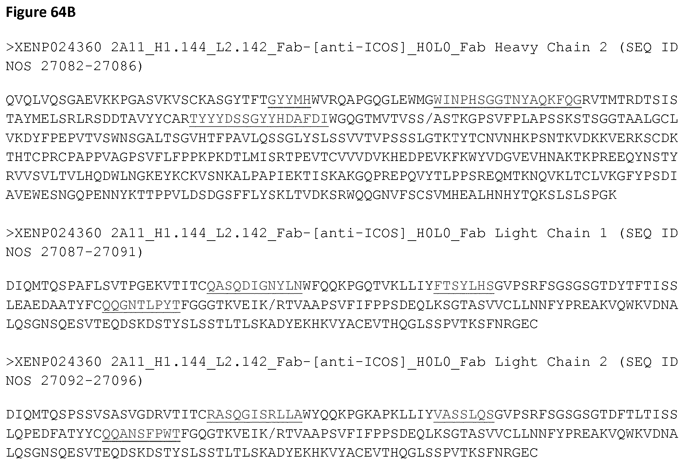

Figure 64A-64B depicts the amino acid sequence of illustrative anti-LAG-3 x anti-ICOS antibodies in the bispecific mAb format. The antibodies are named using the first Fab variable region for a first antigen and the second Fab variable region for a second antigen, separated by a dash, followed by the chain designation (Heavy Chain 1 or Light Chain 1 for the first antigen and Heavy Chain 2 or Light Chain 2 for the second antigen). CDRs are underlined and slashes indicate the border(s) of the variable regions. Each sequence outlined herein can include or exclude the M428L/N434S variants in one or preferably both Fc domains, which results in longer half-life in serum.

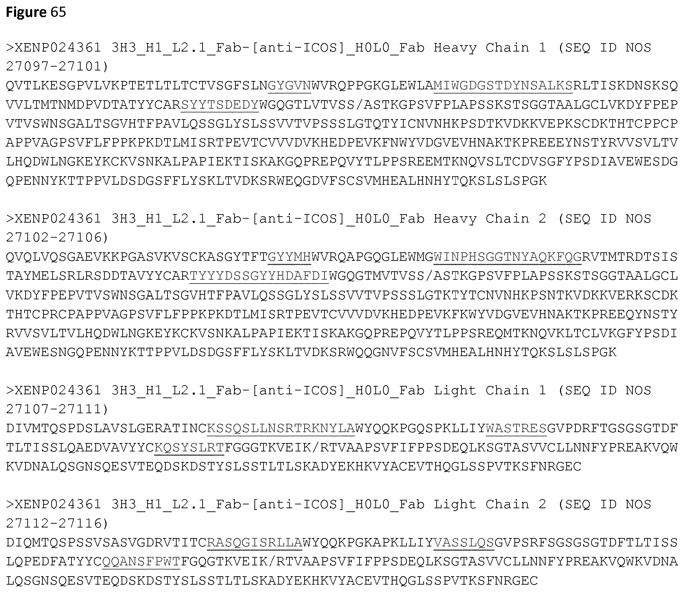

Figure 65 depicts the amino acid sequence of an illustrative anti-TIM-3 x anti-ICOS antibody in the bispecific mAb format. The antibodies are named using the first Fab variable region for a first antigen and the second Fab variable region for a second antigen, separated by a dash, followed by the chain designation (Heavy Chain 1 or Light Chain 1 for the first antigen and Heavy Chain 2 or Light Chain 2 for the second antigen). CDRs are underlined and slashes indicate the border(s) of the variable regions. Each sequence outlined herein can include or exclude the M428L/N434S variants in one or preferably both Fc domains, which results in longer half-life in serum.

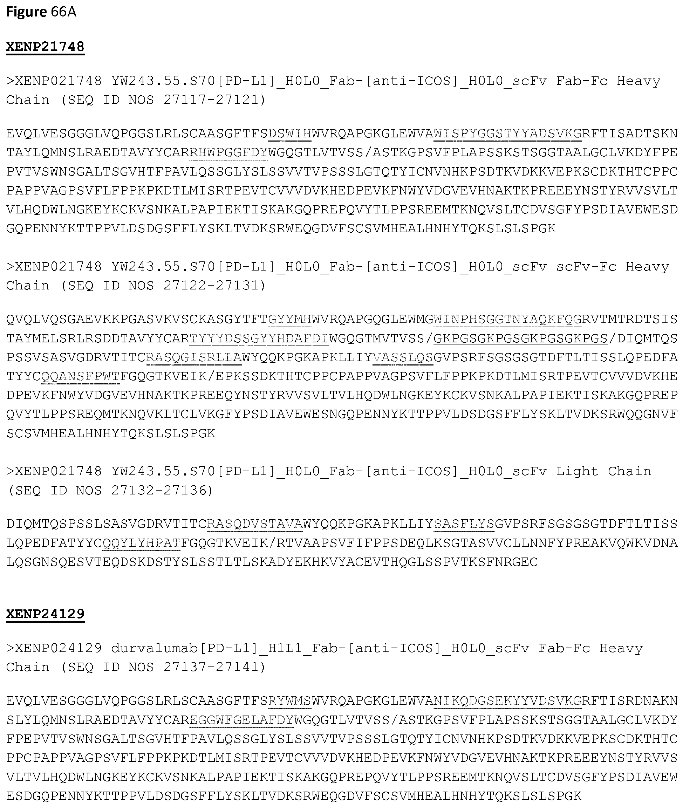

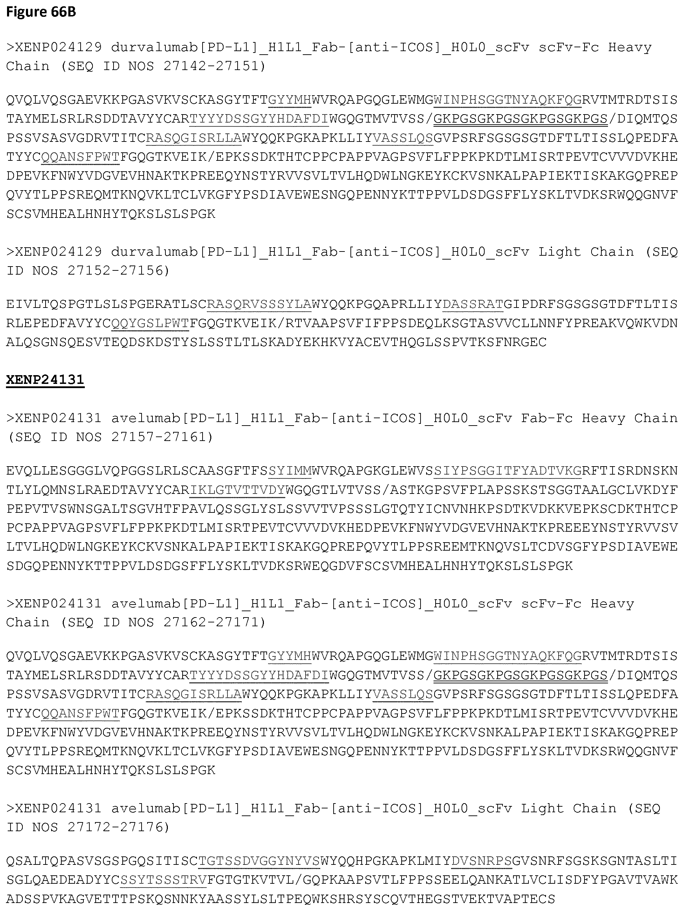

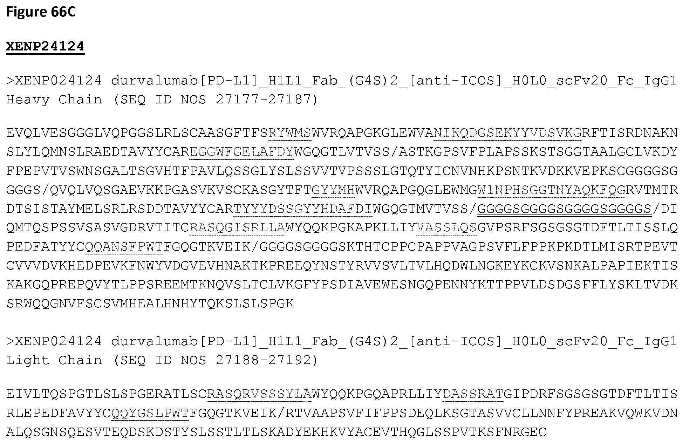

Figure 66A-66C depicts the amino acid sequences of anti-ICOS x anti-PD-L1 antibodies in the bottle-opener format (Fab-scFv-Fc) and central-scFv2 format. The bottle-openers are named using the Fab variable region first and the scFv variable region second, separated by a dash, followed by the chain designation (Fab-Fc heavy chain, scFv-Fc heavy chain or light chain). Central-scFv2s are named using the Fab variable region first and the scFv variable region second, followed by the chain designation (heavy chain or light chain). CDRs are underlined and slashes indicate the border(s) of the variable regions. CDRs are underlined and slashes indicate the border(s) of the variable regions. The scFv domain has different orientations (N- to C-terminus) of either VH-scFv linker-VL or VL-scFv linker-VH as indicated, although this can be reversed. In addition, each sequence outlined herein can include or exclude the M428L/N434S variants in one or preferably both Fc domains, which results in longer half-life in serum.

Figure 67 depicts induction of cytokine secretion (IL-2) by additional costim x checkpoint blockade bispecific antibodies in an SEB-stimulated PBMC assay.

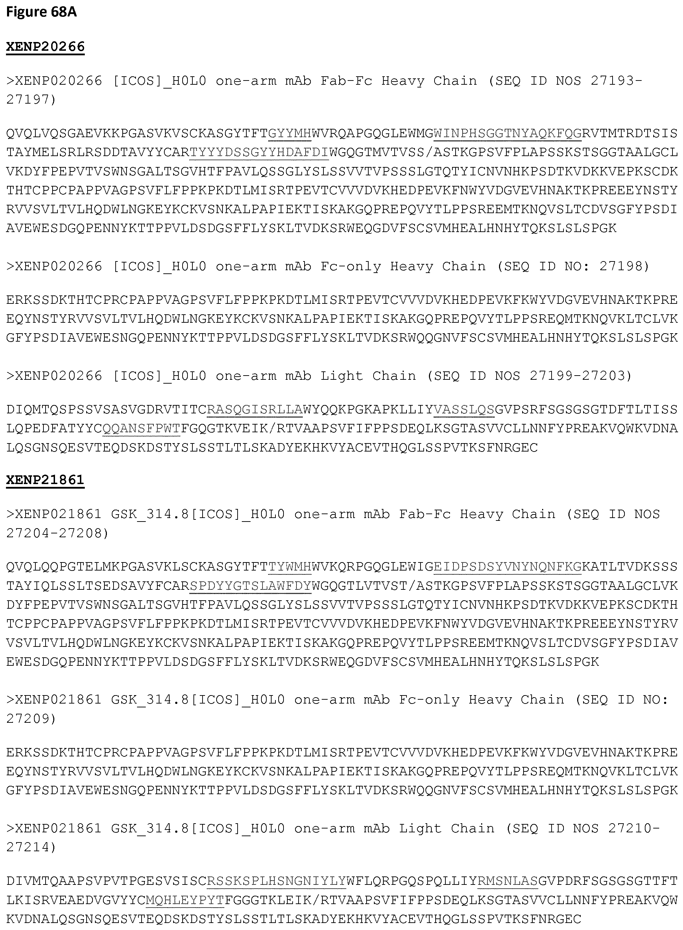

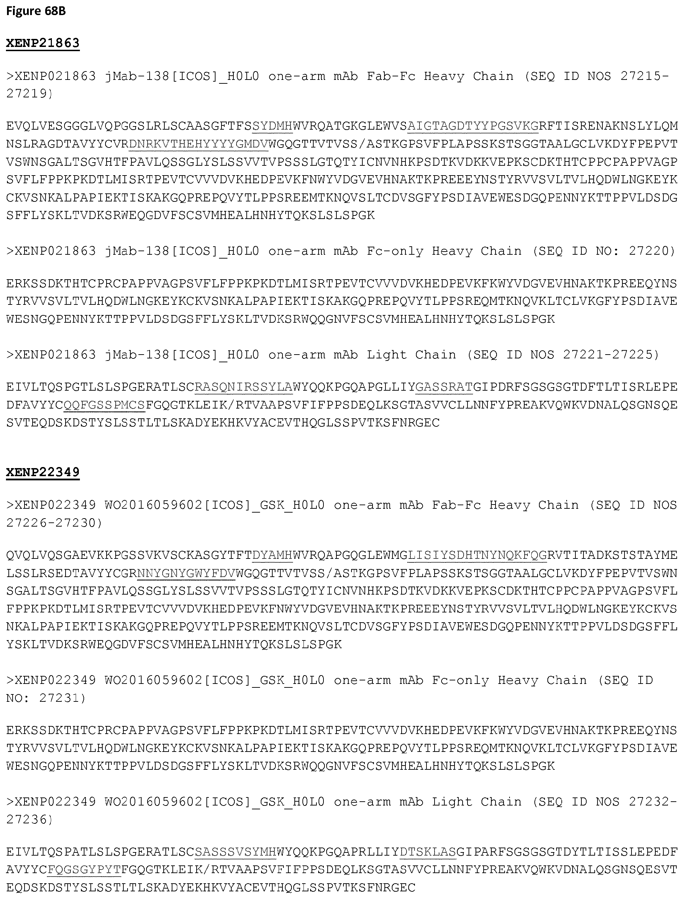

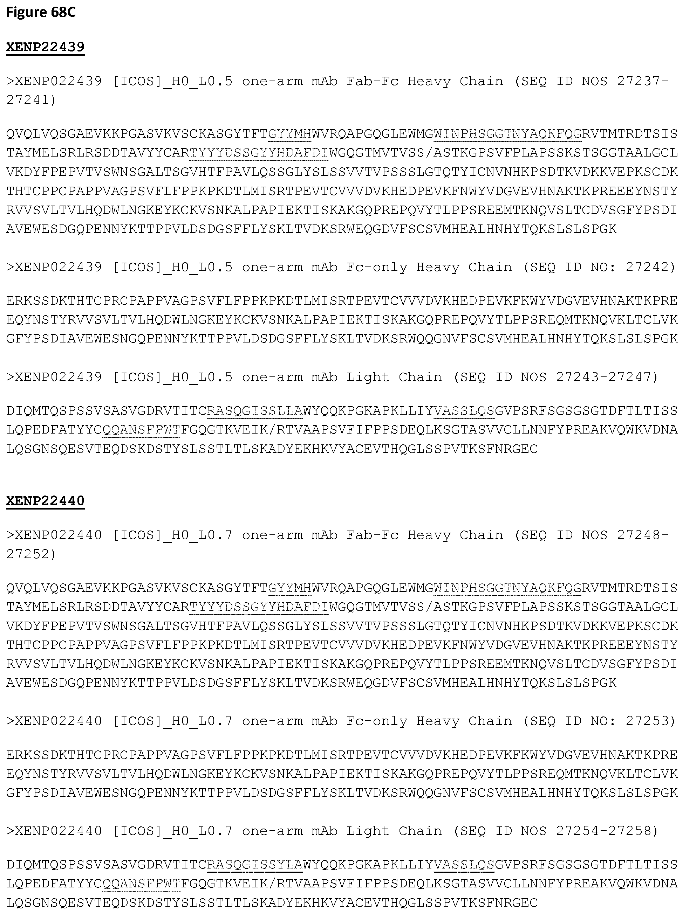

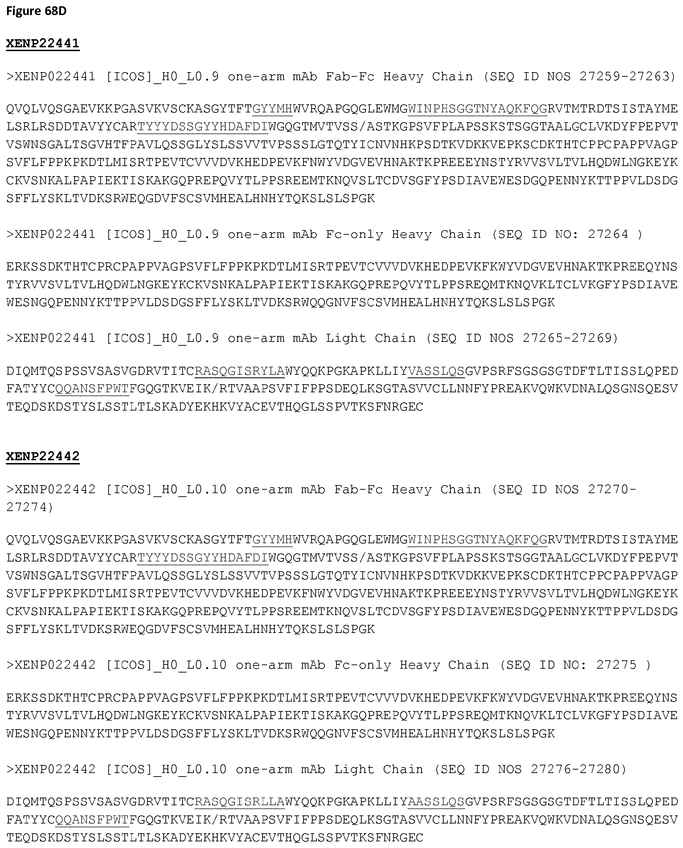

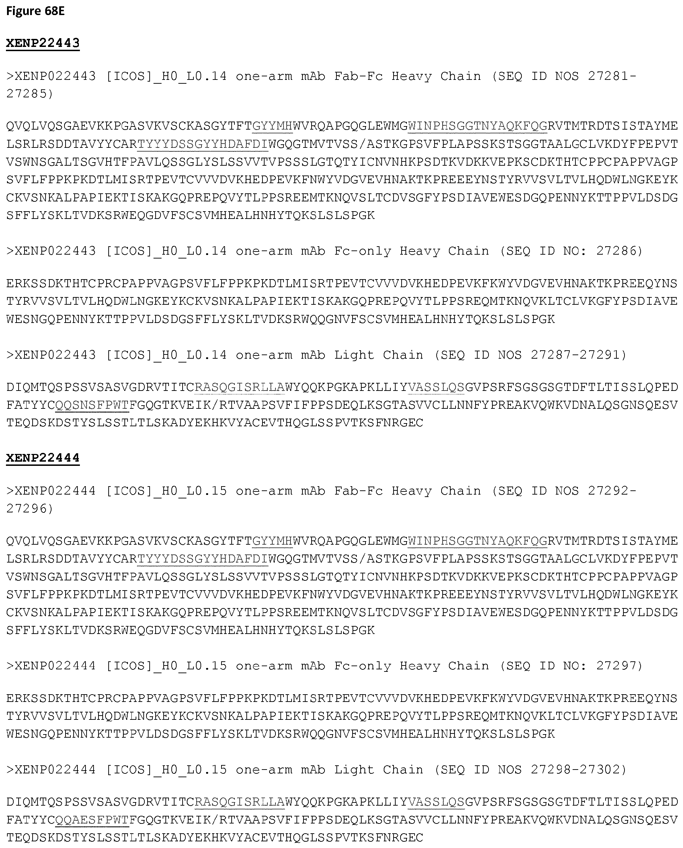

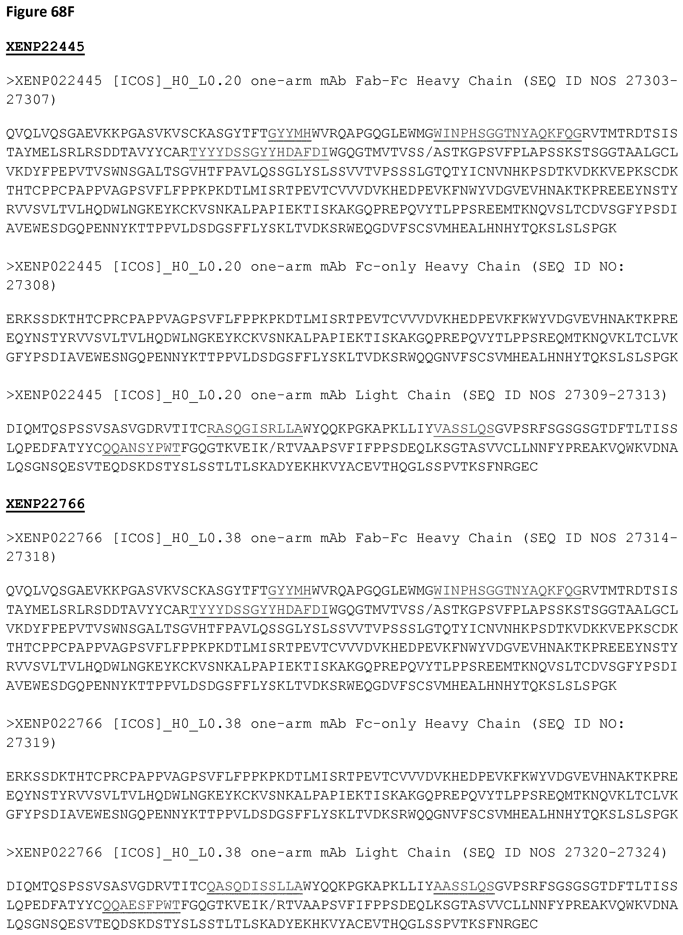

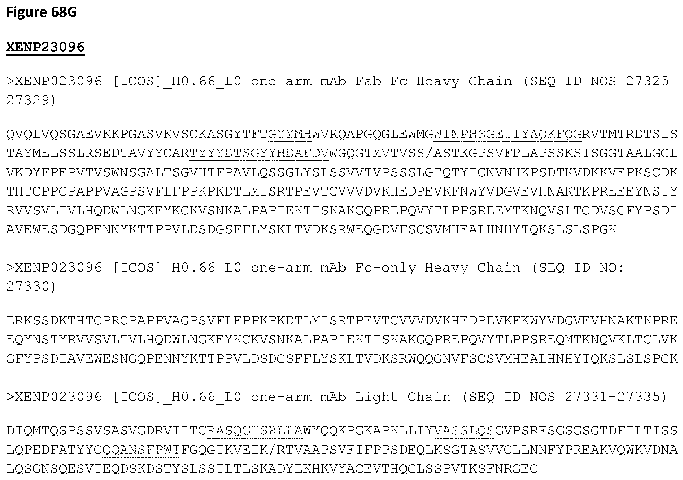

Figure 68A-68G depict amino acid sequences for exemplary one-arm anti-ICOS Fab-Fc antibodies. CDRs are underlined and slashes indicate the border(s) of variable regions. These are referred to as "one-arm" or "one armed" formats as one amino acid chain is only an Fc domain, with the other side being an anti-ICOS Fab side. The Fc domain contains the S364K/E357Q skew variants, as well as the pI(-)_Isosteric_A variants depicted in Figure 68A-68G. The Fab Fc domain contains the L368D/K370S skew variants as well as the pI ISO(+RR) variants depicted in Figure 68A-68G. Both Fc domains include the ablation variants (E233P/L234V/L235A/G236del/S267K).

Figure 69 depicts equilibrium dissociation constants (KD), association rates (ka), and dissociation rates (kd) of variant one-arm anti-ICOS Fab-Fc antibodies for murine Fc fusions of human ICOS captured on AMC biosensors as determined by Octet.

Figure 70 depicts AKT phosphorylation in SEB-stimulated purified CD3+ T cells after treatment with bivalent and monovalent anti-PD-1 antibodies and anti-ICOS x anti-PD-1 bispecific antibodies.

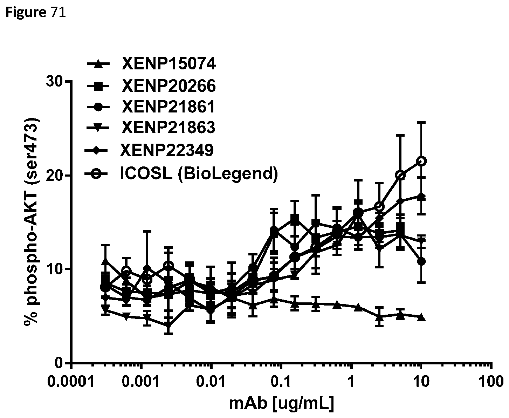

Figure 71 depicts AKT phosphorylation in purified CD3+ T cells after treatment with monovalent anti-ICOS Fab-Fc antibodies with alternative anti-ICOS ABDs.

Figure 72A-72C depict some prototype bispecific antibodies (OX40 X PD-1, GITR X PD-1, 4-1BB X PD-1, CTLA-4 X ICOS).

Figure 73A-73H depict some prototype mAbs (4-1BB, OX40, GITR, ICOS, PD-L1 and PD-1), the Fvs of which can be used in combination with the other Fvs disclosed herein and in any format (bottle opener, mAb-Fv, mAb-scFv, central-scFv, bispecific mAb, central-Fv, one armed central-scFv, one armed scFv-mAb, dual scFv, DVD-Ig or Trident). Some additional ICOS X PD-L1 bottle opener sequences are shown as well.

Figure 74A-74F depict additional PD-1 X ICOS bottle openers, in some cases with the PD-1 Fv being in the Fab format and the ICOS Fv in a scFv format and in other cases reversed.

Figure 75A to 75D shows the sequences of a mAb-scFv backbone of use in the disclosure, to which the Fv sequences of the disclosure may be added. mAb-scFv backbone 1 is based on human IgG1 (356E/358M allotype), and includes the S364K/E357Q : L368D/K370S skew variants, the N208D/Q295E/N384D/Q418E/N421D pI variants on the Fab side and the E233P/L234V/L235A/G236del/S267K ablation variants on both chains. Backbone 2 is based on human IgG1 (356D/358L allotype), and includes the S364K/E357Q : L368D/K370S skew variants, the N208D/Q295E/N384D/Q418E/N421D pI variants on the Fab side and the E233P/L234V/L235A/G236del/S267K ablation variants on both chains. Backbone 3 is based on human IgG1 (356E/358M allotype), and includes the S364K/E357Q : L368D/K370S skew variants, N208D/Q295E/N384D/Q418E/N421D pI variants on the Fab side and the E233P/L234V/L235A/G236del/S267K ablation variants on both chains, as well as an N297A variant on both chains. Backbone 4 is identical to 3 except the mutation is N297S. Alternative formats for mAb-scFv backbones 3 and 4 can exclude the ablation variants E233P/L234V/L235A/G236del/S267K in both chains. Backbone 5 is based on human IgG4, and includes the S364K/E357Q : L368D/K370S skew variants, the N208D/Q295E/N384D/Q418E/N421D pI variants on the Fab side and the E233P/L234V/L235A/G236del/S267K ablation variants on both chains, as well as a S228P (EU numbering, this is S241P in Kabat) variant on both chains that ablates Fab arm exchange as is known in the art Backbone 6 is based on human IgG2, and includes the S364K/E357Q : L368D/K370S skew variants, the N208D/Q295E/N384D/Q418E/N421D pI variants on the Fab side. Backbone 7 is based on human IgG2, and includes the S364K/E357Q : L368D/K370S skew variants, the N208D/Q295E/N384D/Q418E/N421D pI variants on the Fab side as well as a S267K variant on both chains.

As will be appreciated by those in the art and outlined below, these sequences can be used with any vh and vl pairs outlined herein, with one monomer including both a Fab and an scFv (optionally including a charged scFv linker) and the other monomer including the Fab sequence (e.g. a vh attached to the "Fab side heavy chain" and a vl attached to the "constant light chain"). That is, any Fv sequences outlined herein for anti-CTLA-4, anti-PD-1, anti-LAG-3, anti-TIM-3, anti-TIGIT, anti-BTLA, anti-ICOS, anti-GITR, anti-OX40 and anti-4-1BB, whether as scFv (again, optionally with charged scFv linkers) or as Fabs, can be incorporated into this Figure 75A-75D backbone in any combination. The monomer 1 side is the Fab-scFv pI negative side, and includes the heterodimerization variants L368D/K370S, the isosteric pI variants N208D/Q295E/N384D/Q418E/N421D, the ablation variants E233P/L234V/L235A/ G236del/S267K, (all relative to IgG1). The monomer 2 side is the scFv pI positive side, and includes the heterodimerization variants 364K/E357Q. However, other skew variant pairs can be substituted, particularly [S364K/E357Q : L368D/K370S]; [L368D/K370S : S364K]; [L368E/K370S : S364K]; [T411T/E360E/Q362E : D401K]; [L368D/K370S : S364K/E357L], [K370S : S364K/E357Q], [T366S/L368A/Y407V : T366W] and [T366S/L368A/Y407V/Y394C : T366W/S354C].

The constant light chain depicted in Figure 75A can be used for all of the constructs in the figure, although the kappa constant light chain can also be substituted.

It should be noted that these mAb-scFv backbones find use in the both the mAb-Fv format of Figure 1 (where one monomer comprises a vl at the C-terminus and the other a vh at the C-terminus) as well as the scFv-mAb format (with a scFv domain added to the C-terminus of one of the monomers).

Included within each of these backbones are sequences that are 90, 95, 98 and 99% identical (as defined herein) to the recited sequences, and/or contain from 1, 2, 3, 4, 5, 6, 7, 8, 9 or 10 additional amino acid substitutions (as compared to the "parent" of the Figure, which, as will be appreciated by those in the art, already contain a number of amino acid modifications as compared to the parental human IgG1 (or IgG2 or IgG4, depending on the backbone). That is, the recited backbones may contain additional amino acid modifications (generally amino acid substitutions) in addition to the skew, pI and ablation variants contained within the backbones of this figure.

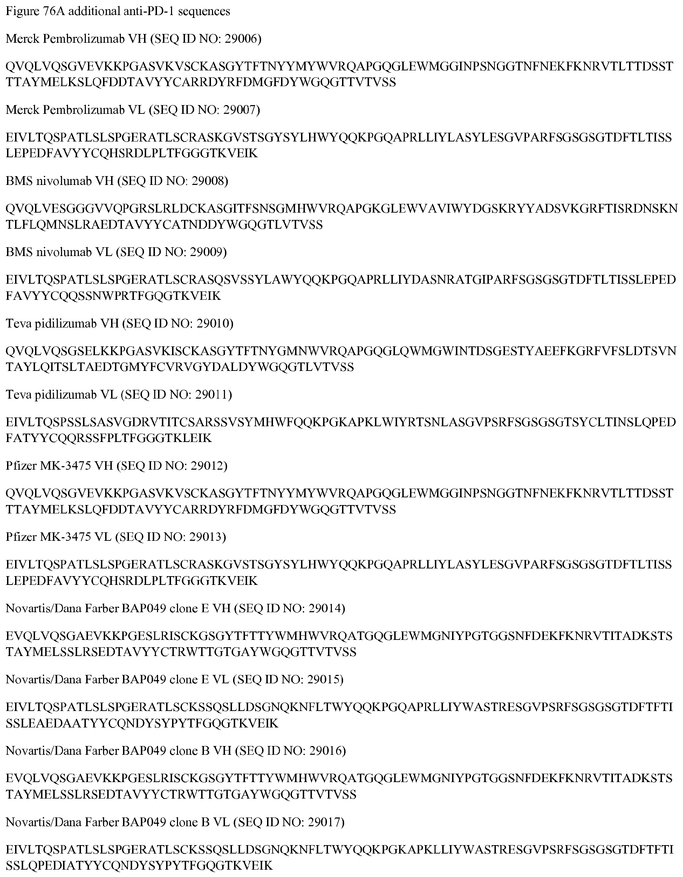

Figure 76A-76F depict a number of prior art sequences for Fvs that bind human PD-1 as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

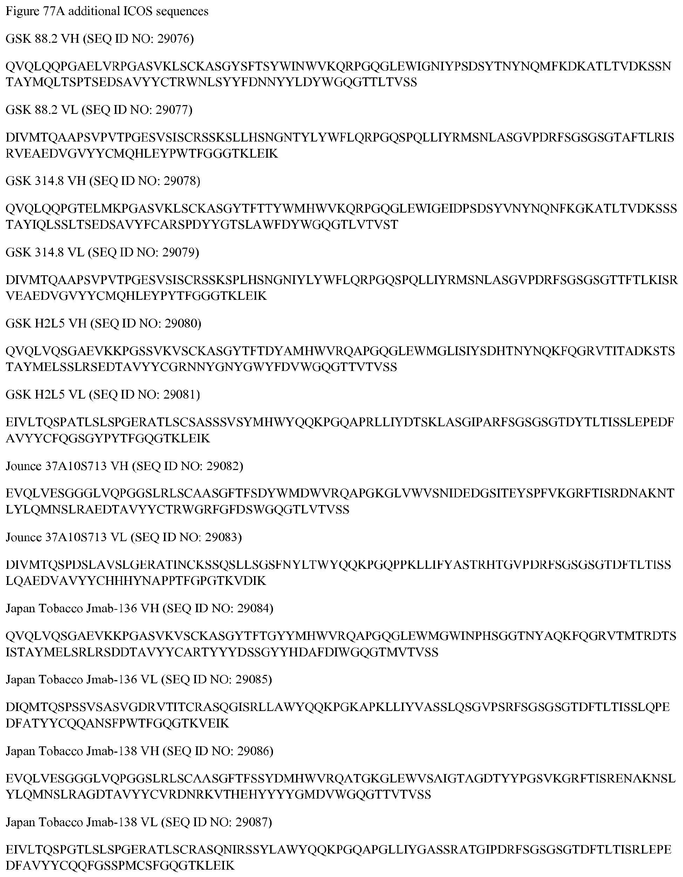

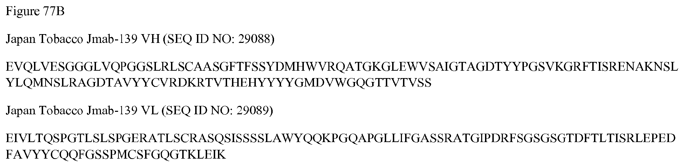

Figure 77A-77B depict a number of prior art sequences for Fvs that bind human ICOS as vh and vl sequences. As will be appreciated by those in the art, any of these Fvs can be combined with an Fv that binds a checkpoint receptor (e.g. PD-1, PD-L1, CTLA-4, TIM-3, LAG-3, TIGIT and BTLA, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with PD-1 ABDs having the identifiers 1G6_H1.279_L1.194; 1G6_H1.280_L1.224; 1G6_L1.194_H1.279; 1G6_L1.210_H1.288; and 2E9_H1L1.

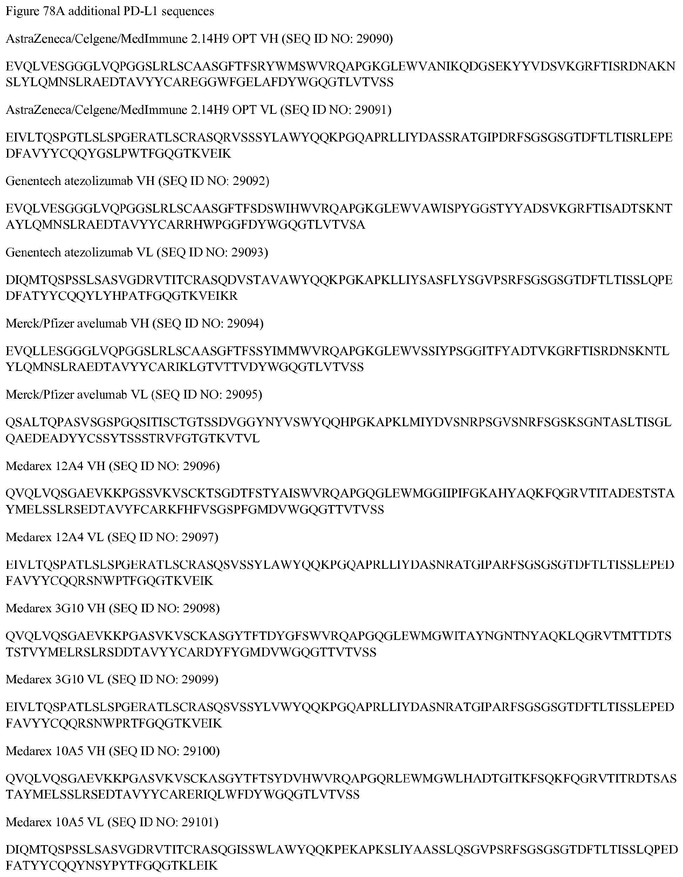

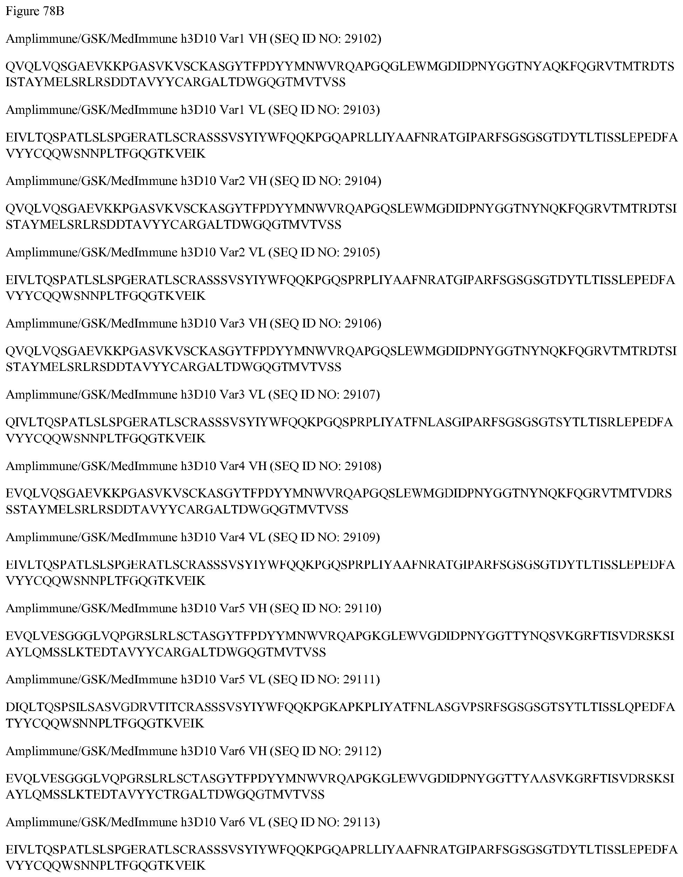

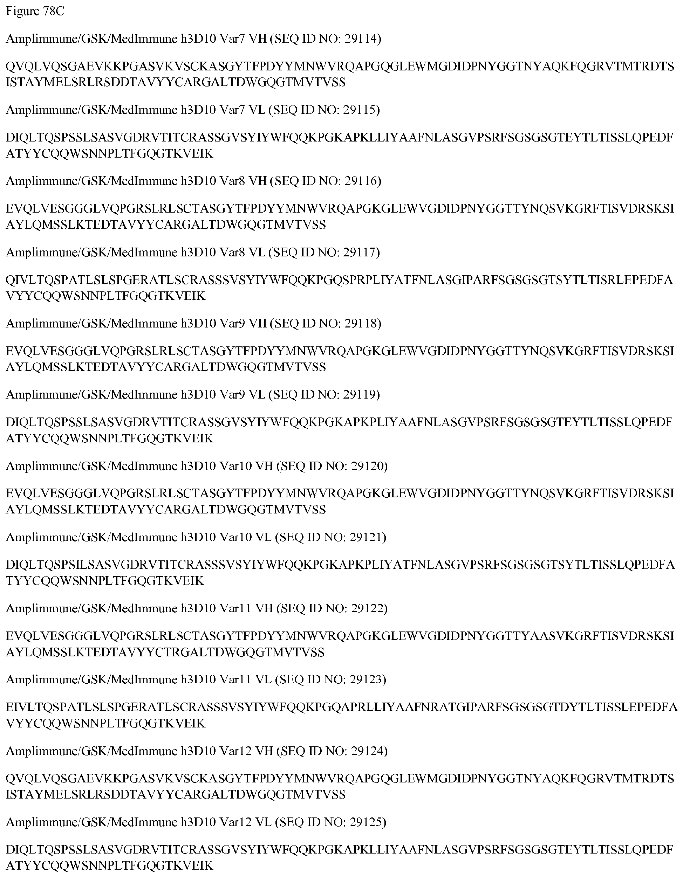

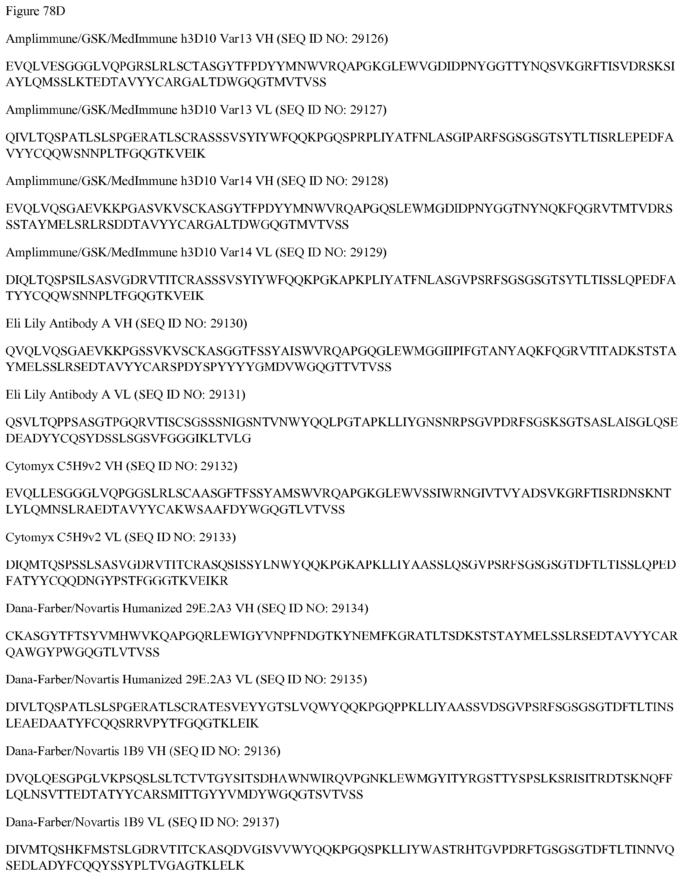

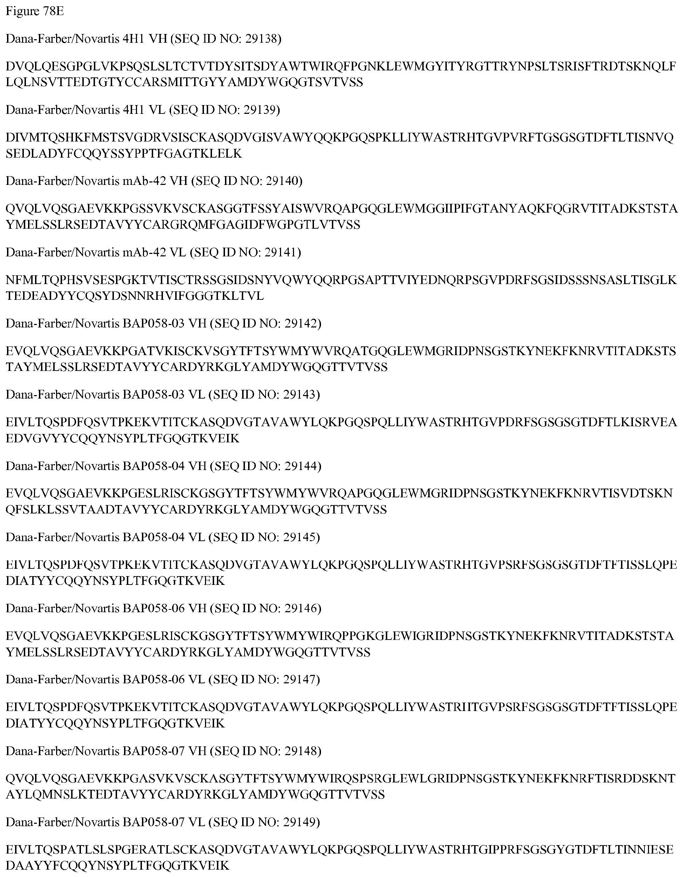

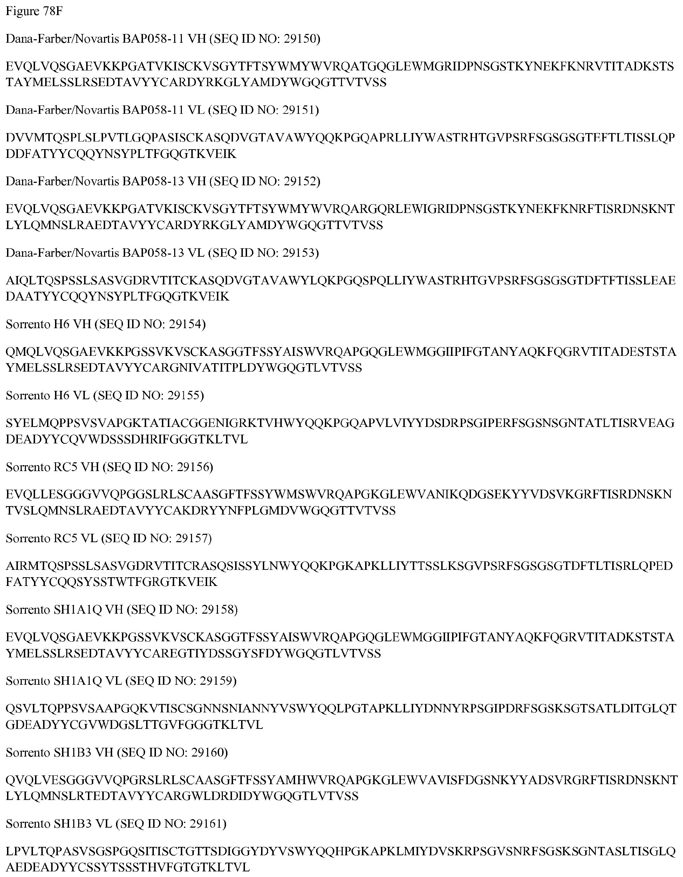

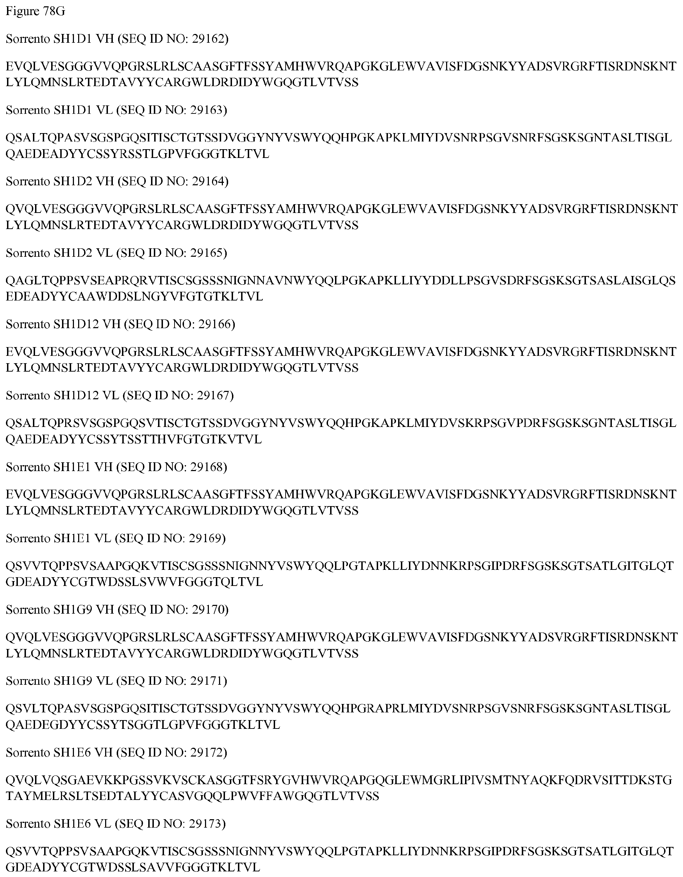

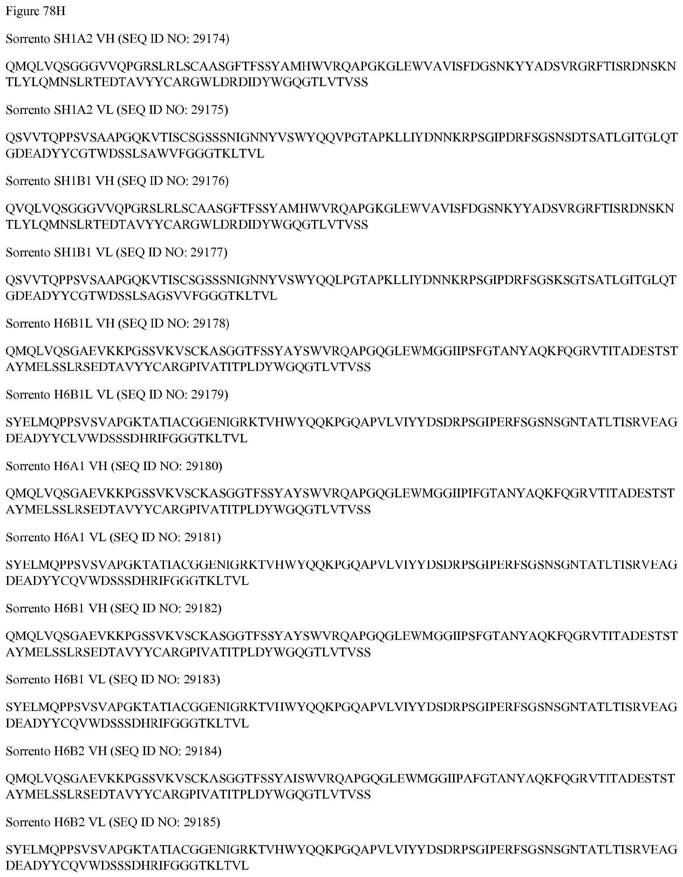

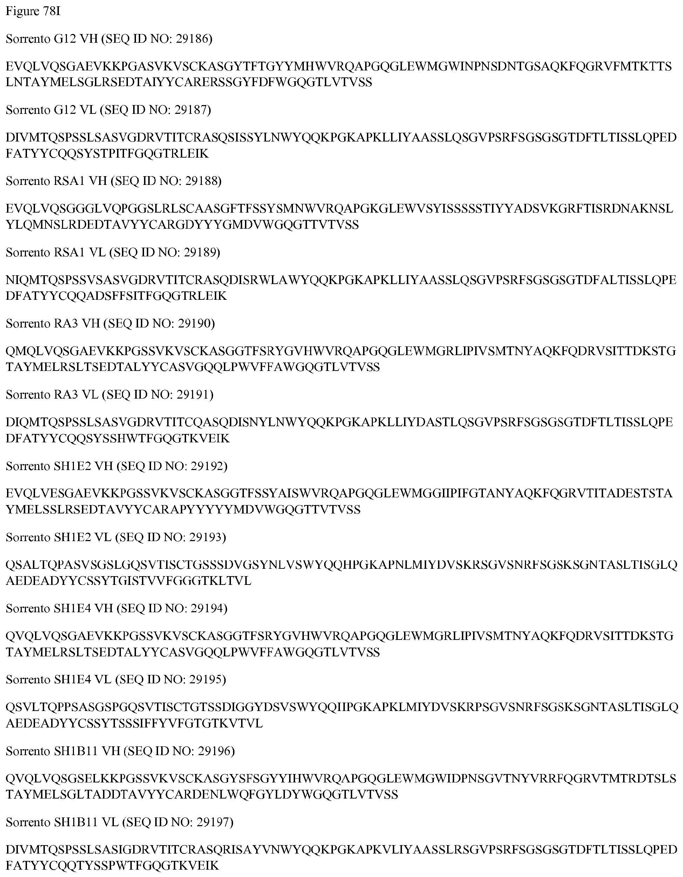

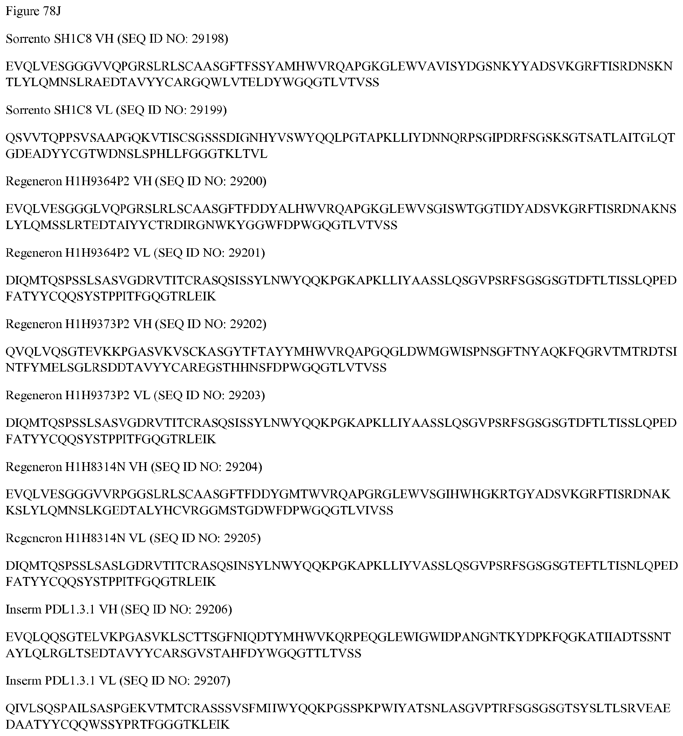

Figure 78A-78J depict a number of prior art sequences for Fvs that bind human PD-L1 as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

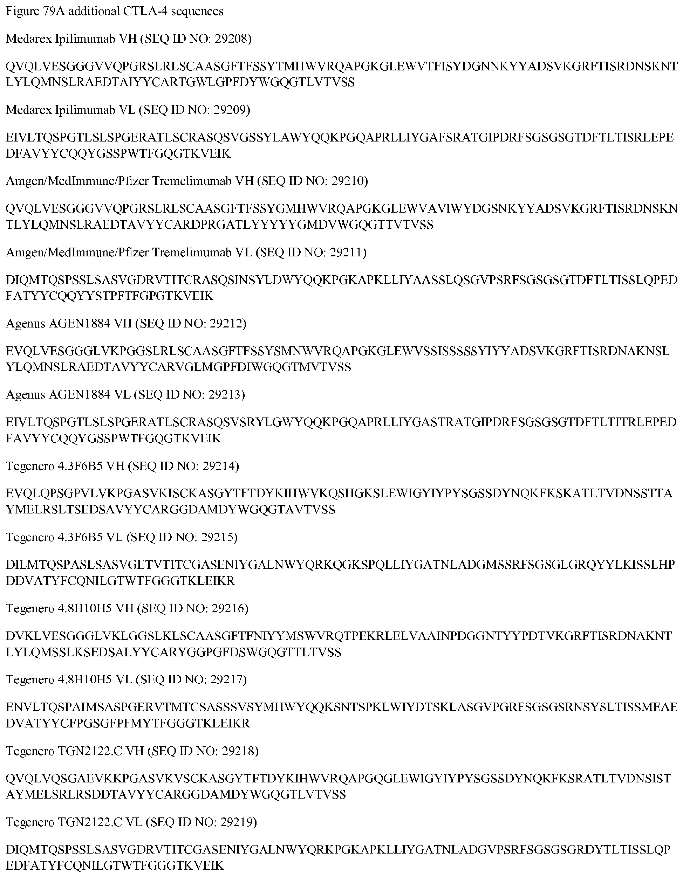

Figure 79A-79B depict a number of prior art sequences for Fvs that bind human CTLA-4 as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

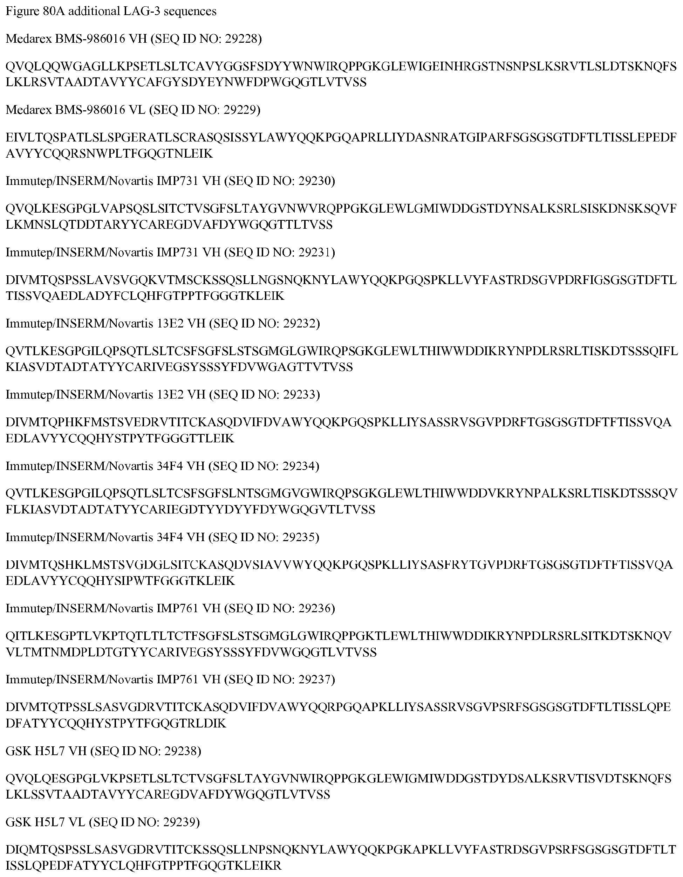

Figure 80A-80C depict a number of prior art sequences for Fvs that bind human LAG-3 as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

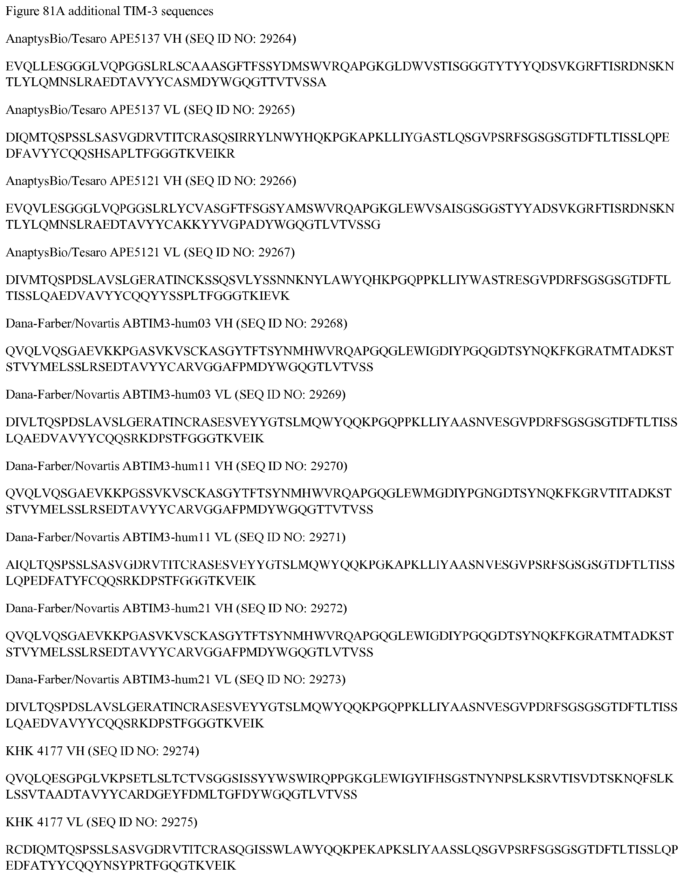

Figure 81A-81C depict a number of prior art sequences for Fvs that bind human TIM-3 as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

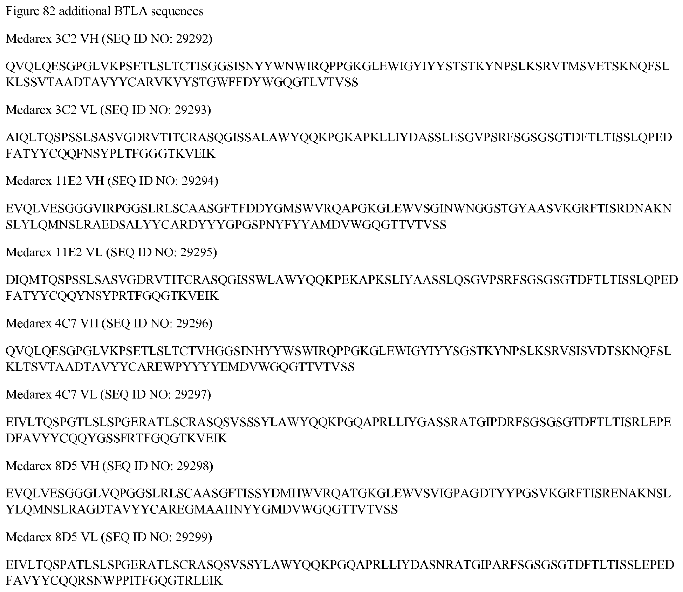

Figure 82 depict a number of prior art sequences for Fvs that bind human BTLA as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

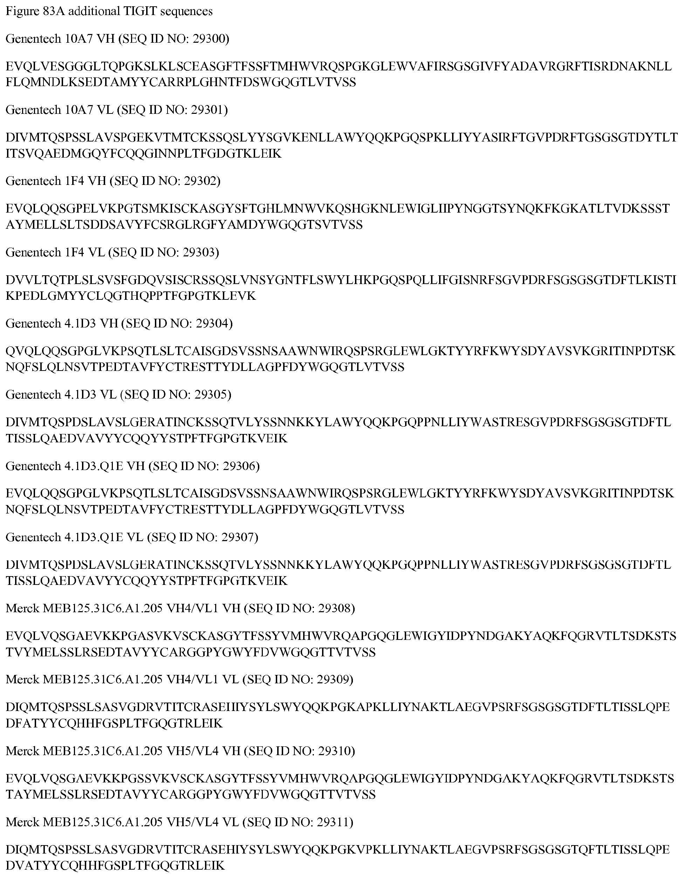

Figure 83A-83D depict a number of prior art sequences for Fvs that bind human TIGIT as vh and vl sequences. As will be appreciated by those in the art, any of these Fvs can be combined with an Fv that binds a costimulatory receptor (e.g. ICOS, GITR, OX40 or 4-1BB, including the Fv sequences contained herein) and in any format (bottle opener, mAb-Fv, mAb-scFv, central-scFv, bispecific mAb, central-Fv, one armed central-scFv, one armed scFv-mAb, dual scFv, DVD-Ig or Trident). In particular they can be combined with ICOS]_H0L0 and [ICOS]H0.66_L0 ABDs.

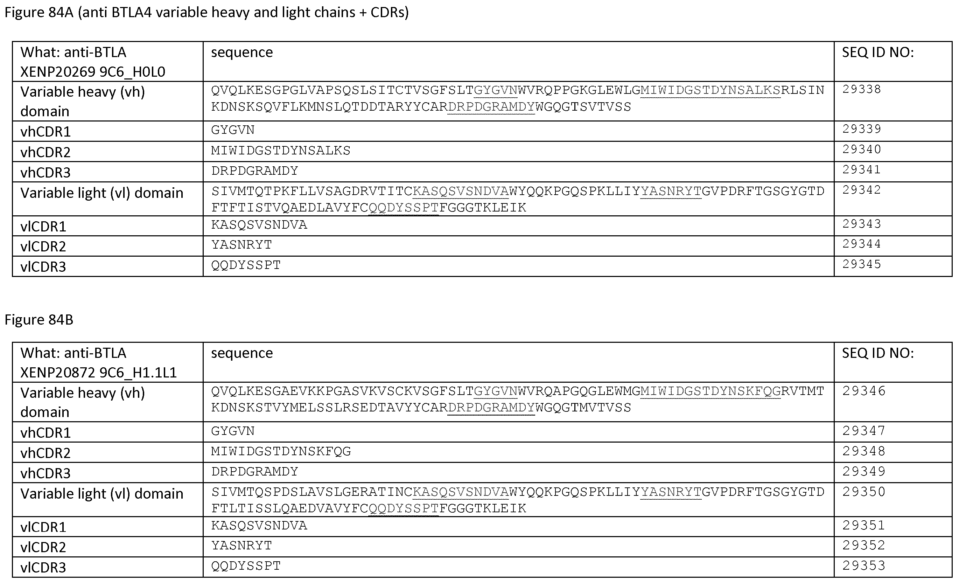

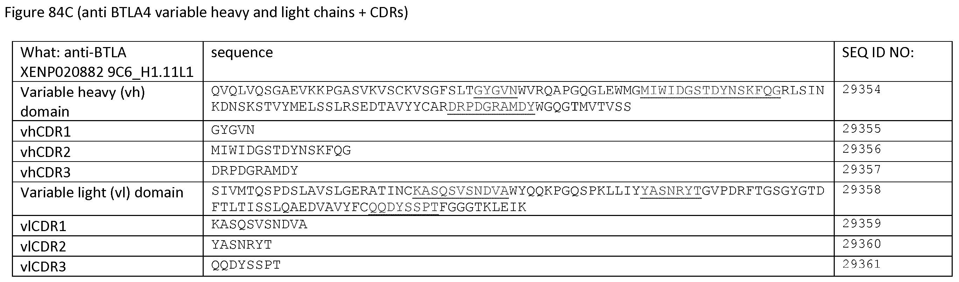

Figure 84A to 84C depict a number of BTLA ABDs, with additional anti-BTLA ABDs being listed as SEQ ID NO: 3705-3736. The CDRs are underlined, the scFv linker is double underlined (in the sequences, the scFv linker is a positively charged scFv (GKPGS)₄ linker (SEQ ID NO: 26209), although as will be appreciated by those in the art, this linker can be replaced by other linkers, including uncharged or negatively charged linkers, some of which are depicted in Figure 8A-8B), and the slashes indicate the border(s) of the variable domains. As above, the naming convention illustrates the orientation of the scFv from N- to C-terminus; in the sequences listed in this figure, they are all oriented as vh-scFv linker-vl (from N- to C-terminus), although these sequences may also be used in the opposite orientation, (from N- to C-terminus) vl-linker-vh. As noted herein and is true for every sequence herein containing CDRs, the exact identification of the CDR locations may be slightly different depending on the numbering used as is shown in Table 1, and thus included herein are not only the CDRs that are underlined but also CDRs included within the vh and vl domains using other numbering systems. Furthermore, as for all the sequences in the Figures, these vh and vl sequences can be used either in a scFv format or in a Fab format.

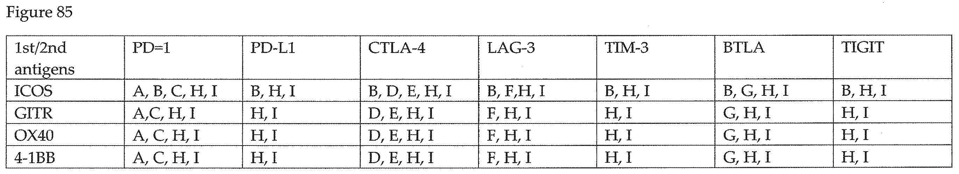

Figure 85 is a matrix of possible combinations of the costim and checkpoint ABDs, with all possible combinations possible. An "A" in the box means that the PD-1 ABD is 1G6_L1.194_H1.279. A "B" in the box means that the ICOS ABD is [ICOS]H.066_L0. A "C" in the box means that the PD-1 is the scFv in the pair. A "D" in the box means the CTLA-4 ABD is a Fab and is [CTLA-4]_H3_L0.22. An "E" in the box means that the CTLA-4 ABD is a scFv and is [CTLA-4]_H3.23_L0.22. An "F" in the box means that the LAG-3 ABD is 7G8_H3.30_L1.34. A "G" in the box means that the BTLA ABD is 9C6_H1.1_L1. An "H" in the box means that combination is a bottle opener. An "I" in the box means the combination is Central-scFv format.

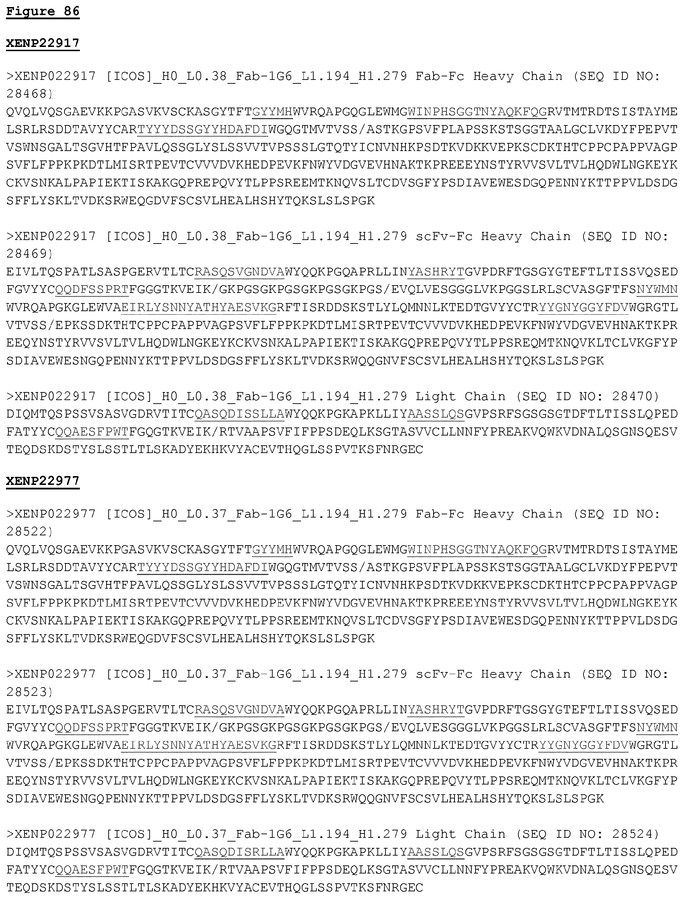

Figure 86 depicts two more ICOS X PD-1 bottle openers.

### DETAILED DESCRIPTION OF THE INVENTION

### I. Nomenclature

The bispecific antibodies disclosed herein are listed in several different formats. Each polypeptide is given a unique "XENP" number, although as will be appreciated in the art, a longer sequence might contain a shorter one. For example, the heavy chain of the scFv side monomer of a bottle opener format for a given sequence will have a first XENP number, while the scFv domain will have a different XENP number. Some molecules have three polypeptides, so the XENP number, with the components, is used as a name. Thus, the molecule XENP, which is in bottle opener format, comprises three sequences, generally referred to as "XENP23104-HC-Fab", XENP23104 HC-scFv" and "XENP23104 LC" or equivalents, although one of skill in the art would be able to identify these easily through sequence alignment. These XENP numbers are in the sequence listing as well as identifiers, and used in the Figures. In addition, one molecule, comprising the three components, gives rise to multiple sequence identifiers. For example, the listing of the Fab monomer has the full length sequence, the variable heavy sequence and the three CDRs of the variable heavy sequence; the light chain has a full length sequence, a variable light sequence and the three CDRs of the variable light sequence; and the scFv-Fc domain has a full length sequence, an scFv sequence, a variable light sequence, 3 light CDRs, a scFv linker, a variable heavy sequence and 3 heavy CDRs; note that all molecules herein with a scFv domain use a single charged scFv linker (+H), although others can be used. In addition, the naming nomenclature of particular variable domains uses a "Hx.xx_Ly.yy" type of format, with the numbers being unique identifiers to particular variable chain sequences. Thus, the variable domain of the scFv side of XENP23104 (which binds PD-1) is "1G6_ L1.194_H1.279", which indicates that the variable heavy domain H1.279 was combined with the light domain L1.194. In the case that these sequences are used as scFvs, the designation "1G6_ L1.194_H1.279", indicates that the variable heavy domain H1.279 was combined with the light domain L1.194 and is in vl-linker-vh orientation, from N- to C-terminus. This molecule with the identical sequences of the heavy and light variable domains but in the reverse order would be named "1G6_H1.279_L1.194". Similarly, different constructs may "mix and match" the heavy and light chains as will be evident from the sequence listing and the Figures.

### II. Materials

### A. Sequences

Target Antigens: The sequence of human PD-1 (sp | Q15116) is SEQ ID NO: 26226. The sequence of human PD-1, extracellular domain (sp | Q15116 | 21-170) is SEQ ID NO: 26227. The sequence of macaca fascicularis PD-1 (tr | B0LAJ3) is SEQ ID NO: 26228. The sequence of macaca fascicularis PD-1, extracellular domain (predicted) (tr | B0LAJ3|21-170) is SEQ ID NO: 26229. The sequence of human CTLA-4 (sp | P16410) is SEQ ID NO: 26230. The sequence of human CTLA-4, extracellular domain (sp | P16410 | 36-161) is SEQ ID NO: 26231. The sequence of macaca fascicularis CTLA-4 (tr | G7PL88) is SEQ ID NO: 26232. The sequence of macaca fascicularis CTLA-4, extracellular domain (predicted) (tr | G7PL88) is SEQ ID NO: 26233. The sequence of human LAG-3 (sp | P18627) is SEQ ID NO: 26234. The sequence of human LAG-3, extracellular domain (sp | P18627129-450) is SEQ ID NO: 26235. The sequence of macaca fascicularis LAG-3 (predicted) (gi |544467815 | ref | XP_005570011.1) is SEQ ID NO: 26236. The sequence of macaca fascicularis LAG-3, extracellular domain (predicted) (gi | 544467815 | ref | XP_005570011.1|29-450) is SEQ ID NO: 26237. The sequence of human TIM-3 (sp | Q8TDQ0) is SEQ ID NO: 26238. The sequence of human TIM-3, extracellular domain (sp | Q8TDQ0 | 22-202) is SEQ ID NO: 26239. The sequence of macaca fascicularis TIM-3 (predicted) (gi |355750365| gb | EHH54703.1) is SEQ ID NO: 26240. The sequence of macaca fascicularis TIM-3, extracellular domain (predicted) (gi | 355750365 | gb | EHH54703.1122-203) is SEQ ID NO: 26241. The sequence of human PD-L1 (sp | Q9NZQ7) is SEQ ID NO: 26242. The sequence of human PD-L1, extracellular domain (sp | Q9NZQ7 | 19-238) is SEQ ID NO: 26243. The sequence of macaca fascicularis PD-L1 (predicted) (gb | XP_005581836.1) is SEQ ID NO: 26244. The sequence of macaca fascicularis PD-L1, extracellular domain (predicted) (gb | XP_005581836.1119-238) is SEQ ID NO: 26245. The sequence of human ICOS (sp | Q9Y6W8) is SEQ ID NO: 26246. The sequence of human ICOS, extracellular domain (sp | Q9Y6W8 | 21-140) is SEQ ID NO: 26247. The sequence of macaca fascicularis ICOS (gi | 544477053 | ref | XP_005574075.1) is SEQ ID NO: 26248. The sequence of macaca fascicularis ICOS, extracellular domain (predicted) (gi | 544477053 | ref| XP_005574075.1121-140) is SEQ ID NO: 26249. The sequence of human GITR (sp | Q9Y5U5) is SEQ ID NO: 26250. The sequence of human GITR, extracellular domain (sp |Q9Y5U5|26-162) is SEQ ID NO: 26251. The sequence of macaca fascicularis GITR (predicted) (ref | XP_005545180.1) is SEQ ID NO: 26252. The sequence of macaca fascicularis GITR, extracellular domain (predicted) (ref | XP_005545180.1126-162) is SEQ ID NO: 26253. The sequence of human OX40 (sp | P43489) is SEQ ID NO: 26254. The sequence of human OX40, extracellular domain (sp | P43489 129-214) is SEQ ID NO: 26255. The sequence of macaca fascicularis OX40 (predicted) (ref | XP_005545179.1) is SEQ ID NO: 26256. The sequence of macaca fascicularis OX40, extracellular domain (predicted) (ref | XP_005545179.1129-214) is SEQ ID NO: 26257. The sequence of human 4-1BB (sp | Q07011) is SEQ ID NO: 26258. The sequence of human 4-1BB, extracellular domain (sp| Q07011 124-186) is SEQ ID NO: 26259. The sequence of macaca fascicularis 4-1BB (predicted) (ref | XP_005544945.1) is SEQ ID NO: 26260. The sequence of macaca fascicularis 4-1BB, extracellular domain (predicted) (ref | XP_005544945.1|24-186 ) is SEQ ID NO: 26261.

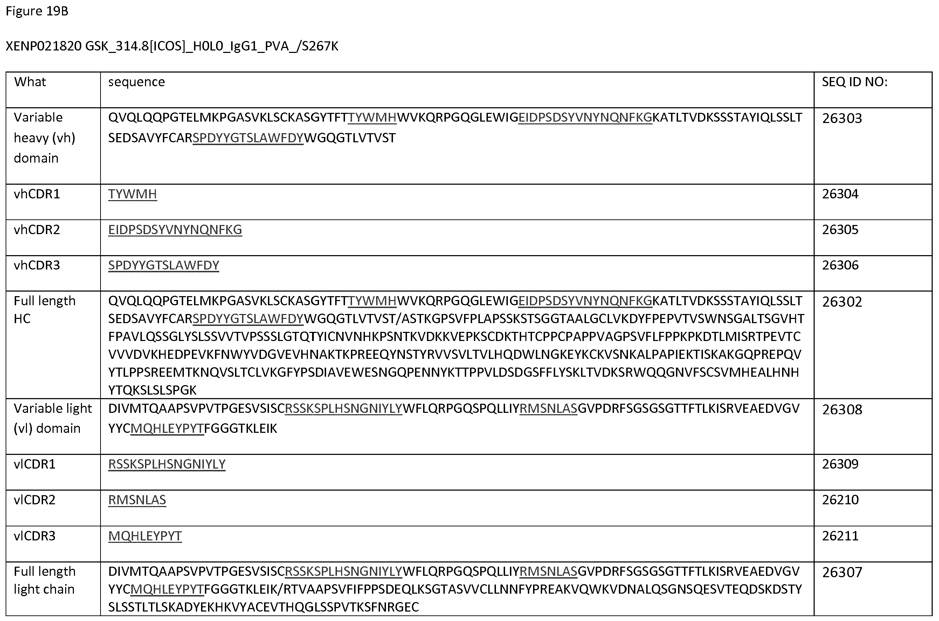

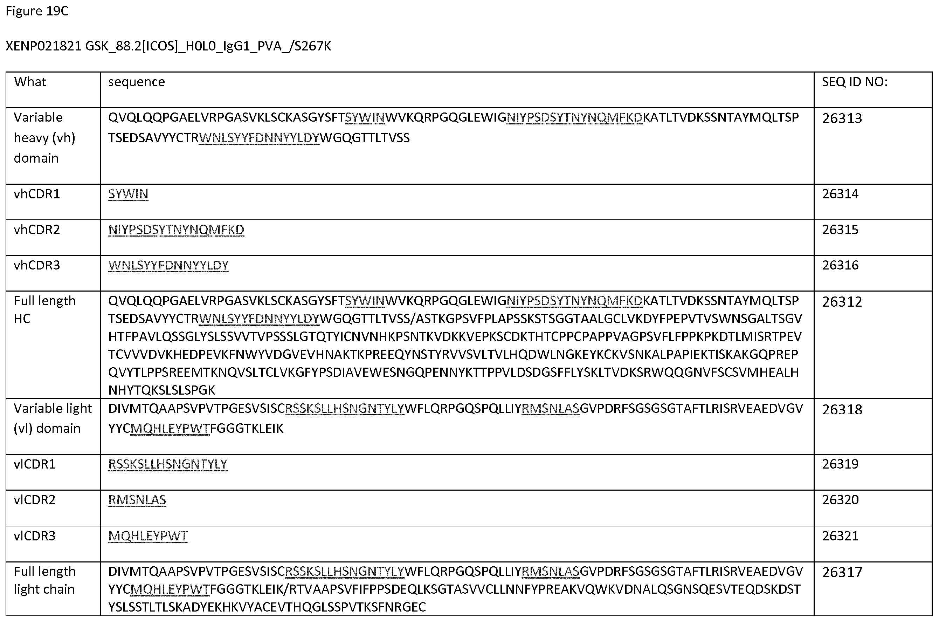

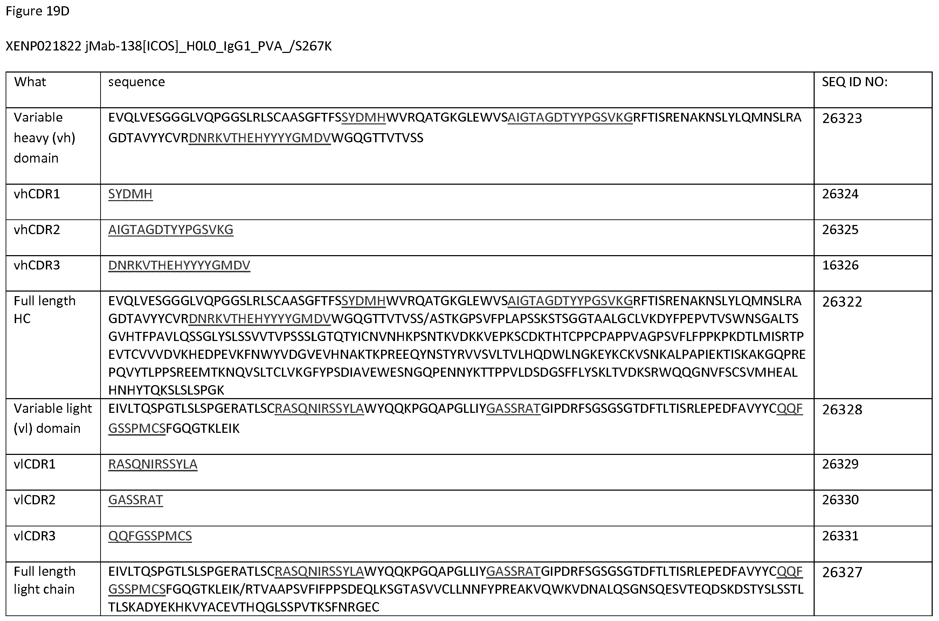

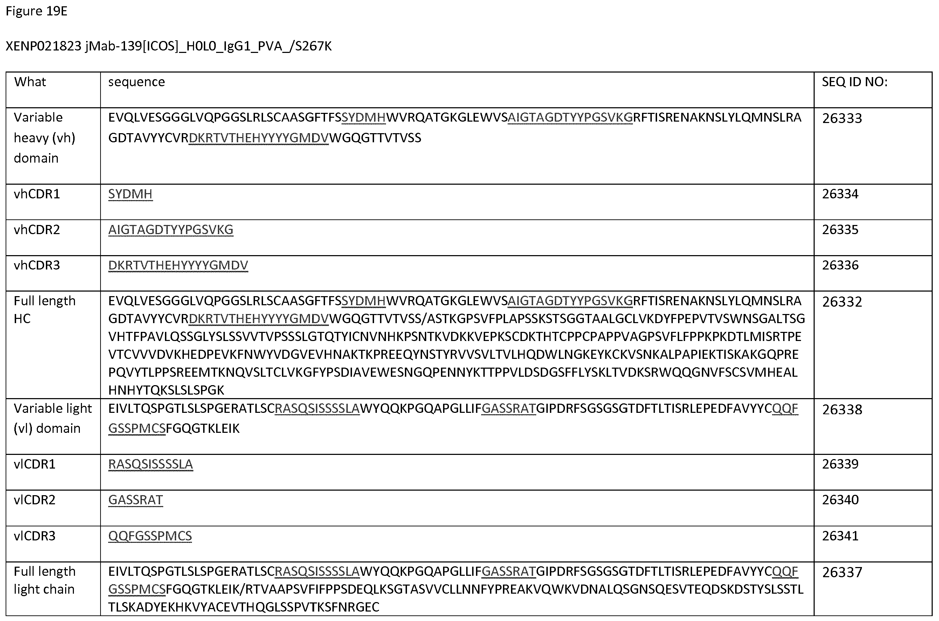

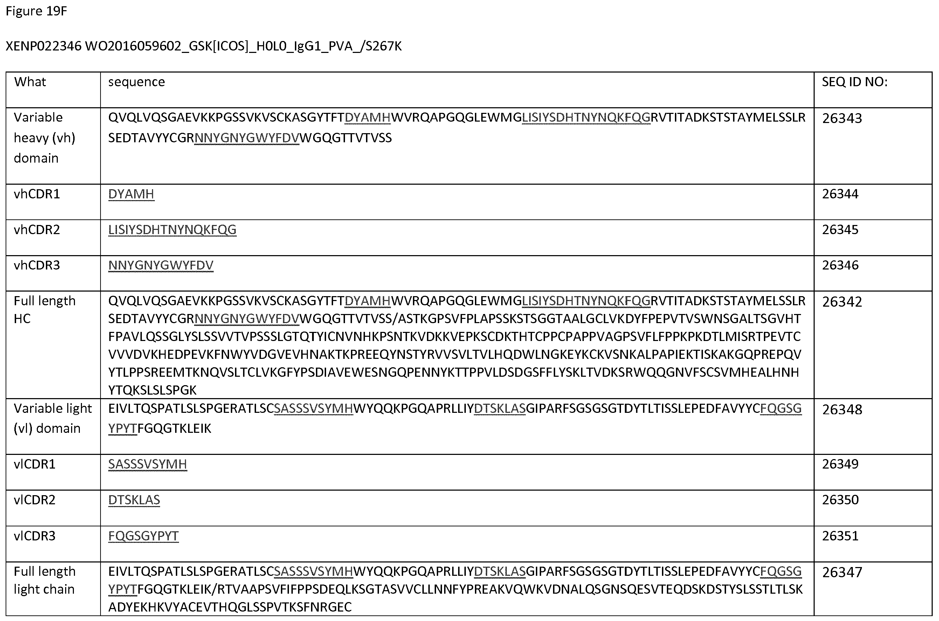

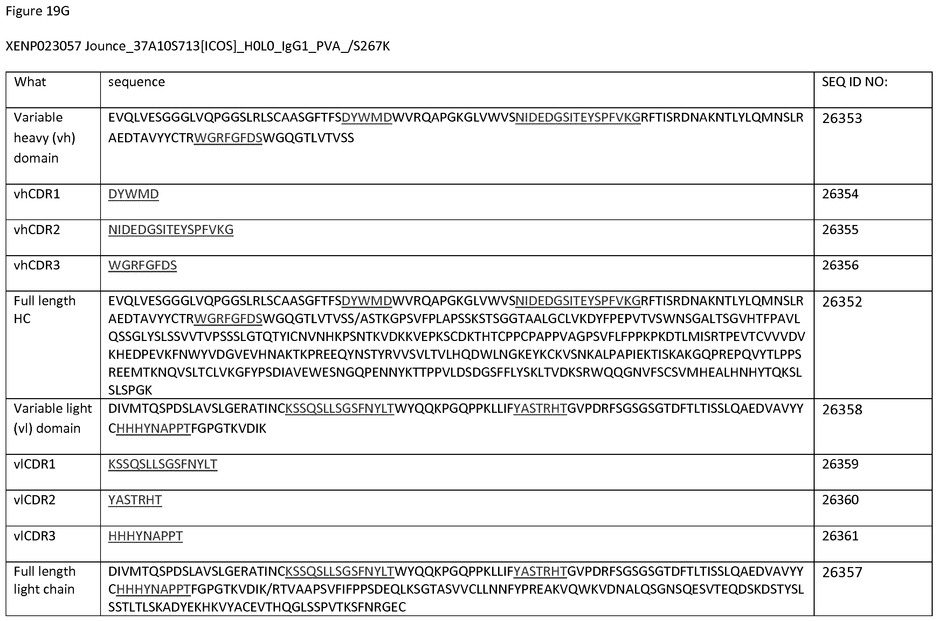

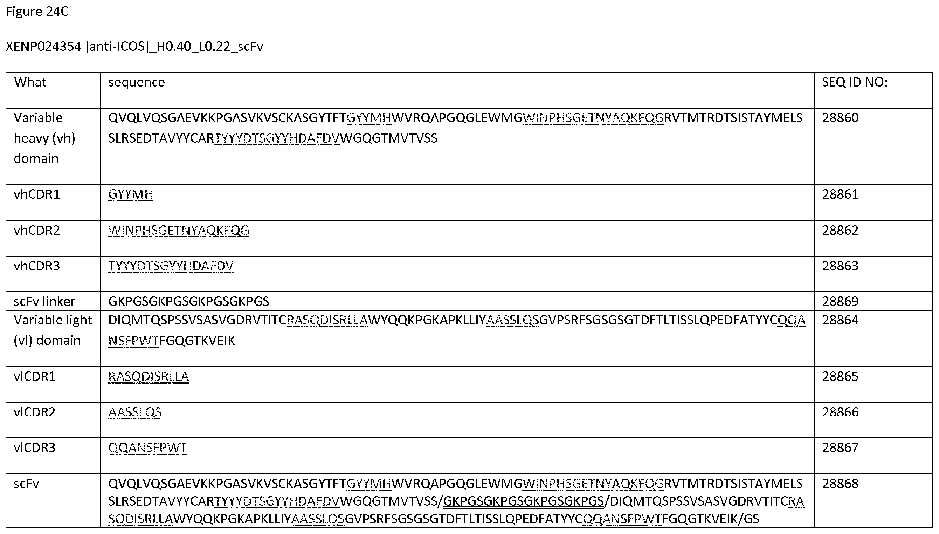

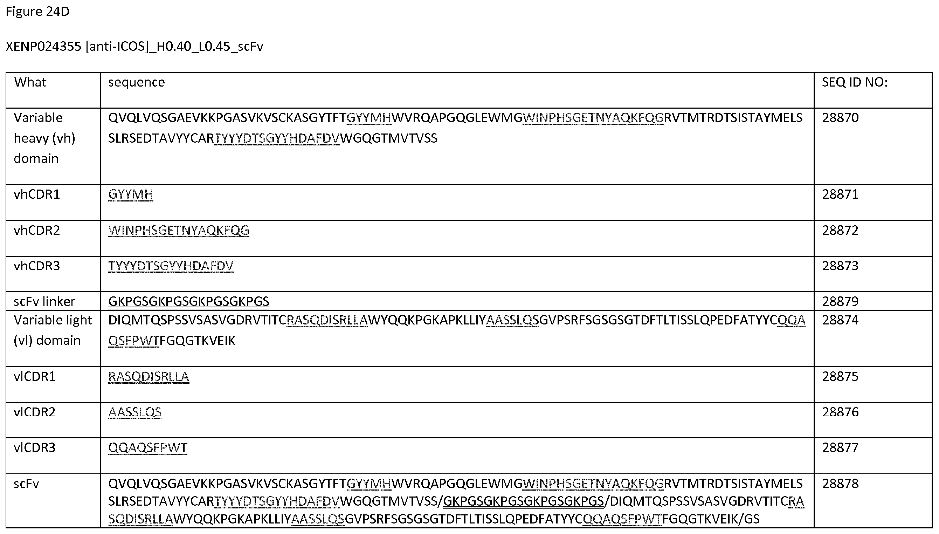

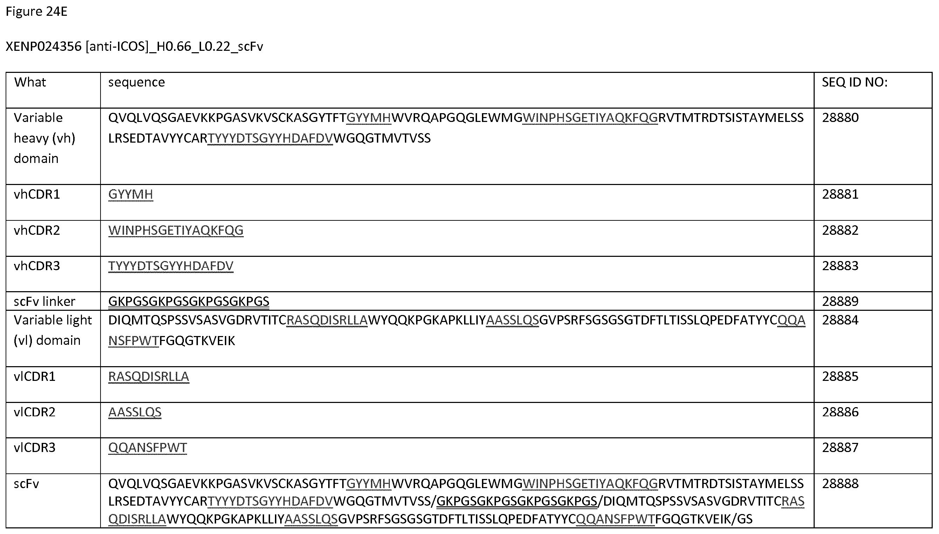

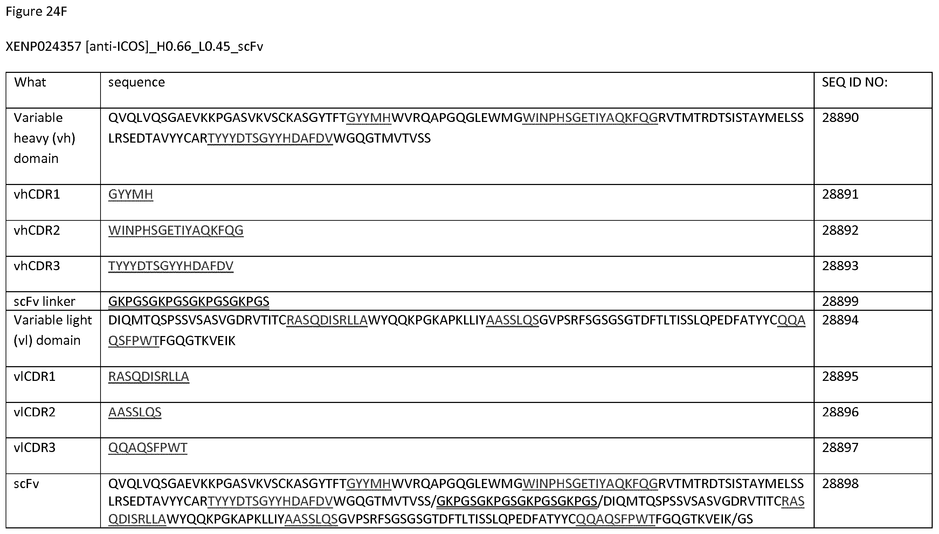

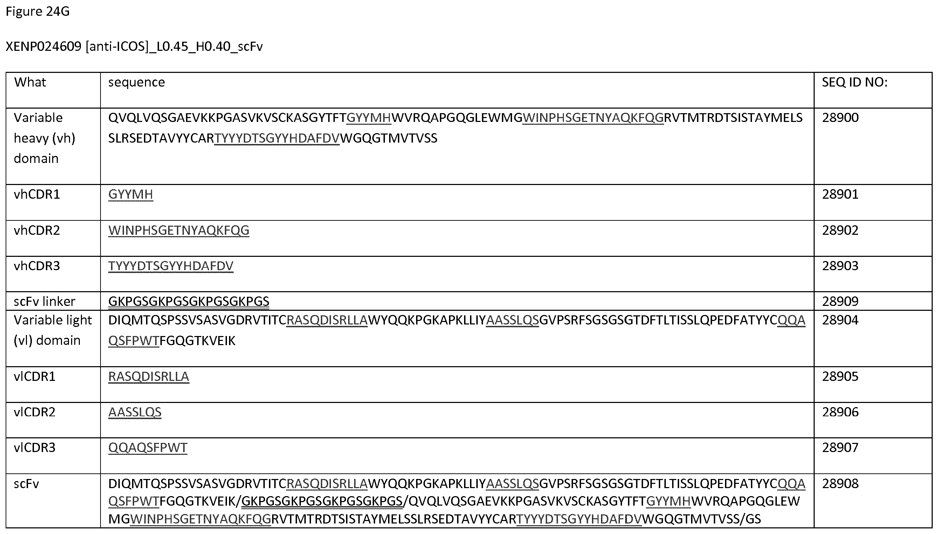

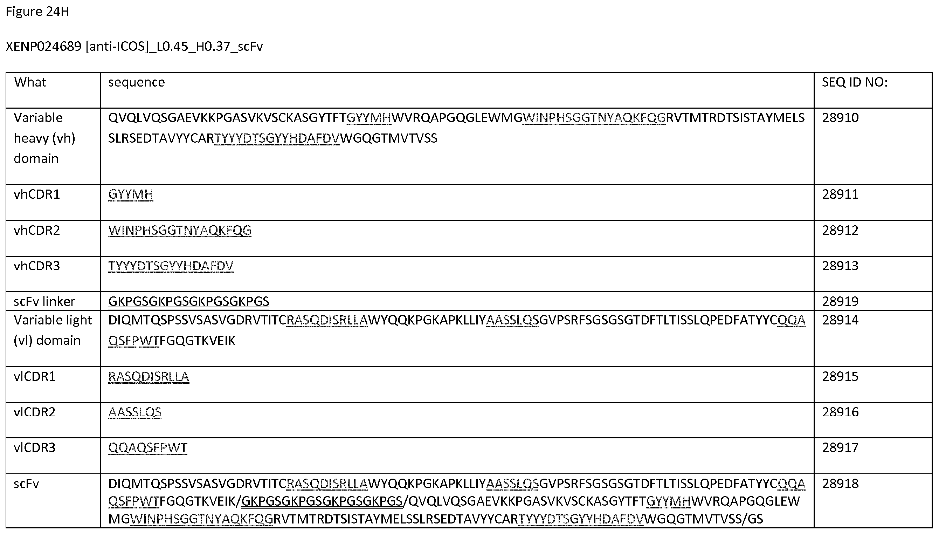

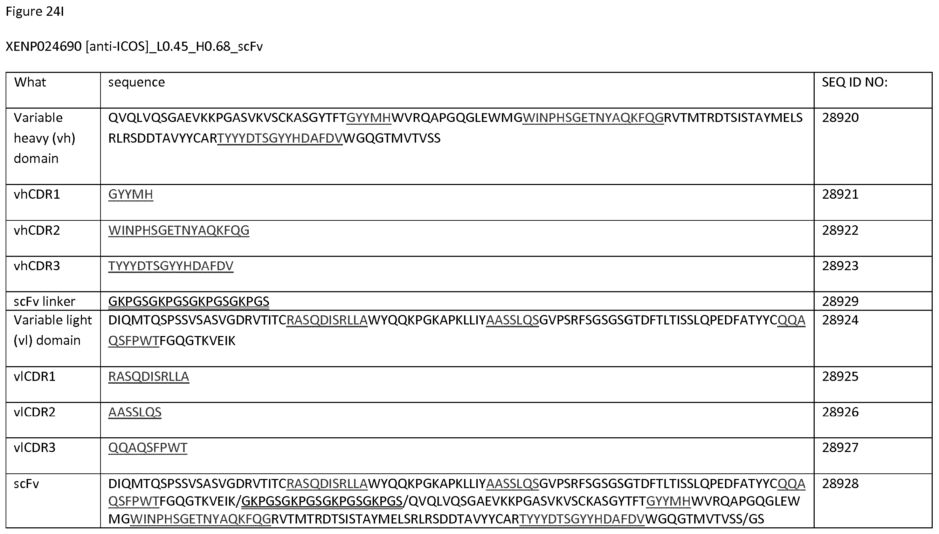

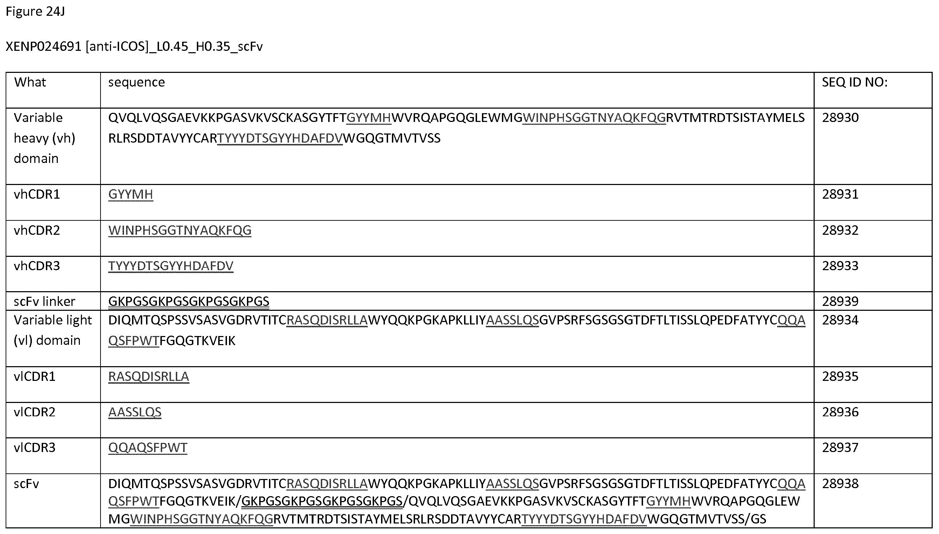

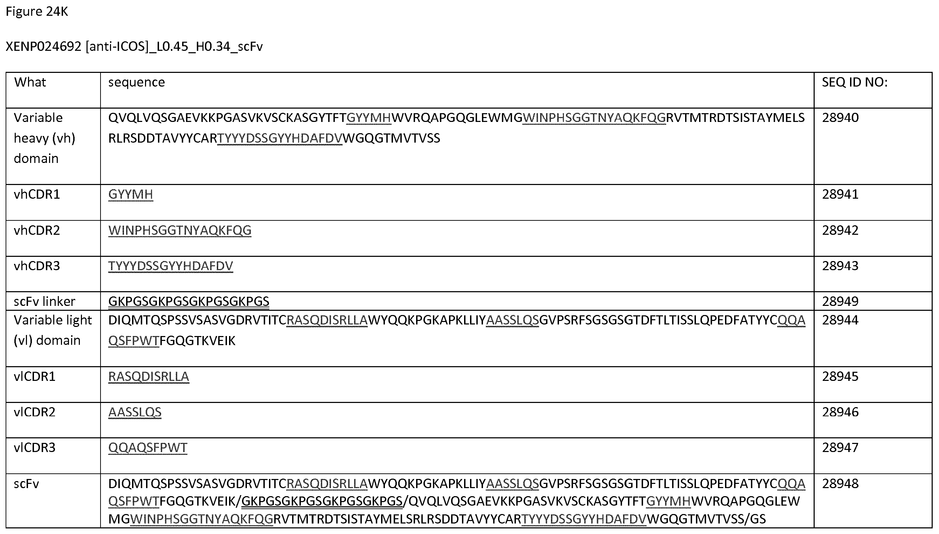

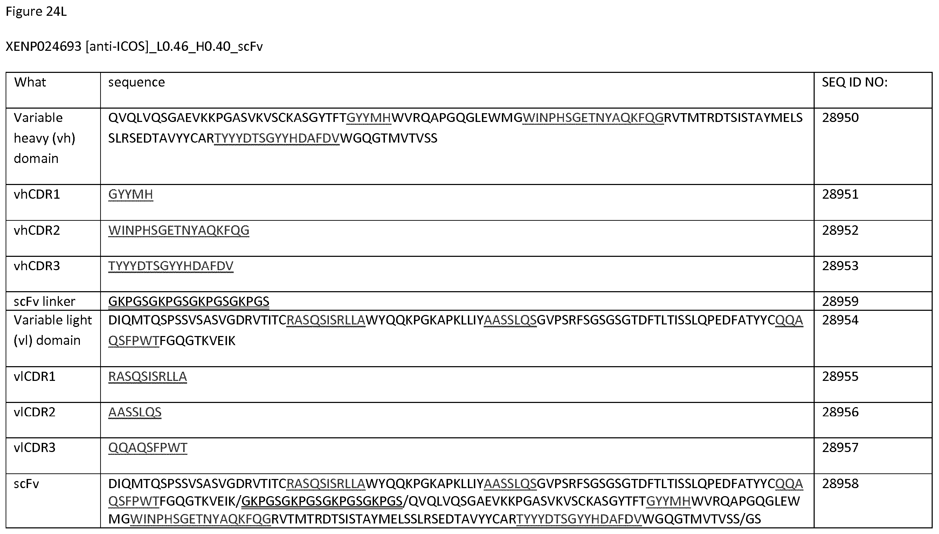

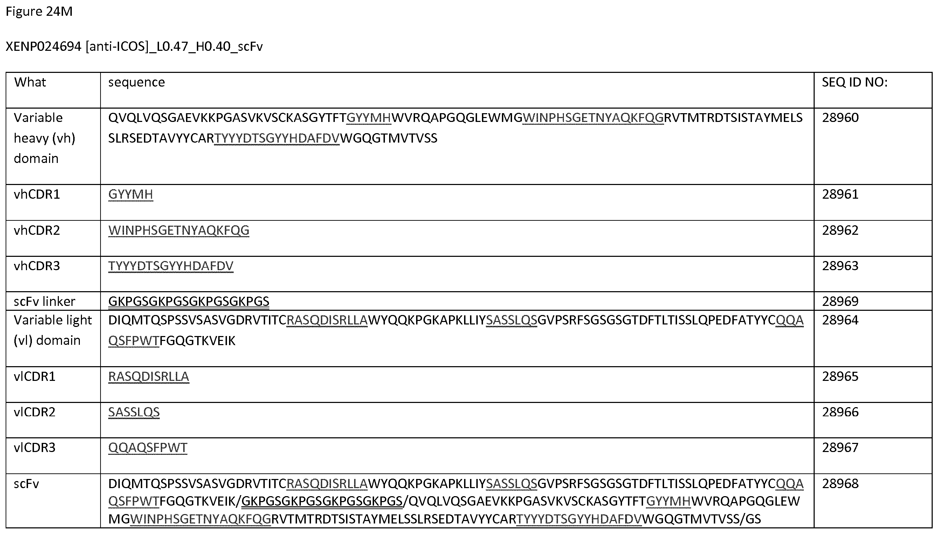

ICOS binding domains: In addition the sequences shown in Figure 19, Figure 20A20G and Figure 24, SEQ ID NO:27869-28086 contain a number of ICOS Fab sequences (heavy chain VH1-CH1 and light chain VL1-CL) as indicated in the naming nomenclature. From the SEQ listing one of skill in the art will be able to ascertain the CDRs (see Table 1 for numbering and/or through sequence alignment) as well as for the junctions (e.g. heavy chain CH1 generally starts with the sequence "ASTK ..." and light chain constant domain generally starts with "RTVA ...". SEQ ID NO:28087-28269 show the three sequences for "one armed mAb" (Figure 2N; Fab-Fc, Fc only and light chain) as shown in the naming nomenclature. From the SEQ listing one of skill in the art will be able to ascertain the CDRs (see Table 1 for numbering and/or through sequence alignment) as well as for the junctions (e.g. heavy chain CH1 generally starts with the sequence "ASTK ..." and light chain constant domain generally starts with "RTVA ...". Additional one armed ICOS molecules are shown in Figure 68A-68G. SEQ ID NO:28549-28556 show some control antibodies (HC and LC) from which the Fvs can be used as ICOS ABDs as well; from the SEQ listing one of skill in the art will be able to ascertain the CDRs (see Table 1 for numbering and/or through sequence alignment) as well as for the junctions (e.g. heavy chain CH1 generally starts with the sequence "ASTK ..." and light chain constant domain generally starts with "RTVA ...". SEQ ID NO:28557-28665 show some ICOS scFvs that find use in combination in the disclosure; from the SEQ listing one of skill in the art will be able to ascertain the CDRs (see Table 1 for numbering and/or through sequence alignment) as well as for the junctions, as the scFvs utilize the charged linker (GKPGS)₄ between the vh and vl domains. Thus, suitable ICOS ABDs for use in combination with ABDs for checkpoint receptors are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and [ICOS]_H0.66_L0 and [ICOS]_H0_L0.

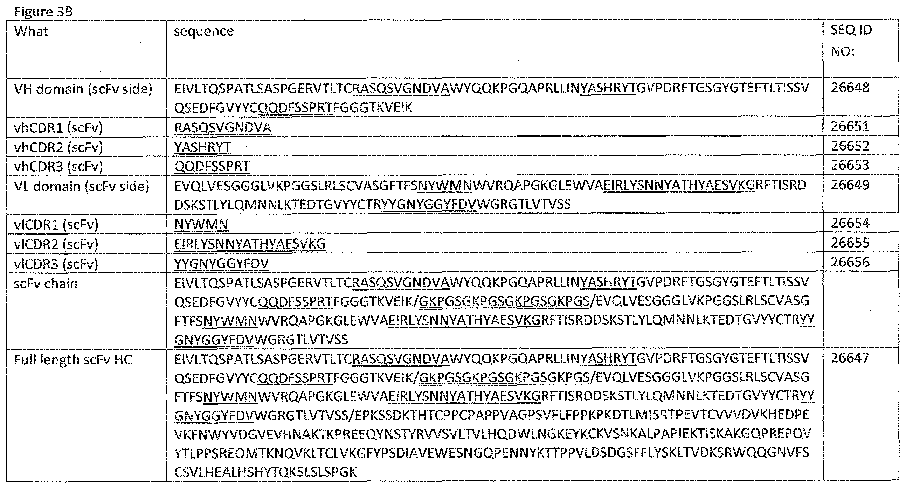

In addition, suitable ICOS X PD-1 bottle opener sequences include those in Figure 3 as well as those in SEQ ID NO: 28270-28548.

### III. Overview

Therapeutic antibodies directed against immune immunomodulatory inhibitors such as PD-1 are showing great promise in limited circumstances in the clinic for the treatment of cancer. Cancer can be considered as an inability of the patient to recognize and eliminate cancerous cells. In many instances, these transformed (e.g. cancerous) cells counteract immunosurveillance. There are natural control mechanisms that limit T-cell activation in the body to prevent unrestrained T-cell activity, which can be exploited by cancerous cells to evade or suppress the immune response. Restoring the capacity of immune effector cells-especially T cells-to recognize and eliminate cancer is the goal of immunotherapy. The field of immuno-oncology, sometimes referred to as "immunotherapy" is rapidly evolving, with several recent approvals of T cell checkpoint inhibitory antibodies such as YERVOY, KEYTRUDA and OPDIVO. These antibodies are generally referred to as "checkpoint inhibitors" because they block normally negative regulators of T cell immunity. It is generally understood that a variety of immunomodulatory signals, both costimulatory and coinhibitory, can be used to orchestrate an optimal antigen-specific immune response.

Checkpoint inhibitor monoclonal antibodies bind to immunomodulatory inhibitor proteins such as PD-1, which under normal circumstances prevent or suppress activation of cytotoxic T cells (CTLs). By inhibiting the immunomodulatory protein, for example through the use of antibodies that bind these proteins, an increased T cell response against tumors can be achieved. That is, these cancer immunomodulatory proteins suppress the immune response; when the proteins are blocked, for example using antibodies to the immunomodulatory protein, the immune system is activated, leading to immune stimulation, resulting in treatment of conditions such as cancer and infectious disease. Antibodies to either the PD-1 protein or its binding partner, PD-L1, leads to T cell activation.

Another area of current interest for harnessing the patient's immune system to fight disease involves the co-stimulation of T cells using agonistic antibodies that bind to costimulatory proteins such as ICOS (Inducible T cell Co-Stimulator, also referred to as CD278) which adds a positive signal to overcome the negative signaling of the immune checkpoint proteins on the T-cells. ICOS is a type I transmembrane protein comprising an extracellular (Ig) V-like domain, and serves as the receptor for the B7h co-stimulatory molecule.

Recent work shows that some tumor infiltrating lymphocytes (TILs) co-express PD-1 and ICOS (see Gros, J. Clinical Invest. 124(5):2246 (2014)).

Bispecific antibodies, which can bind two different targets simultaneously, offer the potential to improve the selectivity of targeting TILs vs peripheral T cells, while also reducing cost of therapy. The bivalent interaction of an antibody with two targets on a cell surface should - in some cases - lead to a higher binding avidity relative to a monovalent interaction with one target at a time. Because of this, normal bivalent antibodies tend to have high avidity for their target on a cell surface. With bispecific antibodies, the potential exists to create higher selectivity for cells that simultaneously express two different targets, utilizing the higher avidity afforded by simultaneous binding to both targets.

Accordingly, disclosed herein are bispecific immunomodulatory antibodies, that bind to cells expressing the two antigens and methods of activating T cells and/or NK cells to treat diseases such as cancer and infectious diseases, and other conditions where increased immune activity results in treatment.

Thus, the invention is directed, in some instances, to solving the issue of toxicity and expense of administering multiple antibodies by providing bispecific antibodies that bind to two different immunomodulatory molecules (one a checkpoint receptor and the other a costimulatory receptor) on a single cell and advantageously requiring administration of only one therapeutic substance.

Bispecific antibodies offer the opportunity to combine immune immunomodulatory blockade with costimulation in one molecule. However, it is not obvious what combination of immune immunomodulatory plus costimulatory protein or what binding stoichiometry (monovalent + monovalent, monovalent + bivalent, etc.) would be efficacious. Here we identify bispecific antibodies that binding monovalently to a costimulatory protein (such as ICOS) and monovalent binding to a checkpoint receptor (such as PD-1) that are capable of inducing robust T cell activation.

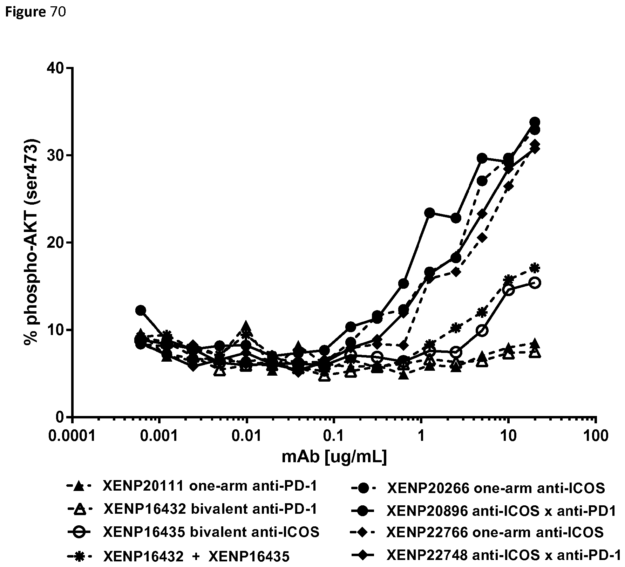

Surprisingly, while conventional wisdom states that monovalent antibodies do not result in agonism, the present work shows the unexpected results of agonism of the ICOS receptor with the monovalent bispecific antibodies of the invention. See Merchant et al., PNAS July 23 2013 E2987-E2996, "[w]hile initial screening of bivalent antibodies produced agonists of MET, engineering them into monovalent antibodies produces antagonists instead." This positive result with only monovalent binding to ICOS is unexpected because it is thought that at least bivalent binding to a costimulatory protein is necessary to provide the required level of receptor clustering on the cell surface for triggering signaling. As shown by Fos et al., J. Immunol. 2008:1969-1977, ICOS ligation induces AKT phosphorylation. The studies here in use AKT phosphorylation as an indicator of ICOS agonism, and this effect is seen for both "one armed ICOS" (see Example 5A(a)) and for bispecific antibodies that bind ICOS monovalently. As shown in Example 7 and in Figure 70, the one-arm XENP20266 that only binds ICOS monovalently promotes more AKT phosphorylation than XENP16435, which binds ICOS bivalently (e.g. as a traditional mAb).

Accordingly, the present invention is directed to novel constructs to provide heterodimeric, bispecific antibodies that allow binding to a checkpoint receptor as well as human ICOS.

Note that generally these bispecific antibodies are named "anti-PD-1 X anti-ICOS", or generally simplistically or for ease (and thus interchangeably) as "PD-1 X ICOS", etc. for each pair.

The heterodimeric bispecific immunomodulatory antibodies of the invention are useful to treat a variety of types of cancers. As will be appreciated by those in the art, in contrast to traditional monoclonal antibodies that bind to tumor antigens, or to the newer classes of bispecific antibodies that bind, for example, CD3 and tumor antigens, immunomodulatory antibodies are used to increase the immune response but are not generally tumor specific in their action. That is, the bispecific immunomodulatory antibodies of the invention inhibit the suppression of the immune system, generally leading to T cell activation, which in turn leads to greater immune response to cancerous cells and thus treatment.

As discussed below, there are a variety of ways that T cell activation can be measured. Functional effects of the bispecific immunomodulatory antibodies on NK and T-cells can be assessed in vitro (and in some cases in vivo, as described more fully below) by measuring changes in the following parameters: proliferation, cytokine release and cell-surface makers. For NK cells, increases in cell proliferation, cytotoxicity (ability to kill target cells as measured by increases in CD107a, granzyme, and perforin expression, or by directly measuring target cells killing), cytokine production (e.g. IFN-γ and TNF), and cell surface receptor expression (e.g. CD25) is indicative of immune modulation, e.g. enhanced killing of cancer cells. For T-cells, increases in proliferation, increases in expression of cell surface markers of activation (e.g. CD25, CD69, CD137, and PD1), cytotoxicity (ability to kill target cells), and cytokine production (e.g. IL-2, IL-4, IL-6, IFN, TNF-a, IL-10, IL-17A) are indicative of immune modulation, e.g. enhanced killing of cancer cells. Accordingly, assessment of treatment can be done using assays that evaluate one or more of the following: (i) increases in immune response, (ii) increases in activation of αβ and/or γδ T cells, (iii) increases in cytotoxic T cell activity, (iv) increases in NK and/or NKT cell activity, (v) alleviation of αβ and/or γδ T-cell suppression, (vi) increases in pro-inflammatory cytokine secretion, (vii) increases in IL-2 secretion; (viii) increases in interferon-γ production, (ix) increases in Th1 response, (x) decreases in Th2 response, (xi) decreases or eliminates cell number and/or activity of at least one of regulatory T cells, (xii) increases in IL-2 secretion.

Thus, provided herein are the use of bispecific immunomodulatory antibodies to perform one or more of the following in a subject in need thereof: (a) upregulating pro-inflammatory cytokines; (b) increasing T-cell proliferation and/or expansion; (c) increasing interferon-γ or TNF-α production by T-cells; (d) increasing IL-2 secretion; (e) stimulating antibody responses; (f) inhibiting cancer cell growth; (g) promoting antigenic specific T cell immunity; (h) promoting CD4+ and/or CD8+ T cell activation; (i) alleviating T-cell suppression; (j) promoting NK cell activity; (k) promoting apoptosis or lysis of cancer cells; and/or (l) cytotoxic or cytostatic effect on cancer cells.

Accordingly, the present disclosure provides bispecific immunomodulatory antibodies. There are a number of formats that can be used in the present disclosure, as generally shown in Figure 2, many of which are heterodimeric (although not all, as DVD-Ig, for example).

The heterodimeric antibodies constructs are based on the self-assembling nature of the two Fc domains of the heavy chains of antibodies, e.g. two "monomers" that assemble into a "dimer". Heterodimeric antibodies are made by altering the amino acid sequence of each monomer as more fully discussed below. Thus, the present invention is directed to the creation of heterodimeric antibodies, which can co-engage two antigens in several ways, relying on amino acid variants in the constant regions that are different on each chain to promote heterodimeric formation and/or allow for ease of purification of heterodimers over the homodimers.

Thus, the present invention provides bispecific immunomodulatory antibodies. An ongoing problem in antibody technologies is the desire for "bispecific" antibodies that bind to two (or more) different antigens simultaneously, in general thus allowing the different antigens to be brought into proximity and resulting in new functionalities and new therapies. In general, these antibodies are made by including genes for each heavy and light chain into the host cells (i.e., in the present invention, genes for two heavy chain monomers and a light chain as defined in the claims). This generally results in the formation of the desired heterodimer (A-B), as well as the two homodimers (A-A and B-B). However, a major obstacle in the formation of bispecific antibodies is the difficulty in purifying the heterodimeric antibodies away from the homodimeric antibodies and/or biasing the formation of the heterodimer over the formation of the homodimers.

To solve this issue, there are a number of mechanisms that can be used to generate the heterodimers of the present invention. In addition, as will be appreciated by those in the art, these mechanisms can be combined to ensure high heterodimerization. Thus, amino acid variants that lead to the production of heterodimeric antibodies are referred to as "heterodimerization variants". As discussed below, heterodimerization variants can include steric variants (e.g. the "knobs and holes" or "skew" variants described below and the "charge pairs" variants described below) as well as "pI variants", which allows purification of homodimers away from heterodimers.

One mechanism is generally referred to in the art as "knobs and holes" ("KIH") or sometimes herein as "skew" variants, referring to amino acid engineering that creates steric influences to favor heterodimeric formation and disfavor homodimeric formation can also optionally be used; this is sometimes referred to as "knobs and holes"; as described in Ridgway et al., Protein Engineering 9(7):617 (1996); Atwell et al., J. Mol. Biol. 1997 270:26; US Patent No. 8,216,805, US 2012/0149876. The Figures identify a number of "monomer A - monomer B" pairs that include "knobs and holes" amino acid substitutions. In addition, as described in Merchant et al., Nature Biotech. 16:677 (1998), these "knobs and hole" mutations can be combined with disulfide bonds to skew formation to heterodimerization. Of use in the present disclosure are T366S/L368A/Y407V paired with T366W, as well as this variant with a bridging disulfide, T366S/L368A/Y407V/Y349C paired with T366W/S354C, particularly in combination with other heterodimerization variants including pI variants as outlined below.

An additional mechanism that finds use in the generation of heterodimeric antibodies is sometimes referred to as "electrostatic steering" or "charge pairs" as described in Gunasekaran et al., J. Biol. Chem. 285(25):19637 (2010). This is sometimes referred to herein as "charge pairs". In this example, electrostatics are used to skew the formation towards heterodimerization. As those in the art will appreciate, these may also have an effect on pI, and thus on purification, and thus could in some cases also be considered pI variants. However, as these were generated to force heterodimerization and were not used as purification tools, they are classified as "steric variants". These include, but are not limited to, D221E/P228E/L368E paired with D221R/P228R/K409R (e.g. these are "monomer corresponding sets) and C220E/P228E/368E paired with C220R/E224R/P228R/K409R and others shown in the Figures.

pI variants may be used to alter the pI of one or both of the monomers and thus allowing the isoelectric purification of A-A, A-B and B-B dimeric proteins.

Disclosed herein are several basic mechanisms that can lead to ease of purifying heterodimeric proteins; one relies on the use of pI variants, such that each monomer has a different pI, thus allowing the isoelectric purification of A-A, A-B and B-B dimeric proteins. Alternatively, some scaffold formats, such as the "triple F" or "bottle opener" format, also allows separation on the basis of size. As is further outlined below, it is also possible to "skew" the formation of heterodimers over homodimers. Thus, a combination of steric heterodimerization variants and pI or charge pair variants find particular use. Additionally, as more fully outlined below, scaffolds that utilize scFv(s) such as the Triple F format can include charged scFv linkers (either positive or negative), that give a further pI boost for purification purposes. As will be appreciated by those in the art, some Triple F formats are useful with just charged scFv linkers and no additional pI adjustments, although the disclosure does provide the use of skew variants with charged scFv linkers as well (and combinations of Fc, FcRn and KO variants discussed herein).

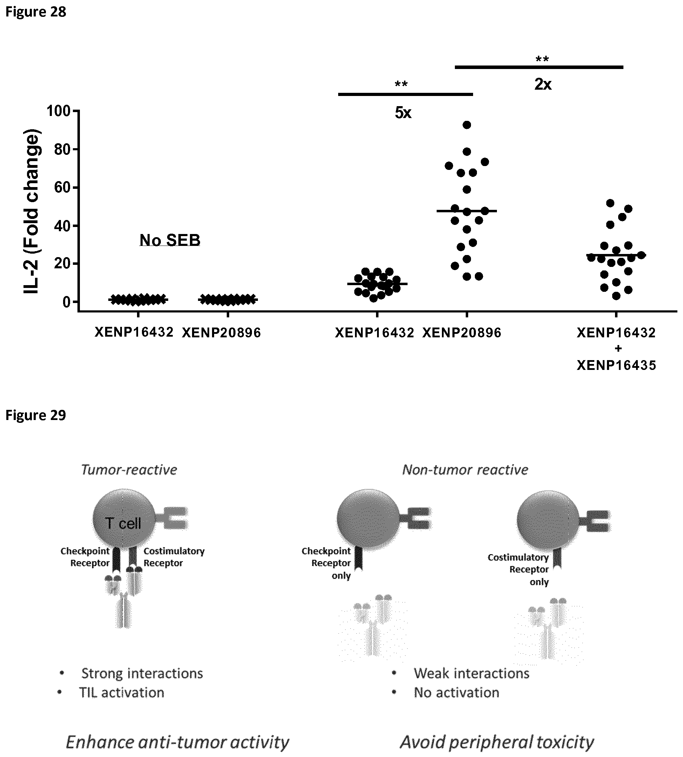

In the present disclosure that utilizes pI as a separation mechanism to allow the purification of heterodimeric proteins, amino acid variants can be introduced into one or both of the monomer polypeptides; that is, the pI of one of the monomers (referred to herein for simplicity as "monomer A") can be engineered away from monomer B, or both monomer A and B change be changed, with the pI of monomer A increasing and the pI of monomer B decreasing. As is outlined more fully below, the pI changes of either or both monomers can be done by removing or adding a charged residue (e.g. a neutral amino acid is replaced by a positively or negatively charged amino acid residue, e.g. glycine to glutamic acid), changing a charged residue from positive or negative to the opposite charge (aspartic acid to lysine) or changing a charged residue to a neutral residue (e.g. loss of a charge; lysine to serine). A number of these variants are shown in the Figures. In addition, suitable pI variants for use in the creation of heterodimeric antibodies herein are those that are isotypic, e.g. importing pI variants from different IgG isotypes such that pI is changed without introducing significant immunogenicity; see Figure 29 from US Publication No. 20140288275.

Accordingly, the present disclosure provides for creating a sufficient change in pI in at least one of the monomers such that heterodimers can be separated from homodimers. As will be appreciated by those in the art, and as discussed further below, this can be done by using a "wild type" heavy chain constant region and a variant region that has been engineered to either increase or decrease its pI (wt A-+B or wt A - -B), or by increasing one region and decreasing the other region (A+ -B- or A- B+).

Thus, in general, a component of the present disclosure are amino acid variants in the constant regions of antibodies that are directed to altering the isoelectric point (pI) of at least one, if not both, of the monomers of a dimeric protein to form "pI heterodimers" (when the protein is an antibody, these are referred to as "pI antibodies") by incorporating amino acid substitutions ("pI variants" or "pI substitutions") into one or both of the monomers. As shown herein, the separation of the heterodimers from the two homodimers can be accomplished if the pIs of the two monomers differ by as little as 0.1 pH unit, with 0.2, 0.3, 0.4 and 0.5 or greater all finding use herein.

As will be appreciated by those in the art, the number of pI variants to be included on each or both monomer(s) to get good separation will depend in part on the starting pI of the scFv and Fab of interest. That is, to determine which monomer to engineer or in which "direction" (e.g. more positive or more negative), the Fv sequences of the two target antigens are calculated and a decision is made from there. As is known in the art, different Fvs will have different starting pIs which are exploited herein. In general, as outlined herein, the pIs are engineered to result in a total pI difference of each monomer of at least about 0.1 logs, with 0.2 to 0.5 being preferred as outlined herein. Furthermore, as will be appreciated by those in the art and outlined herein, in some cases (depending on the format) heterodimers can be separated from homodimers on the basis of size (e.g. Molecular weight). For example, as shown in some examples of Figure 2, some formats result in homodimers and heterodimers with different sizes (e.g. for bottle openers, one homodimer is a "dual scFv" format, one homodimer is a standard antibody, and the heterodimer has one Fab and one scFv).

In the case where pI variants are used to achieve purified heterodimers over homodimers, by using the constant region(s) of the heavy chain(s), a more modular approach to designing and purifying multispecific proteins, including antibodies, is provided. Thus, in some examples, heterodimerization variants (including skew and purification heterodimerization variants) are not included in the variable regions, such that each individual antibody must be engineered. In addition, in some examples, the possibility of immunogenicity resulting from the pI variants is significantly reduced by importing pI variants from different IgG isotypes such that pI is changed without introducing significant immunogenicity. Thus, an additional problem to be solved is the elucidation of low pI constant domains with high human sequence content, e.g. the minimization or avoidance of non-human residues at any particular position.

A side benefit that can occur with this pI engineering is also the extension of serum half-life and increased FcRn binding. That is, **,** lowering the pI of antibody constant domains (including those found in antibodies and Fc fusions) can lead to longer serum retention in vivo. These pI variants for increased serum half life also facilitate pI changes for purification.

In addition, it should be noted that the pI variants of the heterodimerization variants give an additional benefit for the analytics and quality control process of bispecific antibodies, as the ability to either eliminate, minimize and distinguish when homodimers are present is significant. Similarly, the ability to reliably test the reproducibility of the heterodimeric protein production is important.

First and second antigens of the invention are herein referred to as antigen-1 and antigen-2 respectively, wth one being a costimulatory receptor and one being a checkpoint receptor. One heterodimeric scaffold that finds particular use in the present invention is the "triple f" or "bottle opener" scaffold format. In this embodiment, one heavy chain of the antibody contains an single chain fv ("scfv", as defined below) and the other heavy chain is a "regular" fab format, comprising a variable heavy chain and a light chain. This structure is sometimes referred to herein as "triple f" format (scfv-fab-fc) or the "bottle-opener" format, due to a rough visual similarity to a bottle-opener (see Figure 2). The two chains are brought together by the use of amino acid variants in the constant regions (e.g. the Fc domain and/or the hinge region) that promote the formation of heterodimeric antibodies as is described more fully below.

There are several distinct advantages to the present "triple F" format. As is known in the art, antibody analogs relying on two scFv constructs often have stability and aggregation problems, which can be alleviated in the present invention by the addition of a "regular" heavy and light chain pairing. In addition, as opposed to formats that rely on two heavy chains and two light chains, there is no issue with the incorrect pairing of heavy and light chains (e.g. heavy 1 pairing with light 2, etc.)

Furthermore, as outlined herein, additional amino acid variants may be introduced into the bispecific antibodies of the disclosure, to add additional functionalities. For example, amino acid changes within the Fc region can be added (either to one monomer or both) to facilitate increased ADCC or CDC (e.g. altered binding to Fcγ receptors) as well as to increase binding to FcRn and/or increase serum half-life of the resulting molecules. As is further described herein and as will be appreciated by those in the art, any and all of the variants outlined herein can be optionally and independently combined with other variants.

Similarly, another category of functional variants are "Fcγ ablation variants" or "Fc knock out (FcKO or KO) variants. In these examples, for some therapeutic applications, it is desirable to reduce or remove the normal binding of the Fc domain to one or more or all of the Fcγ receptors (e.g. FcγR1, FcγRIIa, FcγRIIb, FcγRIIIa, etc.) to avoid additional mechanisms of action. That is, for example, it is generally desirable to ablate FcγRIIIa binding to eliminate or significantly reduce ADCC activity. Suitable ablation variants are shown in Figure 6.

### IV. Definitions

In order that the application may be more completely understood, several definitions are set forth below.

By "ablation" herein is meant a decrease or removal of activity. Thus for example, "ablating FcγR binding" means the Fc region amino acid variant has less than 50% starting binding as compared to an Fc region not containing the specific variant, with less than 70-80-90-95-98% loss of activity being preferred, and in general, with the activity being below the level of detectable binding in a Biacore assay. Of particular use in the ablation of FcγR binding are those shown in Figure 6.

By "ADCC" or "antibody dependent cell-mediated cytotoxicity" as used herein is meant the cell-mediated reaction wherein nonspecific cytotoxic cells that express FcγRs recognize bound antibody on a target cell and subsequently cause lysis of the target cell. ADCC is correlated with binding to FcγRIIIa; increased binding to FcγRIIIa leads to an increase in ADCC activity. As is discussed herein, many examples of the disclosure ablate ADCC activity entirely.

By "ADCP" or antibody dependent cell-mediated phagocytosis as used herein is meant the cell-mediated reaction wherein nonspecific cytotoxic cells that express FcγRs recognize bound antibody on a target cell and subsequently cause phagocytosis of the target cell.

By "antigen binding domain" or "ABD" herein is meant a set of six Complementary Determining Regions (CDRs) that, when present as part of a polypeptide sequence, specifically binds a target antigen as discussed herein. Thus, a "immunomodulatoryantigen binding domain" binds a target immunomodulatoryantigen as outlined herein. As is known in the art, these CDRs are generally present as a first set of variable heavy CDRs (vhCDRs or V_{H}CDRs) and a second set of variable light CDRs (v1CDRs or V_{L}CDRs), each comprising three CDRs: vhCDR1, vhCDR2, vhCDR3 for the heavy chain and vlCDR1, vlCDR2 and vlCDR3 for the light. The CDRs are present in the variable heavy and variable light domains, respectively, and together form an Fv region. Thus, in some cases, the six CDRs of the antigen binding domain are contributed by a variable heavy and variable light chain. In a "Fab" format, the set of 6 CDRs are contributed by two different polypeptide sequences, the variable heavy domain (vh or V_{H}; containing the vhCDR1, vhCDR2 and vhCDR3) and the variable light domain (vl or V_{L}; containing the vlCDR1, vlCDR2 and vlCDR3), with the C-terminus of the vh domain being attached to the N-terminus of the CH1 domain of the heavy chain and the C-terminus of the vl domain being attached to the N-terminus of the constant light domain (and thus forming the light chain). In a scFv format, the vh and vl domains are covalently attached, generally through the use of a linker as outlined herein, into a single polypeptide sequence, which can be either (starting from the N-terminus) vh-linker-vl or vl-linker-vh, with the former being generally preferred (including optional domain linkers on each side, depending on the format used.

By "modification" herein is meant an amino acid substitution, insertion, and/or deletion in a polypeptide sequence or an alteration to a moiety chemically linked to a protein. For example, a modification may be an altered carbohydrate or PEG structure attached to a protein. By "amino acid modification" herein is meant an amino acid substitution, insertion, and/or deletion in a polypeptide sequence. For clarity, unless otherwise noted, the amino acid modification is always to an amino acid coded for by DNA, e.g. the 20 amino acids that have codons in DNA and RNA.

By "amino acid substitution" or "substitution" herein is meant the replacement of an amino acid at a particular position in a parent polypeptide sequence with a different amino acid. In particular, in some embodiments, the substitution is to an amino acid that is not naturally occurring at the particular position, either not naturally occurring within the organism or in any organism. For example, the substitution E272Y refers to a variant polypeptide, in this case an Fc variant, in which the glutamic acid at position 272 is replaced with tyrosine. For clarity, a protein which has been engineered to change the nucleic acid coding sequence but not change the starting amino acid (for example exchanging CGG (encoding arginine) to CGA (still encoding arginine) to increase host organism expression levels) is not an "amino acid substitution"; that is, despite the creation of a new gene encoding the same protein, if the protein has the same amino acid at the particular position that it started with, it is not an amino acid substitution.

By "amino acid insertion" or "insertion" as used herein is meant the addition of an amino acid sequence at a particular position in a parent polypeptide sequence. For example, -233E or 233E designates an insertion of glutamic acid after position 233 and before position 234. Additionally, -233ADE or A233ADE designates an insertion of AlaAspGlu after position 233 and before position 234.

By "amino acid deletion" or "deletion" as used herein is meant the removal of an amino acid sequence at a particular position in a parent polypeptide sequence. For example, E233- or E233#, E233() or E233del designates a deletion of glutamic acid at position 233. Additionally, EDA233- or EDA233# designates a deletion of the sequence GluAspAla that begins at position 233.

By "variant protein" or "protein variant", or "variant" as used herein is meant a protein that differs from that of a parent protein by virtue of at least one amino acid modification. Protein variant may refer to the protein itself, a composition comprising the protein, or the amino sequence that encodes it. Preferably, the protein variant has at least one amino acid modification compared to the parent protein, e.g. from about one to about seventy amino acid modifications, and preferably from about one to about five amino acid modifications compared to the parent. As described below, in some embodiments the parent polypeptide, for example an Fc parent polypeptide, is a human wild type sequence, such as the Fc region from IgG1, IgG2, IgG3 or IgG4, although human sequences with variants can also serve as "parent polypeptides", for example the IgG1/2 hybrid can be included. The protein variant sequence herein will preferably possess at least about 80% identity with a parent protein sequence, and most preferably at least about 90% identity, more preferably at least about 95-98-99% identity. Variant protein can refer to the variant protein itself, compositions comprising the protein variant, or the DNA sequence that encodes it.

Accordingly, by "antibody variant" or "variant antibody" as used herein is meant an antibody that differs from a parent antibody by virtue of at least one amino acid modification, "IgG variant" or "variant IgG" as used herein is meant an antibody that differs from a parent IgG (again, in many cases, from a human IgG sequence) by virtue of at least one amino acid modification, and "immunoglobulin variant" or "variant immunoglobulin" as used herein is meant an immunoglobulin sequence that differs from that of a parent immunoglobulin sequence by virtue of at least one amino acid modification. "Fc variant" or "variant Fc" as used herein is meant a protein comprising an amino acid modification in an Fc domain. The Fc variants disclosed herein may be defined according to the amino acid modifications that compose them. Thus, for example, N434S or 434S is an Fc variant with the substitution serine at position 434 relative to the parent Fc polypeptide, wherein the numbering is according to the EU index. Likewise, M428L/N434S defines an Fc variant with the substitutions M428L and N434S relative to the parent Fc polypeptide. The identity of the WT amino acid may be unspecified, in which case the aforementioned variant is referred to as 428L/434S. It is noted that the order in which substitutions are provided is arbitrary, that is to say that, for example, 428L/434S is the same Fc variant as M428L/N434S, and so on. For all positions discussed in the present disclosure that relate to antibodies, unless otherwise noted, amino acid position numbering is according to the EU index. The EU index or EU index as in Kabat or EU numbering scheme refers to the numbering of the EU antibody (Edelman et al., 1969, Proc Natl Acad Sci USA 63:78-85) The modification can be an addition, deletion, or substitution. Substitutions can include naturally occurring amino acids and, in some cases, synthetic amino acids. Examples include U.S. Pat. No. 6,586,207; WO 98/48032; WO 03/073238; US2004-0214988A1; WO 05/35727A2; WO 05/74524A2; J. W. Chin et al., (2002), Journal of the American Chemical Society 124:9026-9027; J. W. Chin, & P. G. Schultz, (2002), ChemBioChem 11:1135-1137; J. W. Chin, et al., (2002), PICAS United States of America 99:11020-11024; and, L. Wang, & P. G. Schultz, (2002), Chem. 1-10.

As used herein, "protein" herein is meant at least two covalently attached amino acids, which includes proteins, polypeptides, oligopeptides and peptides. The peptidyl group may comprise naturally occurring amino acids and peptide bonds, or synthetic peptidomimetic structures, i.e. "analogs", such as peptoids (see Simon et al., PNAS USA 89(20):9367 (1992)). The amino acids may either be naturally occurring or synthetic (e.g. not an amino acid that is coded for by DNA); as will be appreciated by those in the art. For example, homo-phenylalanine, citrulline, ornithine and noreleucine are considered synthetic amino acids for the purposes of the invention, and both D- and L-(R or S) configured amino acids may be utilized. The variants of the present invention may comprise modifications that include the use of synthetic amino acids incorporated using, for example, the technologies developed by Schultz and colleagues, including but not limited to methods described by Cropp & Shultz, 2004, Trends Genet. 20(12):625-30, Anderson et al., 2004, Proc Natl Acad Sci USA 101 (2):7566-71, Zhang et al., 2003, 303(5656):371-3, and Chin et al., 2003, Science 301(5635):964-7. In addition, polypeptides may include synthetic derivatization of one or more side chains or termini, glycosylation, PEGylation, circular permutation, cyclization, linkers to other molecules, fusion to proteins or protein domains, and addition of peptide tags or labels.

By "residue" as used herein is meant a position in a protein and its associated amino acid identity. For example, Asparagine 297 (also referred to as Asn297 or N297) is a residue at position 297 in the human antibody IgG1.

By "Fab" or "Fab region" as used herein is meant the polypeptide that comprises the VH, CH1, VL, and CL immunoglobulin domains. Fab may refer to this region in isolation, or this region in the context of a full length antibody, antibody fragment or Fab fusion protein.

By "Fv" or "Fv fragment" or "Fv region" as used herein is meant a polypeptide that comprises the VL and VH domains of a single ABD. As will be appreciated by those in the art, these generally are made up of two chains, or can be combined (generally with a linker as discussed herein) to form an scFv. In some cases, for example in the "central-Fv" and "DVD-Ig" formats, an "extra" vh and vl domain is added that serves as a scFv but where the vh and vl domains are not linked using a scFv linker between them.

By "single chain Fv" or "scFv" herein is meant a variable heavy domain covalently attached to a variable light domain, generally using a scFv linker as discussed herein, to form a scFv or scFv domain. A scFv domain can be in either orientation from N- to C-terminus (vh-linker-vl or vl-linker-vh).

By "IgG subclass modification" or "isotype modification" as used herein is meant an amino acid modification that converts one amino acid of one IgG isotype to the corresponding amino acid in a different, aligned IgG isotype. For example, because IgG1 comprises a tyrosine and IgG2 a phenylalanine at EU position 296, a F296Y substitution in IgG2 is considered an IgG subclass modification.

By "non-naturally occurring modification" as used herein is meant an amino acid modification that is not isotypic. For example, because none of the IgGs comprise a serine at position 434, the substitution 434S in IgG1, IgG2, IgG3, or IgG4 (or hybrids thereof) is considered a non-naturally occurring modification.

By "amino acid" and "amino acid identity" as used herein is meant one of the 20 naturally occurring amino acids that are coded for by DNA and RNA.

By "effector function" as used herein is meant a biochemical event that results from the interaction of an antibody Fc region with an Fc receptor or ligand. Effector functions include but are not limited to ADCC, ADCP, and CDC.

By "IgG Fc ligand" as used herein is meant a molecule, preferably a polypeptide, from any organism that binds to the Fc region of an IgG antibody to form an Fc/Fc ligand complex. Fc ligands include but are not limited to FcγRIs, FcγRIIs, FcγRIIIs, FcRn, C1q, C3, mannan binding lectin, mannose receptor, staphylococcal protein A, streptococcal protein G, and viral FcγR. Fc ligands also include Fc receptor homologs (FcRH), which are a family of Fc receptors that are homologous to the FcγRs(Davis et al., 2002, Immunological Reviews 190:123-136). Fc ligands may include undiscovered molecules that bind Fc. Particular IgG Fc ligands are FcRn and Fc gamma receptors. By "Fc ligand" as used herein is meant a molecule, preferably a polypeptide, from any organism that binds to the Fc region of an antibody to form an Fc/Fc ligand complex.

By "Fc gamma receptor", "FcγR" or "FcgammaR" as used herein is meant any member of the family of proteins that bind the IgG antibody Fc region and is encoded by an FcγR gene. In humans this family includes but is not limited to FcγRI (CD64), including isoforms FcγRIa, FcγRIb, and FcγRIc; FcγRII (CD32), including isoforms FcγRIIa (including allotypes H131 and R131), FcγRIIb (including FcγRIIb-1 and FcγRIIb-2), and FcγRIIc; and FcγRIII (CD16), including isoforms FcγRIIIa (including allotypes V158 and F158) and FcγRIIIb (including allotypes FcγRIIb-NA1 and FcγRIIb-NA2) (Jefferis et al., 2002, Immunol Lett 82:57-65), as well as any undiscovered human FcγRs or FcγR isoforms or allotypes. An FcγR may be from any organism, including but not limited to humans, mice, rats, rabbits, and monkeys. Mouse FcγRs include but are not limited to FcγRI (CD64), FcγRII (CD32), FcγRIII (CD16), and FcγRIII-2 (CD16-2), as well as any undiscovered mouse FcγRs or FcγR isoforms or allotypes.

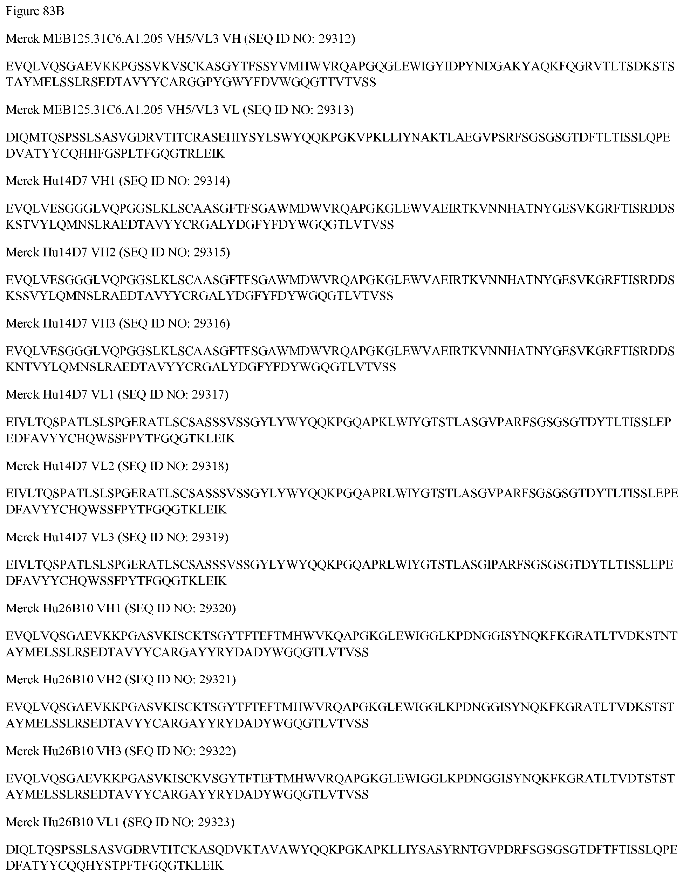

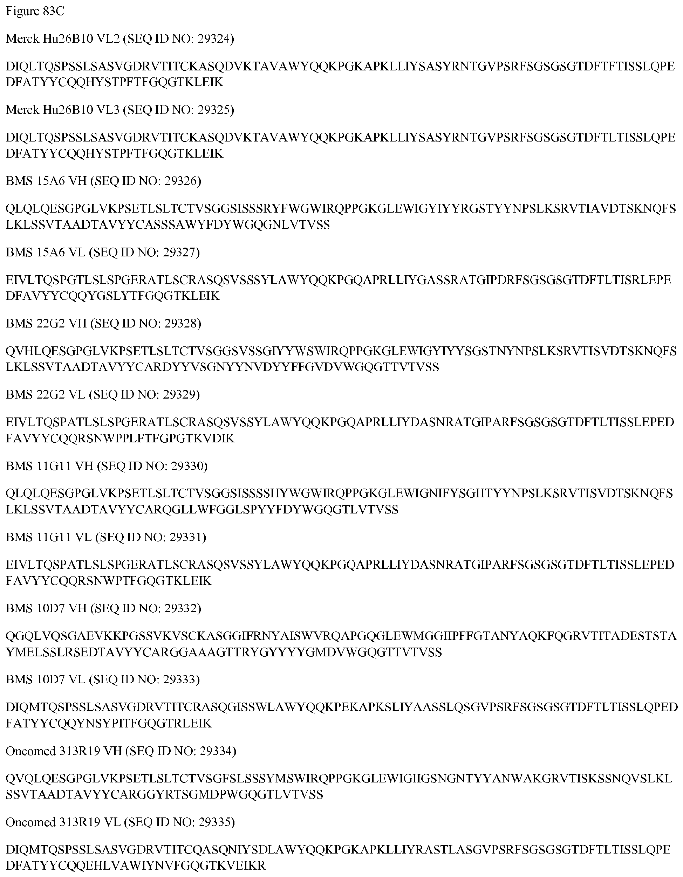

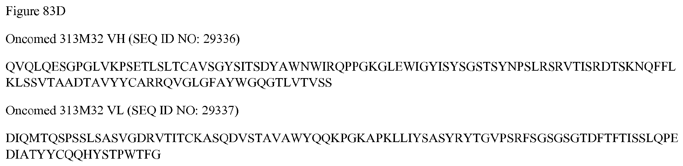

By "FcRn" or "neonatal Fc Receptor" as used herein is meant a protein that binds the IgG antibody Fc region and is encoded at least in part by an FcRn gene. The FcRn may be from any organism, including but not limited to humans, mice, rats, rabbits, and monkeys. As is known in the art, the functional FcRn protein comprises two polypeptides, often referred to as the heavy chain and light chain. The light chain is beta-2-microglobulin and the heavy chain is encoded by the FcRn gene. Unless otherwise noted herein, FcRn or an FcRn protein refers to the complex of FcRn heavy chain with beta-2-microglobulin. A variety of FcRn variants used to increase binding to the FcRn receptor, and in some cases, to increase serum half-life, are shown in the Figure Legend of Figure 83.

By "parent polypeptide" as used herein is meant a starting polypeptide that is subsequently modified to generate a variant. The parent polypeptide may be a naturally occurring polypeptide, or a variant or engineered version of a naturally occurring polypeptide. Parent polypeptide may refer to the polypeptide itself, compositions that comprise the parent polypeptide, or the amino acid sequence that encodes it. Accordingly, by "parent immunoglobulin" as used herein is meant an unmodified immunoglobulin polypeptide that is modified to generate a variant, and by "parent antibody" as used herein is meant an unmodified antibody that is modified to generate a variant antibody. It should be noted that "parent antibody" includes known commercial, recombinantly produced antibodies as outlined below.

By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some examples, as is more fully described below, amino acid modifications are made to the Fc region, for example to alter binding to one or more FcγR receptors or to the FcRn receptor.

By "heavy constant region" herein is meant the CH1-hinge-CH2-CH3 portion of an antibody.

By "Fc fusion protein" or "immunoadhesin" herein is meant a protein comprising an Fc region, generally linked (optionally through a linker moiety, as described herein) to a different protein, such as a binding moiety to a target protein, as described herein. In some cases, one monomer of the heterodimeric antibody comprises an antibody heavy chain (either including an scFv or further including a light chain) and the other monomer is a Fc fusion, comprising a variant Fc domain and a ligand. In some examples, these "half antibody-half fusion proteins" are referred to as "Fusionbodies".

By "position" as used herein is meant a location in the sequence of a protein. Positions may be numbered sequentially, or according to an established format, for example the EU index for antibody numbering.

By "target antigen" as used herein is meant the molecule that is bound specifically by the variable region of a given antibody. A target antigen may be a protein, carbohydrate, lipid, or other chemical compound. Suitable target antigens are described below.

By "strandedness" in the context of the monomers of the heterodimeric antibodies of the invention herein is meant that, similar to the two strands of DNA that "match", heterodimerization variants are incorporated into each monomer so as to preserve the ability to "match" to form heterodimers. For example, if some pI variants are engineered into monomer A (e.g. making the pI higher) then steric variants that are "charge pairs" that can be utilized as well do not interfere with the pI variants, e.g. the charge variants that make a pI higher are put on the same "strand" or "monomer" to preserve both functionalities. Similarly, for "skew" variants that come in pairs of a set as more fully outlined below, the skilled artisan will consider pI in deciding into which strand or monomer that incorporates one set of the pair will go, such that pI separation is maximized using the pI of the skews as well.

By "target cell" as used herein is meant a cell that expresses a target antigen.

By "variable region" as used herein is meant the region of an immunoglobulin that comprises one or more Ig domains substantially encoded by any of the V.kappa., V.lamda., and/or VH genes that make up the kappa, lambda, and heavy chain immunoglobulin genetic loci respectively.

By "wild type or WT" herein is meant an amino acid sequence or a nucleotide sequence that is found in nature, including allelic variations. A WT protein has an amino acid sequence or a nucleotide sequence that has not been intentionally modified.

The antibodies of the present invention are generally isolated or recombinant. "Isolated," when used to describe the various polypeptides disclosed herein, means a polypeptide that has been identified and separated and/or recovered from a cell or cell culture from which it was expressed. Ordinarily, an isolated polypeptide will be prepared by at least one purification step. An "isolated antibody," refers to an antibody which is substantially free of other antibodies having different antigenic specificities. "Recombinant" means the antibodies are generated using recombinant nucleic acid techniques in exogeneous host cells.

"Percent (%) amino acid sequence identity" with respect to a protein sequence is defined as the percentage of amino acid residues in a candidate sequence that are identical with the amino acid residues in the specific (parental) sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. One particular program is the ALIGN-2 program outlined at paragraphs [0279] to [0280] of US Pub. No. 20160244525.

The degree of identity between an amino acid sequence of the present invention ("invention sequence") and the parental amino acid sequence is calculated as the number of exact matches in an alignment of the two sequences, divided by the length of the "invention sequence," or the length of the parental sequence, whichever is the shortest. The result is expressed in percent identity.

In some examples, two or more amino acid sequences are at least 50%, 60%, 70%, 80%, or 90% identical. In some examples, two or more amino acid sequences are at least 95%, 97%, 98%, 99%, or even 100% identical.

"Specific binding" or "specifically binds to" or is "specific for" a particular antigen or an epitope means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target.

Specific binding for a particular antigen or an epitope can be exhibited, for example, by an antibody having a KD for an antigen or epitope of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the antigen or epitope.

Also, specific binding for a particular antigen or an epitope can be exhibited, for example, by an antibody having a KA or Ka for an antigen or epitope of at least 20-, 50-, 100-, 500-, 1000-, 5,000-, 10,000- or more times greater for the epitope relative to a control, where KA or Ka refers to an association rate of a particular antibody-antigen interaction. Binding affinity is generally measured using a Biacore assay.

### V. Antibodies

The present disclosure relates to the generation of bispecific immunomodulatory antibodies that bind two different immunomodulatory antigens as discussed herein. As is discussed below, the term "antibody" is used generally. Antibodies that find use herein can take on a number of formats as described herein.

Traditional antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. The present disclosure is directed to bispecific antibodies that generally are based on the IgG class, which has several subclasses, including, but not limited to IgG1, IgG2, IgG3, and IgG4. In general, IgG1, IgG2 and IgG4 are used more frequently than IgG3. It should be noted that IgG1 has different allotypes with polymorphisms at 356 (D or E) and 358 (L or M). The sequences depicted herein use the 356D/358M allotype, however the other allotype is included herein. That is, any sequence inclusive of an IgG1 Fc domain included herein can have 356E/358L replacing the 356D/358M allotype.

In addition, many of the sequences herein have at least one of the cysteines at position 220 replaced by a serine; generally, this is the on the "scFv monomer" side for most of the sequences depicted herein, although it can also be on the "Fab monomer" side, or both, to reduce disulfide formation. Specifically included within the sequences herein are one or both of these cysteines replaced (C220S).

Thus, "isotype" as used herein is meant any of the subclasses of immunoglobulins defined by the chemical and antigenic characteristics of their constant regions. It should be understood that therapeutic antibodies can also comprise hybrids of isotypes and/or subclasses. For example, as shown in US Publication 2009/0163699, , the present disclosure covers pI engineering of IgG1/G2 hybrids.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions" that are each 9-15 amino acids long or longer.

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region; Kabat et al., SEQUENCES OF PROTEINS OF IMMUNOLOGICAL INTEREST, 5th Ed. Public Health Service, National Institutes of Health, Bethesda, Md. (1991) and/or those residues forming a hypervariable loop (e.g. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the disclosure are described below.

As will be appreciated by those in the art, the exact numbering and placement of the CDRs can be different among different numbering systems. However, it should be understood that the disclosure of a variable heavy and/or variable light sequence includes the disclosure of the associated (inherent) CDRs. Accordingly, the disclosure of each variable heavy region is a disclosure of the vhCDRs (e.g. vhCDR1, vhCDR2 and vhCDR3) and the disclosure of each variable light region is a disclosure of the vlCDRs (e.g. vlCDR1, vlCDR2 and vlCDR3).

A useful comparison of CDR numbering is as below, see Lafranc et al., Dev. Comp. Immunol. 27(1):55-77 (2003):

**TABLE 1**

| | Kabat+ Chothia | IMGT | Kabat | AbM | Chothia | Contact | Xencor |
|---|---|---|---|---|---|---|---|
| vhCDR1 | 26-35 | 27-38 | 31-35 | 26-35 | 26-32 | 30-35 | 27-35 |
| vhCDR2 | 50-65 | 56-65 | 50-65 | 50-58 | 52-56 | 47-58 | 54-61 |
| vhCDR3 | 95-102 | 105-117 | 95-102 | 95-102 | 95-102 | 93-101 | 103-116 |
| vlCDR1 | 24-34 | 27-38 | 24-34 | 24-34 | 24-34 | 30-36 | 27-38 |
| vlCDR2 | 50-56 | 56-65 | 50-56 | 50-56 | 50-56 | 46-55 | 56-62 |
| vlCDR3 | 89-97 | 105-117 | 89-97 | 89-97 | 89-97 | 89-96 | 97-105 |

Throughout the present specification, the Kabat numbering system is generally used when referring to a residue in the variable domain (approximately, residues 1-107 of the light chain variable region and residues 1-113 of the heavy chain variable region) and the EU numbering system for Fc regions (e.g, Kabat et al., supra (1991)).

Disclosed herein are a large number of different CDR sets. In this case, a "full CDR set" comprises the three variable light and three variable heavy CDRs, e.g. a vlCDR1, vlCDR2, vlCDR3, vhCDR1, vhCDR2 and vhCDR3. These can be part of a larger variable light or variable heavy domain, respectfully. In addition, as more fully outlined herein, the variable heavy and variable light domains can be on separate polypeptide chains, when a heavy and light chain is used (for example when Fabs are used), or on a single polypeptide chain in the case of scFv sequences.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning." As outlined below, the disclosure not only includes the enumerated antigen binding domains and antibodies herein, but those that compete for binding with the epitopes bound by the enumerated antigen binding domains.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E.A. Kabat et al..

In the IgG subclass of immunoglobulins, there are several immunoglobulin domains in the heavy chain. By "immunoglobulin (Ig) domain" herein is meant a region of an immunoglobulin having a distinct tertiary structure. Of interest are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat. As shown herein and described below, the pI variants can be in one or more of the CH regions, as well as the hinge region, discussed below.

Another type of Ig domain of the heavy chain is the hinge region. By "hinge" or "hinge region" or "antibody hinge region" or "immunoglobulin hinge region" herein is meant the flexible polypeptide comprising the amino acids between the first and second constant domains of an antibody. Structurally, the IgG CH1 domain ends at EU position 220, and the IgG CH2 domain begins at residue EU position 237. Thus for IgG the antibody hinge is herein defined to include positions 221 (D221 in IgG1) to 236 (G236 in IgG1), wherein the numbering is according to the EU index as in Kabat. In some embodiments, for example in the context of an Fc region, the lower hinge is included, with the "lower hinge" generally referring to positions 226 or 230. As noted herein, pI variants can be made in the hinge region as well.

The light chain generally comprises two domains, the variable light domain (containing the light chain CDRs and together with the variable heavy domains forming the Fv region), and a constant light chain region (often referred to as CL or Cκ).

Another region of interest for additional substitutions, outlined below, is the Fc region.

As described herein and known in the art, the heterodimeric antibodies of the invention comprise different domains within the heavy and light chains, which can be overlapping as well. These domains are as defined in the claims and include the Fc domain, the CH1 domain, the CH2 domain, the CH3 domain, the hinge domain, the heavy constant domain (CH1-hinge-Fc domain or CH1-hinge-CH2-CH3), the variable heavy domain, the variable light domain, the light constant domain, Fab domains and scFv domains.

Thus, the "Fc domain" includes the -CH2-CH3 domain, and optionally a hinge domain. In the embodiments herein, when a scFv is attached to an Fc domain, it is the C-terminus of the scFv construct that is attached to all or part of the hinge of the Fc domain; for example, it is generally attached to the sequence EPKS which is the beginning of the hinge. The heavy chain comprises a variable heavy domain and a constant domain, which includes a CH1-optional hinge-Fc domain comprising a CH2-CH3. The light chain comprises a variable light chain and the light constant domain. A scFv comprises a variable heavy chain, an scFv linker, and a variable light domain. In most of the constructs and sequences outlined herein, C-terminus of the variable light chain is attached to the N-terminus of the scFv linker, the C-terminus of which is attached to the N-terminus of a variable heavy chain (N-vh-linker-vl-C) although that can be switched (N-vl-linker-vh-C). Some examples of the disclosure comprise at least one scFv domain, which, while not naturally occurring, generally includes a variable heavy domain and a variable light domain, linked together by a scFv linker. As outlined herein, while the scFv domain is generally from N- to C-terminus oriented as vh-scFv linker-vl, this can be reversed for any of the scFv domains (or those constructed using vh and vl sequences from Fabs), to vl-scFv linker-vh, with optional linkers at one or both ends depending on the format (see generally Figure 2).

As shown herein, there are a number of suitable scFv linkers that can be used, including traditional peptide bonds, generated by recombinant techniques. The linker peptide may predominantly include the following amino acid residues: Gly, Ser, Ala, or Thr. The linker peptide should have a length that is adequate to link two molecules in such a way that they assume the correct conformation relative to one another so that they retain the desired activity. In one example, the linker is from about 1 to 50 amino acids in length, preferably about 1 to 30 amino acids in length. In one example, linkers of 1 to 20 amino acids in length may be used, with from about 5 to about 10 amino acids finding use in some examples. Useful linkers include glycine-serine polymers, including for example (GS)n, (GSGGS)n, (GGGGS)n, and (GGGS)n, where n is an integer of at least one (and generally from 3 to 4), glycine-alanine polymers, alanine-serine polymers, and other flexible linkers. Alternatively, a variety of nonproteinaceous polymers, including but not limited to polyethylene glycol (PEG), polypropylene glycol, polyoxyalkylenes, or copolymers of polyethylene glycol and polypropylene glycol, may find use as linkers, that is may find use as linkers.

Other linker sequences may include any sequence of any length of CL/CH1 domain but not all residues of CL/CH1 domain; for example the first 5-12 amino acid residues of the CL/CH1 domains. Linkers can be derived from immunoglobulin light chain, for example Cκ or Cλ. Linkers can be derived from immunoglobulin heavy chains of any isotype, including for example Cγ1, Cy2, Cy3, Cy4, Cα1, Cα2, Cδ, Cε, and Cµ. Linker sequences may also be derived from other proteins such as Ig-like proteins (e.g. TCR, FcR, KIR), hinge region-derived sequences, and other natural sequences from other proteins.

In some examples, the linker is a "domain linker", used to link any two domains as outlined herein together. While any suitable linker can be used, many examples utilize a glycine-serine polymer, including for example (GS)n, (GSGGS)n, (GGGGS)n, and (GGGS)n, where n is an integer of at least one (and generally from 3 to 4 to 5) as well as any peptide sequence that allows for recombinant attachment of the two domains with sufficient length and flexibility to allow each domain to retain its biological function.. In some cases, and with attention being paid to "strandedness", as outlined below, charged domain linkers, as used in some examples of scFv linkers can be used.

In some exampless, the scFv linker is a charged scFv linker, a number of which are shown in Figure 8. Accordingly, disclosed herein are charged scFv linkers, to facilitate the separation in pI between a first and a second monomer. That is, by incorporating a charged scFv linker, either positive or negative (or both, in the case of scaffolds that use scFvs on different monomers), this allows the monomer comprising the charged linker to alter the pI without making further changes in the Fc domains. These charged linkers can be substituted into any scFv containing standard linkers. Again, as will be appreciated by those in the art, charged scFv linkers are used on the correct "strand" or monomer, according to the desired changes in pI. For example, as discussed herein, to make triple F format heterodimeric antibody, the original pI of the Fv region for each of the desired antigen binding domains are calculated, and one is chosen to make an scFv, and depending on the pI, either positive or negative linkers are chosen.

Charged domain linkers can also be used to increase the pI separation of the monomers disclosed herein as well, and thus those included in Figure 8 can be used in any example herein where a linker is utilized.

The antibody may be an antibody fragment, as long as it contains at least one constant domain which can be engineered to produce heterodimers, such as pI engineering. Other antibody fragments that can be used include fragments that contain one or more of the CH1, CH2, CH3, hinge and CL domains disclosed herein that have been pI engineered. In particular, the formats depicted in Figure 1 are antibodies, usually referred to as "heterodimeric antibodies", meaning that the protein has at least two associated Fc sequences self-assembled into a heterodimeric Fc domain and at least two Fv regions, whether as Fabs or as scFvs.

### A. Chimeric and Humanized Antibodies

In some examples, the antibodies herein can be derived from a mixture from different species, e.g. a chimeric antibody and/or a humanized antibody. In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536. "Backmutation" of selected acceptor framework residues to the corresponding donor residues is often required to regain affinity that is lost in the initial grafted construct (US 5530101; US 5585089; US 5693761; US 5693762; US 6180370; US 5859205; US 5821337; US 6054297; US 6407213). The humanized antibody optimally also will comprise at least a portion of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein). Humanization methods include but are not limited to methods described in Jones et al., 1986, Nature 321:522-525; Riechmann et al.,1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res. 57(20):4593-9; Gorman et al., 1991, Proc. Natl. Acad. Sci. USA 88:4181-4185; O'Connor et al., 1998, Protein Eng 11:321-8. Humanization or other methods of reducing the immunogenicity of nonhuman antibody variable regions may include resurfacing methods, as described for example in Roguska et al., 1994, Proc. Natl. Acad. Sci. USA 91:969-973.

In certain embodiments, the antibodies comprise a heavy chain variable region from a particular germline heavy chain immunoglobulin gene and/or a light chain variable region from a particular germline light chain immunoglobulin gene. For example, such antibodies may comprise or consist of a human antibody comprising heavy or light chain variable regions that are "the product of" or "derived from" a particular germline sequence. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a humanized antibody typically is at least 90% identical in amino acids sequence to an amino acid sequence encoded by a human germline immunoglobulin gene and contains amino acid residues that identify the antibody as being derived from human sequences when compared to the germline immunoglobulin amino acid sequences of other species (e.g., murine germline sequences). In certain cases, a humanized antibody may be at least 95, 96, 97, 98 or 99%, or even at least 96%, 97%, 98%, or 99% identical in amino acid sequence to the amino acid sequence encoded by the germline immunoglobulin gene. Typically, a humanized antibody derived from a particular human germline sequence will display no more than 10-20 amino acid differences from the amino acid sequence encoded by the human germline immunoglobulin gene (prior to the introduction of any skew, pI and ablation variants herein; that is, the number of variants is generally low, prior to the introduction of the variants). In certain cases, the humanized antibody may display no more than 5, or even no more than 4, 3, 2, or 1 amino acid difference from the amino acid sequence encoded by the germline immunoglobulin gene (again, prior to the introduction of any skew, pI and ablation variants herein; that is, the number of variants is generally low, prior to the introduction of the variants of the invention).

In one embodiment, the parent antibody has been affinity matured, as is known in the art. Structure-based methods may be employed for humanization and affinity maturation. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759. Other humanization methods may involve the grafting of only parts of the CDRs, including but not limited to methods described in Tan et al., 2002, J. Immunol. 169:1119-1125; De Pascalis et al., 2002, J. Immunol. 169:3076-3084.

### VI. Heterodimeric Antibodies

Accordingly, the present invention provides heterodimeric immunomodulatory antibodies that rely on the use of two different heavy chain variant Fc sequences, that will self-assemble to form heterodimeric Fc domains and heterodimeric antibodies.

The present invention is directed to novel constructs to provide heterodimeric antibodies that allow binding to more than one immunomodulatory antigen or ligand, e.g. to allow for bispecific binding. The heterodimeric antibody constructs are based on the self-assembling nature of the two Fc domains of the heavy chains of antibodies, e.g. two "monomers" that assemble into a "dimer". Heterodimeric antibodies are made by altering the amino acid sequence of each monomer as more fully discussed below. Thus, the present invention is directed to the creation of heterodimeric immunomodulatory antibodies which can co-engage antigens in several ways, relying on amino acid variants in the constant regions that are different on each chain to promote heterodimeric formation and/or allow for ease of purification of heterodimers over the homodimers.

Thus, the present invention provides bispecific antibodies. An ongoing problem in antibody technologies is the desire for "bispecific" antibodies that bind to two different antigens simultaneously, in general thus allowing the different antigens to be brought into proximity and resulting in new functionalities and new therapies. In general, these antibodies are made by including genes for each heavy and light chain into the host cells. This generally results in the formation of the desired heterodimer (A-B), as well as the two homodimers (A-A and B-B (not including the light chain heterodimeric issues)). However, a major obstacle in the formation of bispecific antibodies is the difficulty in purifying the heterodimeric antibodies away from the homodimeric antibodies and/or biasing the formation of the heterodimer over the formation of the homodimers.

There are a number of mechanisms that can be used to generate the heterodimers of the present invention. In addition, as will be appreciated by those in the art, these mechanisms can be combined to ensure high heterodimerization. Thus, amino acid variants that lead to the production of heterodimers are referred to as "heterodimerization variants". As discussed below, heterodimerization variants can include steric variants (e.g. the "knobs and holes" or "skew" variants described below and the "charge pairs" variants described below) as well as "pI variants", which allows purification of homodimers away from heterodimers. As is generally described in WO2014/145806 as below for the discussion of "heterodimerization variants", useful mechanisms for heterodimerization include "knobs and holes" ("KIH"; sometimes herein as "skew" variants (see discussion in WO2014/145806), "electrostatic steering" or "charge pairs" as described in WO2014/145806, pI variants as described in WO2014/145806, and general additional Fc variants as outlined in WO2014/145806 and below.

Disclosed herein are several basic mechanisms that can lead to ease of purifying heterodimeric antibodies; one relies on the use of pI variants, such that each monomer has a different pI, thus allowing the isoelectric purification of A-A, A-B and B-B dimeric proteins. Alternatively, some scaffold formats, such as the "triple F" format, also allows separation on the basis of size. As is further outlined below, it is also possible to "skew" the formation of heterodimers over homodimers. Thus, a combination of steric heterodimerization variants and pI or charge pair variants find particular use herein.

In general, examples of particular use in the present disclosure rely on sets of variants that include skew variants, that encourage heterodimerization formation over homodimerization formation, coupled with pI variants, which increase the pI difference between the two monomers.

Additionally, as more fully outlined below, depending on the format of the heterodimer antibody, pI variants can be either contained within the constant and/or Fc domains of a monomer, or charged linkers, either domain linkers or scFv linkers, can be used. That is, scaffolds that utilize scFv(s) such as the Triple F format can include charged scFv linkers (either positive or negative), that give a further pI boost for purification purposes. As will be appreciated by those in the art, some Triple F formats are useful with just charged scFv linkers and no additional pI adjustments, although the disclosure does provide pI variants that are on one or both of the monomers, and/or charged domain linkers as well. In addition, additional amino acid engineering for alternative functionalities may also confer pI changes, such as Fc, FcRn and KO variants.

In the present disclosure that utilizes pI as a separation mechanism to allow the purification of heterodimeric proteins, amino acid variants can be introduced into one or both of the monomer polypeptides; that is, the pI of one of the monomers (referred to herein for simplicity as "monomer A") can be engineered away from monomer B, or both monomer A and B change be changed, with the pI of monomer A increasing and the pI of monomer B decreasing. As discussed, the pI changes of either or both monomers can be done by removing or adding a charged residue (e.g. a neutral amino acid is replaced by a positively or negatively charged amino acid residue, e.g. glycine to glutamic acid), changing a charged residue from positive or negative to the opposite charge (e.g. aspartic acid to lysine) or changing a charged residue to a neutral residue (e.g. loss of a charge; lysine to serine.). A number of these variants are shown in the Figures.

Accordingly, the present disclosure provides for creating a sufficient change in pI in at least one of the monomers such that heterodimers can be separated from homodimers. As will be appreciated by those in the art, and as discussed further below, this can be done by using a "wild type" heavy chain constant region and a variant region that has been engineered to either increase or decrease its pI (wt A-+B or wt A - -B), or by increasing one region and decreasing the other region (A+ -B- or A- B+).

Thus, in general, a component of some examples of the present disclosure are amino acid variants in the constant regions of antibodies that are directed to altering the isoelectric point (pI) of at least one, if not both, of the monomers of a dimeric protein to form "pI antibodies" by incorporating amino acid substitutions ("pI variants" or "pI substitutions") into one or both of the monomers. As shown herein, the separation of the heterodimers from the two homodimers can be accomplished if the pIs of the two monomers differ by as little as 0.1 pH unit, with 0.2, 0.3, 0.4 and 0.5 or greater all finding use herein.

As will be appreciated by those in the art, the number of pI variants to be included on each or both monomer(s) to get good separation will depend in part on the starting pI of the components, for example in the triple F format, the starting pI of the scFv and Fab of interest. That is, to determine which monomer to engineer or in which "direction" (e.g. more positive or more negative), the Fv sequences of the two target antigens are calculated and a decision is made from there. As is known in the art, different Fvs will have different starting pIs which may be exploited in the present disclosure. In general, as outlined herein, the pIs are engineered to result in a total pI difference of each monomer of at least about 0.1 logs, with 0.2 to 0.5 being preferred as outlined herein. Furthermore, as will be appreciated by those in the art and outlined herein, in some embodiments, heterodimers can be separated from homodimers on the basis of size. As shown in Figure 2, for example, several of the formats allow separation of heterodimers and homodimers on the basis of size.

### A. Heterodimerization Variants

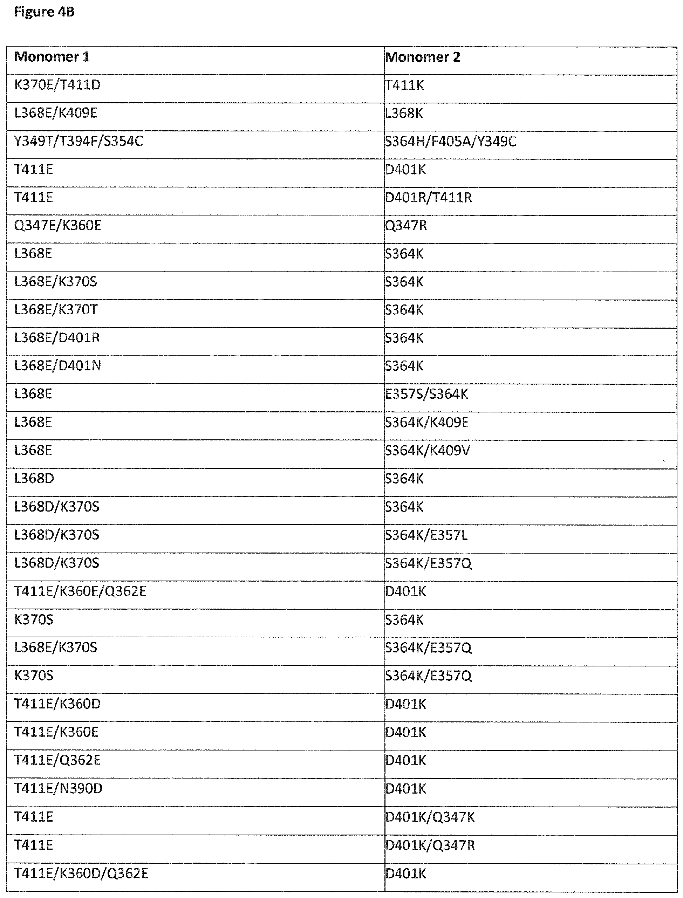

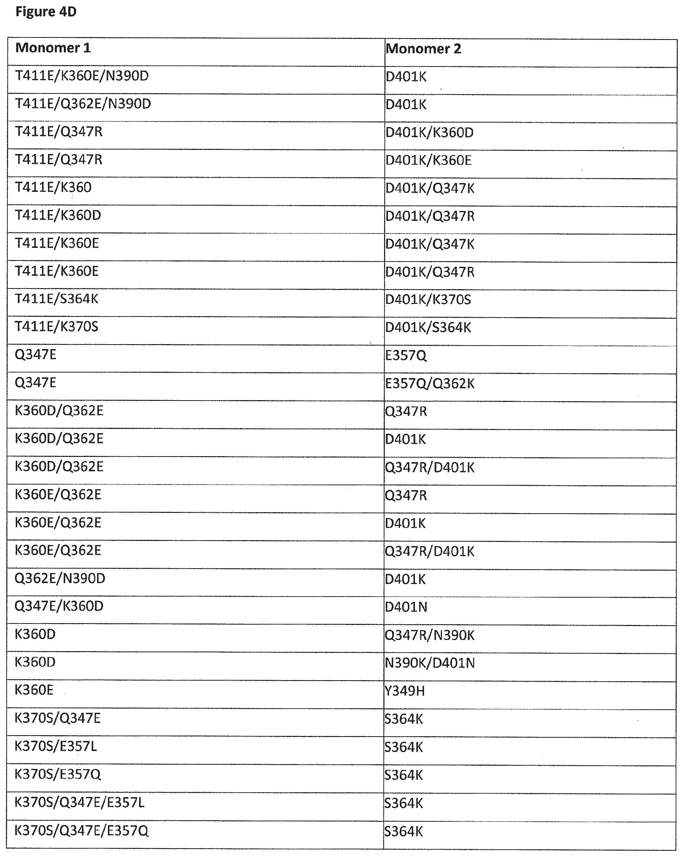

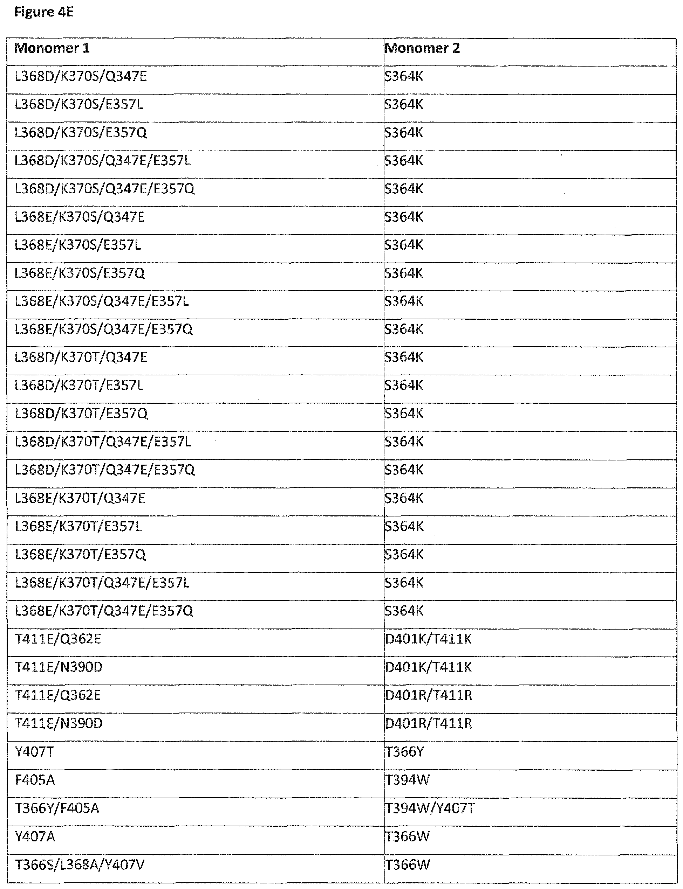

The present disclosure provides heterodimeric proteins, including heterodimeric antibodies in a variety of formats, which utilize heterodimeric variants to allow for heterodimeric formation and/or purification away from homodimers. A number of heterodimerization variants are shown in Figure 4.

There are a number of suitable pairs of sets of heterodimerization skew variants. These variants come in "pairs" of "sets". That is, one set of the pair is incorporated into the first monomer and the other set of the pair is incorporated into the second monomer. It should be noted that these sets do not necessarily behave as "knobs in holes" variants, with a one-to-one correspondence between a residue on one monomer and a residue on the other; that is, these pairs of sets form an interface between the two monomers that encourages heterodimer formation and discourages homodimer formation, allowing the percentage of heterodimers that spontaneously form under biological conditions to be over 90%, rather than the expected 50% (25 % homodimer A/A:50% heterodimer A/B:25% homodimer B/B).

### B. Steric Variants

In some examples, the formation of heterodimers can be facilitated by the addition of steric variants. That is, by changing amino acids in each heavy chain, different heavy chains are more likely to associate to form the heterodimeric structure than to form homodimers with the same Fc amino acid sequences. Suitable steric variants are included in in the Figures.

One mechanism is generally referred to in the art as "knobs and holes", referring to amino acid engineering that creates steric influences to favor heterodimeric formation and disfavor homodimeric formation can also optionally be used; this is sometimes referred to as "knobs and holes", as described in Ridgway et al., Protein Engineering 9(7):617 (1996); Atwell et al., J. Mol. Biol. 1997 270:26; US Patent No. 8,216,805. The Figures identify a number of "monomer A - monomer B" pairs that rely on "knobs and holes". In addition, as described in Merchant et al., Nature Biotech. 16:677 (1998), these "knobs and hole" mutations can be combined with disulfide bonds to skew formation to heterodimerization.

An additional mechanism that finds use in the generation of heterodimers is sometimes referred to as "electrostatic steering" as described in Gunasekaran et al., J. Biol. Chem. 285(25):19637 (2010). This is sometimes referred to herein as "charge pairs". In this example, electrostatics may be used to skew the formation towards heterodimerization. As those in the art will appreciate, these may also have have an effect on pI, and thus on purification, and thus could in some cases also be considered pI variants. However, as these were generated to force heterodimerization and were not used as purification tools, they are classified as "steric variants". These include, but are not limited to, D221E/P228E/L368E paired with D221R/P228R/K409R (e.g. these are "monomer corresponding sets) and C220E/P228E/368E paired with C220R/E224R/P228R/K409R.

Additional monomer A and monomer B variants that can be combined with other variants, optionally and independently in any amount, such as pI variants outlined herein or other steric variants that are shown in Figure 37 of US 2012/0149876, the figure and legend and SEQ ID NOs.

In some examples, the steric variants outlined herein can be optionally and independently incorporated with any pI variant (or other variants such as Fc variants, FcRn variants, etc.) into one or both monomers, and can be independently and optionally included or excluded from the proteins disclosed herein.

A list of suitable skew variants is found in Figure 4 showing some pairs of particular utility in many examples. Of particular use in many examples are the pairs of sets including, but not limited to, S364K/E357Q : L368D/K370S; L368D/K370S : S364K; L368E/K370S : S364K; T411T/E360E/Q362E : D401K; L368D/K370S : S364K/E357L, K370S : S364K/E357Q and T366S/L368A/Y407V : T366W (optionally including a bridging disulfide, T366S/L368A/Y407V/Y349C : T366W/S354C). In terms of nomenclature, the pair "S364K/E357Q: L368D/K370S" means that one of the monomers has the double variant set S364K/E357Q and the other has the double variant set L368D/K370S; as above, the "strandedness" of these pairs depends on the starting pI.

### C. pI (Isoelectric point) Variants for Heterodimers

In general, as will be appreciated by those in the art, there are two general categories of pI variants: those that increase the pI of the protein (basic changes) and those that decrease the pI of the protein (acidic changes). As described herein, all combinations of these variants can be done: one monomer may be wild type, or a variant that does not display a significantly different pI from wild-type, and the other can be either more basic or more acidic. Alternatively, each monomer is changed, one to more basic and one to more acidic.

Preferred combinations of pI variants are shown in Figure 5. As outlined herein and shown in the figures, these changes are shown relative to IgG1, but all isotypes can be altered this way, as well as isotype hybrids. In the case where the heavy chain constant domain is from IgG2-4, R133E and R133Q can also be used.

In one example, for example in the bottle opener format, a preferred combination of pI variants has one monomer (the negative Fab side) comprising 208D/295E/384D/418E/421D variants (N208D/Q295E/N384D/Q418E/N421D when relative to human IgG1) and a second monomer (the positive scFv side) comprising a positively charged scFv linker, including (GKPGS)₄. However, as will be appreciated by those in the art, the first monomer includes a CH1 domain, including position 208. Accordingly, in constructs that do not include a CH1 domain (for example for heterodimeric Fc fusion proteins that do not utilize a CH1 domain on one of the domains, for example in a dual scFv format), a preferred negative pI variant Fc set includes 295E/384D/418E/421D variants (Q295E/N384D/Q418E/N421D when relative to human IgG1).

Accordingly, in some examples, one monomer has a set of substitutions from Figure 5 and the other monomer has a charged linker (either in the form of a charged scFv linker because that monomer comprises an scFv or a charged domain linker, as the format dictates).

### 1. Isotypic Variants

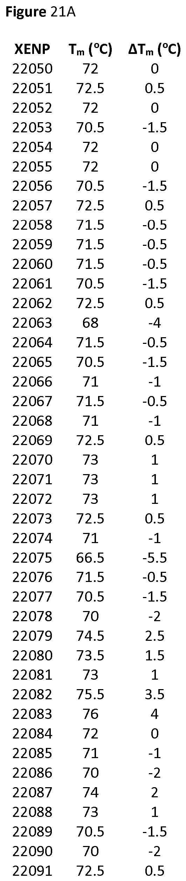

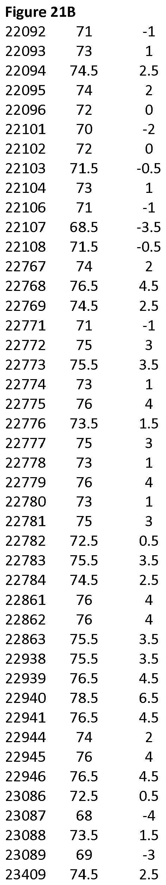

In addition, many examples of the disclosure rely on the "importation" of pI amino acids at particular positions from one IgG isotype into another, thus reducing or eliminating the possibility of unwanted immunogenicity being introduced into the variants. A number of these are shown in Figure 21 of US Publ. 2014/0370013. That is, IgG1 is a common isotype for therapeutic antibodies for a variety of reasons, including high effector function. However, the heavy constant region of IgG1 has a higher pI than that of IgG2 (8.10 versus 7.31). By introducing IgG2 residues at particular positions into the IgG1 backbone, the pI of the resulting monomer is lowered (or increased) and additionally exhibits longer serum half-life. For example, IgG1 has a glycine (pI 5.97) at position 137, and IgG2 has a glutamic acid (pI 3.22); importing the glutamic acid will affect the pI of the resulting protein. As is described below, a number of amino acid substitutions are generally required to significant affect the pI of the variant antibody. However, it should be noted as discussed below that even changes in IgG2 molecules allow for increased serum half-life.

In other examples, non-isotypic amino acid changes may be made, either to reduce the overall charge state of the resulting protein (e.g. by changing a higher pI amino acid to a lower pI amino acid), or to allow accommodations in structure for stability, etc. as is more further described below.

In addition, by pI engineering both the heavy and light constant domains, significant changes in each monomer of the heterodimer can be seen. As discussed herein, having the pIs of the two monomers differ by at least 0.5 can allow separation by ion exchange chromatography or isoelectric focusing, or other methods sensitive to isoelectric point.

### D. Calculating pI

The pI of each monomer can depend on the pI of the variant heavy chain constant domain and the pI of the total monomer, including the variant heavy chain constant domain and the fusion partner. Thus, in some examples, the change in pI may be calculated on the basis of the variant heavy chain constant domain, using the chart in the Figure 19 of US Pub. 2014/0370013. As discussed herein, which monomer to engineer is generally decided by the inherent pI of the Fv and scaffold regions. Alternatively, the pI of each monomer can be compared.

### E. pI Variants that also confer better FcRn in vivo binding

In the case where the pI variant decreases the pI of the monomer, they can have the added benefit of improving serum retention in vivo.

Although still under examination, Fc regions are believed to have longer half-lives in vivo, because binding to FcRn at pH 6 in an endosome sequesters the Fc (Ghetie and Ward, 1997 Immunol Today. 18(12): 592-598). The endosomal compartment then recycles the Fc to the cell surface. Once the compartment opens to the extracellular space, the higher pH, ~7.4, induces the release of Fc back into the blood. In mice, Dall' Acqua et al. showed that Fc mutants with increased FcRn binding at pH 6 and pH 7.4 actually had reduced serum concentrations and the same half life as wild-type Fc (Dall' Acqua et al. 2002, J. Immunol. 169:5171-5180). The increased affinity of Fc for FcRn at pH 7.4 is thought to forbid the release of the Fc back into the blood. Therefore, the Fc mutations that will increase Fc's half-life in vivo will ideally increase FcRn binding at the lower pH while still allowing release of Fc at higher pH. The amino acid histidine changes its charge state in the pH range of 6.0 to 7.4. Therefore, it is not surprising to find His residues at important positions in the Fc/FcRn complex.

Recently it has been suggested that antibodies with variable regions that have lower isoelectric points may also have longer serum half-lives (Igawa et al., 2010 PEDS. 23(5): 385-392). However, the mechanism of this is still poorly understood. Moreover, variable regions differ from antibody to antibody. Constant region variants with reduced pI and extended half-life would provide a more modular approach to improving the pharmacokinetic properties of antibodies, as described herein.

### F. Additional Fc Variants for Additional Functionality

In addition to pI amino acid variants, there are a number of useful Fc amino acid modification that can be made for a variety of reasons, including, but not limited to, altering binding to one or more FcyR receptors, altered binding to FcRn receptors, etc.

Accordingly, the proteins of the disclosure can include amino acid modifications, including the heterodimerization variants outlined herein, which includes the pI variants and steric variants. Each set of variants can be independently and optionally included or excluded from any particular heterodimeric protein.

### G. FcyR Variants

Accordingly, there are a number of useful Fc substitutions that can be made to alter binding to one or more of the FcyR receptors. Substitutions that result in increased binding as well as decreased binding can be useful. For example, it is known that increased binding to FcyRIIIa results in increased ADCC (antibody dependent cell-mediated cytotoxicity; the cell-mediated reaction wherein nonspecific cytotoxic cells that express FcyRs recognize bound antibody on a target cell and subsequently cause lysis of the target cell). Similarly, decreased binding to FcyRIIb (an inhibitory receptor) can be beneficial as well in some circumstances. Particular variants that find use include, but are not limited to, 236A, 239D, 239E, 332E, 332D, 239D/332E, 267D, 267E, 328F, 267E/328F, 236A/332E, 239D/332E/330Y, 239D, 332E/330L, 243A, 243L, 264A, 264V and 299T.

In addition, there are additional Fc substitutions that find use in increased binding to the FcRn receptor and increased serum half life, for example, but not limited to, 434S, 434A, 428L, 308F, 259I, 428L/434S, 259I/308F, 436I/428L, 436I or V/434S, 436V/428L and 259I/308F/428L.

### H. Ablation Variants

Similarly, another category of functional variants are "FcγR ablation variants" or "Fc knock out (FcKO or KO)" variants. In these examples, for some therapeutic applications, it may be desirable to reduce or remove the normal binding of the Fc domain to one or more or all of the Fcγ receptors (e.g. FcyR1, FcγRIIa, FcγRIIb, FcγRIIIa, etc.) to avoid additional mechanisms of action. That is, for example, in many examples, particularly in the use of bispecific immunomodulatory antibodies desirable to ablate FcγRIIIa binding to eliminate or significantly reduce ADCC activity such that one of the Fc domains comprises one or more Fcy receptor ablation variants. These ablation variants are depicted in Figure 6, and each can be independently and optionally included or excluded, with preferred aspects utilizing ablation variants selected from the group consisting of G236R/L328R, E233P/L234V/L235A/G236del/S239K, E233P/L234V/L235A/G236del/S267K, E233P/L234V/L235A/G236del/S239K/A327G, E233P/L234V/L235A/G236del/S267K/A327G and E233P/L234V/L235A/G236del. It should be noted that the ablation variants referenced herein ablate FcyR binding but generally not FcRn binding.

### I. Combination of Heterodimeric and Fc Variants

As will be appreciated by those in the art, all of the recited heterodimerization variants (including skew and/or pI variants) can be optionally and independently combined in any way, as long as they retain their "strandedness" or "monomer partition". In addition, all of these variants can be combined into any of the heterodimerization formats.

In the case of pI variants, while examples finding particular use are shown in the Figures, other combinations can be generated, following the basic rule of altering the pI difference between two monomers to facilitate purification.

In addition, any of the heterodimerization variants, skew and pI, are also independently and optionally combined with Fc ablation variants, Fc variants, FcRn variants, as generally outlined herein.

### VII. Useful Formats

As will be appreciated by those in the art and discussed more fully below, the bispecific heterodimeric antibodies disclosed herein can take on a wide variety of configurations, as are generally depicted in Figure 2. Some figures depict "single ended" configurations, where there is one type of specificity on one "arm" of the molecule and a different specificity on the other "arm". Other figures depict "dual ended" configurations, where there is at least one type of specificity at the "top" of the molecule and one or more different specificities at the "bottom" of the molecule. Thus, the present disclosure is directed to novel immunoglobulin compositions that co-engage a different first and a second antigen.

As will be appreciated by those in the art, the heterodimeric formats (see Figure 2) disclosed herein can have different valencies as well as be bispecific. That is, antibodies disclosed herein can be bivalent and bispecific, wherein a checkpoint target is bound by one ABD and the costimulatory target is bound by a second ABD (see for example the bottle opener format which is heterodimeric) or the bispecific mAb which is homodimeric). The heterodimeric antibodies can also be trivalent and bispecific, wherein the first antigen is bound by two ABDs and the second antigen by a second ABD (see for example the Central-scFv format and the trident format). The heterodimeric antibodies can also be bispecific and tetravalent (such as the Central scFv2 format and the DVD-Ig format).

Again, with the exception of the DVD-Ig format and the central-scFv2 format, the antibodies are generally formatted such that the co-stimulatory target is bound monovalently.

### A. Bottle opener format

The heterodimeric scaffold that finds particular use in the present invention is the "triple F" or "bottle opener" scaffold format. In this embodiment, one heavy chain of the antibody contains a single chain Fv ("scFv", as defined below) and the other heavy chain is a "regular" Fab format, comprising a variable heavy chain and a light chain. This structure is sometimes referred to herein as "triple F" format (scFv-Fab-Fc) or the "bottle-opener" format, due to a rough visual similarity to a bottle-opener. The two chains are brought together by the use of amino acid variants in the constant regions (e.g. the Fc domain, the CH1 domain and/or the hinge region) that promote the formation of heterodimeric antibodies as is described more fully below.

There are several distinct advantages to the present "triple F" format. As is known in the art, antibody analogs relying on two scFv constructs often have stability and aggregation problems, which can be alleviated in the present invention by the addition of a "regular" heavy and light chain pairing. In addition, as opposed to formats that rely on two heavy chains and two light chains, there is no issue with the incorrect pairing of heavy and light chains (e.g. heavy 1 pairing with light 2, etc.).

Many of the embodiments outlined herein rely in general on the bottle opener format that comprises a first monomer comprising an scFv, comprising a variable heavy and a variable light domain, covalently attached using an scFv linker (charged, in many but not all instances), where the scFv is covalently attached to the N-terminus of a first Fc domain usually through a domain linker (which, as outlined herein can either be un-charged or charged and can be exogeneous or endogeneous (e.g. all or part of the native hinge domain). The second monomer of the bottle opener format is a heavy chain, and the composition further comprises a light chain.

In addition, the Fc domains of the bottle opener format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the bottle opener format includes skew variants, pI variants, and ablation variants. Accordingly, some examples include bottle opener formats that comprise: a) a first monomer (the "scFv monomer") that comprises a charged scFv linker (with the +H sequence of Figure 8 being preferred in some embodiments), the skew variants S364K/E357Q, the ablation variants E233P/L234V/L235A/G236del/S267K, and an Fv that binds to one target as outlined herein; b) a second monomer (the "Fab monomer") that comprises the skew variants L368D/K370S, the pI variants N208D/Q295E/N384D/Q418E/N421D, the ablation variants E233P/L234V/L235A/ G236del/S267K, and a variable heavy domain that, with the variable light domain, makes up an Fv that binds to a second target as outlined herein; and c) a light chain. In this particular example, suitable monomer Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

Accordingly, some examples include bottle opener formats that comprise: a) a first monomer (the "scFv monomer") that comprises a charged scFv linker (with the +H sequence of Figure 8 being preferred in some examples), the skew variants S364K/E357Q, the ablation variants E233P/L234V/L235A/G236del/S267K, and an Fv that binds to one target as outlined herein; b) a second monomer (the "Fab monomer") that comprises the skew variants L368D/K370S, the pI variants N208D/Q295E/N384D/Q418E/N421D, the ablation variants E233P/L234V/L235A/ G236del/S267K, and a variable heavy domain that, with the variable light domain, makes up an Fv that binds to a second target as outlined herein; and c) a light chain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the bottle opener format includes skew variants, pI variants, ablation variants and FcRn variants. Accordingly, some examples include bottle opener formats that comprise: a) a first monomer (the "scFv monomer") that comprises a charged scFv linker (with the +H sequence of Figure 8 being preferred in some examples), the skew variants S364K/E357Q, the ablation variants E233P/L234V/L235A/G236del/S267K, the FcRn variants M428L/N434S and an Fv that binds to a first receptor (either a costimulatory or checkpoint receptor) as outlined herein; b) a second monomer (the "Fab monomer") that comprises the skew variants L368D/K370S, the pI variants N208D/Q295E/N384D/ Q418E/N421D, the ablation variants E233P/L234V/L235A/G236del/S267K, the FcRn variants M428L/N434S and a variable heavy domain that, with the variable light domain, makes up an Fv that binds to a second receptor as outlined herein (the other of the costimulatory or checkpoint receptor); and c) a light chain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

Specifically, Figure 9 shows some bottle opener "backbone" sequences that are missing the Fv sequences that can be used herein. That is, Fv sequences for the scFv portion and the Fab portion can be used from any combination of ICOS and PD-1, ICOS and CTLA-4, ICOS and LAG-3, ICOS and TIM-3, ICOS and PD-L1, ICOS and BTLA, ICOS and TIGIT, GITR and TIGIT, GITR and PD-1, GITR and CTLA-4, GITR and LAG-3, GITR and TIM-3, GITR and PD-L1, GITR and BTLA, OX40 and PD-1, OX40 and TIGIT, OX40 and CTLA-4, IC OX40 OS and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1, TIGIT and 4-1BB and 4-1BB and BTLA, in combination with any or all of backbones 1 to 10, with backbone 1 of particular use in these combinations.

For bottle opener backbone 1 from Figure 9 (optionally including the 428L/434S variants) specific Fv combinations of use in the present disclosure include ICOS and PD-1, ICOS and PD-L1 and ICOS and CTLA-4.

For bottle opener backbone 1 from Figure 9 (optionally including the 428L/434S variants) specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

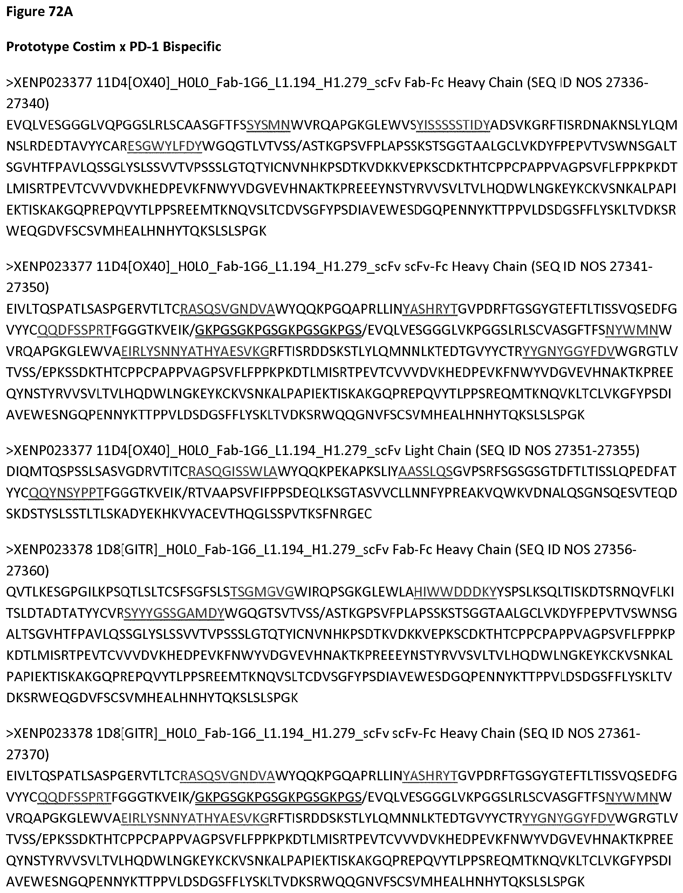

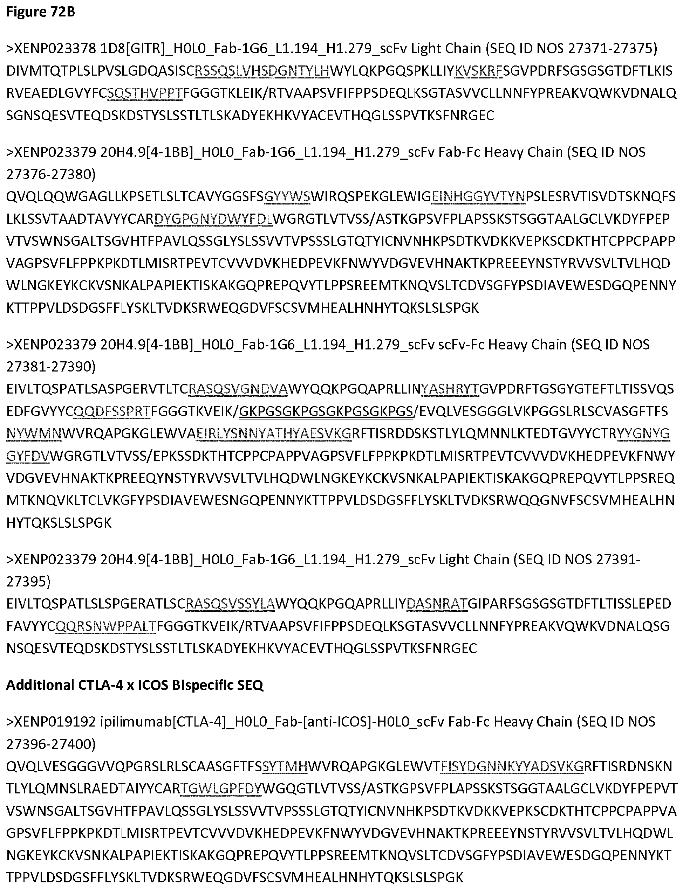

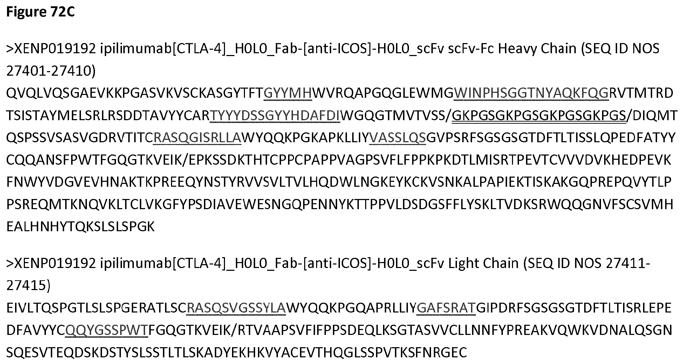

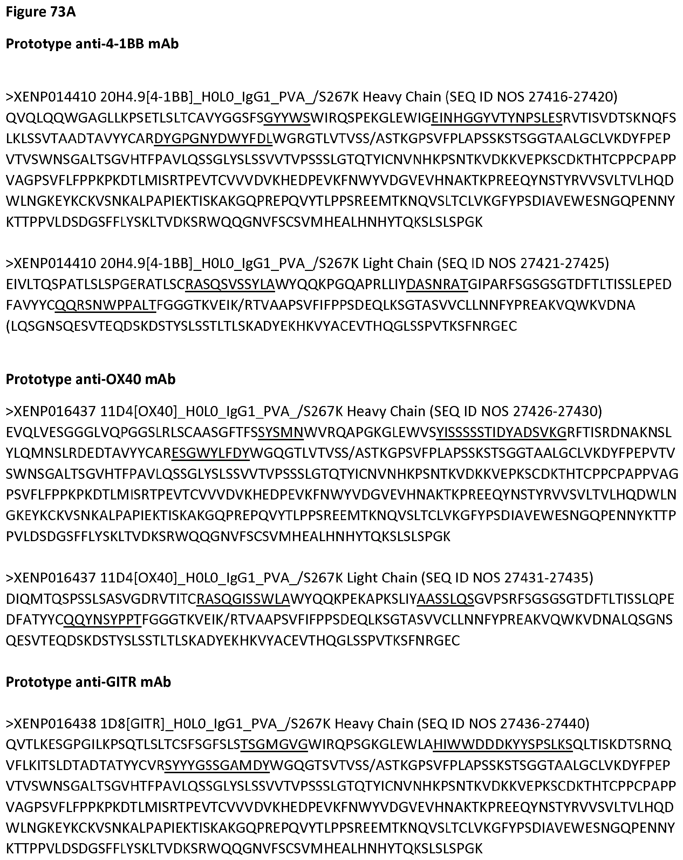

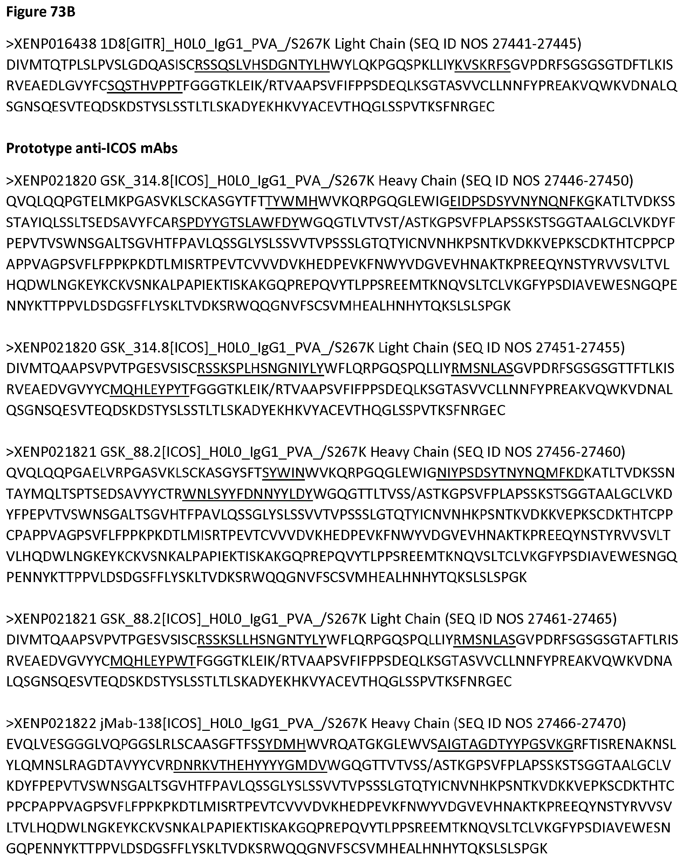

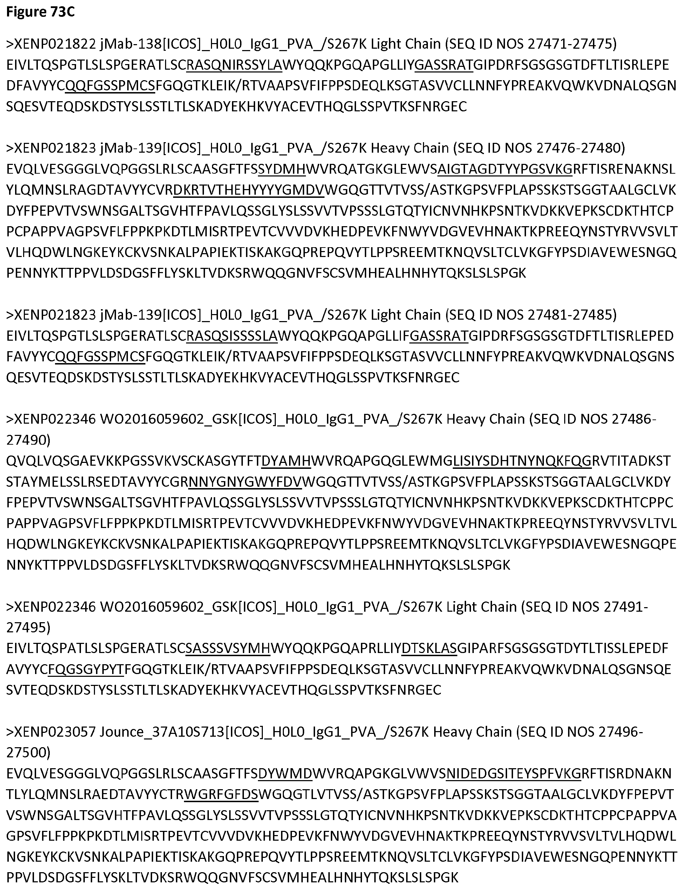

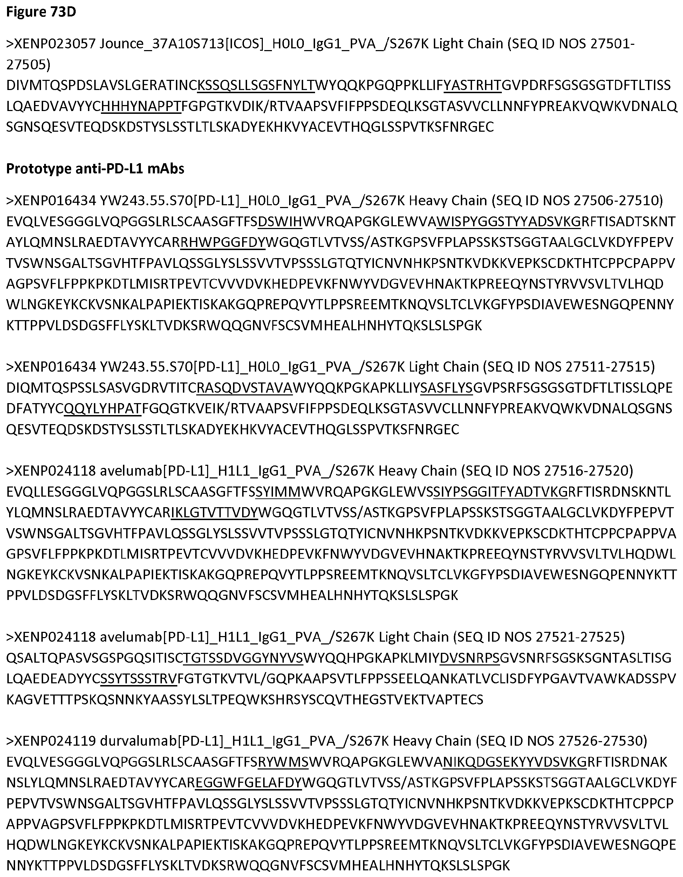

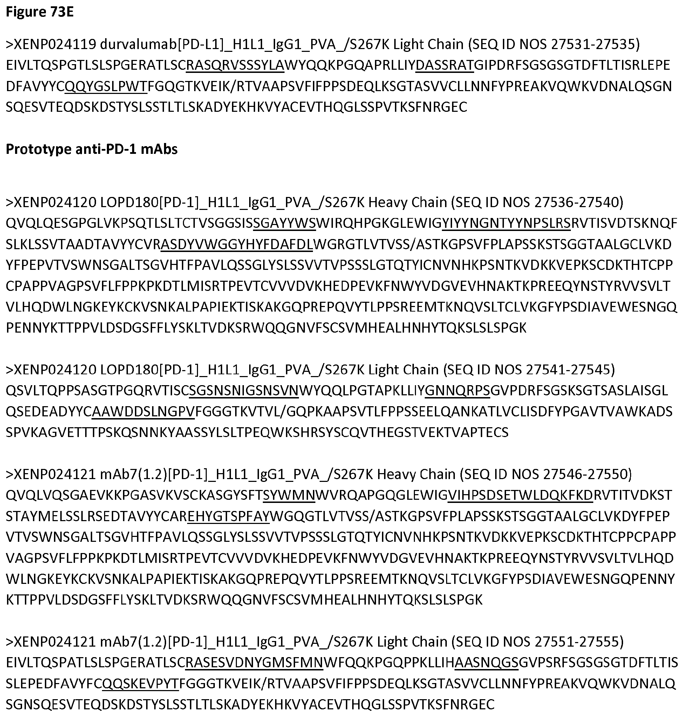

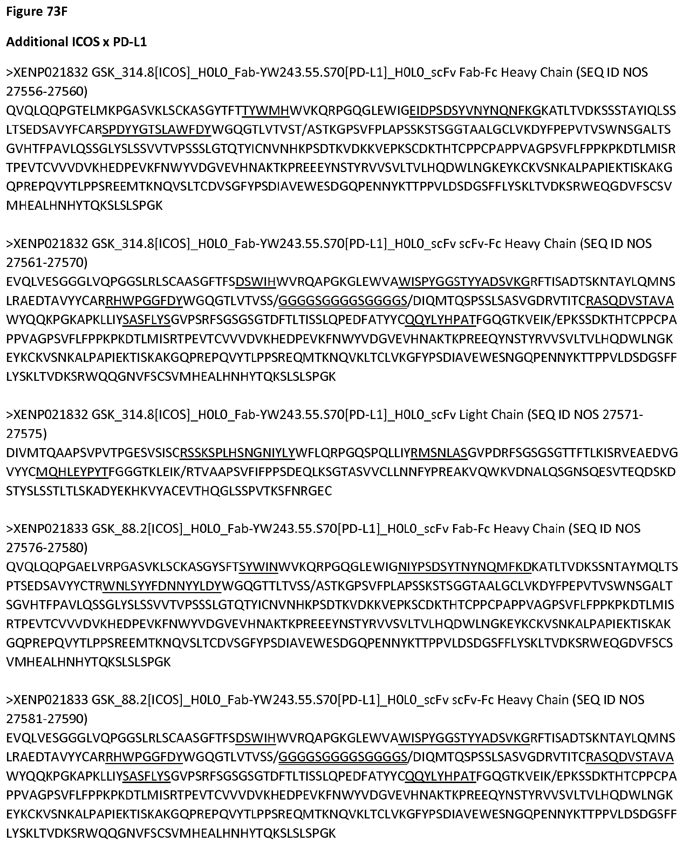

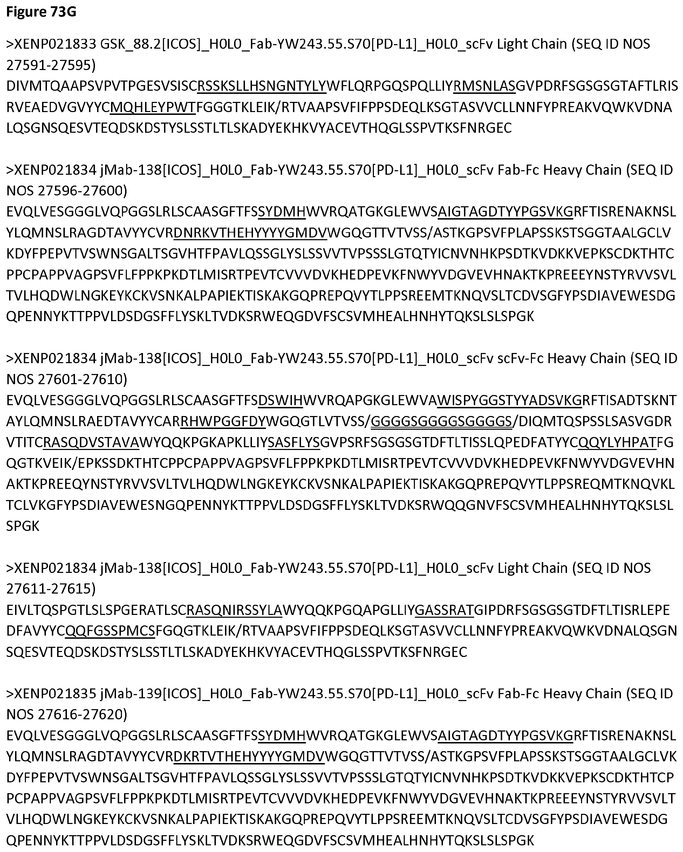

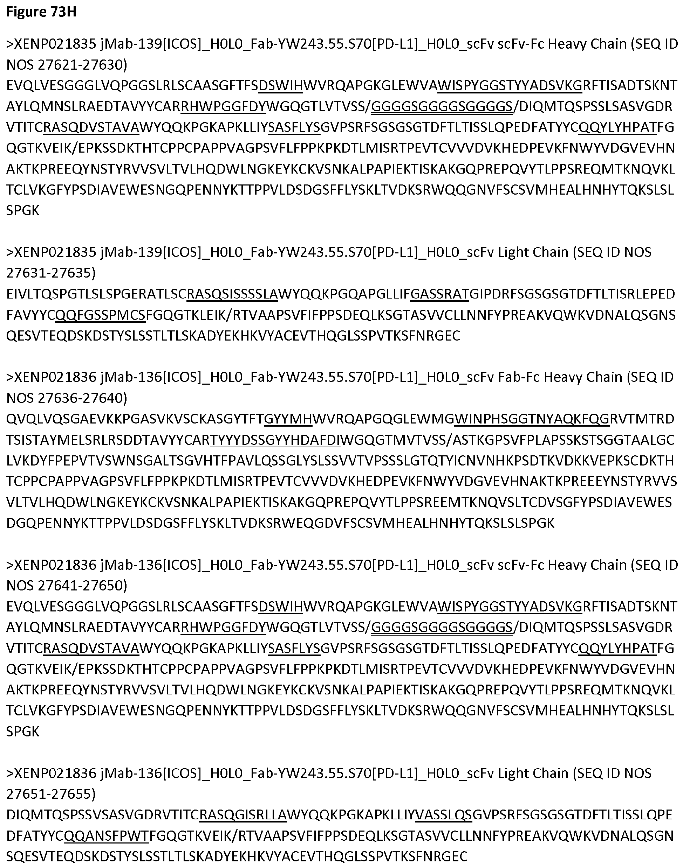

For bottle opener backbone 1 from Figure 9 (optionally including the 428L/434S variants), specific ABDs that bind human GITR include, but are not limited to, those in Figure 18, Figure 72 and Figure 73 and those listed in SEQ ID NO:26282-26290.

For bottle opener backbone 1 from Figure 9 (optionally including the 428L/434S variants), specific ABDs that bind OX40 include, but are not limited to, Figure 17, Figure 72 and Figure 73 and those listed in SEQ ID NO: 26272-26281.

For bottle opener backbone 1 from Figure 9 (optionally including the 428L/434S variants), specific ABDs that bind human 4-1BB include, but are not limited to, Figure 16, Figure 72 and Figure 73 and SEQ ID NO: 26262-2671.

For bottle opener backbone 1 from Figure 9 (optionally including the 428L/434S variants), specific ABDs that bind human PD-L1 include, but are not limited to, Figure 15A-15C, Figure 73 and Figure 78 and SEQ ID NO: 3961-4432.

Specific bottle opener examples are outlined below.

### B. mAb-Fv format

One heterodimeric scaffold that finds particular use in the present disclosure is the mAb-Fv format. In this example, the format relies on the use of a C-terminal attachment of an "extra" variable heavy domain to one monomer and the C-terminal attachment of an "extra" variable light domain to the other monomer, thus forming a third antigen binding domain (i.e. an "extra" Fv domain), wherein the Fab portions of the two monomers bind one checkpoint target and the "extra" Fv domain binds a costimulatory target.

In this example, the first monomer comprises a first heavy chain, comprising a first variable heavy domain and a first constant heavy domain comprising a first Fc domain, with a first variable light domain covalently attached to the C-terminus of the first Fc domain using a domain linker (vh1-CH1-hinge-CH2-CH3-[optional linker]-v12). The second monomer comprises a second variable heavy domain, a second constant heavy domain comprising a second Fc domain, and a third variable heavy domain covalently attached to the C-terminus of the second Fc domain using a domain linker (vh1-CH1-hinge-CH2-CH3-[optional linker]-vh2. This example further utilizes a common light chain comprising a variable light domain and a constant light domain, which associates with the heavy chains to form two identical Fabs that include two identical Fvs. The two C-terminally attached variable domains make up the "extra" third Fv. As for many of the examples herein, these constructs include skew variants, pI variants, ablation variants, additional Fc variants, etc. as desired and described herein. In this example, suitable Fv pairs include (Fabs listed first, "extra" Fv listed second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the mAb-Fv format comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6, optionally charged scFv linkers (including those shown in Figure 8) and the heavy chain comprises pI variants (including those shown in Figure 5).

In some examples, the mAb-Fv format includes skew variants, pI variants, and ablation variants. Accordingly, some examples include mAb-Fv formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the first receptor (either a costimulatory receptor or a checkpoint receptor) as outlined herein, and a second variable light chain, that together with the second variable heavy chain forms an Fv (ABD) that binds a second receptor (e.g. the other of the costimulatory or checkpoint receptor; and c) a light chain comprising a first variable light domain and a constant light domain. Of particular use in some examples in this format, are (Fab-scFv order) ICOS X PD-1, PD-1 X ICOS, PD-L1 X ICOS, ICOS x PD-L1, GITR X PD-1, OX40 X PD-1 and 4-1BB X PD-1. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the mAb-Fv format includes skew variants, pI variants, ablation variants and FcRn variants. Accordingly, some examples include mAb-Fv formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein, and a second variable light chain, that together with the second variable heavy chain forms an Fv (ABD) that binds a second checkpoint inhibitors; and c) a light chain comprising a first variable light domain and a constant light domain. Of particular use in some examples in this format, are (Fab-scFv order) ICOS X PD-1, ICOS x PD-L1, GITR X PD-1, OX40 X PD-1 and 4-1BB X PD-1.

For mAb-Fv sequences that are similar to the mAb-scFv backbone 1 (optionally including M428L/N434S) from Figure 75, specific ABDs that bind human ICOS are [ICOS]_H0L0 and [ICOS]_H0.66_L0, as well as those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

For mAb-Fv sequences that are similar to the mAb-scFv backbone 1 (optionally including M428L/N434S) from Figure 75, specific ABDs that bind human PD-L1 are shown in Figure 15A-15C, Figure 73 and Figure 78 and SEQ ID NO: 3961-4432.

For mAb-Fv sequences that are similar to the mAb-scFv backbone 1 (optionally including M428L/N434S) fromFigure 75, specific ABDs that bind human GITR are those in Figure 18, Figure 72 and Figure 73 and those listed in SEQ ID NO:26282-26290.

For mAb-Fv sequences that are similar to the mAb-scFv backbone 1 (optionally including M428L/N434S) fromFigure 75, specific ABDs that bind human 4-1BB are those in Figure 16, Figure 72 and Figure 73 and SEQ ID NO: 26262-2671.

For mAb-Fv sequences that are similar to the mAb-scFv backbone 1 (optionally including M428L/N434S) fromfigure 75, specific ABDs that bind human OX40 are those in Figure 17, Figure 72 and Figure 73 and those listed in SEQ ID NO: 26272-26281.

For mAb-Fv sequences that are similar to the mAb-scFv backbone 1 (optionally including M428L/N434S) fromfigure 75, specific ABDs that bind human PD-L1 from Figure 15A-15C, Figure 73 and Figure 78 and SEQ ID NO: 3961-4432.

### C. mAb-scFv

One heterodimeric scaffold that finds particular use herein is the mAb-scFv format. In this example, the format relies on the use of a C-terminal attachment of an scFv to one of the monomers, thus forming a third antigen binding domain, wherein the Fab portions of the two monomers bind one receptor target and the "extra" scFv domain binds the other receptor target (generally the monovalently bound costimulatory receptor).

In this example, the first monomer comprises a first heavy chain (comprising a variable heavy domain and a constant domain), with a C-terminally covalently attached scFv comprising a scFv variable light domain, an scFv linker and a scFv variable heavy domain in either orientation (vh1-CH1-hinge-CH2-CH3-[optional linker]-vh2-scFv linker-vl2 or vh1-CH1-hinge-CH2-CH3-[optional linker]-vl2-scFv linker-vh2). This example further utilizes a common light chain comprising a variable light domain and a constant light domain, which associates with the heavy chains to form two identical Fabs that bind one of the target receptors. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the mAb-scFv format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the mAb-scFv format includes skew variants, pI variants, and ablation variants. Accordingly, some examples include bottle opener formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first receptor, and a scFv that binds to the second receptor; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the first receptor as outlined herein; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the mAb-scFv format includes skew variants, pI variants, ablation variants and FcRn variants. Accordingly, some examples include mAb-scFv formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first receptor, and a scFv that binds to the second receptor; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein, and c) a light chain comprising a first variable light domain and a constant light domain.

In mAb-scFv backbone 1 (optionally including M428L/N434S) from Figure 75, specific ABDs that bind human ICOS are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In mAb-scFv backbone 1 (optionally including M428L/N434S) from Figure 75, specific ABDs that bind human GITR are those in Figure 18, Figure 72 and Figure 73 and those listed in SEQ ID NO:26282-26290.

In mAb-scFv backbone 1 (optionally including M428L/N434S) from Figure 75, specific ABDs that bind human 4-1BB include those in Figure 16, Figure 72 and Figure 73 and SEQ ID NO: 26262-2671.

In mAb-scFv backbone 1 (optionally including M428L/N434S) from Figure 75, specific ABDs that bind human OX-40 Figure 17, Figure 72 and Figure 73 and those listed in SEQ ID NO: 26272-26281.

In mAb-scFv backbone 1 (optionally including M428L/N434S) from Figure 75, specific ABDs that bind human PD-L1 include Figure 15A-15C, Figure 73 and Figure 78 and SEQ ID NO: 3961-4432.

### D. Central scFv

One heterodimeric scaffold that finds particular use in the present disclosure is the Central-scFv format. In this example, the format relies on the use of an inserted scFv domain thus forming a third antigen binding domain, wherein the Fab portions of the two monomers bind one receptor target and the "extra" scFv domain binds another (again, generally the costimulatory receptor is bound monovalently). The scFv domain is inserted between the Fc domain and the CH1-Fv region of one of the monomers, thus providing a third antigen binding domain.

In this example, one monomer comprises a first heavy chain comprising a first variable heavy domain, a CH1 domain (and optional hinge) and Fc domain, with a scFv comprising a scFv variable light domain, an scFv linker and a scFv variable heavy domain. The scFv is covalently attached between the C-terminus of the CH1 domain of the heavy constant domain and the N-terminus of the first Fc domain using optional domain linkers (vh1-CH1-[optional linker]-vh2-scFv linker-vl2-[optional linker including the hinge]-CH2-CH3, or the opposite orientation for the scFv, vh1-CH1-[optional linker]-vl2-scFv linker-vh2-[optional linker including the hinge]-CH2-CH3). In some examples, the optional linker is a hinge or fragment thereof. The other monomer is a standard Fab side (e.g. vh1-CH1-hinge-CH2-CH3). This example further utilizes a common light chain comprising a variable light domain and a constant light domain, which associates with the heavy chains to form two identical Fabs that bind a checkpoint inhibitor. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the central scFv format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the central scFv format includes skew variants, pI variants, and ablation variants. Accordingly, some examples include central scFv formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first receptor; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the second receptor as outlined herein; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the central scFv format includes skew variants, pI variants, ablation variants and FcRn variants. Accordingly, some examples include central-scFv formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first receptor and an scFv domain that binds to a second receptor; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein; and c) a light chain comprising a first variable light domain and a constant light domain. In this example, suitable Fv pairs include (Fabs listed first, scFvs second) ICOS X PD-1, PD-1 X ICOS, ICOS X PD-L1, PD-L1 X ICOS, ICOS X CTLA-4 and CTLA-4 X ICOS.

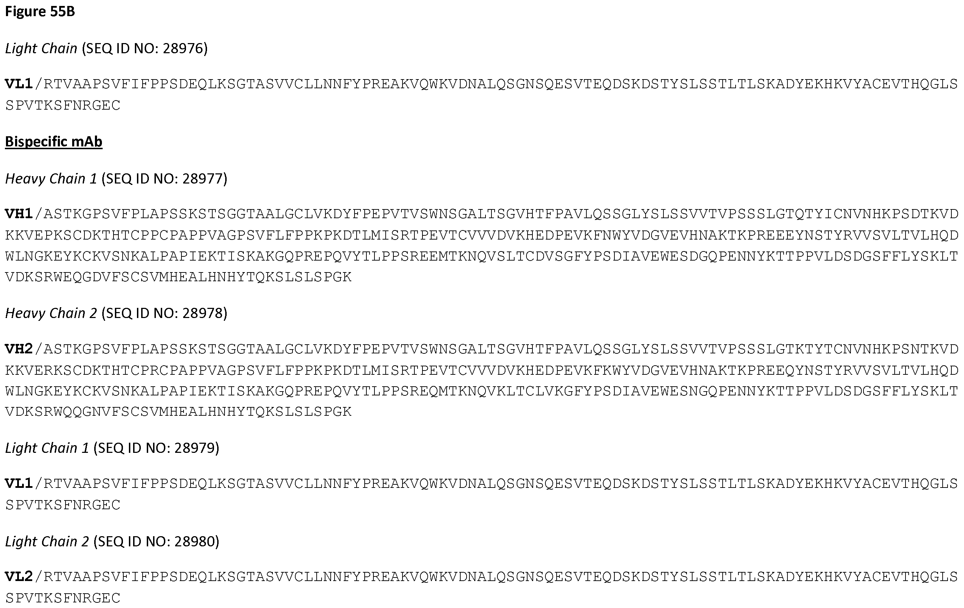

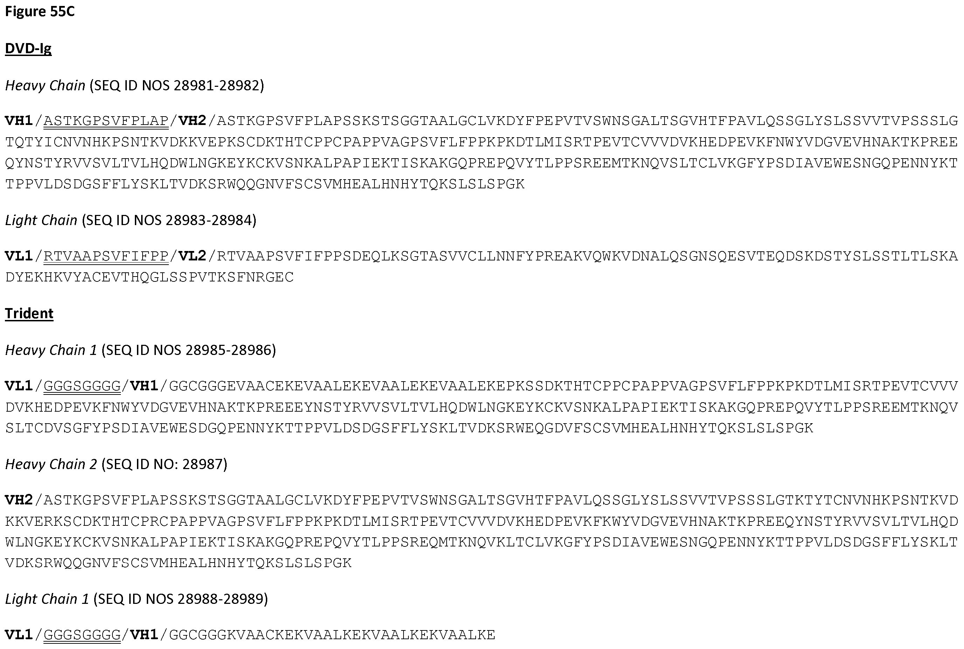

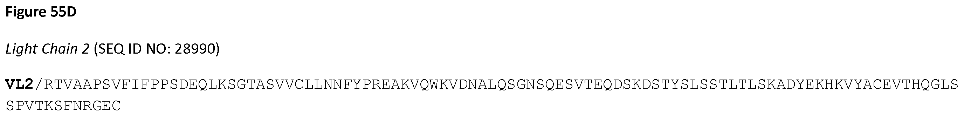

For central-scFv sequences that utilize the central-scFv sequences of Figure 55 suitable Fvs that bind ICOS include, but are not limited to, shown in Figure 19, Figure 20A- 20G, Figure 24, Figure 68A-68G and Figure 77A-77B as well as SEQ ID NO: 27869-28086, 28087-28269, 27193-27335, 28549-28556 and 28557-28665.

For central-scFv sequences that utilize the central-scFv sequences of Figure 55 suitable Fvs that bind PD-L1 include, but are not limited to, those shown in Figure 15A-15C, Figure 73 and Figure 78 and SEQ ID NO: 3961-4432.

For central-scFv sequences that utilize the central-scFv sequences of Figure 55 suitable Fvs that bind GITR include, but are not limited to, those in Figure 18, Figure 72 and Figure 73 and those listed in SEQ ID NO:26282-26290.

For central-scFv sequences that utilize the central-scFv sequences of Figure 55 suitable Fvs that bind OX40 include, but are not limited to, Figure 17, Figure 72 and Figure 73 and those listed in SEQ ID NO: 26272-26281.

For central-scFv sequences that utilize the central-scFv sequences of Figure 55 suitable Fvs that bind 4-1BB include, but are not limited to, Figure 16, Figure 72 and Figure 73 and SEQ ID NO: 26262-2671.

### E. Central-scFv2

One heterodimeric scaffold that finds particular use herein is the Central-scFv2 format, which is bispecific and tetravalent. In this example, the format relies on the use of two inserted scFv domains thus forming third and fourth antigen binding domains, wherein the Fab portions of the two monomers bind one receptor target and the "extra" scFv domains bind another. The scFv domain is inserted between the Fc domain and the CH1-Fv region of the monomers.

In this example, both monomers comprise a first heavy chain comprising a first variable heavy domain, a CH1 domain (and optional hinge) and Fc domain, with a scFv comprising a scFv variable light domain, an scFv linker and a scFv variable heavy domain. The scFv is covalently attached between the C-terminus of the CH1 domain of the heavy constant domain and the N-terminus of the first Fc domain using optional domain linkers (vh1-CH1-[optional linker]-vh2-scFv linker-vl2-[optional linker including the hinge]-CH2-CH3, or the opposite orientation for the scFv, vh1-CH1-[optional linker]-vl2-scFv linker-vh2-[optional linker including the hinge]-CH2-CH3). In some embodiments, the optional linker is a hinge or fragment thereof. This example further utilizes a common light chain comprising a variable light domain and a constant light domain, which associates with the heavy chains to form two identical Fabs that bind a receptor. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the central scFv2 format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the central scFv2 format includes skew variants, pI variants, and ablation variants. Accordingly, some examples include central scFv formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first receptor; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the second receptor as outlined herein; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the central scFv2 format includes skew variants, pI variants, ablation variants and FcRn variants. Accordingly, some examples include central-scFv formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first receptor and an scFv domain that binds to a second receptor; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein; and c) a light chain comprising a first variable light domain and a constant light domain. In this example, suitable Fv pairs include (Fabs listed first, scFvs second) ICOS X PD-1, PD-1 X ICOS, ICOS X PD-L1, PD-L1 X ICOS, ICOS X CTLA-4 and CTLA-4 X ICOS.

For central-scFv2 sequences that utilize the central-scFv2 sequences of Figure 55 suitable Fvs that bind ICOS include, but are not limited to, shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

For central-scFv2 sequences that utilize the central-scFv2 sequences of Figure 55 suitable Fvs that bind PD-L1 include, but are not limited to, those shown in Figure 15A-15C, Figure 73 and Figure 78 and SEQ ID NO: 3961-4432.

For central-scFv2 sequences that utilize the central-scFv sequences of Figure 55 suitable Fvs that bind GITR include, but are not limited to, those in Figure 18, Figure 72 and Figure 73 and those listed in SEQ ID NO:26282-26290.

For central-scFv2 sequences that utilize the central-scFv sequences of Figure 55 suitable Fvs that bind OX40 include, but are not limited to, Figure 17, Figure 72 and Figure 73 and those listed in SEQ ID NO: 26272-26281.

For central-scFv2 sequences that utilize the central-scFv sequences of Figure 55 suitable Fvs that bind 4-1BB include, but are not limited to, Figure 16, Figure 72 and Figure 73 and SEQ ID NO: 26262-2671.

### F. One armed mAb

As noted above, surprisingly and unexpectedly, monovalent costimulatory antibodies comprising a single ABD to the target show efficacy in activating T cells.

Accordingly, in some examples, the disclosure provides monovalent, monospecific antibodies as shown in Figure 2N that comprise a heterodimeric Fc domain (for stability). In this example, one monomer comprises just an Fc domain, while the other monomer is a HC (VH1-CH1-hinge-CH2-CH3). This example further utilizes a light chain comprising a variable light domain and a constant light domain, that associates with the heavy chain to form a Fab. As for many of the examples herein, these constructs include skew variants, pI variants, ablation variants, additional Fc variants, etc. as desired and described herein. In this example, suitable ABDs bind a costimulatory receptor such as ICOS, GITR, OX40 or 4-1BB.

The ABD sequences for these combinations can be as disclosed in the sequence listing or as shown in the Figures.

In addition, the Fc domains of the comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), and the heavy chain comprises pI variants (including those shown in Figure 5).

In some examples, the one armed scFv-mAb format includes skew variants, pI variants, and ablation variants. Accordingly, some examples include formats that comprise: a) a first (Fc) monomer that comprises the skew variants S364K/E357Q, the ablation variants E233P/L234V/L235A/G236del/S267K b) a second monomer that comprises the skew variants L368D/K370S, the pI variants Q295E/N384D/Q418E/N421D, the ablation variants E233P/L234V/L235A/ G236del/S267K, and a first variable heavy domain that, with the first variable light domain, makes up the Fv that binds to the costimulatory receptor as outlined herein

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

Specific "one armed mAbs" are shown in the Figures and sequence listing.

### G. Bispecific mAb

One heterodimeric scaffold that finds particular use in the present disclosure is the bispecific mAb format, which is bispecific and tetravalent. In this example, the format relies on the generation of separate homodimeric antibodies which are then recombined. In this format, there is one HC-LC pair (VH1-CH1-hinge-CH2-CH3 and VL1-LC) and a second Hc-LC pair (VH2-CH1-hinge-CH2-CH3 and VL2-LC), e.g. two different heavy chains and two different light chains. Reference is made to Example 5I(d).

In this format, suitable pairs include ICOS and PD-1, ICOS and CTLA-4, ICOS and LAG-3, ICOS and TIM-3, ICOS and PD-L1, ICOS and BTLA, ICOS and TIGIT, GITR and TIGIT, GITR and PD-1, GITR and CTLA-4, GITR and LAG-3, GITR and TIM-3, GITR and PD-L1, GITR and BTLA, OX40 and PD-1, OX40 and TIGIT, OX40 and CTLA-4, IC OX40 OS and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1, TIGIT and 4-1BB and 4-1BB and BTLA.

In some particular examples with these variants in this format:
(1) the format comprises a Fab ABD binds to ICOS that has the ABD of [ICOS] H0.66_L0;
(2) the format comprises an ICOS ABD of H0.66_L0 combined with the ABD comprising the ABD 1G6_L1.194_H1.279 that binds to PD-1;
(3) the format comprises an ICOS ABD of H0.66_L0 combined with the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

### H. Central-Fv format

One heterodimeric scaffold that finds particular use in the present disclosure is the Central-Fv format shown in Figure 2. In this example, the format relies on the use of an inserted Fv domain thus forming an "extra" third antigen binding domain, wherein the Fab portions of the two monomers bind one receptor and the "extra" central-Fv domain binds another (generally the costimulatory receptor). The Fv domain is inserted between the Fc domain and the CH1-Fv region of the monomers, thus providing a third antigen binding domain, wherein each monomer contains a component of the Fv (e.g. one monomer comprises a variable heavy domain and the other a variable light domain of the "extra" central Fv domain).

In this example, one monomer comprises a first heavy chain comprising a first variable heavy domain, a CH1 domain, and Fc domain and an additional variable light domain. The additional variable light domain is covalently attached between the C-terminus of the CH1 domain of the heavy constant domain and the N-terminus of the first Fc domain using domain linkers (vh1-CH1-[optional linker]-vl2-hinge-CH2-CH3). The other monomer comprises a first heavy chain comprising a first variable heavy domain, a CH1 domain and Fc domain and an additional variable heavy domain (vh1-CH1-[optional linker]-vh2-hinge-CH2-CH3). The additional variable heavy domain domain is covalently attached between the C-terminus of the CH1 domain of the heavy constant domain and the N-terminus of the first Fc domain using domain linkers. This example utilizes a common light chain comprising a variable light domain and a constant light domain, that associates with the heavy chains to form two identical Fabs that each bind a receptor. The additional variable heavy domain and additional variable light domain form an "extra" central Fv that binds a second receptor. As for many of the examples herein, these constructs include skew variants, pI variants, ablation variants, additional Fc variants, etc. as desired and described herein. In this example, suitable Fv pairs include (Fabs listed first, "extra" central Fv second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the central-Fv format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the central-Fv format includes skew variants, pI variants, and ablation variants. Accordingly, some examples include central scFv formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first receptor; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the second receptor as outlined herein; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the central-Fv format includes skew variants, pI variants, ablation variants and FcRn variants. Accordingly, some examples include central-scFv formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first receptor and an scFv domain that binds to a second receptor; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein; and c) a light chain comprising a first variable light domain and a constant light domain. In this example, suitable Fv pairs include (Fabs listed first, scFvs second) ICOS X PD-1, PD-1 X ICOS, ICOS X PD-L1, PD-L1 X ICOS, ICOS X CTLA-4 and CTLA-4 X ICOS.

For central-Fv formats suitable Fvs that bind ICOS include, but are not limited to, shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

For central-Fv formats suitable Fvs that bind PD-L1 include, but are not limited to, those shown in Figure 15A-15C, Figure 73 and Figure 78 and SEQ ID NO: 3961-4432.

For central-Fv formats suitable Fvs that bind GITR include, but are not limited to, those in Figure 18, Figure 72 and Figure 73 and those listed in SEQ ID NO:26282-26290.

For central-Fv formats suitable Fvs that bind OX40 include, but are not limited to, Figure 17, Figure 72 and Figure 73 and those listed in SEQ ID NO: 26272-26281.

For central-Fv formats suitable Fvs that bind 4-1BB include, but are not limited to, Figure 16, Figure 72 and Figure 73 and SEQ ID NO: 26262-2671.

For central-FV formats suitable Fvs that bind PD-L1 include, but are not limited to, those of Figure 15A-15C, Figure 73 and Figure 78 and SEQ ID NO: 3961-4432.

### I. One armed central-scFv

One heterodimeric scaffold that finds particular use in the present disclosure is the one armed central-scFv format shown in Figure 1. In this example, one monomer comprises just an Fc domain, while the other monomer includes a Fab domain (a first antigen binding domain), a scFv domain (a second antigen binding domain) and an Fc domain, where the scFv domain is inserted between the Fc domain and the Fc domain. In this format, the Fab portion binds one receptor target and the scFv binds another.

In this example, one monomer comprises a first heavy chain comprising a first variable heavy domain, a CH1 domain and Fc domain, with a scFv comprising a scFv variable light domain, an scFv linker and a scFv variable heavy domain. The scFv is covalently attached between the C-terminus of the CH1 domain of the heavy constant domain and the N-terminus of the first Fc domain using domain linkers, in either orientation, VH1-CH1-[optional domain linker]-VH2-scFv linker-VL2-[optional domain linker]-CH2-CH3 or VH1-CH1-[optional domain linker]-VL2-scFv linker-VH2-[optional domain linker]-CH2-CH3. The second monomer comprises an Fc domain (CH2-CH3). This example further utilizes a light chain comprising a variable light domain and a constant light domain, that associates with the heavy chain to form a Fab. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figure.

In addition, the Fc domains of the one armed central-scFv format generally comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the one armed central-scFv format includes skew variants, pI variants, and ablation variants. Accordingly, some examples include formats that comprise: a) a first monomer that comprises the skew variants S364K/E357Q, the ablation variants E233P/L234V/L235A/G236del/S267K, and a variable heavy domain that, with the variable light domain of the light chain, makes up an Fv that binds to a first receptor, and a scFv that binds to the other receptor; b) a second monomer that comprises the skew variants L368D/K370S, the pI variants Q295E/N384D/Q418E/N421D, the ablation variants E233P/L234V/L235A/ G236del/S267K, and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the one armed central-scFv format includes skew variants, pI variants, ablation variants and FcRn variants. Accordingly, some examples include formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first receptor and a scFv domain that binds to a second receptor; b) a second monomer that comprises the skew variants L368D/K370S, the pI variants Q295E/N384D/Q418E/N421D, the ablation variants E233P/L234V/L235A/G236del/S267K, the FcRn variants M428L/N434S. and c) a light chain comprising a first variable light domain and a constant light domain.

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

### J. One armed scFv-mAb

One heterodimeric scaffold that finds particular use in the present disclosure is the one armed scFv-mAb format shown in Figure 2D. In this example, one monomer comprises just an Fc domain, while the other monomer uses a scFv domain attached at the N-terminus of the heavy chain, generally through the use of a linker: vh1-scFv linker-vl1-[optional domain linker]-VH2-CH1-hinge-CH2-CH3 or (in the opposite orientation) vl1-scFv linker-vh1-[optional domain linker]-VH2-CH1-hinge-CH2-CH3. In this format, either the Fab portion binds one receptor target and the scFv binds another. This example further utilizes a light chain comprising a variable light domain and a constant light domain, that associates with the heavy chain to form a Fab. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or as shown in the Figures.

In addition, the Fc domains of the comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the one armed scFv-mAb format includes skew variants, pI variants, and ablation variants. Accordingly, some examples include formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/Q418E/N421D, the ablation variants E233P/L234V/L235A/ G236del/S267K, and a first variable heavy domain that, with the first variable light domain, makes up the Fv that binds to the first checkpoint inhibitor as outlined herein, and a second variable light chain, that together with the second variable heavy chain forms an Fv (ABD) that binds a second checkpoint inhibitors; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the one armed scFv-mAb format includes skew variants, pI variants, ablation variants and FcRn variants. Accordingly, some examples include bottle opener formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein, and a second variable light chain, that together with the second variable heavy chain forms an Fv (ABD) that binds a second checkpoint inhibitors; and c) a light chain comprising a first variable light domain and a constant light domain.

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

### K. scFv-mAb format

One heterodimeric scaffold that finds particular use in the present disclosure is the mAb-scFv format shown in Figure 2E. In this example, the format relies on the use of a N-terminal attachment of a scFv to one of the monomers, thus forming a third antigen binding domain, wherein the Fab portions of the two monomers each bind one target and the "extra" scFv domain binds a different target.

In this example, the first monomer comprises a first heavy chain (comprising a variable heavy domain and a constant domain), with a N-terminally covalently attached scFv comprising a scFv variable light domain, an scFv linker and a scFv variable heavy domain in either orientation ((vh1-scFv linker-vl1-[optional domain linker]- vh2-CH1-hinge-CH2-CH3) or (with the scFv in the opposite orientation) ((vl1-scFv linker-vh1-[optional domain linker]-vh2-CH1-hinge-CH2-CH3)). The second monomer comprises a heavy chain VH20CH1-hinge-CH2-CH3. This example further utilizes a common light chain comprising a variable light domain and a constant light domain, that associates with the heavy chains to form two identical Fabs that bind one of the target antigens. As for many of the examples herein, these constructs include skew variants, pI variants, ablation variants, additional Fc variants, etc. as desired and described herein. In this example, suitable Fv pairs include (Fabs listed first, scFvs second) ICOS X PD-1, ICOS X PD-L1, ICOS X CTLA-4, ICOS X LAG-3, ICOS X TIM-3, ICOS X BTLA, ICOS X TIGIT, TIGIT X ICOS, PD-1 X ICOS, PD-L1 X ICOS, CTLA-4 X ICOS, LAG-3 X ICOS, TIM-3 X ICOS, BTLA X ICOS, OX40 X TIGIT, OX40 X PD-1, OX40 X PD-L1, OX40 X CTLA-4, OX40 X LAG-3, OX40 X TIM-3, OX40 X BTLA, TIGIT X OX40, PD-1 X OX40, PD-L1 X OX40, CTLA-4 X OX40, LAG-3 X OX40, TIM-3 X OX40, BTLA X OX40, GITR X TIGIT, GITR X PD-1, GITR X PD-L1, GITR X CTLA-4, GITR X LAG-3, GITR X TIM-3, GITR X BTLA, TIGIT x GITR, PD-1 X GITR, PD-L1 X GITR, CTLA-4 X GITR, LAG-3 X GITR, TIM-3 X GITR, BTLA X GITR, 4-1BB X TIGIT, 4-1BB X PD-1, 4-1BB X PD-L1, 4-1BB X CTLA-4, 4-1BB X LAG-3, 4-1BB X TIM-3, 4-1BB X BTLA, TIGIT X 4-1BB, PD-1 X 4-1BB, PD-L1 X 4-1BB, CTLA-4 X 4-1BB, LAG-3 X 4-1BB, TIM-3 X 4-1BB and BTLA X 4-1BB.

The ABD sequences for these combinations can be as disclosed in the sequence listing or in the Figures.

In addition, the Fc domains of the scFv-mAb format comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the mAb-scFv format includes skew variants, pI variants, and ablation variants. Accordingly, some examples include bottle opener formats that comprise: a) a first monomer that comprises the skew variants S364K/E357Q, the ablation variants E233P/L234V/L235A/G236del/S267K, and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/ Q418E/N421D, the ablation variants E233P/L234V/L235A/G236del/S267K, and a first variable heavy domain that, with the first variable light domain, makes up the Fv that binds to the first checkpoint inhibitor as outlined herein, and a second variable light chain, that together with the second variable heavy chain forms an Fv (ABD) that binds a second checkpoint inhibitors; and c) a light chain comprising a first variable light domain and a constant light domain. In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In some examples, the mAb-scFv format includes skew variants, pI variants, ablation variants and FcRn variants. Accordingly, some examples include bottle opener formats that comprise: a) a first monomer that comprises the skew variants S364K/E357Q, the ablation variants E233P/L234V/L235A/G236del/S267K, the FcRn variants M428L/N434S and a first variable heavy domain that, with the first variable light domain of the light chain, makes up an Fv that binds to a first checkpoint inhibitor, and a second variable heavy domain; b) a second monomer that comprises the skew variants L368D/K370S, the pI variants N208D/Q295E/N384D/Q418E/N421D, the ablation variants E233P/L234V/L235A/G236del/S267K, the FcRn variants M428L/N434S and a first variable heavy domain that, with the first variable light domain, makes up the Fv that binds to the first checkpoint inhibitor as outlined herein, and a second variable light chain, that together with the second variable heavy chain forms an Fv (ABD) that binds a second checkpoint inhibitors; and c) a light chain comprising a first variable light domain and a constant light domain.

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

### L. Dual scFv formats

The present disclosure also provides dual scFv formats as are known in the art and shown in Figure 2B. In this example, the heterodimeric bispecific antibody is made up of two scFv-Fc monomers (both in either (vh-scFv linker-vl-[optional domain linker]-CH2-CH3) format or (vl-scFv linker-vh-[optional domain linker]-CH2-CH3) format, or with one monomer in one orientation and the other in the other orientation.

In this case, all ABDs are in the scFv format, with any combination of ICOS and PD-1, ICOS and CTLA-4, ICOS and LAG-3, ICOS and TIM-3, ICOS and PD-L1, ICOS and BTLA, GITR and PD-1, GITR and CTLA-4, GITR and LAG-3, GITR and TIM-3, GITR and PD-L1, GITR and BTLA, OX40 and PD-1, OX40 and CTLA-4, OX40 and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1 and 4-1BB and BTLA being useful. The ABD sequences for these combinations can be as disclosed in the sequence listing or as shown in the Figures.

In addition, the Fc domains of the dual scFv format comprise skew variants (e.g. a set of amino acid substitutions as shown in Figure 4, with particularly useful skew variants being selected from the group consisting of S364K/E357Q : L368D/K370S; L368D/K370S : S364K; L368E/K370S : S364K; T411T/E360E/Q362E : D401K; L368D/K370S : S364K/E357L, K370S : S364K/E357Q, T366S/L368A/Y407V : T366W and T366S/L368A/Y407V/Y349C : T366W/S354C), optionally ablation variants (including those shown in Figure 6), optionally charged scFv linkers (including those shown in Figure 8) and the heavy chain comprises pI variants (including those shown in Figure 5).

In some examples, the dual scFv format includes skew variants, pI variants, and ablation variants. Accordingly, some examples include formats that comprise: a) a first monomer that comprises the skew variants S364K/E357Q, the ablation variants E233P/L234V/L235A/G236del/S267K, and a scFv that binds a first receptor (VH1-scFv linker-VL1-[optional domain linker]-CH2-CH3 or VL1-scFv linker-VH1-[optional domain linker]-CH2-CH3) and b) a first monomer that comprises the skew variants L368D/K370S, the ablation variants E233P/L234V/L235A/G236del/S267K, and a scFv that binds a first receptor (VH1-scFv linker-VL1-[optional domain linker]-CH2-CH3 or VL1-scFv linker-VH1-[optional domain linker]-CH2-CH3). pI variants can be as outlined herein, but most common will be charged scFv linkers of opposite charge for each monomer. FcRn variants, particularly 428L/434S, can optionally be included. In some particular examples with these variants in this format:
(1) the format comprises a ABD binds to ICOS that has the ABD of [ICOS] H0.66_L0;
(2) the format comprises an ICOS ABD of H0.66_L0 combined with the ABD comprising the ABD 1G6_L1.194_H1.279 that binds to PD-1;
(3) the format comprises an ICOS ABD of H0.66_L0 combined with the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises the ABD 1G6_L1.194_H1.279 that binds to PD-1 and an Fv binds to GITR;
(6) the format comprises the ABD 1G6_L1.194_H1.279 that binds to PD-1 and the Fv binds to OX40;
(7) the format comprises the ABD 1G6_L1.194_H1.279 that binds to PD-1 and the ABD binds to ICOS;
8) the format comprises the ABD 1G6_L1.194_H1.279 that binds to PD-1 and an ABD binds to 4-1BB; and
(9) the format comprises the ABD of H0.66_L0 that binds to ICOS and an ABD that binds to PD-L1.

In this format, specific ABDs that bind human ICOS include, but are not limited to, [ICOS]_H0L0 and [ICOS]H0.66_L0 and and those shown in Figure 19, Figure 20, Figure 24, Figure 68 and Figure 77 as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

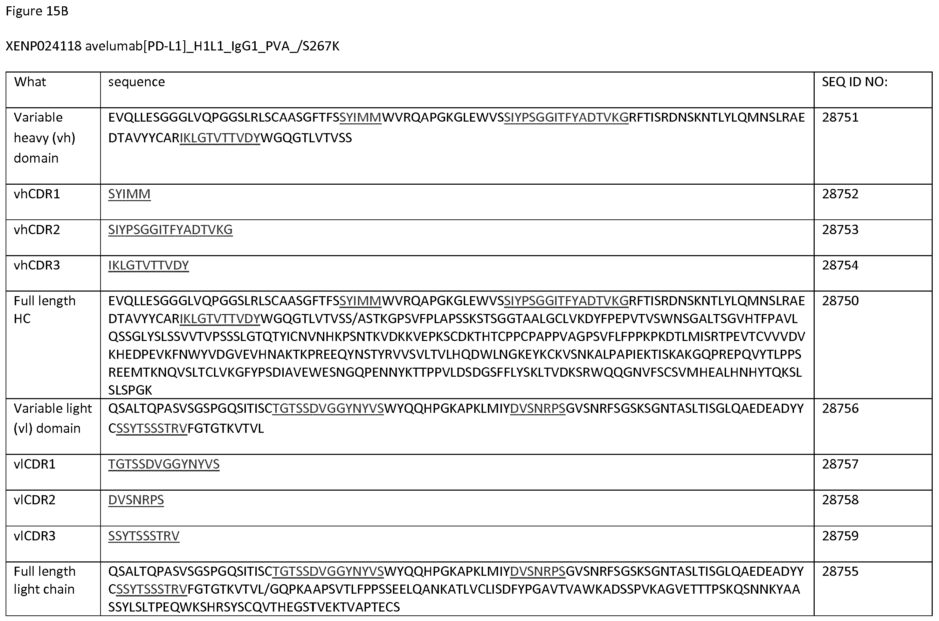

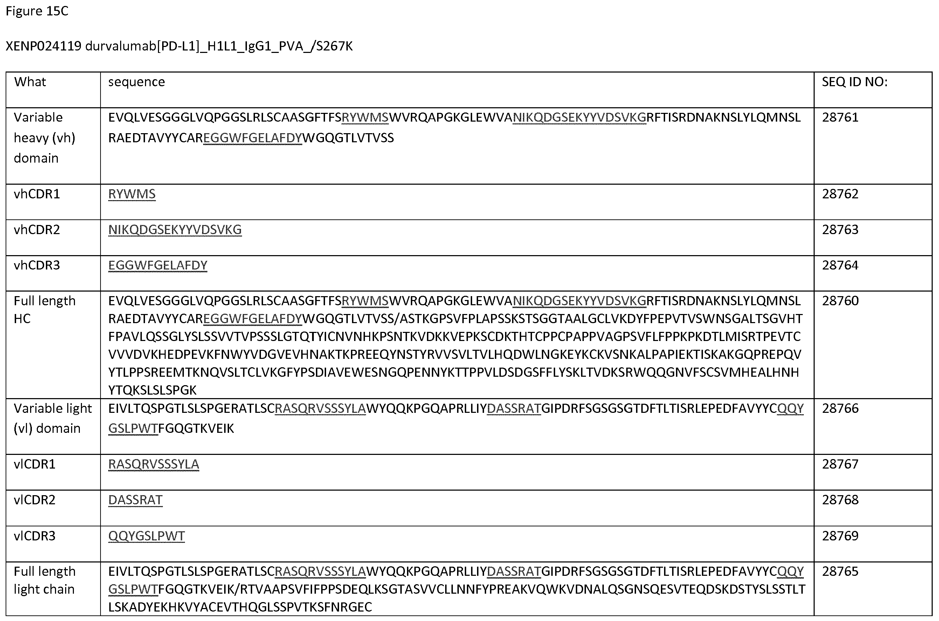

In this format, specific ABDs that bind human PD-L1 include, but are not limited to, Figure 15, Figure 73 and Figure 78 and SEQ ID NO: 3961-4432.

### M. Non-heterodimeric bispecific antibodies

As will be appreciated by those in the art, the Fv sequences outlined herein can also be used in both monospecific antibodies (e.g. "traditional monoclonal antibodies") or non-heterodimeric bispecific formats (see Figure 2J, K and L).

In this format, specific ABDs that bind human ICOS include, but are not limited to, [ICOS]_H0L0 and [ICOS]H0.66_L0 and and those shown in Figure 19, Figure 20, Figure 24, Figure 68 and Figure 77 as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15, Figure 73 and Figure 78 and SEQ ID NO: 3961-4432.

Suitable non-heterodimeric bispecific formats are known in the art, and include a number of different formats as generally depicted in Spiess et al., Molecular Immunology (67):95-106 (2015) and Kontermann, mAbs 4:2, 182-197 (2012) in particular for the figures, legends and citations to the formats therein.

### N. DVD-Ig Format

In some examples, the bispecific antibody is in a "Dual Variable Domain-Ig" or "DVD-Ig^{™}" format (see Figure 2L) such as is generally described in US Patent No. 7,612,181 in particular for the Figures and Legends therein. In the DVD-Ig format, the antibody is tetravalent and bispecific, and comprises 4 chains: two homodimeric heavy chains and two identical light chains. The heavy chains each have a VH1-(optional linker)-VH2-CH1-hinge-CH2-CH3 structure and the two light chains each have a VL1-optional linker-VL2-CL structure, with VH1 and VL1 forming a first ABD and the VH2 and VL2 forming a second ABD, where the first and second ABDs bind a costimulatory and a checkpoint receptor. In this example, suitable combinations include ICOS and PD-1, ICOS and CTLA-4, ICOS and LAG-3, ICOS and TIM-3, ICOS and PD-L1, ICOS and BTLA, GITR and PD-1, GITR and CTLA-4, GITR and LAG-3, GITR and TIM-3, GITR and PD-L1, GITR and BTLA, OX40 and PD-1, OX40 and CTLA-4, OX40 and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1 and 4-1BB and BTLA.

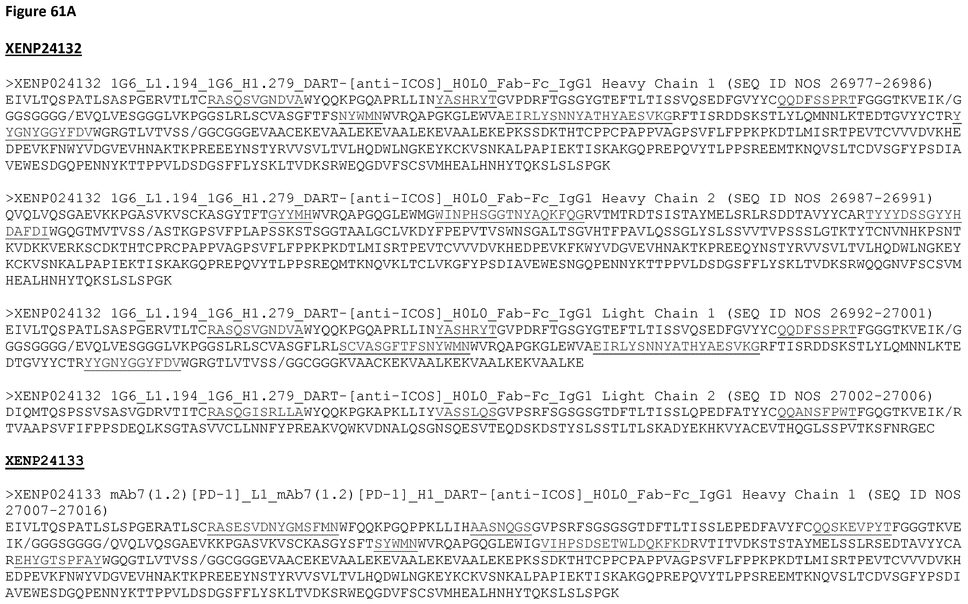

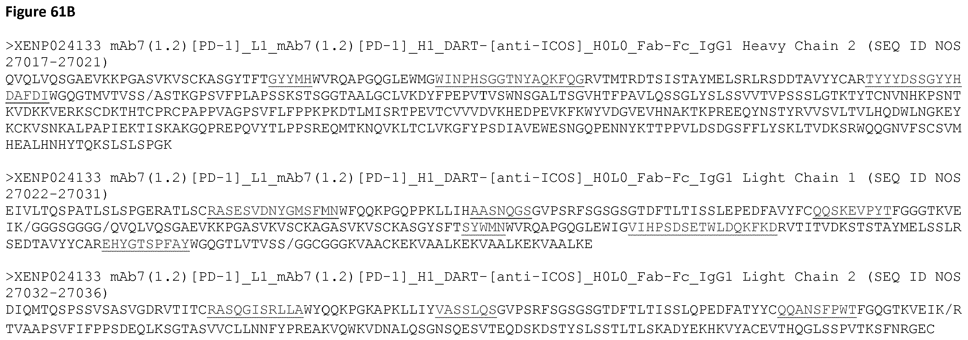

The DVD-Ig^{™} and Central-scFv2 are two formats that are bispecific and tetravalent, and thus do not bind a costimulatory receptor in a monovalent fashion. Exemplary DVD-Ig^{™} constructs are shown in Figure 61.

In some particular examples with these variants in this format:
(1) the format comprises the ABD of [ICOS] H0.66_L0;
(2) the format comprises an ICOS ABD of H0.66_L0 combined with the ABD comprising the ABD 1G6_L1.194_H1.279 that binds to PD-1;
(3) the format comprises an ICOS ABD of H0.66_L0 combined with the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises the ABD 1G6_L1.194_H1.279 that binds to PD-1 and an ABD that binds to GITR;
(6) the format comprises the ABD 1G6_L1.194_H1.279 that binds to PD-1 and and ABD that binds to OX40;
(7) the format comprises the ABD 1G6_L1.194_H1.279 that binds to PD-1 and an ABD that binds to ICOS;
8) the format comprises the ABD 1G6_L1.194_H1.279 that binds to PD-1 and an ABD that binds to 4-1BB; and
(9) the format comprises the ABD of H0.66_L0 that binds to ICOS and an ABD that binds to PD-L1.

In this format, specific ABDs that bind human ICOS include, but are not limited to, [ICOS]_H0L0 and [ICOS]H0.66_L0 and and those shown in Figure 19, Figure 20, Figure 24, Figure 68 and Figure 77 as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15, Figure 73 and Figure 78 and SEQ ID NO: 3961-4432.

### O. Trident Format

In some examples, the bispecific antibodies disclosed herein may be in the "Trident" format as generally described in WO2015/184203 in particular for the Figures, Legends, definitions and sequences of "Heterodimer-Promoting Domains" or "HPDs", including "K-coil" and "E-coil" sequences. Tridents rely on using two different HPDs that associate to form a heterodimeric structure as a component of the structure, see Figure 2M. In this example, the Trident format includes a "traditional" heavy and light chain (e.g. VH1-CH1-hinge-CH2-CH3 and VL1-CL), a third chain comprising a first "diabody-type binding domain" or "DART^{®}", VH2-(linker)-VL3-HPD1 and a fourth chain comprising a second DART^{®}, VH3-(linker)-(linker)-VL2-HPD2. The VH1 and VL1 form a first ABD, the VH2 and VL2 form a second ABD, and the VH3 and VL3 form a third ABD. IN some cases, as is shown in Figure 2M, the second and third ABDs bind the same antigen, in this instance generally the checkpoint receptor, e.g. bivalently, with the first ABD binding a costimulatory receptor monovalently. In this example, suitable combinations include ICOS and PD-1, ICOS and CTLA-4, ICOS and LAG-3, ICOS and TIM-3, ICOS and PD-L1, ICOS and BTLA, GITR and PD-1, GITR and CTLA-4, GITR and LAG-3, GITR and TIM-3, GITR and PD-L1, GITR and BTLA, OX40 and PD-1, OX40 and CTLA-4, OX40 and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1 and 4-1BB and BTLA.

In some particular examples with these variants in this format:
(1) the format comprises a Fab ABD binds to ICOS that has the ABD of [ICOS] H0.66_L0;
(2) the format comprises an ICOS ABD of H0.66_L0 combined with the scFv ABD comprising the ABD 1G6_L1.194_H1.279 that binds to PD-1;
(3) the format comprises an ICOS ABD of H0.66_L0 combined with the scFv comprising the ABD H3.23_L0.129 that binds to CTLA-4;
(4) the format comprises an ICOS ABD of H0.66_L0 is combined with the ABD 7G8_H.303_L1.34 that binds to LAG-3;
(5) the format comprises a scFv comprising the ABD 1G6_L1.194_H1.279 that binds to PD-1 and the Fab binds to GITR;
(6) the format comprises a scFv comprising the ABD 1G6_L1.194_H1.279 that binds to PD-1 and the Fab binds to OX40;
(7) the format comprises a scFv comprising the ABD 1G6_L1.194_H1.279 that binds to PD-1 and the Fab binds to ICOS;
8) the format comprises a scFv comprising the ABD 1G6_L1.194_H1.279 that binds to PD-1 and the Fab binds to 4-1BB; and
(9) the format comprises the ABD of H0.66_L0 that binds to ICOS and an ABD that binds to PD-L1.

In this format, specific ABDs that bind human ICOS include, but are not limited to, [ICOS]_H0L0 and [ICOS]H0.66_L0 and and those shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665.

In this format, specific ABDs that bind human GITR include, but are not limited to, those in Figure 18, Figure 72 and Figure 73 and those listed in SEQ ID NO:26282-26290.

In this format, specific ABDs that bind OX40 include, but are not limited to, Figure 17, Figure 72 and Figure 73 and those listed in SEQ ID NO: 26272-26281.

In this format, specific ABDs that bind human 4-1BB include, but are not limited to, Figure 16, Figure 72 and Figure 73 and SEQ ID NO: 26262-2671.

In this format, specific ABDs that bind human PD-L1 include, but are not limited to, Figure 15A-15C, Figure 73 and Figure 78 and SEQ ID NO: 3961-4432.

### P. Monospecific, monoclonal antibodies

As will be appreciated by those in the art, the novel Fv sequences outlined herein can also be used in both monospecific antibodies (e.g. "traditional monoclonal antibodies") or non-heterodimeric bispecific formats. Accordingly, the present disclosure provides monoclonal (monospecific) antibodies comprising the 6 CDRs and/or the vh and vl sequences from the figures, generally with IgG1, IgG2, IgG3 or IgG4 constant regions, with IgG1, IgG2 and IgG4 (including IgG4 constant regions comprising a S228P amino acid substitution) finding particular use in some examples. That is, any sequence herein with a "H_L" designation can be linked to the constant region of a human IgG1 antibody.

### Vill. Antigen Binding Domains (ABDs) to Target Antigens

The bispecific antibodies disclosed herein have two different antigen binding domains (ABDs) that bind to two different target receptor antigens ("target pairs"), in either bivalent, bispecific formats or trivalent, bispecific formats as generally shown in Figure 2.

The bispecific antibodies may bind to a first target antigen comprising a checkpoint receptor and a second target antigen comprising a costimulatory receptor. Suitable checkpoint receptors as outlined herein include PD-1, PD-L1, LAG-3, TIM-3, CTLA-4, BTLA and TIGIT. Suitable costimulatory receptors as outlined herein include ICOS, GITR, OX40 and 4-1BB. As outlined

Suitable target checkpoint antigens include human (and sometimes cyno) PD-1, CTLA-4, TIM-3, LAG-3, TIGIT and BTLA (sequences in the sequence listing). Accordingly, suitable bispecific antibodies bind ICOS and PD-1, ICOS and CTLA-4, ICOS and LAG-3, ICOS and TIM-3, ICOS and PD-L1, ICOS and BTLA, ICOS and TIGIT, GITR and TIGIT, GITR and PD-1, GITR and CTLA-4, GITR and LAG-3, GITR and TIM-3, GITR and PD-L1, GITR and BTLA, OX40 and PD-1, OX40 and TIGIT, OX40 and CTLA-4, IC OX40 OS and LAG-3, OX40 and TIM-3, OX40 and PD-L1, OX40 and BTLA, 4-1BB and PD-1, 4-1BB and CTLA-4, 4-1BB and LAG-3, 4-1BB and TIM-3, 4-1BB and PD-L1, TIGIT and 4-1BB and 4-1BB and BTLA.

Note that generally these bispecific antibodies are named "anti-PD-1 X anti-CTLA-4", or generally simplistically or for ease (and thus interchangeably) as "PD-1 X CTLA-4", etc. for each pair. Note that unless specified herein, the order of the antigen list in the name does not confer structure; that is a PD-1 X CTLA-4 bottle opener antibody can have the scFv bind to PD-1 or CTLA-4, although in some cases, the order specifies structure as indicated.

As is more fully outlined herein, these combinations of ABDs can be in a variety of formats, as outlined below, generally in combinations where one ABD is in a Fab format and the other is in an scFv format. As discussed herein and shown in Figure 2, some formats use a single Fab and a single scFv (A, C and D), and some formats use two Fabs and a single scFv (E, F, G, H and I).

### A. Antigen Binding Domains

As discussed herein, the bispecific checkpoint heterodimeric antibodies of the invention include two antigen binding domains (ABDs), each of which bind to a different checkpoint protein. As outlined herein, some heterodimeric antibodies disclosed herein can be bispecific and bivalent (each antigen is bound by a single ABD, for example, bispecific and trivalent (one antigen is bound by a single ABD and the other is bound by two ABDs, for example as depicted in Figure 2F, G, H, I or M), or bispecific and tetravalent (both antigens are bound by two ABDs, for example as depicted in Figure 2J and L).

In addition, in general, one of the ABDs comprises a scFv as outlined herein, in an orientation from N- to C-terminus of vh-scFv linker-vl or vl-scFv linker-vh. One or both of the other ABDs, according to the format, generally is a Fab, comprising a vh domain on one protein chain (generally as a component of a heavy chain) and a vl on another protein chain (generally as a component of a light chain). Note that the "trident" format uses DART^{®}s, which are similar to scFvs except the orientation is different and in general the linkers can be slightly longer.

The disclosure provides a number of ABDs that bind to a number of different receptor proteins, as outlined below. As will be appreciated by those in the art, any set of 6 CDRs or vh and vl domains can be in the scFv format or in the Fab format, which is then added to the heavy and light constant domains, where the heavy constant domains comprise variants (including within the CH1 domain as well as the Fc domain). The scFv sequences contained in the sequence listing utilize a particular charged linker, but as outlined herein, uncharged or other charged linkers can be used, including those depicted in Figure 8.

In addition, as discussed above, the numbering used in the Sequence Listing for the identification of the CDRs is Kabat, however, different numbering can be used, which will change the amino acid sequences of the CDRs as shown in Table 1.

For all of the variable heavy and light domains listed herein, further variants can be made. As outlined herein, in some examples the set of 6 CDRs can have from 0, 1, 2, 3, 4 or 5 amino acid modifications (with amino acid substitutions finding particular use), as well as changes in the framework regions of the variable heavy and light domains, as long as the frameworks (excluding the CDRs) retain at least about 80, 85 or 90% identity to a human germline sequence selected from those listed in Figure 1 of U.S. Patent No.7,657,380. Thus, for example, the identical CDRs as described herein can be combined with different framework sequences from human germline sequences, as long as the framework regions retain at least 80, 85 or 90% identity to a human germline sequence selected from those listed in Figure 1 of U.S. Patent No.7,657,380. Alternatively, the CDRs can have amino acid modifications (e.g. from 1, 2, 3, 4 or 5 amino acid modifications in the set of CDRs (that is, the CDRs can be modified as long as the total number of changes in the set of 6 CDRs is less than 6 amino acid modifications, with any combination of CDRs being changed; e.g. there may be one change in vlCDR1, two in vhCDR2, none in vhCDR3, etc.)), as well as having framework region changes, as long as the framework regions retain at least 80, 85 or 90% identity to a human germline sequence selected from those listed in Figure 1 of U.S. Patent No.7,657,380.

### B. PD-1 Antigen Binding Domains

In some embodiments, one of the ABDs binds PD-1. Suitable sets of 6 CDRs and/or vh and vl domains, as well as scFv sequences relating to the disclosure, are depicted in Figure 3, Figure 10, Figure 11, Figure 72, Figure 73, Figure 74, Figure 76 and SEQ ID NO:1-2392, 3125-3144, 4697-7594 and 4697-21810, and include those sequences in the sequence listing with the identifiers 1G6_H1.279_L1.194; 1G6_H1.280_L1.224; 1G6_L1.194_H1.279; 1G6_L1.210_H1.288; and 2E9_H1L1.

As will be appreciated by those in the art, suitable anti-PD-1 ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences of these sequences. Suitable ABDs can also include the entire vh and vl sequences as depicted in these sequences and Figures, used as scFvs or as Fabs. In many of the embodiments herein that contain an Fv to PD-1, it is the scFv monomer that binds PD-1. As discussed herein, the other of the target pair when PD-1 is one of the antigens is selected from ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665 (which can be scFv sequences, CDR sequence sets or vh and vl sequences)), GITR, OX40 and 4-1BB.

Particularly useful ABDs that bind human PD-1 include, but are not limited to, 1G6_H1.279_L1.194, 1G6_H1.280_L1.224; 1G6_L1.194_H1.279, 1G6_L1.210_H1.288 and 2E9_H1L1.

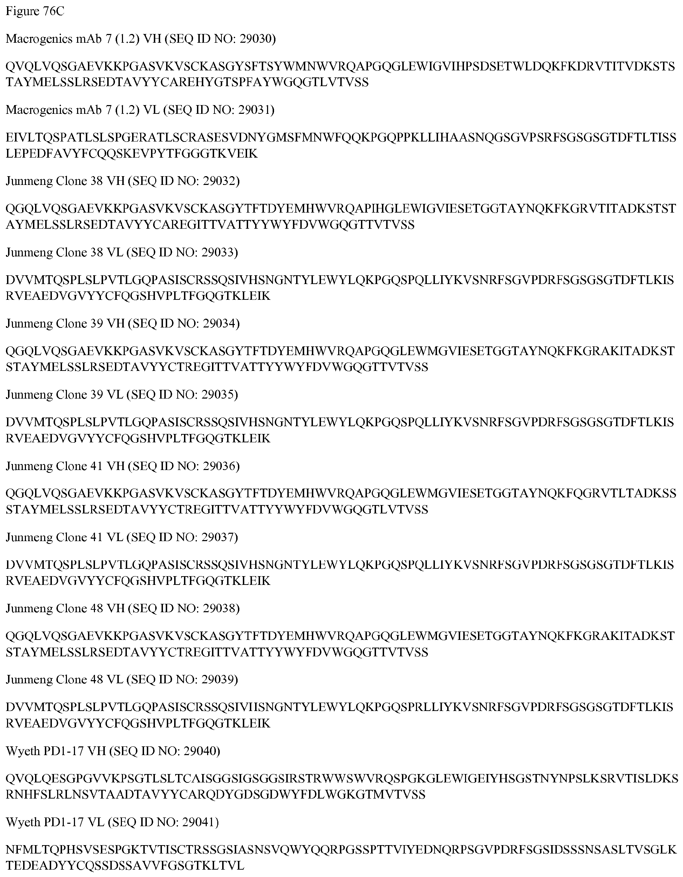

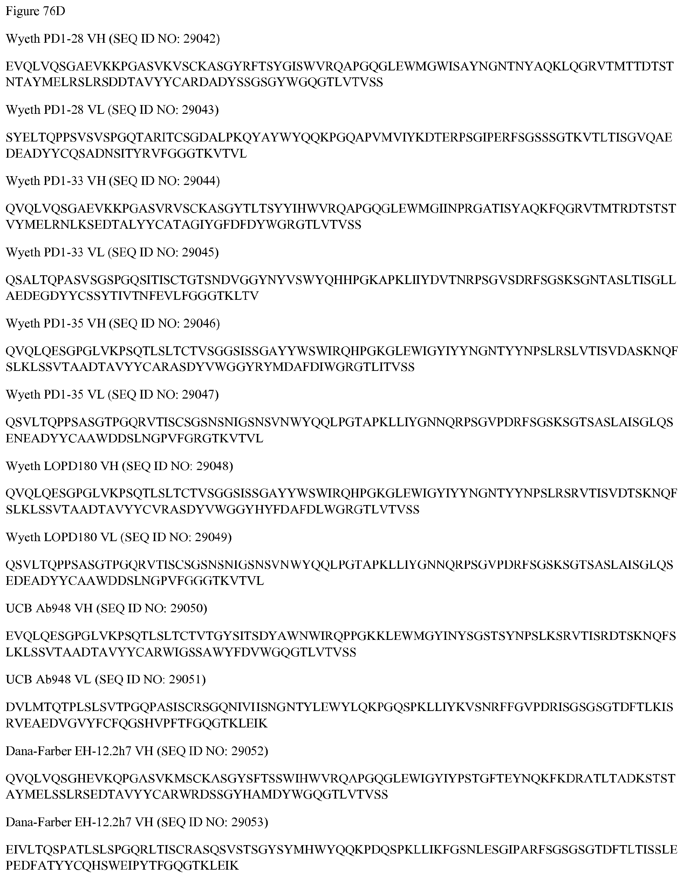

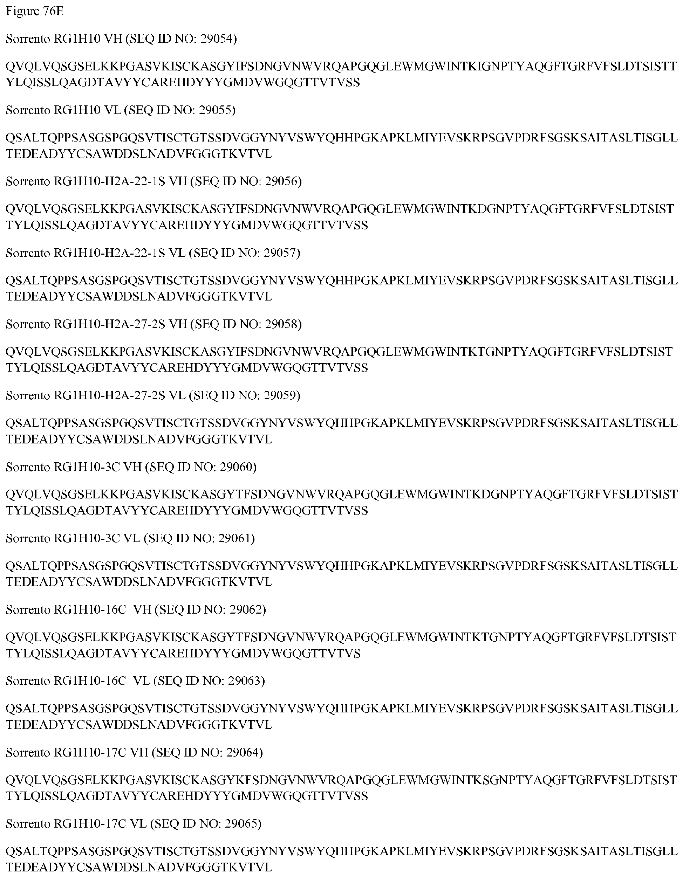

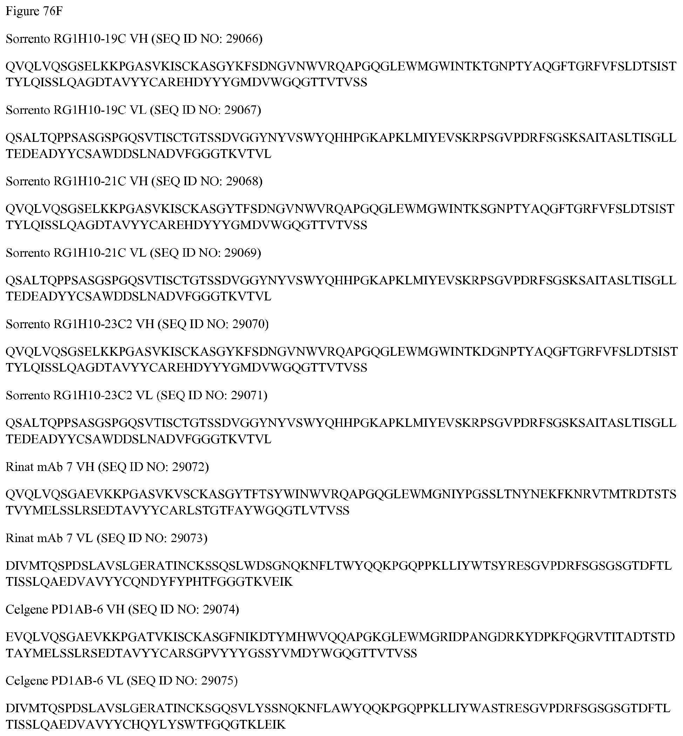

Additionally useful vh and vl sequences that bind human PD-1 are shown in Figure 76.

In addition to the parental CDR sets disclosed in the sequence listing that form an ABD to PD-1, the disclosure provides variant CDR sets. In one example, a set of 6 CDRs can have 1, 2, 3, 4 or 5 amino acid changes from the parental CDRs, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to PD-1, the disclos provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of aBiacore, surface plasmon resonance (SPR) and/or BLI (biolayer interferometry, e.g. Octet assay) assay, with the latter finding particular use in many examples.

Specific preferred embodiments include the 1G6_L1.194_H1.279 anti-PD-1 Fv, in a scFv format, included within any of the bottle opener format backbones of Figure 9.

Specific preferred embodiments include the 1G6_L1.194_H1.279 anti-PD-1 Fv, in a scFv format, included within any of the format backbones of Figure 55.

Specific preferred embodiments include the 1G6_L1.194_H1.279 anti-PD-1 Fv, in a scFv format, included within any of the mAb-scFv format backbones of Figure 75.

Other examples utilize any of the anti-PD-1 vh and vl domain pairs (either as scFvs or Fabs) as shown in Figure 76 in any format shown in Figure 2.

### C. CTLA-4 Antigen Binding Domains

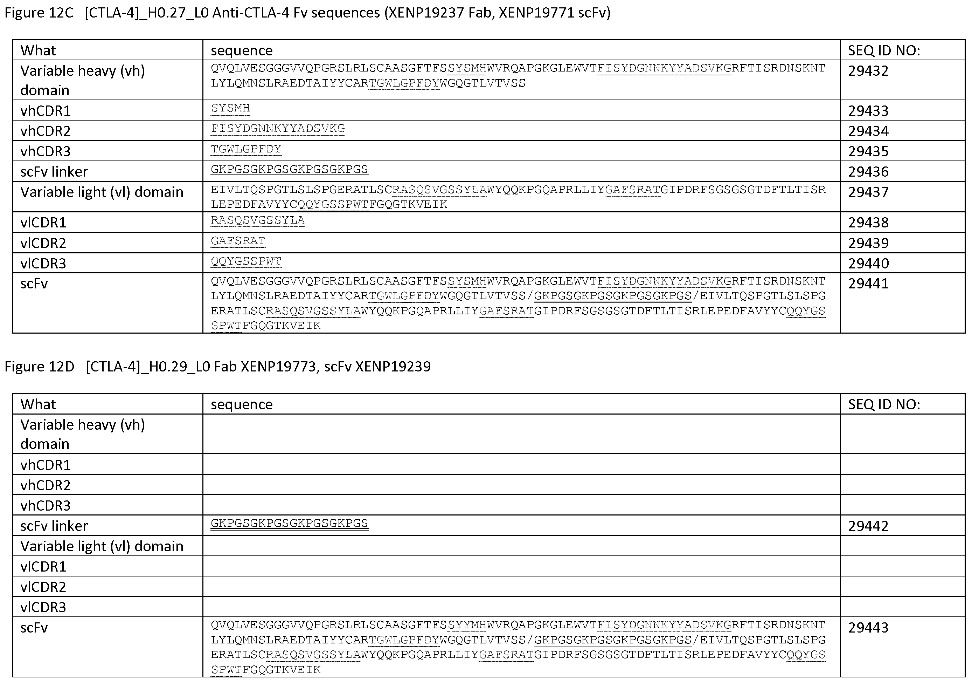

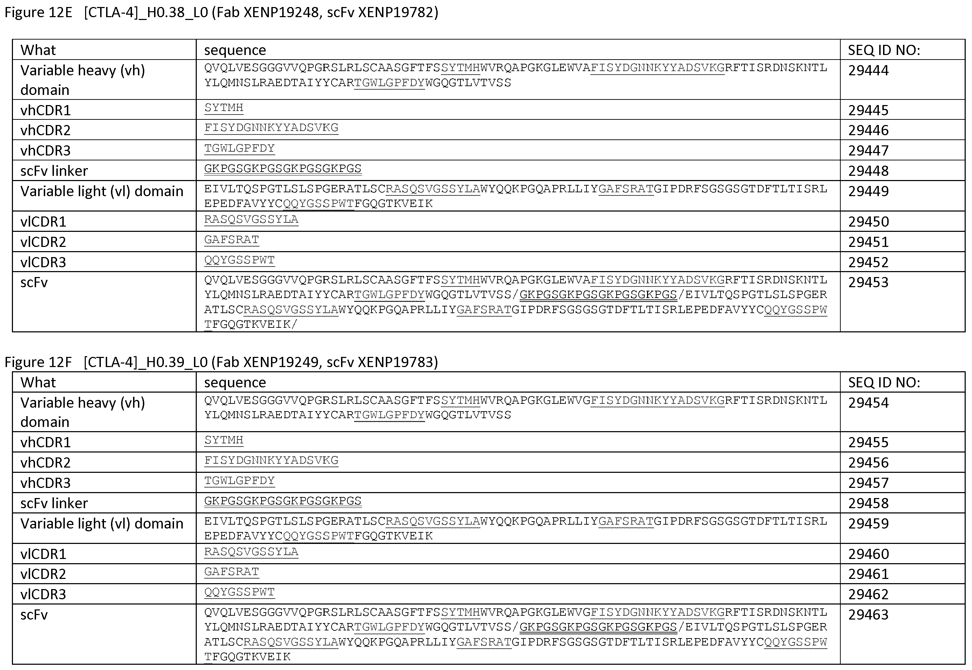

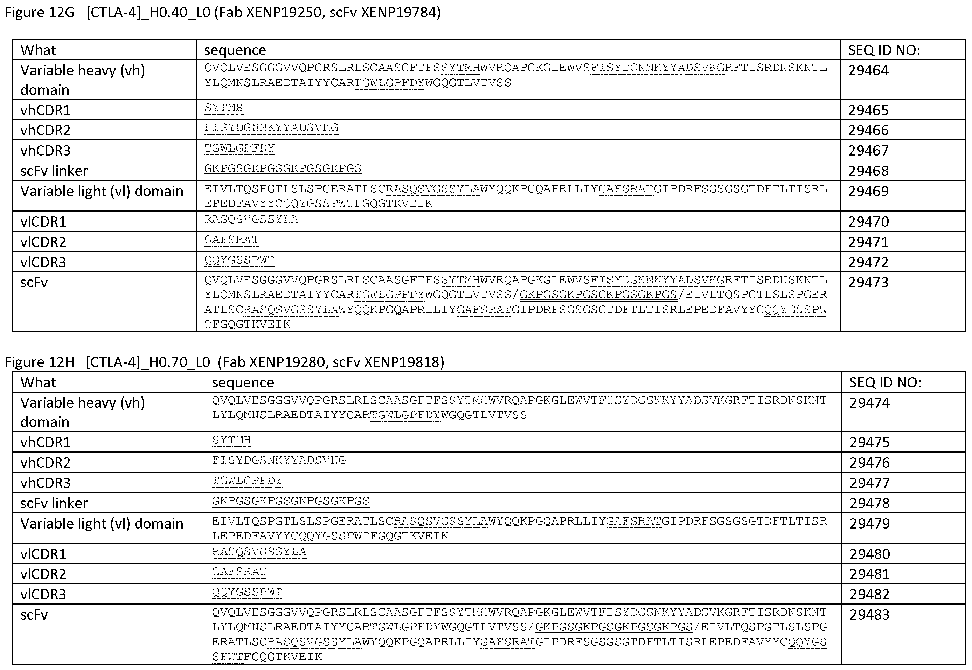

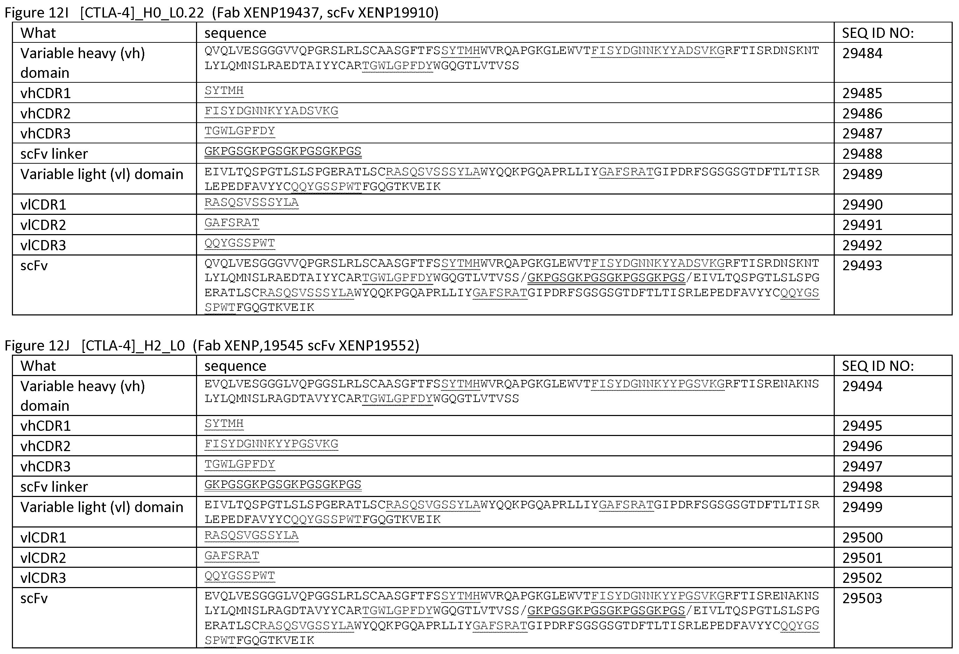

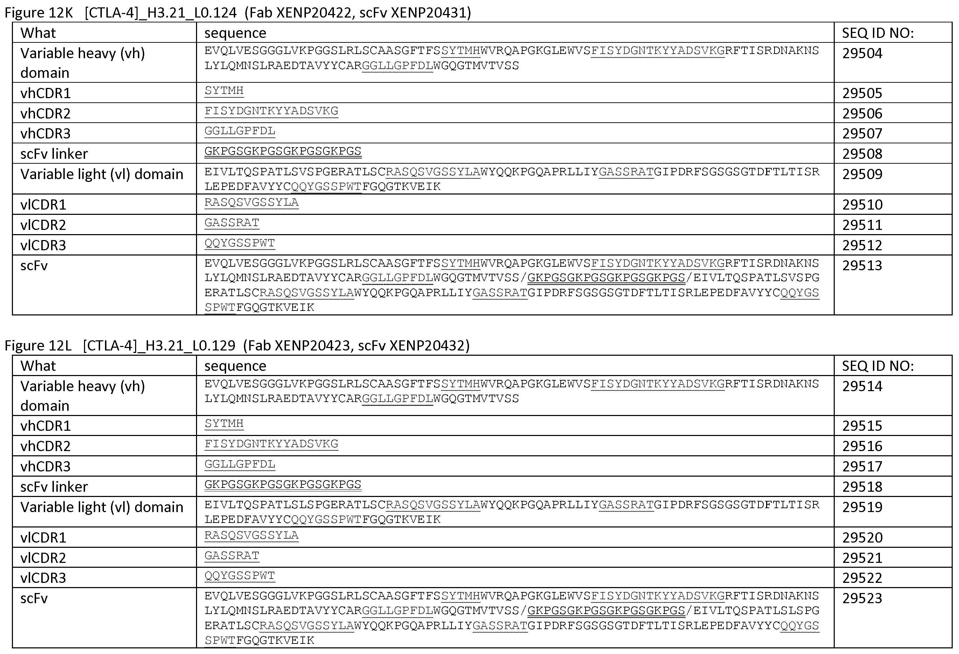

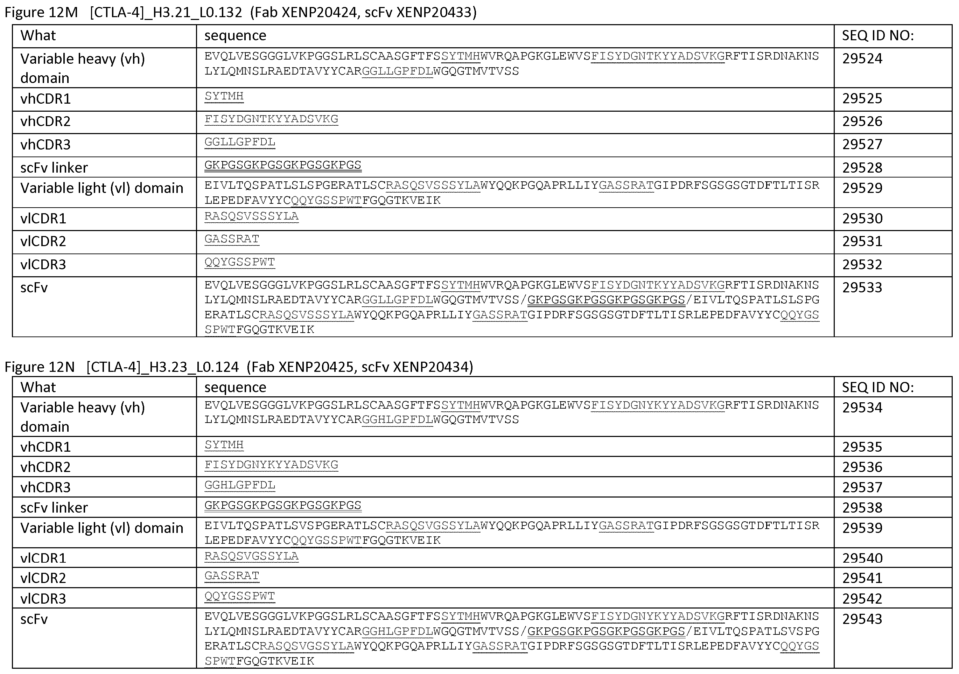

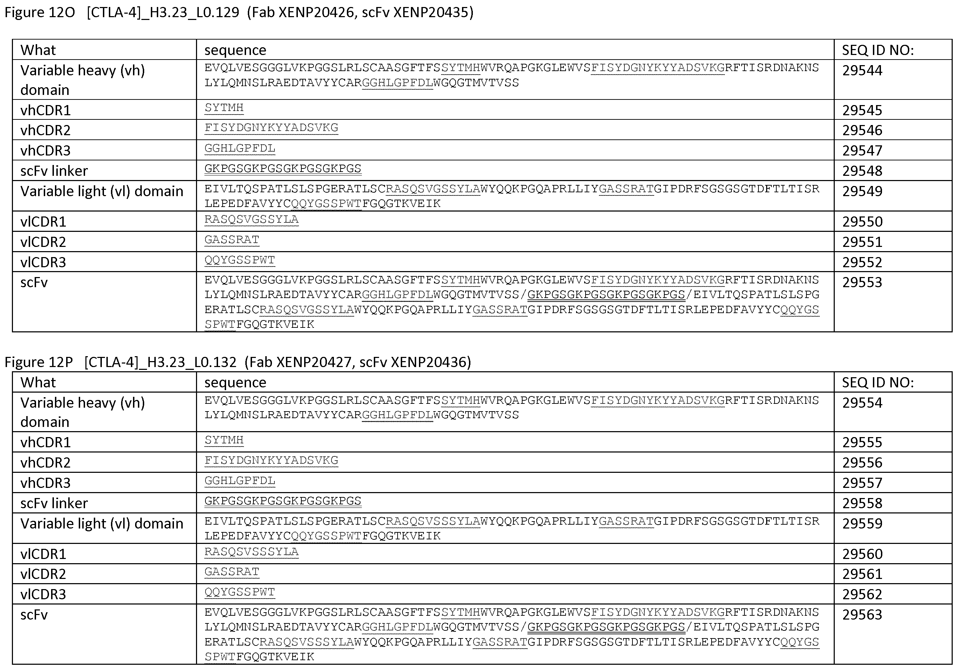

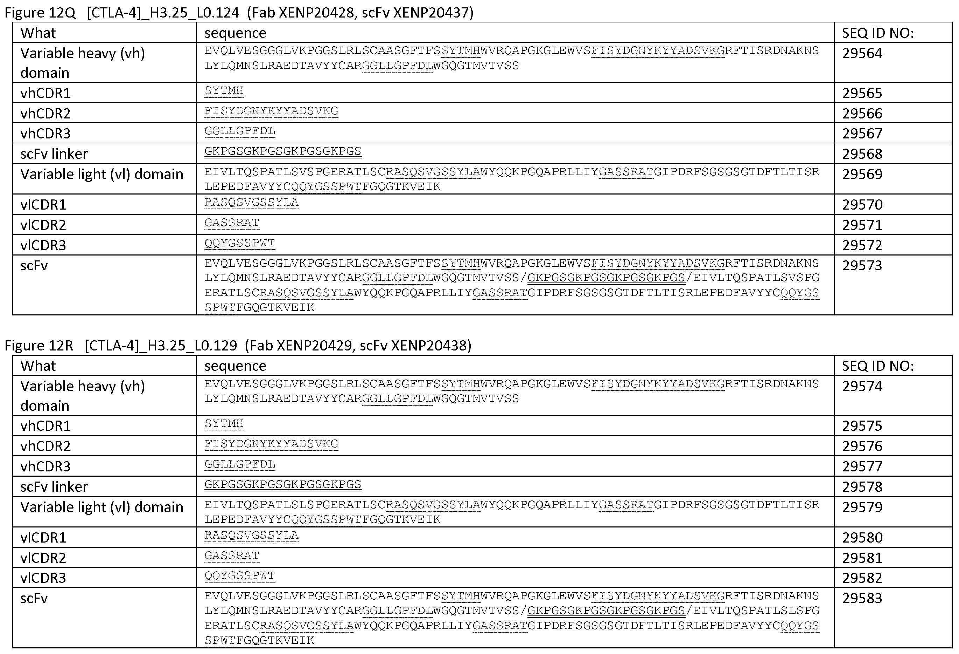

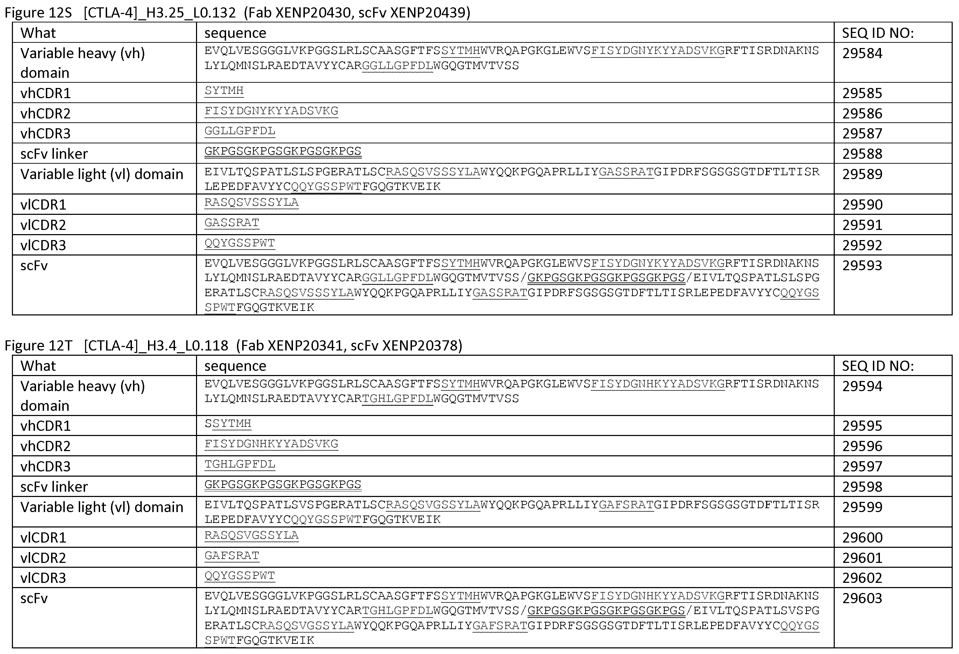

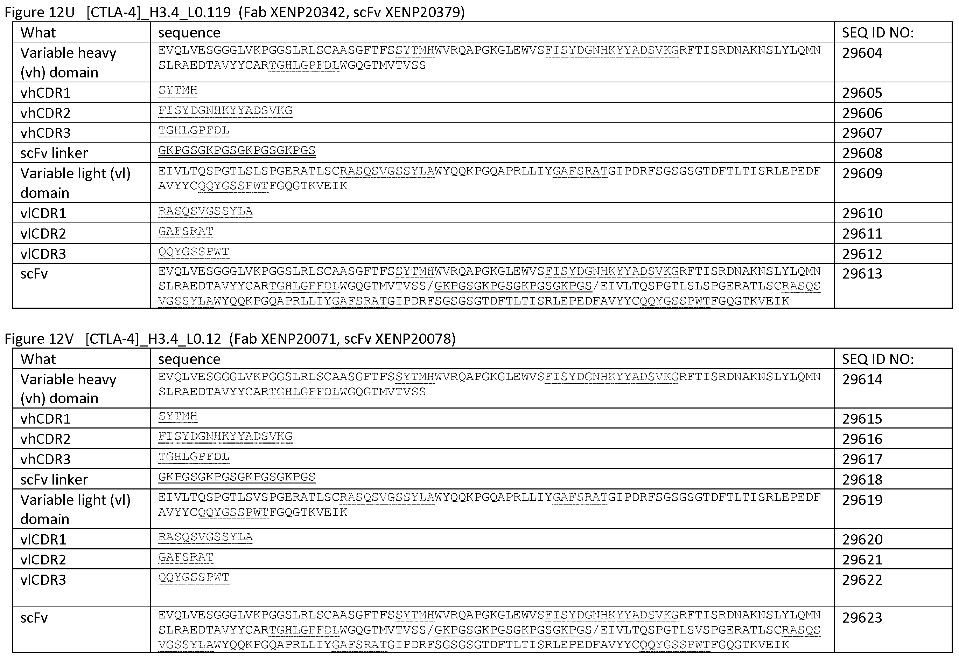

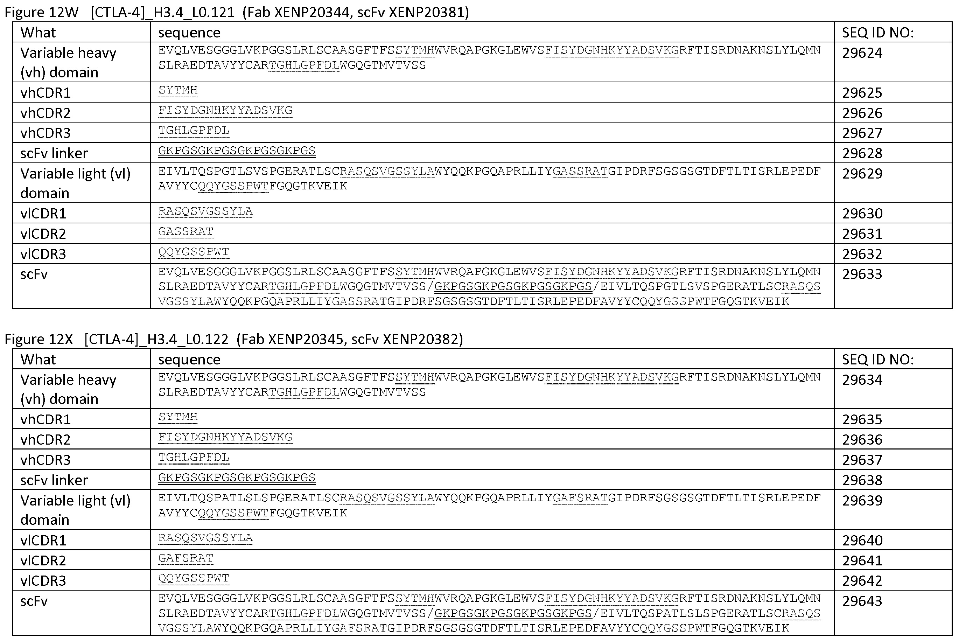

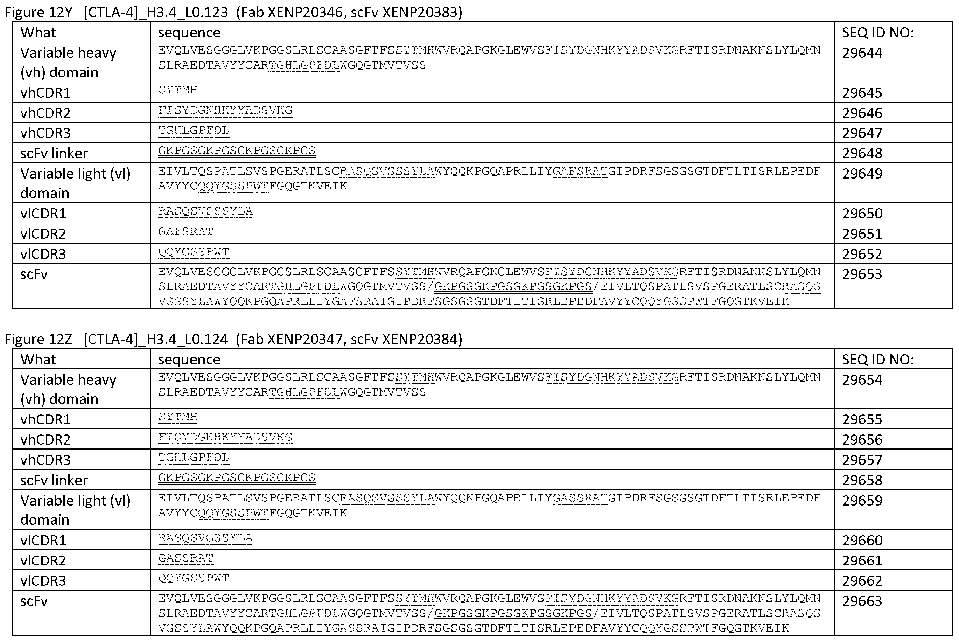

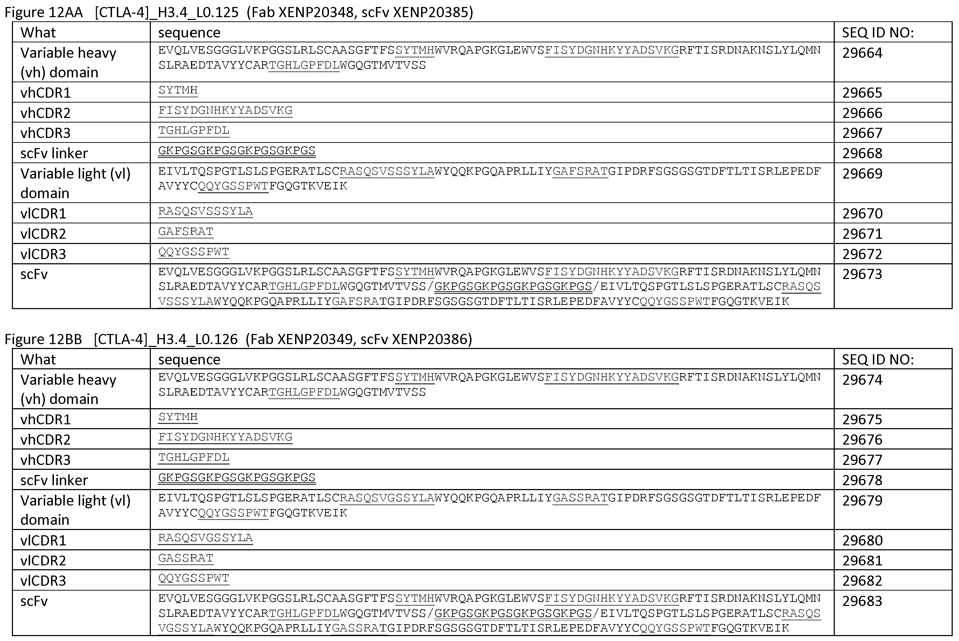

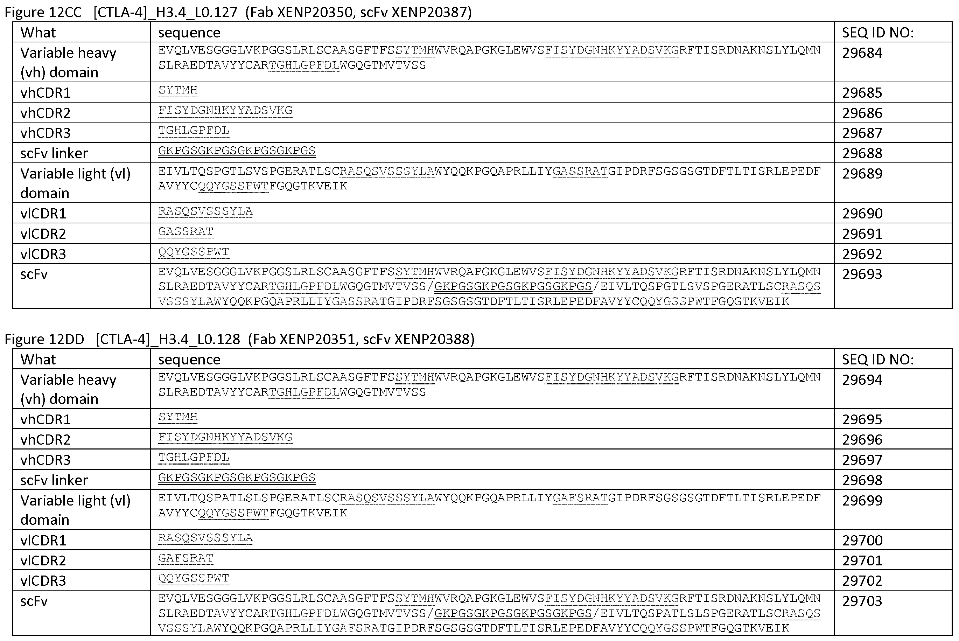

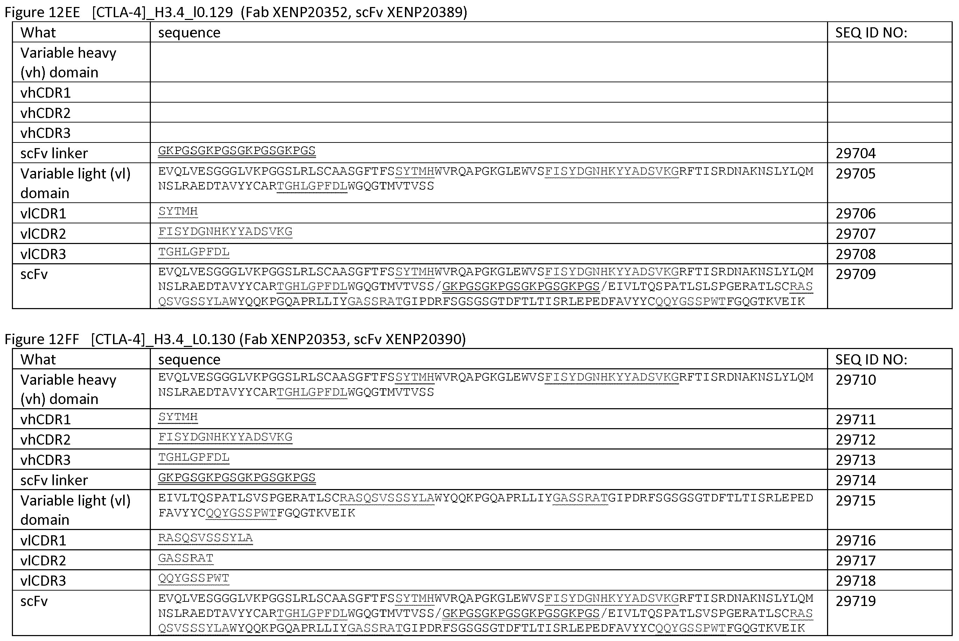

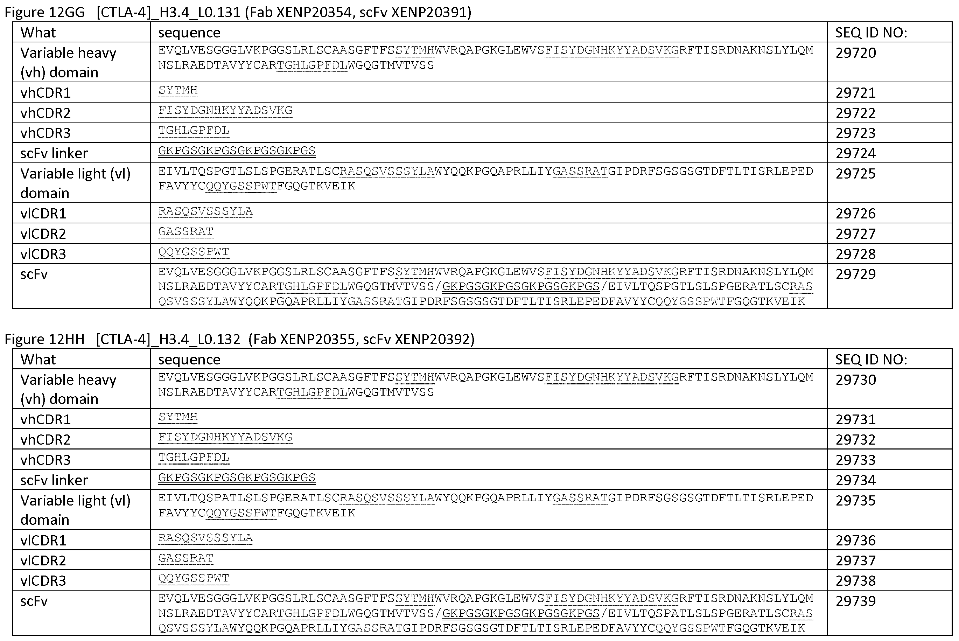

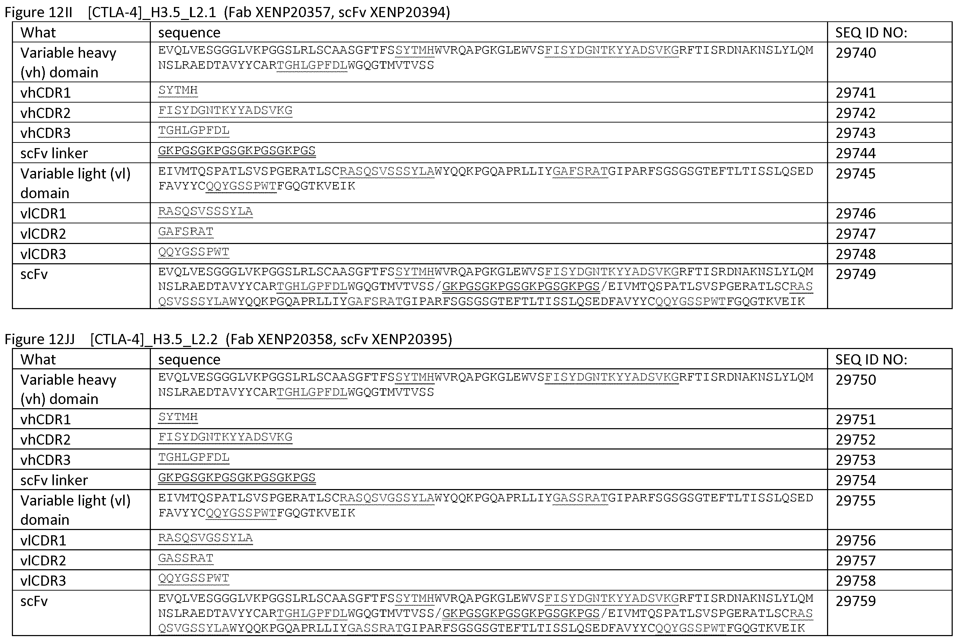

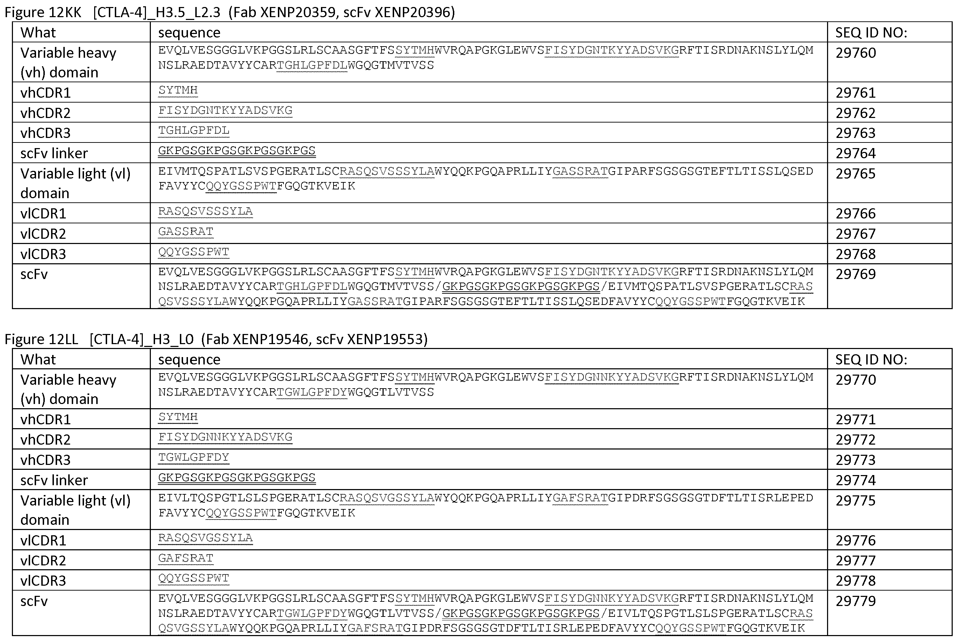

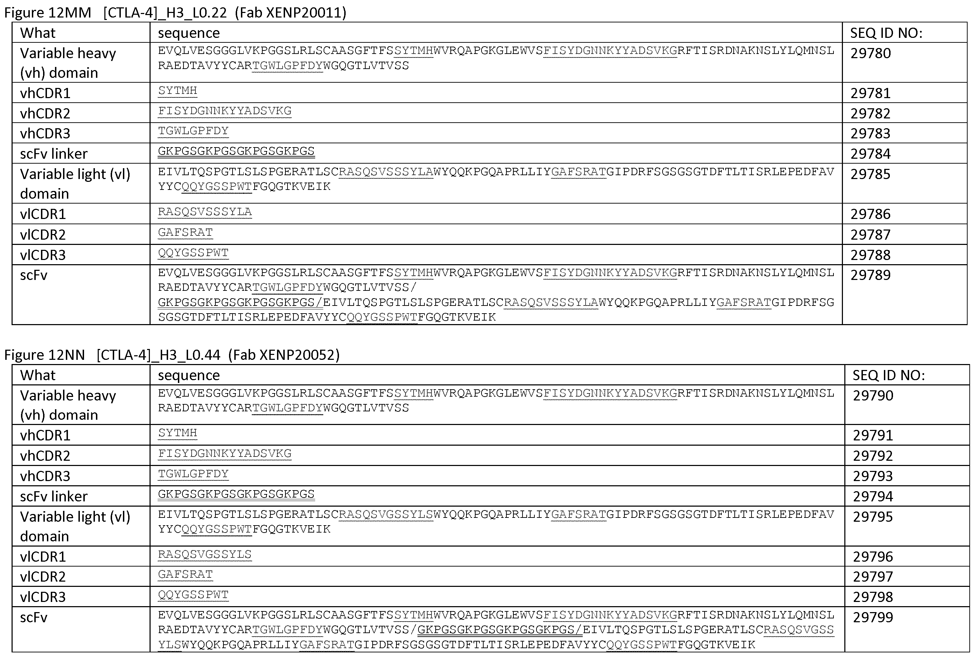

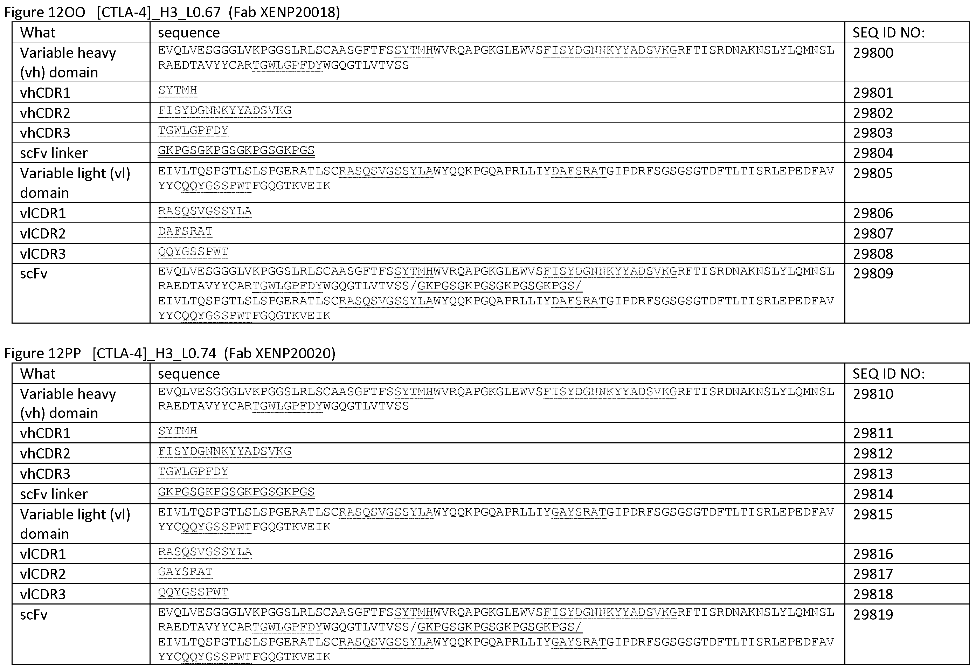

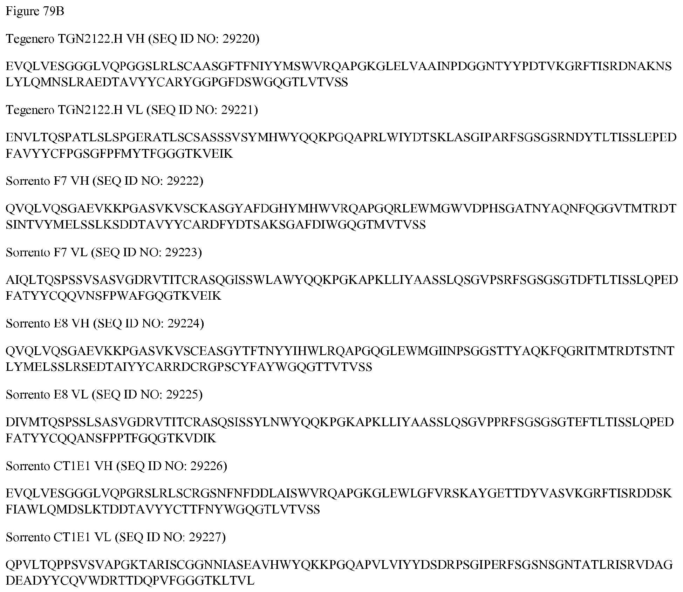

In some examples, one of the ABDs binds CTLA-4. Suitable sets of 6 CDRs and/or vh and vl domains, as well as scFv sequences, are depicted in SEQ ID NOs: 2393-2414 and 3737-3816, as well as sequences of particular interest in some examples are shown in Figure 12 and Figure 79 and also include those sequences in the sequence listing with the identifiers [CTLA-4]_H0.25_L0; [CTLA-4]_H0.26_L0; [CTLA-4]_H0.27_L0; [CTLA-4]_H0.29_L0; [CTLA-4]_H0.38_L0; [CTLA-4]_H0.39_L0; 0[CTLA-4]_H0.40_L0; [CTLA-4]H0.70_L0; [CTLA-4]_H0_L0.22; [CTLA-4]_H2_L0; [CTLA-4]_H3.21_L0.124; [CTLA-4]_H3.21_L0.129; [CTLA-4]_H3.21_L0.132; [CTLA-4]_H3.23_L0.124; [CTLA-4]_H3.23_L0.129; [CTLA-4]_H3.23_L0.132; [CTLA-4]_H3.25_L0.124; [CTLA-4]_H3.25_L0.129; [CTLA-4]_H3.25_L0.132; [CTLA-4]_H3.4_L0.118; [CTLA-4]_H3.4_L0.119; [CTLA-4]_H3.4_L0.12; [CTLA-4]_H3.4_L0.121; [CTLA-4]_H3.4_L0.122; [CTLA-4]_H3.4_L0.123; [CTLA-4]_H3.4_L0.124; [CTLA-4]_H3.4_L0.125; [CTLA-4]_H3.4_L0.126; [CTLA-4]_H3.4_L0.127; [CTLA-4]_H3.4_L0.128; [CTLA-4]_H3.4_L0.129; [CTLA-4]_H3.4_L0.130; [CTLA-4]_H3.4_L0.131; [CTLA-4]_H3.4_L0.132; [CTLA-4]_H3.5_L2.1; [CTLA-4]_H3.5_L2.2; [CTLA-4]_H3.5_L2.3; [CTLA-4]_H3_L0; [CTLA-4]_H3_L0.22; [CTLA-4]_H3_L0.44; [CTLA-4]_H3_L0.67; and [CTLA-4]_H3_L0.74.

As will be appreciated by those in the art, suitable anti-CTLA-4 ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences outlined herein. Suitable ABDs can also include the entire vh and vl sequences as depicted in these sequences and Figures, used as scFvs or as Fabs. In many of the examples herein that contain an Fv to CTLA-4, it is the scFv monomer that binds CTLA-4.
As discussed herein, the other of the target pair when CTLA-4 is one of the antigens is selected from ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

In addition to the parental CDR sets disclosed in the sequence listing that form an ABD to CTLA-4, the invention provides variant CDR sets. In one example, a set of 6 CDRs can have 1, 2, 3, 4 or 5 amino acid changes from the parental CDRs, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to CTLA-4, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of aBiacore, surface plasmon resonance (SPR) and/or BLI (biolayer interferometry, e.g. Octet assay) assay, with the latter finding particular use in many examples.

### D. TIM-3 Antigen Binding Domains

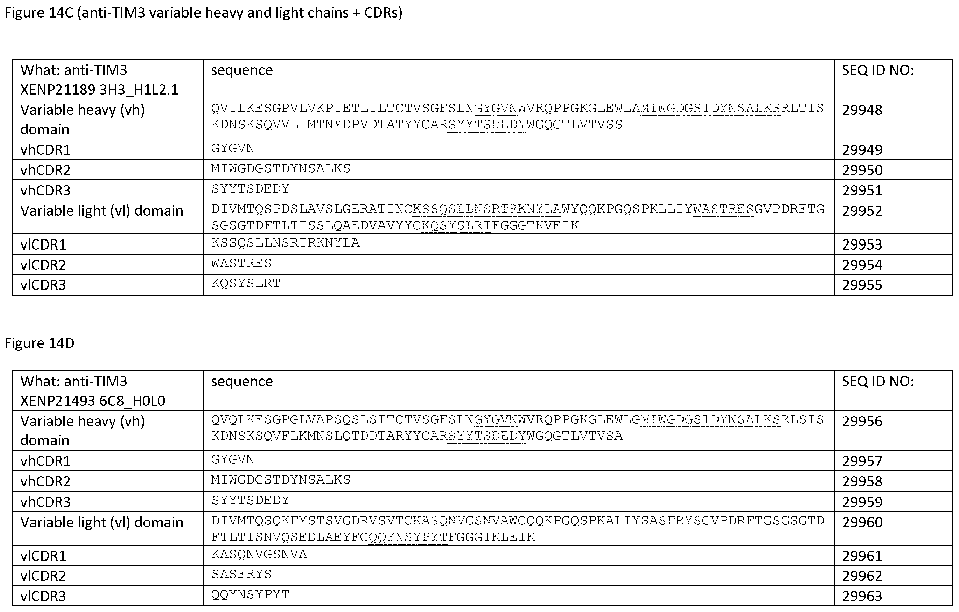

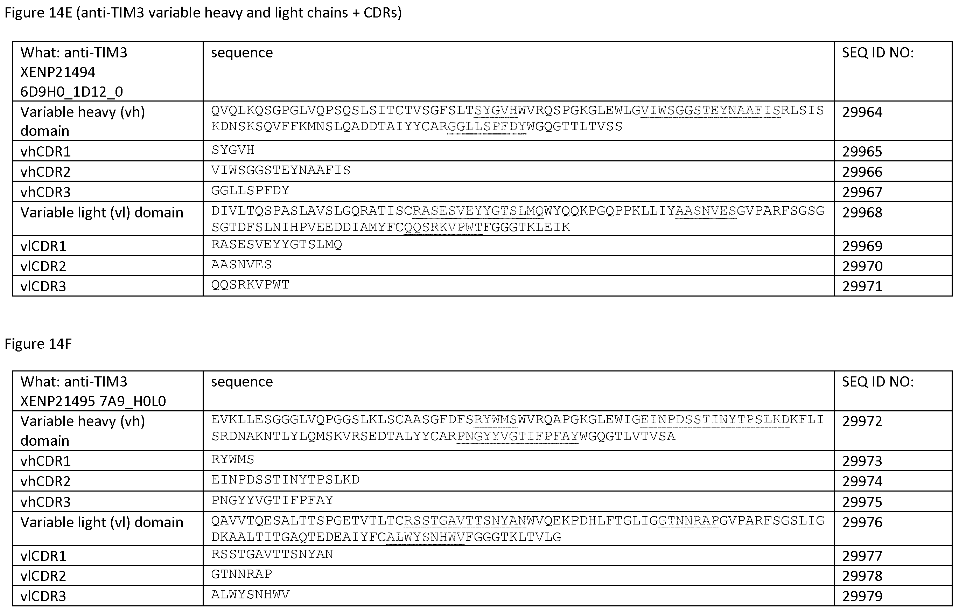

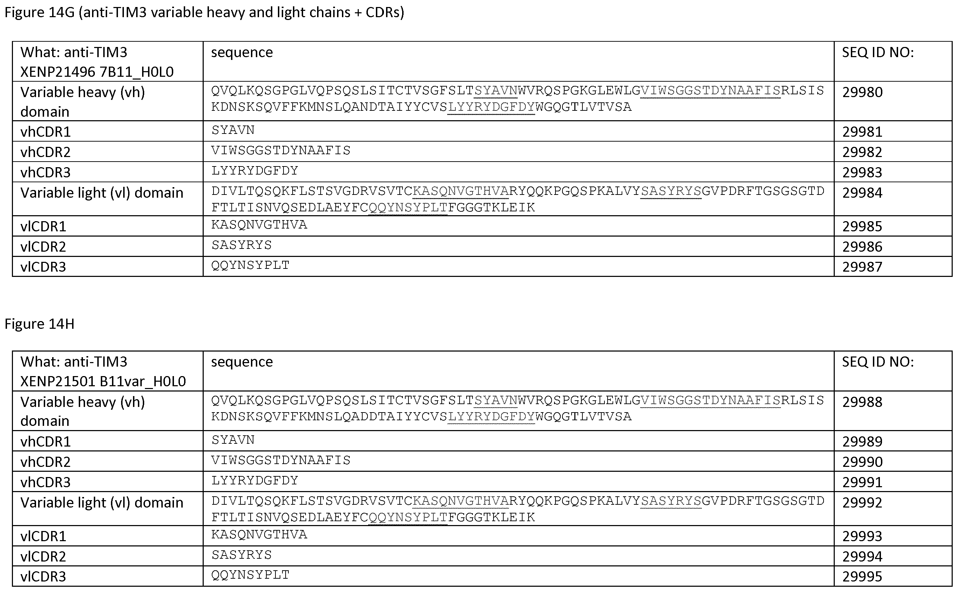

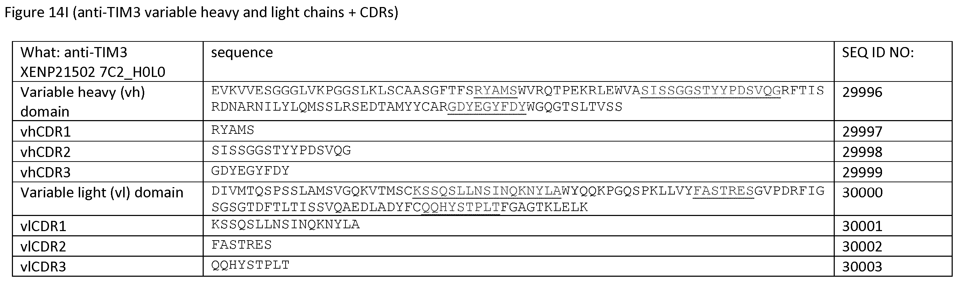

In some examples, one of the ABDs binds TIM-3. Suitable sets of 6 CDRs and/or vh and vl domains, as well as scFv sequences, are depicted in Figure 14 and Figure 81A-81C and SEQ ID NO: 3345-3704, 4585-4696. ABD sequences of particular interest in some examples include those sequences in the sequence listing with the identifiers 1D10_H0L0; 1D12_H0L0; 3H3_H1_L2.1; 6C8_H0L0; 6D9_H0_1D12_L0; 7A9_H0L0; 7B11_H0L0; 7B11var_H0L0; and 7C2_H0L0.

As will be appreciated by those in the art, suitable anti-TIM-3 ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences of those depicted herein. Suitable ABDs can also include the entire vh and vl sequences as depicted in these sequences and Figures, used as scFvs or as Fabs. In many of the examples herein that contain an Fv to TIM-3, it is the Fab monomer that binds TIM-3. As discussed herein, the other of the target pair when TIM-3 is one of the antigens is selected ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

In addition to the parental CDR sets disclosed in the sequence listing that form an ABD to TIM-3, the invention provides variant CDR sets. In one example, a set of 6 CDRs can have 1, 2, 3, 4 or 5 amino acid changes from the parental CDRs, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to TIM-3, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of aBiacore, surface plasmon resonance (SPR) and/or BLI (biolayer interferometry, e.g. Octet assay) assay, with the latter finding particular use in many examples.

### LAG-3 Antigen Binding Domains

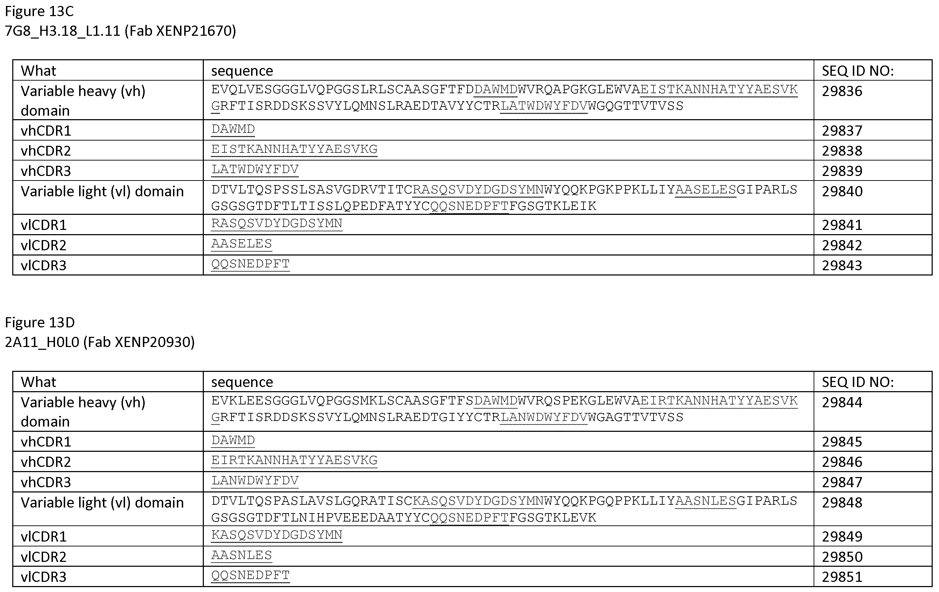

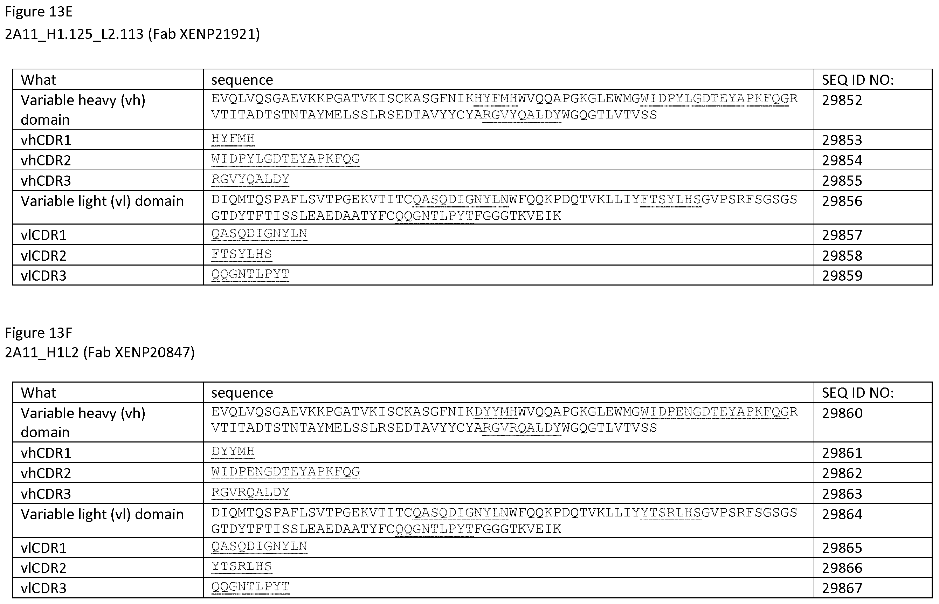

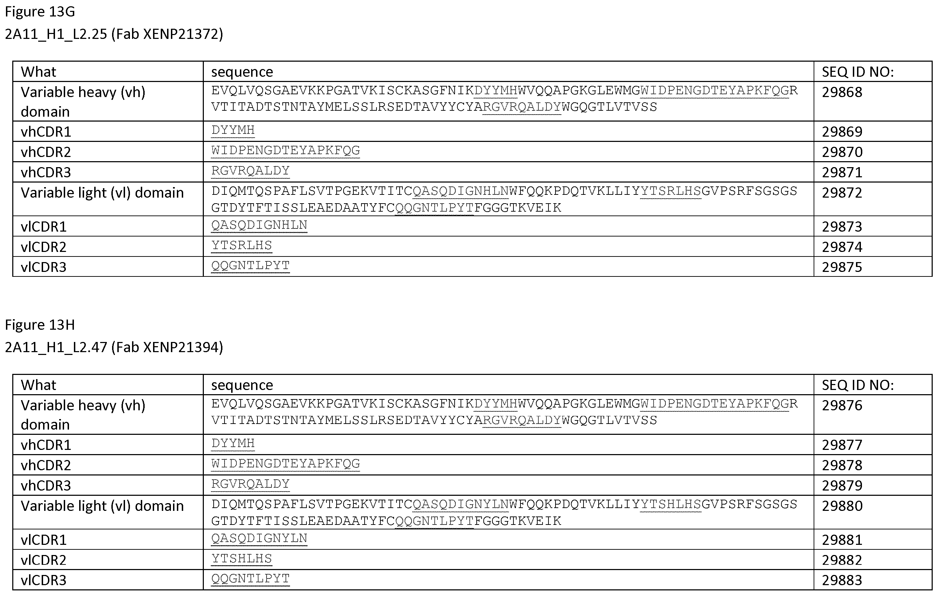

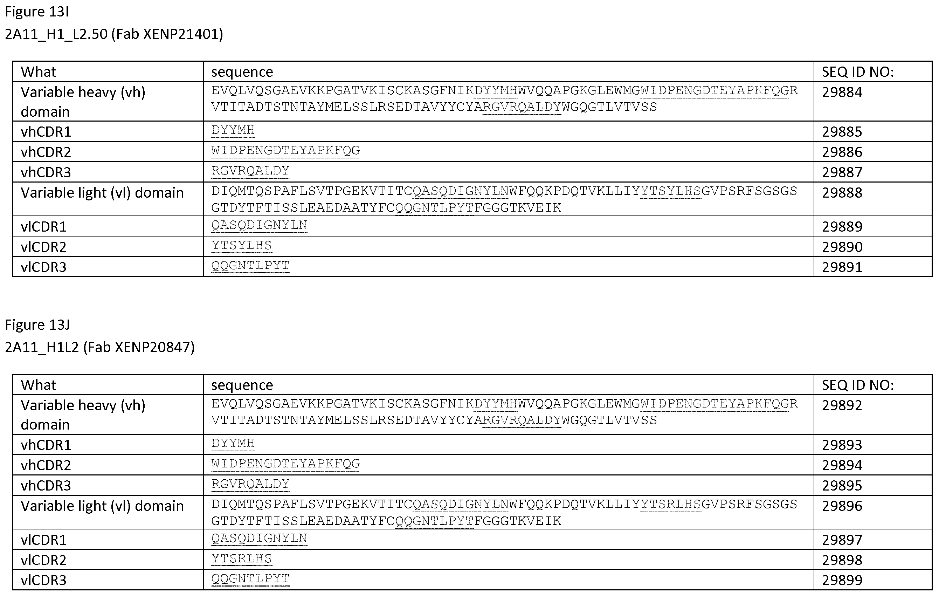

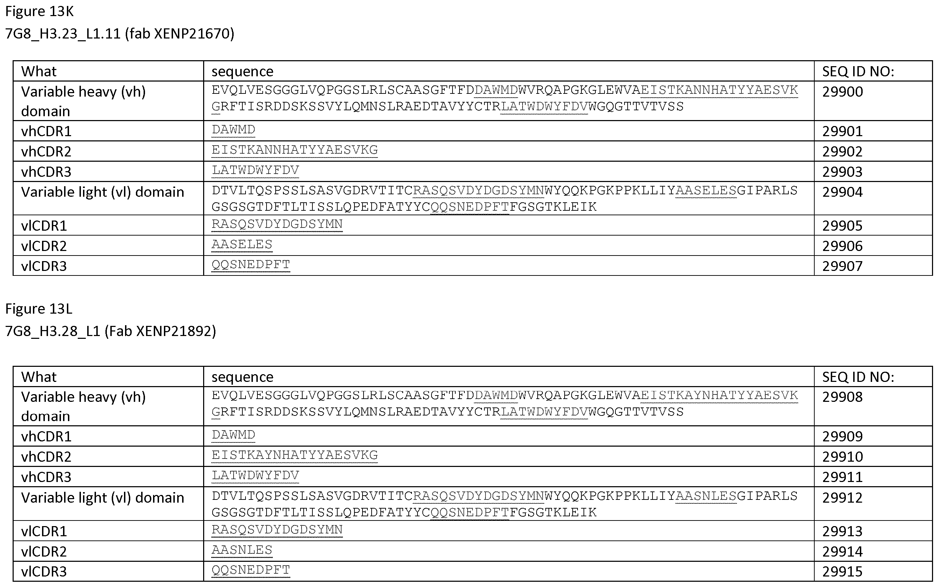

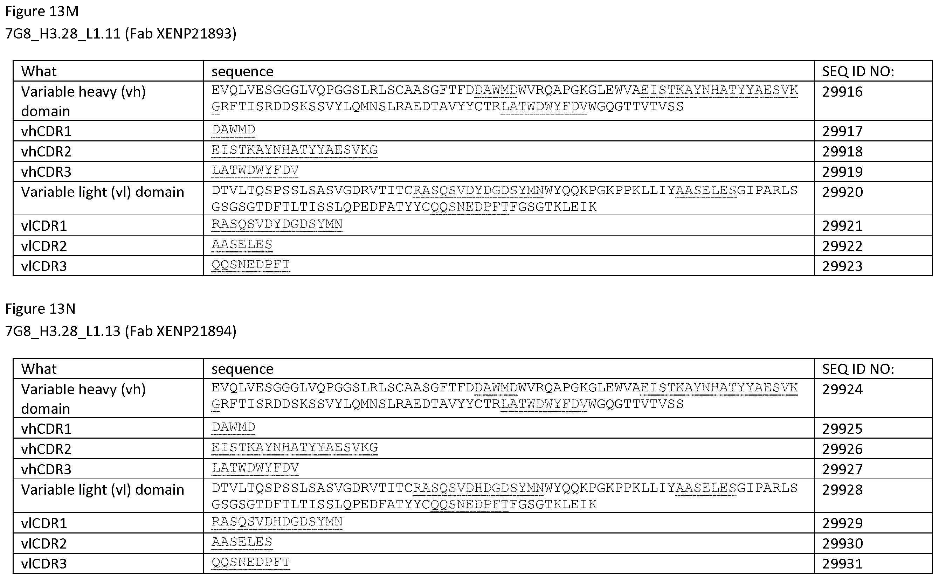

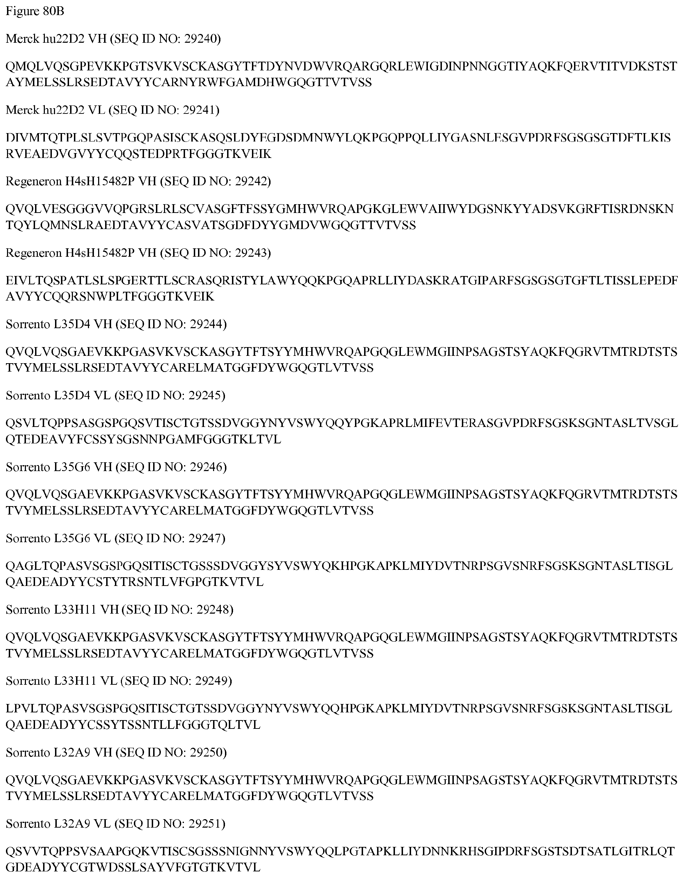

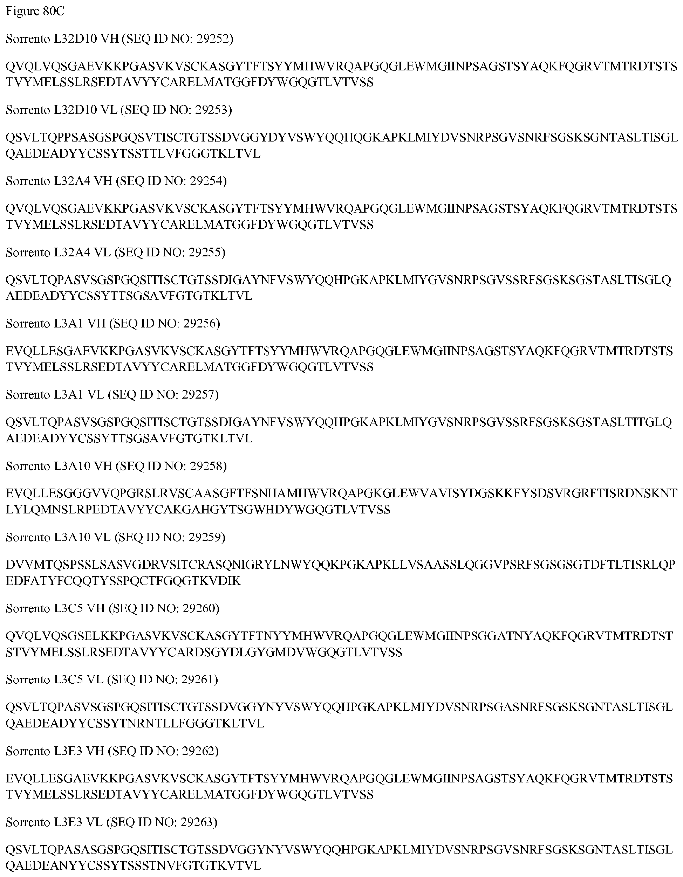

In some examples, one of the ABDs binds LAG-3. Suitable sets of 6 CDRs and/or vh and vl domains, as well as scFv sequences, are depicted in Figure 13, Figure 80 and in SEQ ID NO: 2415-2604, 3817-3960 also include those sequences in the sequence listing with the identifiers 2A11_H0L0; 2A11_H1.125_L2.113; 2A11_H1.144_L2.142; 2A11_H1_L2.122; 2A11_H1_L2.123; 2A11_H1_L2.124; 2A11_H1_L2.25; 2A11_H1_L2.47; 2A11_H1_L2.50; 2A11_H1_L2.91; 2A11_H1_L2.93; 2A11_H1_L2.97; 2A11_H1L1; 2A11_H1L2; 2A11_H2L2; 2A11_H3L1; 2A11_H3L2; 2A11_H4L1; 2A11_H4L2; 7G8_H0L0; 7G8_H1L1; 7G8_H3.18_L1.11; 7G8_H3.23_L1.11; 7G8_H3.28_L1; 7G8_H3.28_L1.11; 7G8_H3.28_L1.13; 7G8_H3.30_L1.34; 7G8_H3.30_L1.34; and 7G8_H3L1.

As will be appreciated by those in the art, suitable anti-LAG-3 ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences of the sequences herein. Suitable ABDs can also include the entire vh and vl sequences as depicted in these sequences and Figures, used as scFvs or as Fabs. In many of the examples herein that contain an Fv to LAG-3, it is the Fab monomer that binds LAG-3. As discussed herein, the other of the target pair when LAG-3 is is one of the antigens is selected from ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

In addition to the parental CDR sets disclosed in the sequence listing that form an ABD to LAG-3, the invention provides variant CDR sets. In one example, a set of 6 CDRs can have 1, 2, 3, 4 or 5 amino acid changes from the parental CDRs, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to LAG-3, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of aBiacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

### E. BTLA Antigen Binding Domains

In some examples, one of the ABDs binds BTLA. Suitable sets of 6 CDRs and/or vh and vl domains, as well as scFv sequences, are depicted in Figure 82, Figure 84A-84C and SEQ ID NO:3705-3736, and also include those sequences in the sequence listing with the identifiers 9C6_H0L0; 9C6_H1.1_L1; and 9C6_H1.11_L1.

As will be appreciated by those in the art, suitable anti-BTLA ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences depicted herein. Suitable ABDs can also include the entire vh and vl sequences as depicted in these sequences and Figures, used as scFvs or as Fabs. In many of the examples herein that contain an Fv to BTLA, it is the Fab monomer that binds BTLA. As discussed herein, the other of the target pair when BTLA is is one of the antigens is selected from ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

In addition to the parental CDR sets disclosed in the sequence listing that form an ABD to BTLA, the disclosure provides variant CDR sets. In one example, a set of 6 CDRs can have 1, 2, 3, 4 or 5 amino acid changes from the parental CDRs, as long as the ABD is still able to bind to the target antigen, as measured at least one of aBiacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to BTLA, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

### F. TIGIT Antigen Binding Domains

In some examples, one of the ABDs binds TIGIT. Suitable sets of 6 CDRs and/or vh and vl domains, as well as scFv sequences, are depicted in Figure 8A-8B And in SEQ ID NO:4433-4585.

As will be appreciated by those in the art, suitable anti- TIGIT ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences depicted herein. Suitable ABDs can also include the entire vh and vl sequences as depicted in these sequences and Figures, used as scFvs or as Fabs. In many of the examples herein that contain an Fv to TIGIT, it is the Fab monomer that binds TIGIT. As discussed herein, the other of the target pair when TIGIT is is one of the antigens is selected ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

### G. PD-L1 Antigen Binding Domains

In some examples, one of the ABDs binds PD-L1. Suitable sets of 6 CDRs and/or vh and vl domains, as well as scFv sequences, are depicted in Figure 15A-15C, Figure 73 and Figure 78, and in SEQ ID NO:3961-4432.

As will be appreciated by those in the art, suitable anti- TIGIT ABDs can comprise a set of 6 CDRs as depicted in these sequences and Figures, either as they are underlined or, in the case where a different numbering scheme is used as described herein and as shown in Table 1, as the CDRs that are identified using other alignments within the vh and vl sequences depicted herein. Suitable ABDs can also include the entire vh and vl sequences as depicted in these sequences and Figures, used as scFvs or as Fabs. In many of the examples herein that contain an Fv to TIGIT, it is the Fab monomer that binds TIGIT. As discussed herein, the other of the target pair when TIGIT is is one of the antigens is selected ICOS (suitable sequences are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0), GITR, OX40 and 4-1BB.

### H. ICOS Antigen Binding Domains

In some embodiments, one of the ABDs binds ICOS. Suitable sets of 6 CDRs and/or vh and vl domains, as well as scFv sequences ICOS (suitable sequences relating to the disclosure are shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

As will be appreciated by those in the art, suitable anti-ICOS ABDs can comprise a set of 6 CDRs as depicted in these sequences and Figures, either as they are underlined or, in the case where a different numbering scheme is used as described herein and as shown in Table 1, as the CDRs that are identified using other alignments within the sequences disclosed herein. Suitable ABDs can also include the entire vh and vl sequences as depicted in these sequences and Figures, used as scFvs or as Fabs. In many of the embodiments herein that contain an Fv to ICOS, it is the Fab monomer that binds ICOS. As discussed herein, the other of the target pair when ICOS is one of the antigens is selected from PD-1 are depicted in Figure 3, Figure 10, Figure 11, Figure 72, Figure 73, Figure 74, Figure 76 and SEQ ID NO:1-2392, 3125-3144, 4697-7594 and 4697-21810, and include those sequences in the sequence listing with the identifiers 1G6_H1.279_L1.194; 1G6_H1.280_L1.224; 1G6_L1.194_H1.279; 1G6_L1.210_H1.288; and 2E9_H1L1 (which can be scFv sequences, CDR sequence sets or vh and vl sequences)), CTLA-4 (suitable sequences are depicted in Figures 12, 72 and 79, as well as SEQ ID NO:2393-2414 and 3737-3816 (which can be scFv sequences, CDR sequence sets or vh and vl sequences)), TIM-3 (suitable sequences are depicted in Figure 14, Figure 81A-81C and SEQ ID NO: 3345-3704, 4585-4696 (which can be scFv sequences, CDR sequence sets or vh and vl sequences)), LAG-3 (suitable sequences are depicted in Figure 13, Figure 80 and SEQ ID NO:2415-2604, 3817-3960 (which can be scFv sequences, CDR sequence sets or vh and vl sequences)), BTLA (suitable sequences are depicted in Fiugre 82 and 84 and SEQ ID NO: 3705-3736 (which can be scFv sequences, CDR sequence sets or vh and vl sequences)), and TIGIT (suitable sequences are depicted in Figure 83A-83D and SEQ ID NO:4433-4585 (which can be scFv sequences, CDR sequence sets or vh and vl sequences)).

In addition to the parental CDR sets disclosed in the sequence listing that form an ABD to ICOS, the invention provides variant CDR sets. In one example, a set of 6 CDRs can have 1, 2, 3, 4 or 5 amino acid changes from the parental CDRs, as long as the ABD is still able to bind to the target antigen, as measured at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to ICOS, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

Specific preferred examples include the [ICOS]_H0.66_L0 anti-ICOS Fv, in a Fab format, included within any of the bottle opener format backbones of Figure 9.

Specific preferred embodiments include the [ICOS]_H0_L0 anti-ICOS Fv, in a scFv format, included within any of the bottle opener format backbones of Figure 9.

Specific preferred examples include the [ICOS]_H0.66_L0 anti-ICOS Fv, in a scFv format, included within any of the mAb-scFv format backbones of Figure 75.

Specific preferred examples include the [ICOS]_H0.66_L0 anti-ICOS Fv, in a Fab format, included within any of the mAb-scFv format backbones of Figure 75.

Specific preferred examples include the [ICOS]_H0.66_L0 anti-ICOS Fv, in a Fab format, included within any of the format backbones of Figure 55.

### I. GITR Antigen Binding Domains

In some examples, one of the ABDs binds GITR. Suitable sets of 6 CDRs and/or vh and vl domains, as well as scFv sequences FITR (suitable sequences are shown in Figure 18, Figure 72 and Figure 73, and in SEQ ID NO: 26282-26290.

As will be appreciated by those in the art, suitable anti-GITR ABDs can comprise a set of 6 CDRs as depicted in these sequences and Figures, either as they are underlined or, in the case where a different numbering scheme is used as described herein and as shown in Table 1, as the CDRs that are identified using other alignments within the sequences disclosed herein. Suitable ABDs can also include the entire vh and vl sequences as depicted in these sequences and Figures, used as scFvs or as Fabs. In many of the examples herein that contain an Fv to GITR, it is the Fab monomer that binds GITR. As discussed herein, the other of the target pair when GITR is one of the antigens is selected from PD-1 are depicted in Figure 3, Figure 10, Figure 11, Figure 72, Figure 73, Figure 74, Figure 76 and SEQ ID NO:1-2392, 3125-3144, 4697-7594 and 4697-21810, and include those sequences in the sequence listing with the identifiers 1G6_H1.279_L1.194; 1G6_H1.280_L1.224; 1G6_L1.194_H1.279; 1G6_L1.210_H1.288; and 2E9_H1L1 (which can be scFv sequences, CDR sequence sets or vh and vl sequences)), CTLA-4 (suitable sequences are depicted in Figures 12, 72 and 79, as well as SEQ ID NO:2393-2414 and 3737-3816 (which can be scFv sequences, CDR sequence sets or vh and vl sequences)), TIM-3 (suitable sequences are depicted in Figure 14, Figure 81A-81C and SEQ ID NO: 3345-3704, 4585-4696 (which can be scFv sequences, CDR sequence sets or vh and vl sequences)), LAG-3 (suitable sequences are depicted in Figure 13, Figure 80 and SEQ ID NO:2415-2604, 3817-3960 (which can be scFv sequences, CDR sequence sets or vh and vl sequences)), BTLA (suitable sequences are depicted in Fiugre 82 and 84 and SEQ ID NO: 3705-3736 (which can be scFv sequences, CDR sequence sets or vh and vl sequences)), and TIGIT (suitable sequences are depicted in Figure 83A-83D and SEQ ID NO:4433-4585 (which can be scFv sequences, CDR sequence sets or vh and vl sequences)).

In addition to the parental CDR sets disclosed in the sequence listing that form an ABD to GITR, the invention provides variant CDR sets. In one example, a set of 6 CDRs can have 1, 2, 3, 4 or 5 amino acid changes from the parental CDRs, as long as the ABD is still able to bind to the target antigen, as measured at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to GITR, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

### J. OX40 Antigen Binding Domains

In some examples, one of the ABDs binds OX40. Suitable sets of 6 CDRs and/or vh and vl domains, as well as scFv sequences for OX40 are provided (suitable sequences are shown in Figure 17, Figure 72 and Figure 73, and in SEQ ID NO: 26272-26281.

As will be appreciated by those in the art, suitable anti-OX40 ABDs can comprise a set of 6 CDRs as depicted in these sequences and Figures, either as they are underlined or, in the case where a different numbering scheme is used as described herein and as shown in Table 1, as the CDRs that are identified using other alignments within the sequences disclosed herein. Suitable ABDs can also include the entire vh and vl sequences as depicted in these sequences and Figures, used as scFvs or as Fabs. In many of the examples herein that contain an Fv to OX40, it is the Fab monomer that binds GITR. As discussed herein, the other of the target pair when OX40 is one of the antigens is selected from PD-1 are depicted in Figure 3, Figure 10, Figure 11, Figure 72, Figure 73, Figure 74, Figure 76 and SEQ ID NO:1-2392, 3125-3144, 4697-7594 and 4697-21810, and include those sequences in the sequence listing with the identifiers 1G6_H1.279_L1.194; 1G6_H1.280_L1.224; 1G6_L1.194_H1.279; 1G6_L1.210_H1.288; and 2E9_H1L1 (which can be scFv sequences, CDR sequence sets or vh and vl sequences)), CTLA-4 (suitable sequences are depicted in Figures 12, 72 and 79, as well as SEQ ID NO:2393-2414 and 3737-3816 (which can be scFv sequences, CDR sequence sets or vh and vl sequences)), TIM-3 (suitable sequences are depicted in Figure 14, Figure 81A-81C and SEQ ID NO: 3345-3704, 4585-4696 (which can be scFv sequences, CDR sequence sets or vh and vl sequences)), LAG-3 (suitable sequences are depicted in Figure 13, Figure 80 and SEQ ID NO:2415-2604, 3817-3960 (which can be scFv sequences, CDR sequence sets or vh and vl sequences)), BTLA (suitable sequences are depicted in Fiugre 82 and 84 and SEQ ID NO:3705-3736 (which can be scFv sequences, CDR sequence sets or vh and vl sequences)), and TIGIT (suitable sequences are depicted in Figure 83A-83D and SEQ ID NO:4433-4585 (which can be scFv sequences, CDR sequence sets or vh and vl sequences)).

In addition to the parental CDR sets disclosed in the sequence listing that form an ABD to OX40, the invention provides variant CDR sets. In one example, a set of 6 CDRs can have 1, 2, 3, 4 or 5 amino acid changes from the parental CDRs, as long as the ABD is still able to bind to the target antigen, as measured at least one of a Biacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to OX40, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

### K. 4-1BB Antigen Binding Domains

In some examples, one of the ABDs binds 4-1BB. Suitable sets of 6 CDRs and/or vh and vl domains, as well as scFv sequences 4-1BB (suitable sequences are shown in Figure 16, Figure 72 and Figure 73, and in SEQ ID NO:26262-2671.

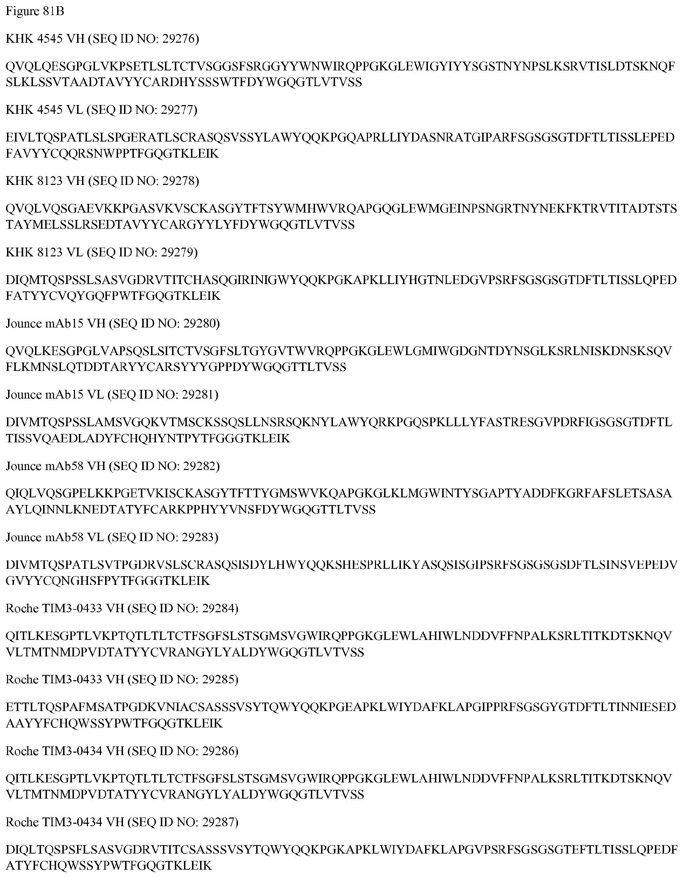

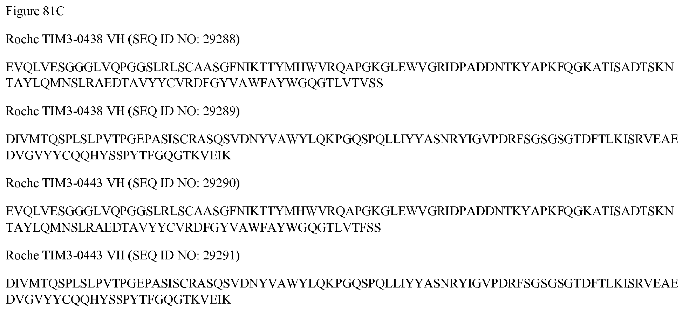

As will be appreciated by those in the art, suitable anti-4-1BB ABDs can comprise a set of 6 CDRs as depicted in these sequences and Figures, either as they are underlined or, in the case where a different numbering scheme is used as described herein and as shown in Table 1, as the CDRs that are identified using other alignments within the sequences disclosed herein. Suitable ABDs can also include the entire vh and vl sequences as depicted in these sequences and Figures, used as scFvs or as Fabs. In many of the examples herein that contain an Fv to 4-1BB, it is the Fab monomer that binds 4-1BB. As discussed herein, the other of the target pair when ICOS is one of the antigens is selected from PD-1 are depicted in Figure 3, Figure 10, Figure 11, Figure 72, Figure 73, Figure 74, Figure 76 and SEQ ID NO:1-2392, 3125-3144, 4697-7594 and 4697-21810, and include those sequences in the sequence listing with the identifiers 1G6_H1.279_L1.194; 1G6_H1.280_L1.224; 1G6_L1.194_H1.279; 1G6_L1.210_H1.288; and 2E9_H1L1 (which can be scFv sequences, CDR sequence sets or vh and vl sequences)), CTLA-4 (suitable sequences are depicted in Figures 12, 72 and 79, as well as SEQ ID NO:2393-2414 and 3737-3816 (which can be scFv sequences, CDR sequence sets or vh and vl sequences)), TIM-3 (suitable sequences are depicted in Figure 14, Figure 81 and SEQ ID NO: 3345-3704, 4585-4696 (which can be scFv sequences, CDR sequence sets or vh and vl sequences)), LAG-3 (suitable sequences are depicted in Figure 13, Figure 80 and SEQ ID NO:2415-2604, 3817-3960 (which can be scFv sequences, CDR sequence sets or vh and vl sequences)), BTLA (suitable sequences are depicted in Fiugre 82 and 84 and SEQ ID NO: 3705-3736 (which can be scFv sequences, CDR sequence sets or vh and vl sequences)), and TIGIT (suitable sequences are depicted in Figure 83A-83DXX and SEQ ID NO:4433-4585 (which can be scFv sequences, CDR sequence sets or vh and vl sequences)).

In addition to the parental CDR sets disclosed in the sequence listing that form an ABD to 4-1BB, the invention provides variant CDR sets. In one example, a set of 6 CDRs can have 1, 2, 3, 4 or 5 amino acid changes from the parental CDRs, as long as the ABD is still able to bind to the target antigen, as measured at least one of aBiacore, surface plasmon resonance (SPR) and/or BLI (biolayer interferometry, e.g. Octet assay) assay, with the latter finding particular use in many examples.

In addition to the parental variable heavy and variable light domains disclosed herein that form an ABD to 4-1BB, the invention provides variant vh and vl domains. In one example, the variant vh and vl domains each can have from 1, 2, 3, 4, 5, 6, 7, 8, 9 or 10 amino acid changes from the parental vh and vl domain, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples. In another example, the variant vh and vl are at least 90, 95, 97, 98 or 99% identical to the respective parental vh or vl, as long as the ABD is still able to bind to the target antigen, as measured by at least one of a Biacore, surface plasmon resonance (SPR) and/or BLI (biolayer interferometry, e.g. Octet assay) assay, with the latter finding particular use in many examples.

### L. Specific Bispecific examples

The disclosure provides a number of particular bispecific antibodies as outlined below.

### 1. ICOS X PD-1

The invention provides bispecific heterodimeric antibodies that bind ICOS and PD-1 each monovalently.

In one example of the disclosure, the PD-1 ABD is 1G6_L1.194_H1.279 and the ICOS ABD is selected from sequences shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

In one example, the ICOS X PD-1 bispecific antibody is selected from XENP numbers 20261, 20730, 20896, 22432-22438, 22731-22748, 22878-22894, 22931-22932, 22950-22961, 23090-23093, 23295-23296, 23301, 23405, 23408, 23410 (all without 428L/434S, although they can have those substitutions); XENP numbers 22730, 22917-22928, 22935-22937, 22974-22979, 22995-22996, 23001, 23103, 23104 (all with 428L/434S, although they can not have those); XENP numbers 23411, 21828, 21829, 21830, 21831, 22348, 23059 (using prior art ICOS sequences); XENP numbers 18920, 24125, 24130 (additional bottle openers); XENP 23406, 23407, 24128 (ICOS x PD-1 central-scFv), XENP24123 (ICOS x PD-1 central-scFv2); XENP24134 (ICOS x PD-1 bispecific mAb) 24122 (ICOS x PD-1 DVD-Ig), XENP 24132, 24133 (ICOS x PD-1 Trident).

### 2. ICOS X PD-L1

In this example, the ICOS ABD is selected from sequences shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

### 3. ICOS X CTLA-4

In this example, the ICOS ABD is selected from sequences shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

In one example, a bottle opener format with a Fab ICOS ABD of [ICOS]H0.66_L0 is paired with a scFv CTLA-4 ABD of [CTLA-4]_H3.32_L0.129, particularly in bottle opener backbone 1 from Figure 9.

### 4. ICOS X LAG-3

In this example, the ICOS ABD is selected from sequences shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

### 5. ICOS X TIM-3

In this example, the ICOS ABD is selected from sequences shown in Figure 19, Figure 20A-20G, Figure 24, Figure 68A-68G and Figure 77A-77B as well as SEQ ID NO: 27869-28086, 28087-28269, 27193-27335, 28549-28556 and 28557-28665, and those with the identifiers [ICOS]_H0.66_L0 and [ICOS]_H0L0.

### M. Homologous Antibodies

The disclosure further provides antibodies that share amino acid sequence identity with the antibodies outlined herein.

In one example, bispecific antibodies are made that have amino acid variants in one or more of the CDRs of the Vh and Vl sequences outlined herein and in the Figures. In one example, antibodies are provided that have 1, 2, 3, 4 or 5 amino acid differences in one or more of the CDRs of the vh and vl chains outlined herein. These amino acid variants can be in one CDR or spread out between more than one CDR. These amino acid variants can also be in one or both of the Fvs of the bispecific antibody; e.g. there can be 2 amino acid variants in CDRs on the ICOS Fv side (generally a Fab but can be a scFv as outlined herein) and one on the PD-1 side, etc.

Similarly, the disclosure provides for antibodies that have at least 95, 96, 97 98 or 99% amino acid identity to the sequences outlined herein, and particularly in the variable heavy and/or variable light domains. This sequence identity can also be on one Fv or both Fv of the bispecific antibodies. That is, bispecific antibodies are provided that are 95-99% identical to the variable heavy and/or variable light domains outlined in the figures.

### IX. Nucleic acids of the disclosure

The disclosure further provides nucleic acid compositions encoding the bispecific antibodies of the invention (or, in the case of "monospecific" antibodies, nucleic acids encoding those as well).

As will be appreciated by those in the art, the nucleic acid compositions will depend on the format and scaffold of the heterodimeric protein. Thus, for example, when the format requires three amino acid sequences, , three nucleic acid sequences can be incorporated into one or more expression vectors for expression. Similarly, some formats (e.g. dual scFv formats such as disclosed in Figure 2) only two nucleic acids are needed; again, they can be put into one or two expression vectors. Some formats need 4 amino acids (bispecific mAbs).

As is known in the art, the nucleic acids encoding the components disclosed herein can be incorporated into expression vectors as is known in the art, and depending on the host cells used to produce the heterodimeric antibodies of the invention. Generally ,the nucleic acids are operably linked to any number of regulatory elements (promoters, origin of replication, selectable markers, ribosomal binding sites, inducers, etc.). The expression vectors can be extra-chromosomal or integrating vectors.

The nucleic acids and/or expression vectors disclosed herein may then transformed into any number of different types of host cells as is well known in the art, including mammalian, bacterial, yeast, insect and/or fungal cells, with mammalian cells (e.g. CHO cells), finding use in many examples.

In some examples, nucleic acids encoding each monomer and the optional nucleic acid encoding a light chain, as applicable depending on the format, are each contained within a single expression vector, generally under different or the same promoter controls. In examples of particular use in the present disclosure, each of these two or three nucleic acids are contained on a different expression vector. As shown herein, different vector ratios can be used to drive heterodimer formation. That is, surprisingly, while the proteins comprise first monomer:second monomer:light chains (in the case of many of the exampless herein that have three polypeptides comprising the heterodimeric antibody) in a 1:1:2 ratio, these are not the ratios that give the best results.

The heterodimeric antibodies of the invention are made by culturing host cells comprising the expression vector(s) as is well known in the art. Once produced, traditional antibody purification steps are done, including an ion exchange chromotography step. As discussed herein, having the pIs of the two monomers differ by at least 0.5 can allow separation by ion exchange chromatography or isoelectric focusing, or other methods sensitive to isoelectric point. That is, the inclusion of pI substitutions that alter the isoelectric point (pI) of each monomer so that such that each monomer has a different pI and the heterodimer also has a distinct pI, thus facilitating isoelectric purification of the "triple F" heterodimer (e.g., anionic exchange columns, cationic exchange columns). These substitutions also aid in the determination and monitoring of any contaminating dual scFv-Fc and mAb homodimers post-purification (e.g., IEF gels, cIEF, and analytical IEX columns).

### X. Biological and Biochemical Functionality of the Heterodimeric Immunomodulatory Antibodies

Generally the bispecific immunomodulatory antibodies of the invention are administered to patients with cancer, and efficacy is assessed, in a number of ways as described herein. Thus, while standard assays of efficacy can be run, such as cancer load, size of tumor, evaluation of presence or extent of metastasis, etc., immuno-oncology treatments can be assessed on the basis of immune status evaluations as well. This can be done in a number of ways, including both in vitro and in vivo assays. For example, evaluation of changes in immune status (e.g. presence of ICOS+ CD4+ T cells following ipi treatment) along with "old fashioned" measurements such as tumor burden, size, invasiveness, LN involvement, metastasis, etc. can be done. Thus, any or all of the following can be evaluated: the inhibitory effects of PVRIG on CD4⁺ T cell activation or proliferation, CD8⁺ T (CTL) cell activation or proliferation, CD8⁺ T cell-mediated cytotoxic activity and/or CTL mediated cell depletion, NK cell activity and NK mediated cell depletion, the potentiating effects of PVRIG on Treg cell differentiation and proliferation and Treg- or myeloid derived suppressor cell (MDSC)- mediated immunosuppression or immune tolerance, and/or the effects of PVRIG on proinflammatory cytokine production by immune cells, e.g., IL-2, IFN-γ or TNF-α production by T or other immune cells.

In some examples, assessment of treatment is done by evaluating immune cell proliferation, using for example, CFSE dilution method, Ki67 intracellular staining of immune effector cells, and 3H-Thymidine incorporation method,

In some examples, assessment of treatment is done by evaluating the increase in gene expression or increased protein levels of activation-associated markers, including one or more of: CD25, CD69, CD137, ICOS, PD1, GITR, OX40, and cell degranulation measured by surface expression of CD107A.

In general, gene expression assays are done as is known in the art.

In general, protein expression measurements are also similarly done as is known in the art.

In some examples, assessment of treatment is done by assessing cytotoxic activity measured by target cell viability detection via estimating numerous cell parameters such as enzyme activity (including protease activity), cell membrane permeability, cell adherence, ATP production, co-enzyme production, and nucleotide uptake activity. Specific examples of these assays include, but are not limited to, Trypan Blue or PI staining, ⁵¹Cr or ³⁵S release method, LDH activity, MTT and/or WST assays, Calcein-AM assay, Luminescent based assay, and others.

In some examples, assessment of treatment is done by assessing T cell activity measured by cytokine production, measure either intracellularly in culture supernatant using cytokines including, but not limited to, IFNy, TNFα, GM-CSF, IL2, IL6, IL4, IL5, IL10, IL13 using well known techniques.

Accordingly, assessment of treatment can be done using assays that evaluate one or more of the following: (i) increases in immune response, (ii) increases in activation of αβ and/or γδ T cells, (iii) increases in cytotoxic T cell activity, (iv) increases in NK and/or NKT cell activity, (v) alleviation of αβ and/or γδ T-cell suppression, (vi) increases in proinflammatory cytokine secretion, (vii) increases in IL-2 secretion; (viii) increases in interferon-γ production, (ix) increases in Th1 response, (x) decreases in Th2 response, (xi) decreases or eliminates cell number and/or activity of at least one of regulatory T cells (Tregs.

### Assays to measure efficacy

T cell activation may be assessed using a Mixed Lymphocyte Reaction (MLR) assay as is known in the art. An increase in activity indicates immunostimulatory activity. Appropriate increases in activity are outlined below.

In one example, the signaling pathway assay measures increases or decreases in immune response as measured for an example by phosphorylation or de-phosphorylation of different factors, or by measuring other post translational modifications. An increase in activity indicates immunostimulatory activity. Appropriate increases in activity are outlined below.

In one example, the signaling pathway assay measures increases or decreases in activation of αβ and/or γδ T cells as measured for an example by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in cytotoxic T cell activity as measured for an example by direct killing of target cells like for an example cancer cells or by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in NK and/or NKT cell activity as measured for an example by direct killing of target cells like for an example cancer cells or by cytokine secretion or by changes in expression of activation markers like for an example CD107a, etc. An increase in activity indicates immunostimulatory activity. Appropriate increases in activity are outlined below.

In one example, the signaling pathway assay measures increases or decreases in αβ and/or γδ T-cell suppression, as measured for an example by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in proinflammatory cytokine secretion as measured for example by ELISA or by Luminex or by Multiplex bead based methods or by intracellular staining and FACS analysis or by Alispot etc. An increase in activity indicates immunostimulatory activity. Appropriate increases in activity are outlined below.

In one example, the signaling pathway assay measures increases or decreases in IL-2 secretion as measured for example by ELISA or by Luminex or by Multiplex bead based methods or by intracellular staining and FACS analysis or by Alispot etc. An increase in activity indicates immunostimulatory activity. Appropriate increases in activity are outlined below.

In one example, the signaling pathway assay measures increases or decreases in interferon-γ production as measured for example by ELISA or by Luminex or by Multiplex bead based methods or by intracellular staining and FACS analysis or by Alispot etc. An increase in activity indicates immunostimulatory activity. Appropriate increases in activity are outlined below.

In one example, the signaling pathway assay measures increases or decreases in Th1 response as measured for an example by cytokine secretion or by changes in expression of activation markers. An increase in activity indicates immunostimulatory activity. Appropriate increases in activity are outlined below.

In one example, the signaling pathway assay measures increases or decreases in Th2 response as measured for an example by cytokine secretion or by changes in expression of activation markers. An increase in activity indicates immunostimulatory activity. Appropriate increases in activity are outlined below.

In one example, the signaling pathway assay measures increases or decreases cell number and/or activity of at least one of regulatory T cells (Tregs), as measured for example by flow cytometry or by IHC. A decrease in response indicates immunostimulatory activity. Appropriate decreases are the same as for increases, outlined below.

In one example, the signaling pathway assay measures increases or decreases in M2 macrophages cell numbers, as measured for example by flow cytometry or by IHC. A decrease in response indicates immunostimulatory activity. Appropriate decreases are the same as for increases, outlined below.

In one example, the signaling pathway assay measures increases or decreases in M2 macrophage pro-tumorigenic activity, as measured for an example by cytokine secretion or by changes in expression of activation markers. A decrease in response indicates immunostimulatory activity. Appropriate decreases are the same as for increases, outlined below.

In one example, the signaling pathway assay measures increases or decreases in N2 neutrophils increase, as measured for example by flow cytometry or by IHC. A decrease in response indicates immunostimulatory activity. Appropriate decreases are the same as for increases, outlined below.

In one example, the signaling pathway assay measures increases or decreases in N2 neutrophils pro-tumorigenic activity, as measured for an example by cytokine secretion or by changes in expression of activation markers. A decrease in response indicates immunostimulatory activity. Appropriate decreases are the same as for increases, outlined below.

In one example, the signaling pathway assay measures increases or decreases in inhibition of T cell activation, as measured for an example by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in inhibition of CTL activation as measured for an example by direct killing of target cells like for an example cancer cells or by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in αβ and/or γδ T cell exhaustion as measured for an example by changes in expression of activation markers. A decrease in response indicates immunostimulatory activity. Appropriate decreases are the same as for increases, outlined below.

In one example, the signaling pathway assay measures increases or decreases αβ and/or γδ T cell response as measured for an example by cytokine secretion or by proliferation or by changes in expression of activation markers like for an example CD137, CD107a, PD1, etc. An increase in activity indicates immunostimulatory activity. Appropriate increases in activity are outlined below.

In one example, the signaling pathway assay measures increases or decreases in stimulation of antigen-specific memory responses as measured for an example by cytokine secretion or by proliferation or by changes in expression of activation markers like for an example CD45RA, CCR7 etc. An increase in activity indicates immunostimulatory activity. Appropriate increases in activity are outlined below..

In one example, the signaling pathway assay measures increases or decreases in apoptosis or lysis of cancer cells as measured for an example by cytotoxicity assays such as for an example MTT, Cr release, Calcine AM, or by flow cytometry based assays like for an example CFSE dilution or propidium iodide staining etc. An increase in activity indicates immunostimulatory activity. Appropriate increases in activity are outlined below.

In one example, the signaling pathway assay measures increases or decreases in stimulation of cytotoxic or cytostatic effect on cancer cells. as measured for an example by cytotoxicity assays such as for an example MTT, Cr release, Calcine AM, or by flow cytometry based assays like for an example CFSE dilution or propidium iodide staining etc. An increase in activity indicates immunostimulatory activity. Appropriate increases in activity are outlined below.

In one example, the signaling pathway assay measures increases or decreases direct killing of cancer cells as measured for an example by cytotoxicity assays such as for an example MTT, Cr release, Calcine AM, or by flow cytometry based assays like for an example CFSE dilution or propidium iodide staining etc. An increase in activity indicates immunostimulatory activity. Appropriate increases in activity are outlined below.

In one example, the signaling pathway assay measures increases or decreases Th17 activity as measured for an example by cytokine secretion or by proliferation or by changes in expression of activation markers. An increase in activity indicates immunostimulatory activity. Appropriate increases in activity are outlined below.

In one example, the signaling pathway assay measures increases or decreases in induction of complement dependent cytotoxicity and/or antibody dependent cell-mediated cytotoxicity, as measured for an example by cytotoxicity assays such as for an example MTT, Cr release, Calcine AM, or by flow cytometry based assays like for an example CFSE dilution or propidium iodide staining etc. An increase in activity indicates immunostimulatory activity. Appropriate increases in activity are outlined below.

In one example, T cell activation is measured for an example by direct killing of target cells like for an example cancer cells or by cytokine secretion or by proliferation or by changes in expression of activation markers like for an example CD137, CD107a, PD1, etc. For T-cells, increases in proliferation, cell surface markers of activation (e.g. CD25, CD69, CD137, PD1), cytotoxicity (ability to kill target cells), and cytokine production (e.g. IL-2, IL-4, IL-6, IFNy, TNF-a, IL-10, IL-17A) would be indicative of immune modulation that would be consistent with enhanced killing of cancer cells.

In one example, NK cell activation is measured for example by direct killing of target cells like for an example cancer cells or by cytokine secretion or by changes in expression of activation markers like for an example CD107a, etc. For NK cells, increases in proliferation, cytotoxicity (ability to kill target cells and increases CD107a, granzyme, and perforin expression), cytokine production (e.g. IFNγ and TNF ), and cell surface receptor expression (e.g. CD25) would be indicative of immune modulation that would be consistent with enhanced killing of cancer cells.

In one example, γδ T cell activation is measured for example by cytokine secretion or by proliferation or by changes in expression of activation markers.

In one example, Th1 cell activation is measured for example by cytokine secretion or by changes in expression of activation markers.

Appropriate increases in activity or response (or decreases, as appropriate as outlined above), are increases of 10%, 20%, 30%, 40%, 50%, 60%, 70%, 80%, 90%, 95% or 98 to 99% percent over the signal in either a reference sample or in control samples, for example test samples that do not contain an anti-PVRIG antibody of the invention. Similarly, increases of at least one-, two-, three-, four- or five-fold as compared to reference or control samples show efficacy.

### XI. Treatments

Once made, the compositions of the invention find use in a number of oncology applications, by treating cancer, generally by inhibiting the suppression of T cell activation (e.g. T cells are no longer suppressed) with the binding of the bispecific immunomodulatory antibodies of the invention.

Accordingly, the heterodimeric compositions disclosed herein find use in the treatment of these cancers.

### XII. Antibody Compositions for In Vivo Administration

Formulations of the antibodies used in accordance with the present disclosure are prepared for storage by mixing an antibody having the desired degree of purity with optional pharmaceutically acceptable carriers, excipients or stabilizers (as generally outlined in Remington's Pharmaceutical Sciences 16th edition, Osol, A. Ed. [1980]), in the form of lyophilized formulations or aqueous solutions. Acceptable carriers, buffers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG).

### Administrative modalities

The antibodies and chemotherapeutic agents disclosed herein may be administered to a subject, in accord with known methods, such as intravenous administration as a bolus or by continuous infusion over a period of time.

### Treatment modalities

In the methods disclosed herein, therapy may be used to provide a positive therapeutic response with respect to a disease or condition. By "positive therapeutic response" is intended an improvement in the disease or condition, and/or an improvement in the symptoms associated with the disease or condition. For example, a positive therapeutic response would refer to one or more of the following improvements in the disease: (1) a reduction in the number of neoplastic cells; (2) an increase in neoplastic cell death; (3) inhibition of neoplastic cell survival; (5) inhibition (i.e., slowing to some extent, preferably halting) of tumor growth; (6) an increased patient survival rate; and (7) some relief from one or more symptoms associated with the disease or condition.

Positive therapeutic responses in any given disease or condition can be determined by standardized response criteria specific to that disease or condition. Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bone scan imaging, endoscopy, and tumor biopsy sampling including bone marrow aspiration (BMA) and counting of tumor cells in the circulation.

In addition to these positive therapeutic responses, the subject undergoing therapy may experience the beneficial effect of an improvement in the symptoms associated with the disease.

Treatment according to the present invention includes a "therapeutically effective amount" of the medicaments used. A "therapeutically effective amount" refers to an amount effective, at dosages and for periods of time necessary, to achieve a desired therapeutic result.

A therapeutically effective amount may vary according to factors such as the disease state, age, sex, and weight of the individual, and the ability of the medicaments to elicit a desired response in the individual. A therapeutically effective amount is also one in which any toxic or detrimental effects of the antibody or antibody portion are outweighed by the therapeutically beneficial effects.

A "therapeutically effective amount" for tumor therapy may also be measured by its ability to stabilize the progression of disease. The ability of a compound to inhibit cancer may be evaluated in an animal model system predictive of efficacy in human tumors.

Alternatively, this property of a composition may be evaluated by examining the ability of the compound to inhibit cell growth or to induce apoptosis by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

The efficient dosages and the dosage regimens for the bispecific antibodies used in the present invention depend on the disease or condition to be treated and may be determined by the persons skilled in the art.

An exemplary, non-limiting range for a therapeutically effective amount of an bispecific antibody used in the present disclosure may be about 0.1-100 mg/kg.

Whereas particular embodiments of the invention have been described above for purposes of illustration, it will be appreciated by those skilled in the art that numerous variations of the details may be made without departing from the invention as described in the appended claims.

### XIII. Examples

Examples are provided below to illustrate the present disclosure. These examples are not meant to constrain the present disclosure to any particular application or theory of operation. For all constant region positions discussed in the present disclosure, numbering is according to the EU index as in Kabat (Kabat et al., 1991, Sequences of Proteins of Immunological Interest, 5th Ed., United States Public Health Service, National Institutes of Health, Bethesda). Those skilled in the art of antibodies will appreciate that this convention consists of nonsequential numbering in specific regions of an immunoglobulin sequence, enabling a normalized reference to conserved positions in immunoglobulin families. Accordingly, the positions of any given immunoglobulin as defined by the EU index will not necessarily correspond to its sequential sequence.

General and specific scientific techniques are outlined in US Publications 2015/0307629, 2014/0288275 and WO2014/145806.

### A. Example 1: TILs from multiple cancer types co-express PD-1 and T cell costimulatory receptors

To investigate potential associations between PD-1 and various T cell costimulatory receptors, RNA sequencing data from The Cancer Genome Atlas project (TCGA) were used for analysis. V2 RSEM data were downloaded from FireBrowse (http://firebrowse.org/). Analysis was performed using R with custom routines. The correlation between the expression of PD-1 and eight costimulatory receptors is depicted in Figure 1, along with calculated R2 values (square of the Pearson correlation coefficient). The data show that PD-1 and several costimulatory receptors were co-expressed in cancers including bladder, breast, colon, head & neck, kidney, lung-adeno, lung squamous, ovarian, pancreatic, prostate, and melanoma cancer. Notably, expression of ICOS on TILs correlates better with that of PD-1 than several other costims.

### B. Example 2: Immune checkpoint antigen binding domains

### 1. 2A: anti-PD-1 ABDs

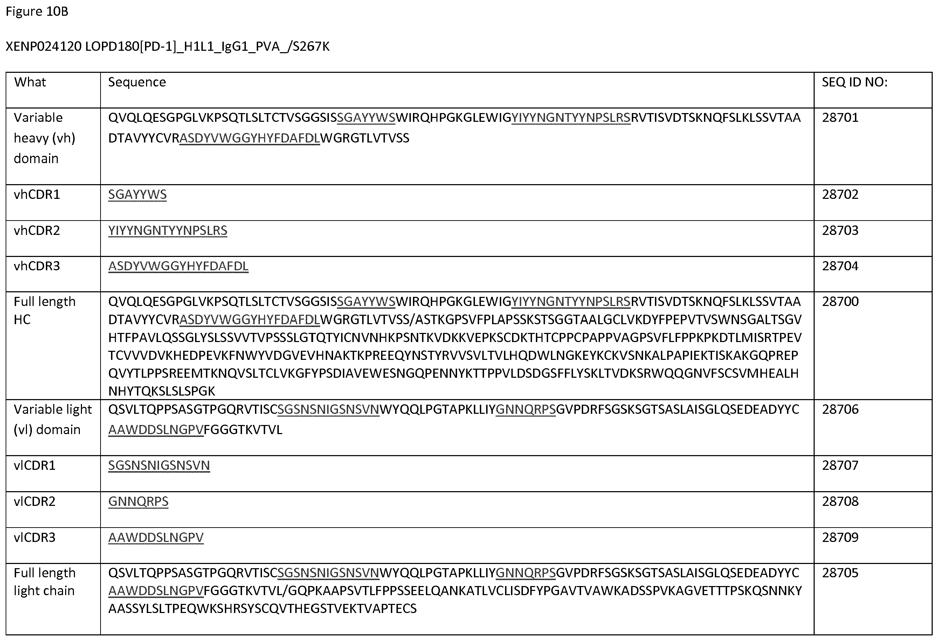

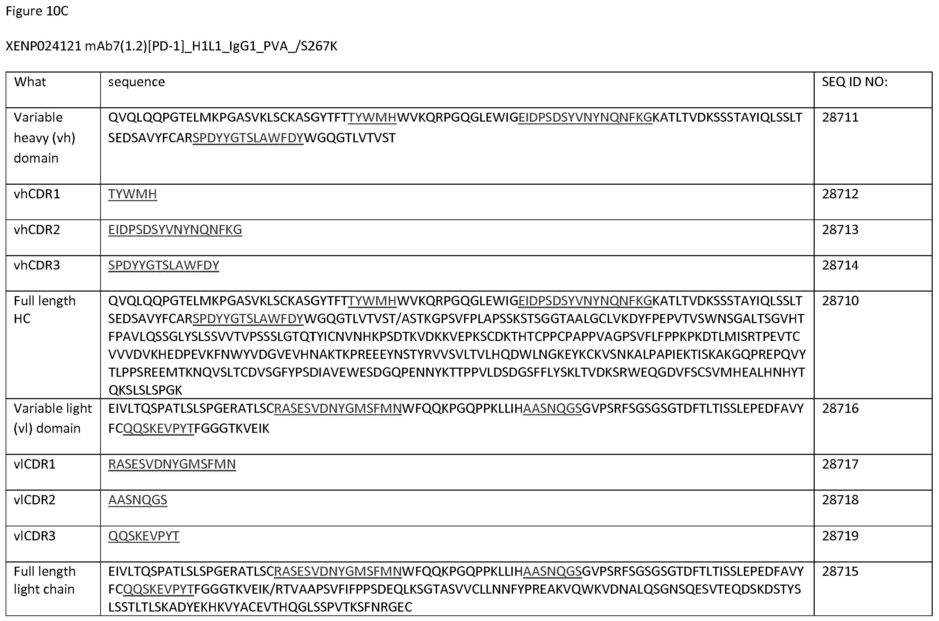

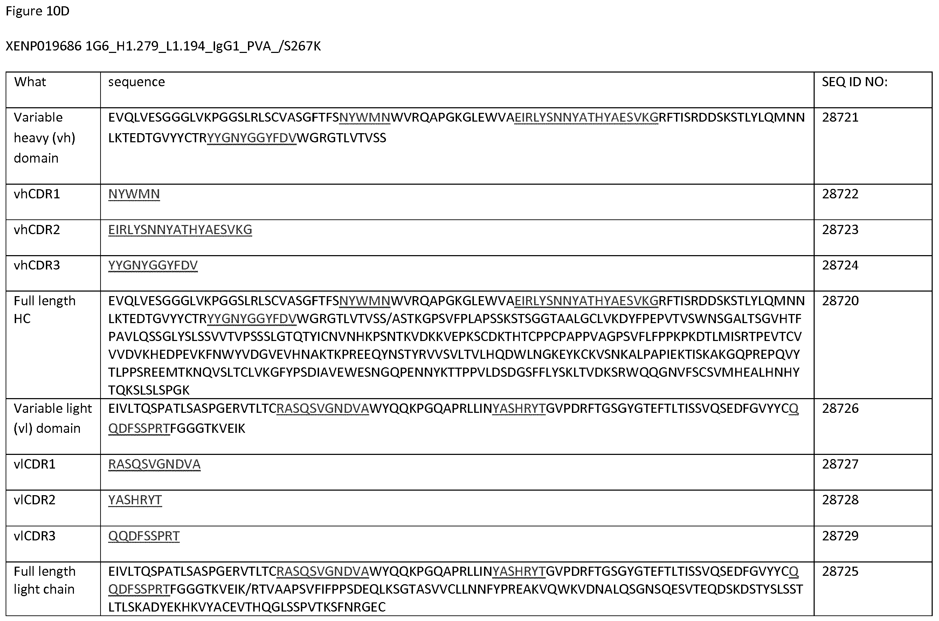

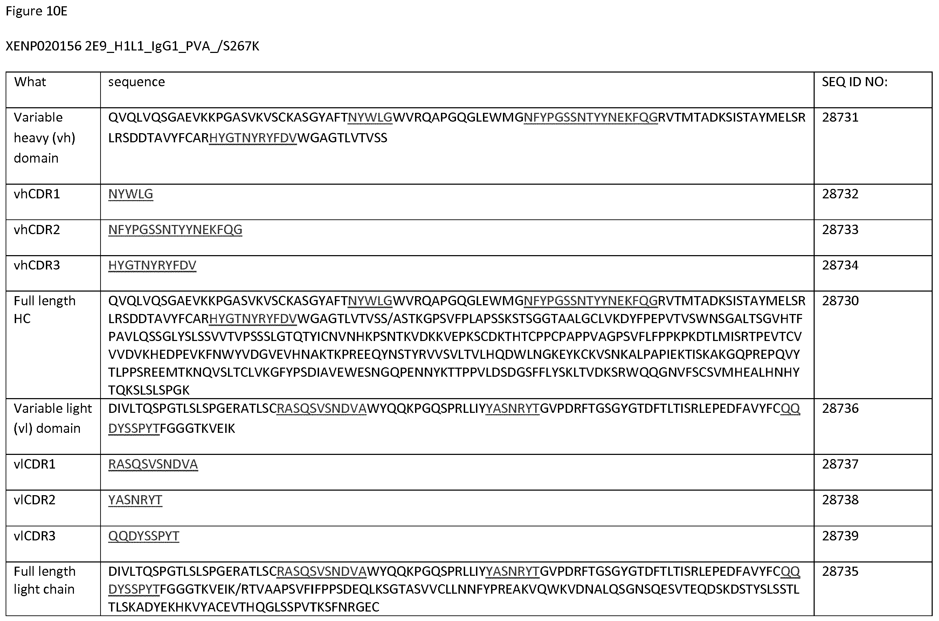

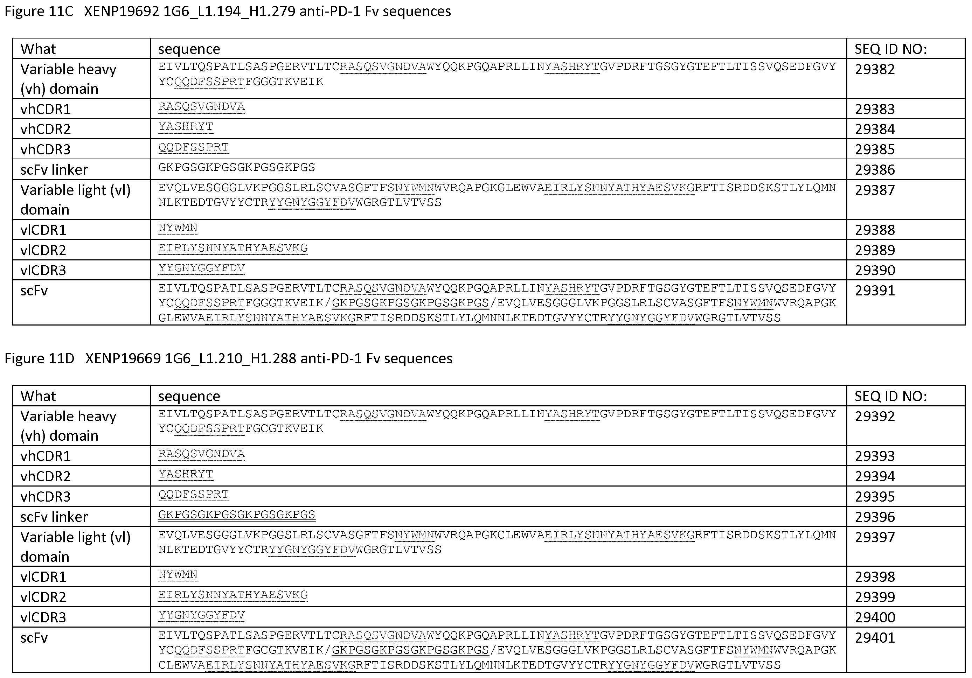

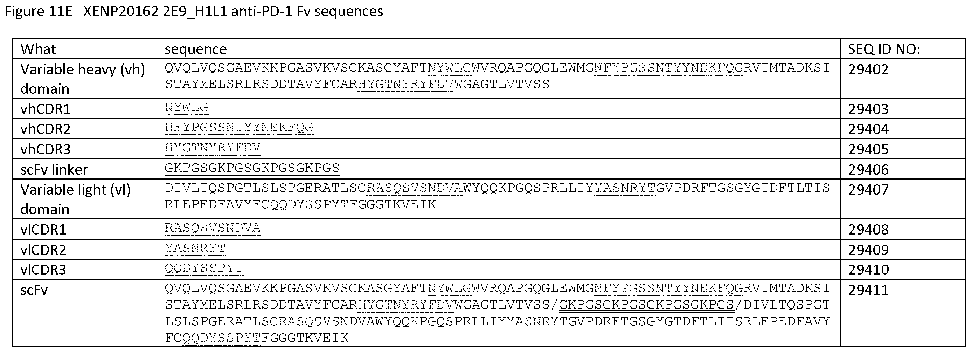

Examples of antibodies which bind PD-1 were generated in bivalent IgG1 format with E233P/L234V/L235A/G236del/S267K substitutions, illustrative sequences for which are depicted in Figure 10. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human Cκ constant region. DNA was transfected into HEK293E cells for expression. Additional PD-1 ABDs (including those derived from the above antibodies) were formatted as Fabs and scFvs for use in costim/checkpoint bispecific antibodies, illustrative sequences for which are depicted respectively in Figure 11 and in the sequence listing.

### 2. 2B: anti-CTLA-4 ABDs

Antibodies which bind CTLA-4 were generated in bivalent IgG1 format with E233P/L234V/L235A/G236del/S267K substitutions. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human C? constant region. DNA was transfected into HEK293E cells for expression. Additional CTLA-4 ABDs (including those derived from the above antibodies) were formatted as scFvs for use in costim/checkpoint bispecific antibodies, illustrative sequences for which are depicted in Figure 12 and in the sequence listing.

### 3. 2C: anti-LAG-3 ABDs

Examples of antibodies which bind LAG-3 were generated in bivalent IgG1 format with E233P/L234V/L235A/G236del/S267K substitutions. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human C? constant region. DNA was transfected into HEK293E cells for expression. Additional LAG-3 ABDs (including those derived from the above antibodies) were formatted as Fabs for use in costim/checkpoint bispecific antibodies, illustrative sequences for which are depicted in Figure 13 and in the sequence listing.

### 4. 2D: anti-TIM-3 ABDs

Examples of antibodies which bind TIM-3 were generated in bivalent IgG1 format with E233P/L234V/L235A/G236del/S267K substitutions, exemplary sequences for which are depicted in Figure 14. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human C? constant region. DNA was transfected into HEK293E cells for expression. The above antibodies were formatted as Fabs for use in costim/checkpoint bispecific antibodies.

### 5. 2E: anti-PD-L1 ABDs

Prototype antibodies which bind PD-L1 were generated in bivalent IgG1 format with E233P/L234V/L235A/G236del/S267K substitutions, exemplary sequences for which are depicted in Figure 15A-15C. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human C? constant region. DNA was transfected into HEK293E cells for expression. The above antibodies were formatted as Fabs and scFvs for use in costim/checkpoint bispecific antibodies.

### C. Example 3: Costimulatory receptor antigen binding domains

Prototype costimulatory receptor antibodies which bind ICOS, GITR, OX40, and 4-1BB were generated in bivalent IgG1 format with E233P/L234V/L235A/G236del/S267K substitutions, sequences for which are depicted in Figures 16-19. DNA encoding the variable regions was generated by gene synthesis and was inserted into the mammalian expression vector pTT5 by the Gibson assembly method. Heavy chain VH genes were inserted via Gibson assembly into pTT5 encoding the human IgG1 constant region with the substitutions mentioned above. Light chains VL genes were inserted into pTT5 encoding the human C? constant region. DNA was transfected into HEK293E cells for expression. The above antibodies were formatted as Fabs or scFvs for use in costim/checkpoint bispecific antibodies.

### D. Example 4: Engineering anti-ICOS ABD for stability and affinity

The parental variable region of an anti-ICOS antibody (XENP16435; depicted in Figure 19) was engineered for use as a component in an ICOS x checkpoint bispecific antibody. A library of Fv variants engineered to have optimal affinity and stability was constructed by site-directed mutagenesis (QUIKCHANGE, Stratagene, Cedar Creek, Tx.) or additional gene synthesis and subcloning in Fab-His and scFv-His formats and produced as described below.

### 1. 4A: anti-ICOS Fabs

Amino acid sequences for variant anti-ICOS Fabs are listed in Figure 20A-20G (the polyhistidine (His6) tags have been removed from the C-terminal of the Fab heavy chains). DNA encoding the two chains needed for Fab expression were generated by gene synthesis and were subcloned using standard molecular biology techniques into the expression vector pTT5. The Fab heavy chain included a C-terminal polyhistidine tag. DNA was transfected into HEK293E cells for expression and resulting proteins were purified from the supernatant using Ni-NTA chromatography. The resultant anti-ICOS Fabs were characterized for stability and affinity.

Differential Scanning Fluorimetry (DSF) experiments were performed using a Bio-Rad CFX CONNECT Real-Time PCR Detection System. Proteins were mixed with SYPRO Orange fluorescent dye and diluted to 0.2 mg/mL in PBS. The final concentration of SYPRO Orange was 10X. After an initial 10 minute incubation period at 25oC, proteins were heated from 25 to 95oC using a heating rate of 1oC/min. A fluorescence measurement was taken every 30 seconds. Melting temperatures (Tm) were calculated using the instrument software. The results are shown in Figure 21.

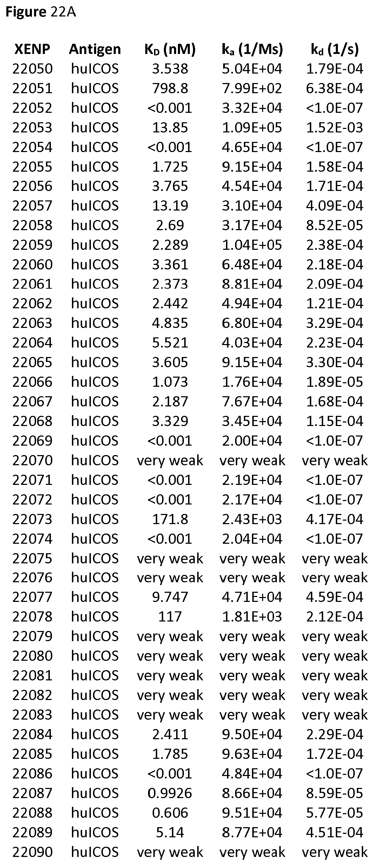

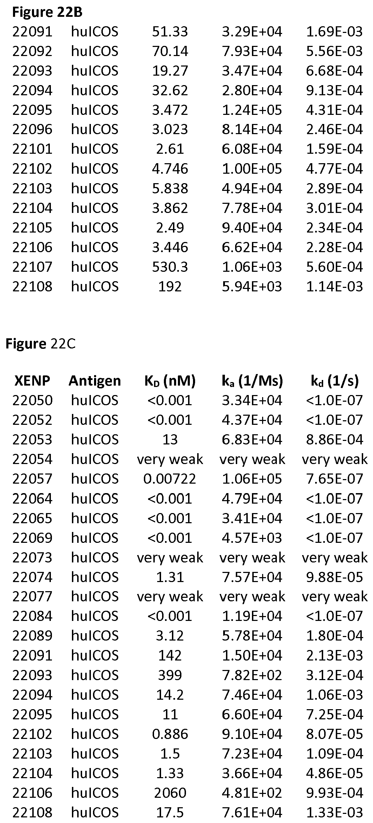

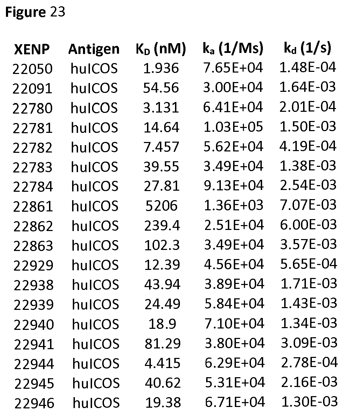

A series of affinity screens of the anti-ICOS Fabs to human ICOS were performed using Octet, a BioLayer Interferometry (BLI)-based method. Experimental steps for Octet generally included the following: Immobilization (capture of ligand or test article onto a biosensor); Association (dipping of ligand- or test article-coated biosensors into wells containing serial dilutions of the corresponding test article or ligand); and Dissociation (returning of biosensors to well containing buffer) in order to determine the monovalent affinity of the test articles. Specifically, anti-mouse Fc (AMC) biosensors were used to capture mouse IgG2a Fc fusion of ICOS and dipped into multiple concentrations of the test articles. The resulting equilibrium dissociation constants (KD), association rates (ka), and dissociation rates (kd) are presented in Figures 22-23. Binding affinities and kinetic rate constants were obtained by analyzing the processed data using a 1:1 binding model using ForteBio Octet Data Analysis software (ForteBio). The data from a two separate experiments are depicted in Figure 22A-22C. In a further experiment, streptavidin (SA) biosensors were used to capture ICOS-TEV-Fc-Avi and dipped into the test articles. The data are depicted in Figure 23. A number of variant anti-ICOS Fabs including XENP22780, XENP22782, XENP22783, and XENP22784 had improved stability while maintaining affinity characteristics similar to a Fab comprising the parental variable regions (XENP22050).

### 2. 4B: anti-ICOS scFvs

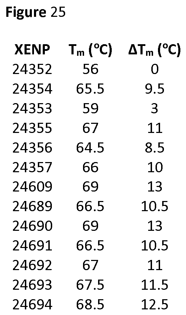

Amino acid sequences for anti-ICOS scFvs are listed in Figure 24 (the polyhistidine (His6) tags have been removed from the C-terminal of the scFvs). DNA encoding the scFv was generated by gene synthesis and were subcloned using standard molecular biology techniques into the expression vector pTT5. The scFv included a C-terminal polyhistidine tag. DNA was transfected into HEK293E cells for expression and resulting proteins were purified from the supernatant using Ni-NTA chromatography. The resultant anti-ICOS scFvs were characterized for stability in DSF experiments as described above. The data is depicted in Figure 25.

### E. Example 5: anti-ICOS x anti-PD-1 bispecific antibodies

### 1. 5A: Prototype costim/checkpoint bottle-openers

Schematics for costim/checkpoint bispecific antibody in the bottle-opener format are depicted as Figure 2A. Prototype bottle-openers with costimulatory receptor binding Fab arms based on prototype anti-GITR mAb (XENP16438), anti-OX40 mAb (XENP16437), anti-4-1BB mAb (XENP14410) and anti-ICOS mAb (XENP16435) and exemplary anti-PD-1 scFv (XENP19692) arm were produced to investigate their effect on cytokine secretion in an SEB-stimulated PBMC assay. Sequences are depicted in Figure 26A-26D. DNA encoding the three chains needed for bottle-opener expression were generated by gene synthesis and were subcloned using standard molecular biology techniques into the expression vector pTT5. DNA transfected into HEK293E cells for expression and resulting proteins were purified using standard techniques.

### a. 5A(a): Prototype anti-ICOS x anti-PD-1 bottle-opener enhances cytokine secretion

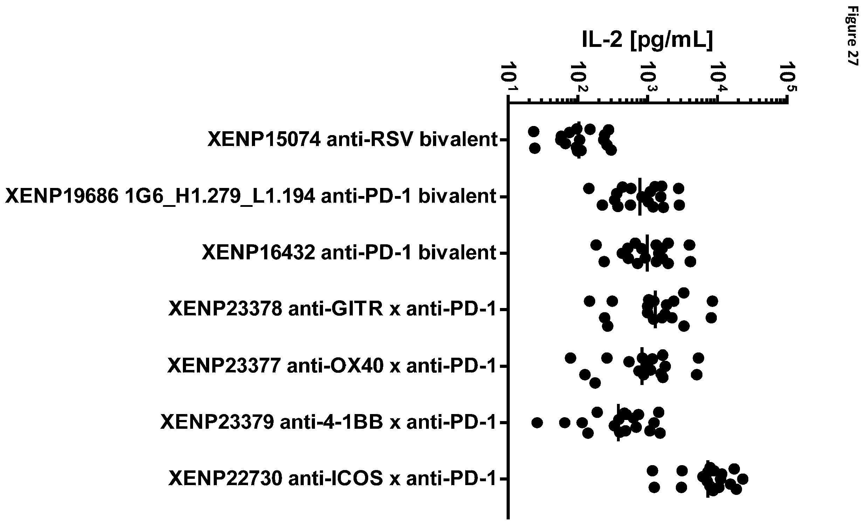

Staphylococcal Enterotoxin B (SEB) is a superantigen that causes T cell activation and proliferation in a manner similar to that achieved by activation via the T cell receptor (TCR). Stimulating human PBMC with SEB is a common method for assaying T cell activation and proliferation. PBMCs were simulated with 100 ng/mL SEB for 2 days. Cells were washed twice and restimulated with 100 ng/mL SEB in combination with 20 µg/mL of the indicated test articles. A first bivalent anti-PD-1 antibody based on nivolumab (XENP16432), a second bivalent anti-PD-1 mAb (XENP19686), and a bivalent anti-RSV mAb (XENP15074) were used as controls. 24 hours after treatment, supernatants were assayed for IL-2. The data depicted in Figure 27 show that each of the costim/checkpoint bottle-openers enhanced cytokine secretion in comparison to the bivalent anti-RSV antibody. Surprisingly, induction of cytokine secretion by XENP22730 is vastly superior to cytokine secretion by bivalent anti-PD-1 antibody alone as well as the other prototype costim/checkpoint bottle-openers, indicating that addition of ICOS binding enhances cytokine production and that ICOS is a better PD-1 partner than other costimulatory receptors for a bispecific antibody.

In another experiment, PBMCs were stimulated with 0.01 µg/mL SEB for 3 days with 20 µg/mL of indicated test articles. As control, test articles were also incubated with naive (non-SEB stimulated) PBMCs. 3 days after treatment, supernatant was assessed for IL-2 secretion as an indicator of T cell activation (depicted in Figure 28). The data show that neither the anti-PD-1 bivalent antibody nor the anti-ICOS x anti-PD-1 bottle-opener stimulate IL-2 secretion in naive cells. Further, the data show again that XENP20896 enhances IL-2 secretion more than the bivalent anti-PD-1 antibody alone does and that XENP20896 enhances IL-2 secretion more than combination of bivalents (XENP16432 and XENP16435) as well as combination of one-arms (XENP20111 and XENP20266) do.

Costimulatory receptors such as ICOS have previously been found to induce cytokine production only following crosslinking by bivalent antibodies or multimerized ligands (Viera et al. 2004; Sanmamed et al. 2015). In view of the crosslinking mechanisms, it is surprising that monovalent ICOS binding by a single arm in a costim x checkpoint blockade bispecific antibody was able to enhance cytokine production. Further, it is notable that the bispecific antibody was able to enhance cytokine secretion more than bivalent anti-PD-1 mAb in combination with bivalent anti-ICOS mAb (XENP16432 + XENP16435).

### b. 5A(b): PD-1 and ICOS double-positive cells are selectively occupied by prototype anti-ICOS x anti-PD-1 bottle-opener

Selective targeting of tumor-reactive TILs co-expressing immune checkpoint receptors (e.g. PD-1) and costimulatory receptors as shown in Example 1 over non-tumor reactive T cells expressing immune checkpoint receptors or costimulatory receptors alone could enhance anti-tumor activity while avoiding peripheral toxicity (as depicted in Figure 29).

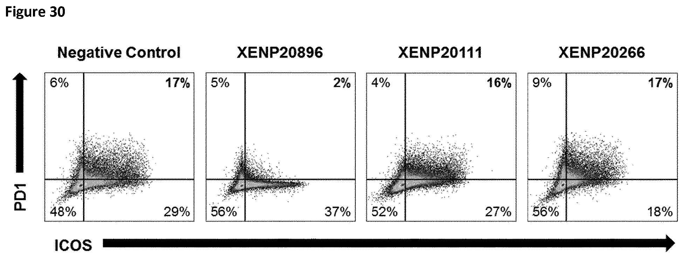

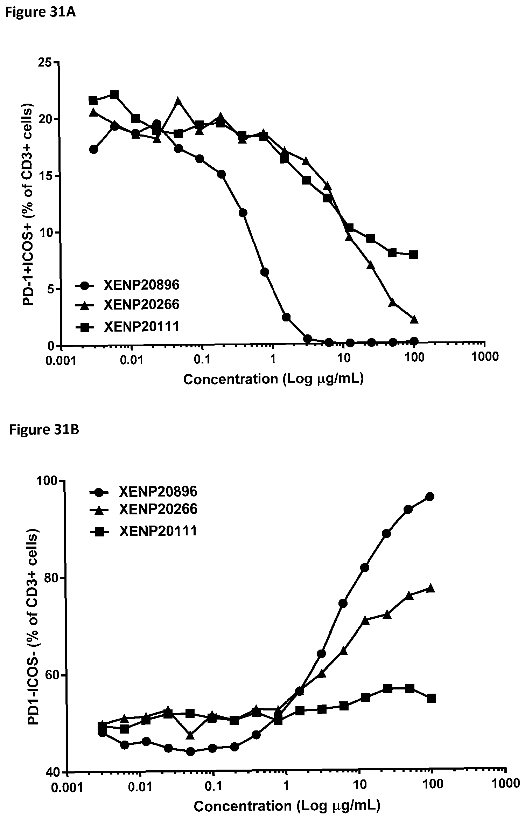

An SEB-stimulated PBMC assay was used to investigate binding of anti-ICOS x anti-PD-1 bottle-opener to T cells. PBMCs were stimulated with 100 ng/mL SEB (staphylococcal enterotoxin B) for 3 days, after which the PBMCs were treated with the indicated test articles for 30 minutes at 4oC. PBMCs were then incubated with APC-labeled one-arm anti-ICOS antibody and FITC-labeled one-arm anti-PD-1 antibody for 30 minutes at 4oC. Figures 30 and 31 shows receptor occupancy of a prototype anti-ICOS x anti-PD-1 bottle-opener (XENP20896), one-arm anti-ICOS antibody (XENP20266) and one-arm anti-PD-1 antibody (XENP20111).

The data show that double-positive cells (expressing both PD-1 and ICOS) are selectively occupied by the anti-ICOS x anti-PD-1 bottle-opener (XENP20896) as depicted in Figure 30, indicating that monovalent ICOS and PD-1 binding is useful for selective targeting. Further, anti-ICOS x anti-PD-1 bottle-opener (e.g. XENP20896) binds more potently to double-positive cells than monovalent, monospecific one-arm anti-PD-1 and anti-ICOS as shown in Figure 31A.

### c. 5A(c): Prototype anti-ICOS x anti-PD-1 bottle-opener enhance engraftment in a GVHD mouse study

The prototype anti-ICOS x anti-PD-1 bottle-opener was evaluated in a Graft-versus-Host Disease (GVHD) model conducted in NSG (NOD-SCID-gamma) immunodeficient mice. The mice were engrafted with human PBMCs. When NSG mice are injected with human PBMCs, they develop an autoimmune response against the human PBMCs. Treatment of NSG mice injected with human PBMCs with immune checkpoint antibodies (e.g. anti-PD-1) enhance engraftment. Thus, increased engraftment shows efficacy of the antibodies.

10 million human PBMCs were engrafted into NSG mice via IV-OSP on Day 0 followed by dosing with the indicated test articles on Day 1, 8, 15, and 22. Human CD45+ cell counts were measured on Day 14 as an indicator of disease.

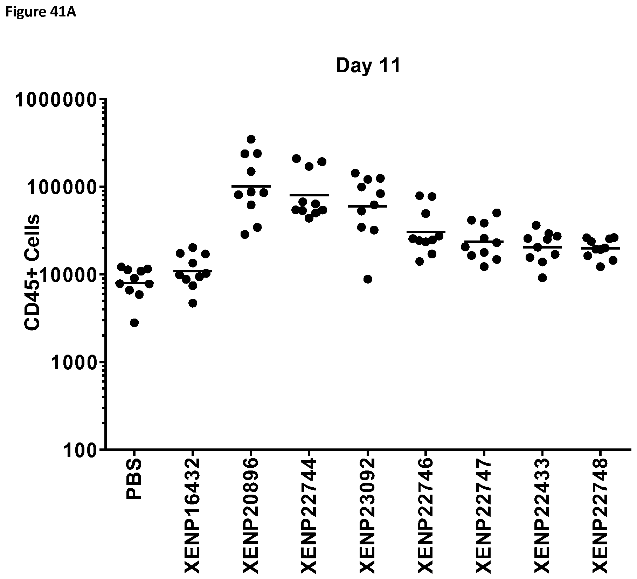

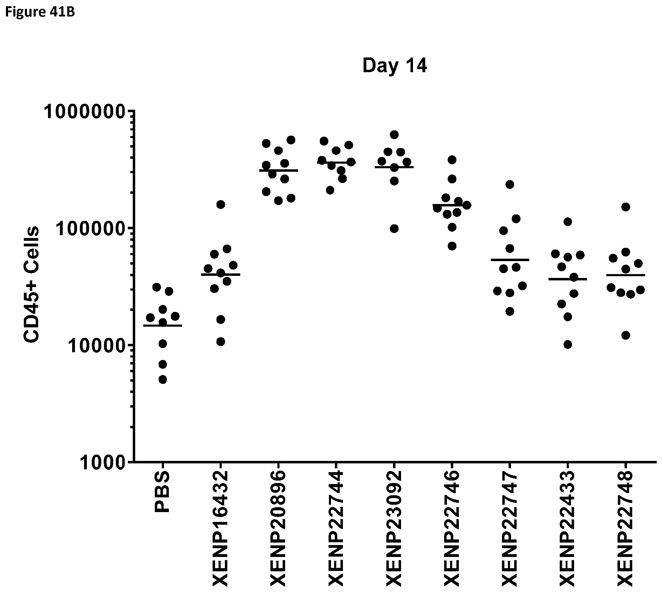

The data depicted in Figure 41A-41B show that the anti-ICOS x anti-PD-1 bottle-openers enhance proliferation of CD45+ cells in human PBMC-engrafted NSG mice as compared to controls (PBS and PBS + PBMC). Further, enhancement is greater using the anti-ICOS x anti-PD-1 bottle-openers than that seen with bivalent anti-PD-1 antibody.

### 2. 5B: Production of variant anti-ICOS x anti-PD-1 bottle-openers with optimized anti-ICOS Fab arms

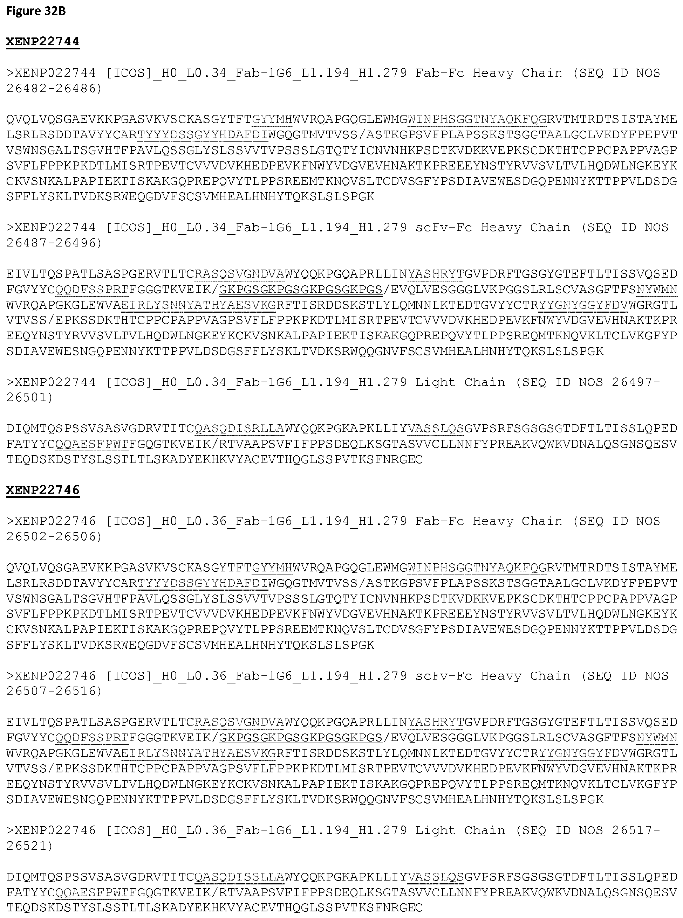

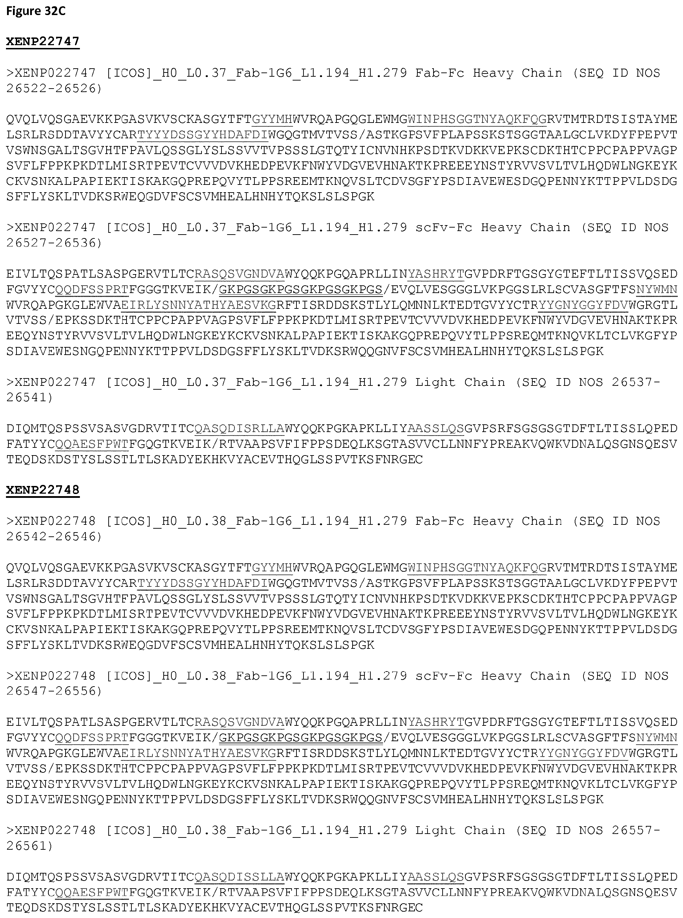

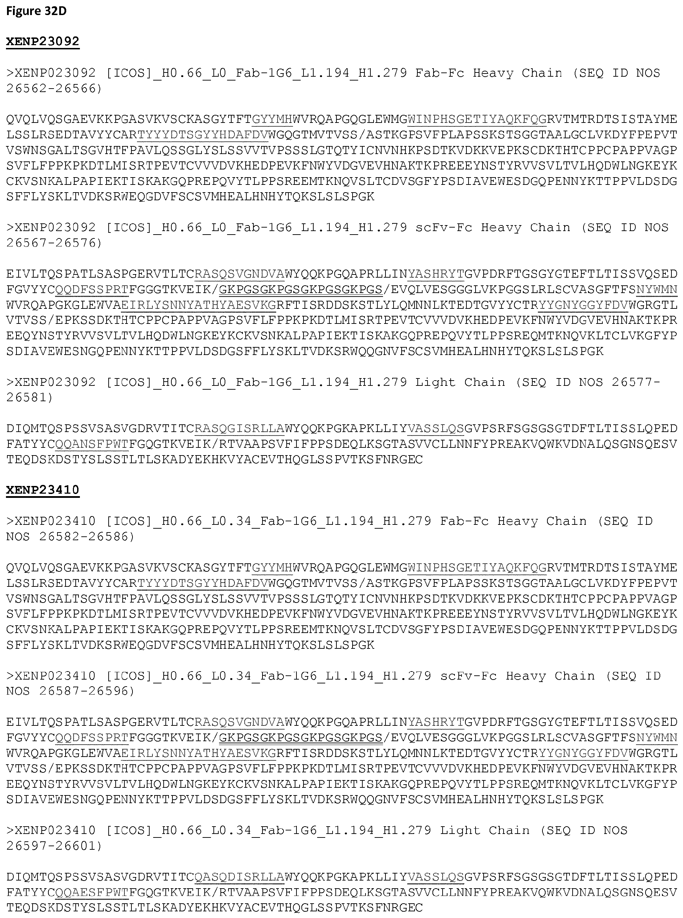

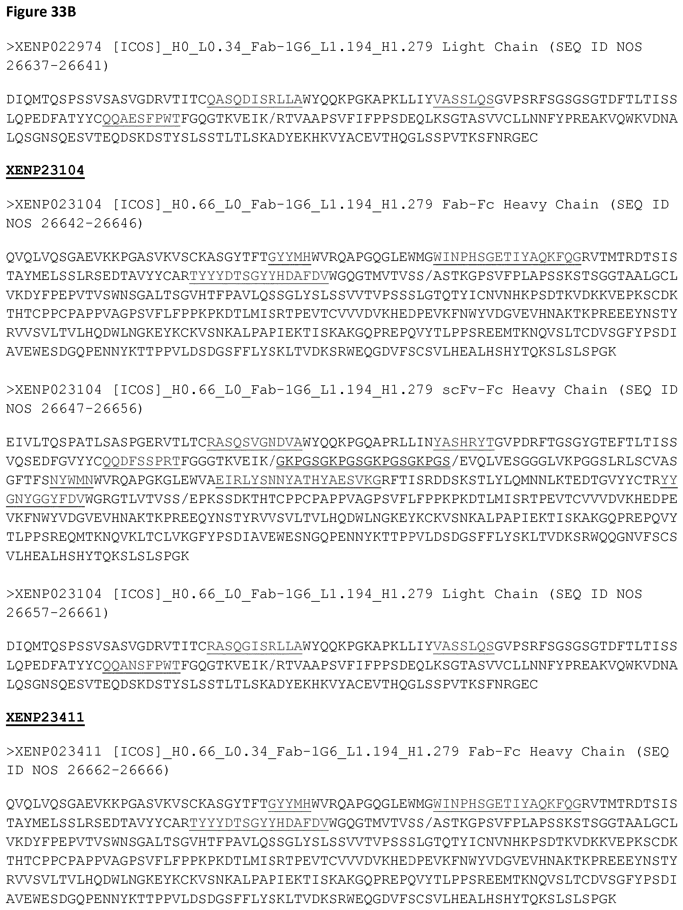

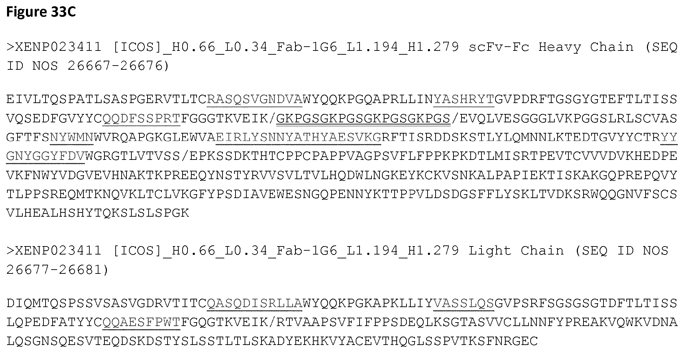

Variant anti-ICOS x anti-PD-1 bottle-openers comprising anti-ICOS Fabs engineered as described in Example 4A were produced as generally described above. Amino acid sequences for variant anti-ICOS x anti-PD-1 bottle-openers are listed in Figure 32. Amino acid sequences for variant anti-ICOS x anti-PD-1 with FcRn pH 6.0 affinity enhancing substitutions are listed in Figure 33.

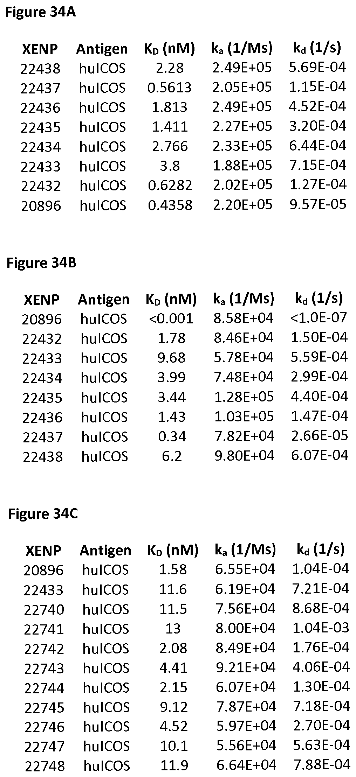

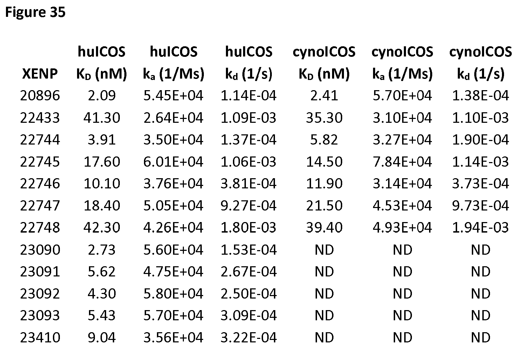

The resultant anti-ICOS x anti-PD-1 bispecific antibodies were characterized for affinity to human and cynomolgus ICOS using Octet as generally described above. In a first set of experiments, AMC biosensors were used to capture mouse IgG2a Fc fusion of ICOS and dipped into multiple concentrations of the test articles (data depicted in Figure 34A-34C). In further experiments, streptavidin (SA or SAX) biosensors were used to capture biotinylated human and cynomolgus IgG1 Fc fusions of human and cynomolgus ICOS and dipped into multiple concentrations of the test articles (data depicted in Figure 35).

### 3. 5C: T cell surface binding of variant anti-ICOS x anti-PD-1 bottle-openers

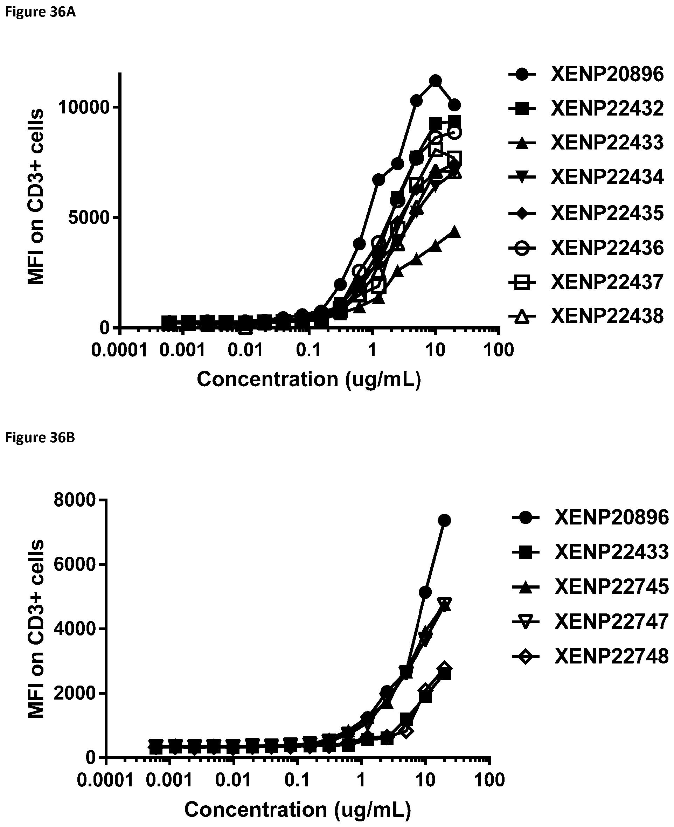

Binding of anti-ICOS x anti-PD-1 bispecifics to T cells was measured in an SEB-stimulated PBMC assay. Human PBMCs were stimulated with 100 ng/mL SEB for 3 days. PBMCs were then treated with the indicated test articles and incubated at 4oC for 30 minutes. After treatment, cells were incubated with FITC-labeled anti-CD3 antibody and APC-labeled anti-human IgG Fc secondary antibody. MFI on CD3+ cells are depicted in Figure 36A-36B.

### 4. 5D: Receptor occupancy of variant anti-ICOS x anti-PD-1 bottle-openers on T cells

Receptor occupancy of variant anti-ICOS x anti-PD-1 bottle-openers on T cells was measured in an SEB-stimulated PBMC assay. PBMCs were stimulated with 100 ng/mL SEB (staphylococcal enterotoxin B) for 3 days, after which the PBMCs were treated with the indicated test articles for 30 minutes at 4oC. PBMCs were then incubated with APC-labeled one-arm anti-ICOS antibody and FITC-labeled one-arm anti-PD-1 antibody for 30 minutes at 4oC. Figure 37 depicts the receptor occupancy of variant anti-ICOS x anti-PD-1 bispecific antibodies, corresponding one-arm anti-ICOS antibodies and one-arm anti-PD-1 antibody (XENP20111) on PD-1 and ICOS double-positive T cells. Consistent with the prototype antibodies investigated in Example 1A, each of the bottle-openers binds more potently to double-positive cells than monovalent, monospecific one-arm anti-PD-1 and one-arm anti-ICOS antibodies.

### 5. 5E: In vitro activity of variant anti-ICOS x anti-PD-1 bottle-openers in a cytokine release assay

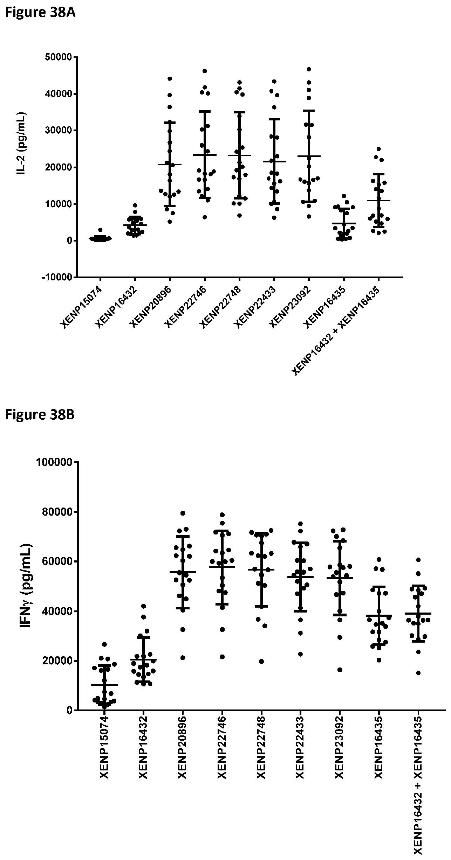

Human PBMCs were stimulated with 100 ng/mL SEB for 2 days. Cells were washed and stimulated again with 100 ng/mL SEB in combination with 20 µg/mL of indicated test articles. 24 hours after treatment, cells were assayed for IL-2 (Figure 38A) and IFNγ (Figure 38B). The data show that anti-ICOS x anti-PD-1 bottle-openers stimulated significantly more cytokine release than bivalent anti-PD-1 antibody (XENP16432) alone, bivalent anti-ICOS antibody (XENP16435) alone, or bivalent anti-PD-1 antibody plus bivalent anti-ICOS antibody in combination.

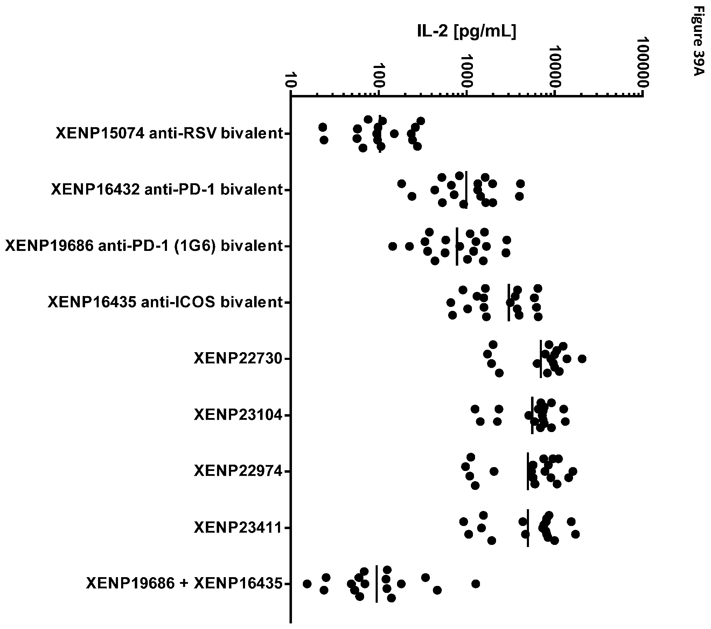

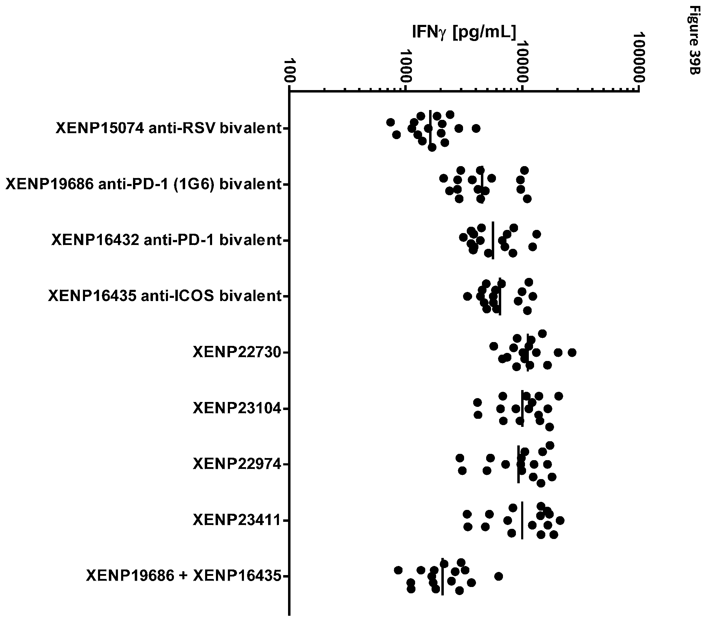

In a further experiment, human PBMCs were stimulated with 100 ng/mL SEB for 2 days. Cells were washed and stimulated again with 100 ng/mL SEB in combination with 20 µg/mL of indicated test articles. 24 hours after treatment, cells were assayed for IL-2 (Figure 39A) and IFNγ (Figure 39B).

### 6. 5F: In vivo activity of variant anti-ICOS x anti-PD-1 bottle openers in a GVHD mouse study

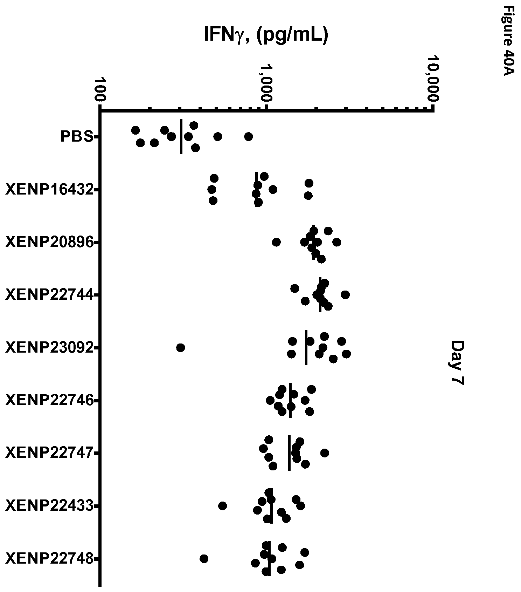

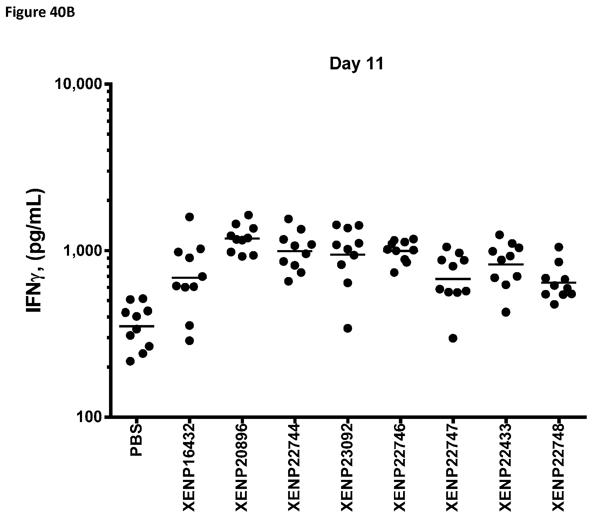

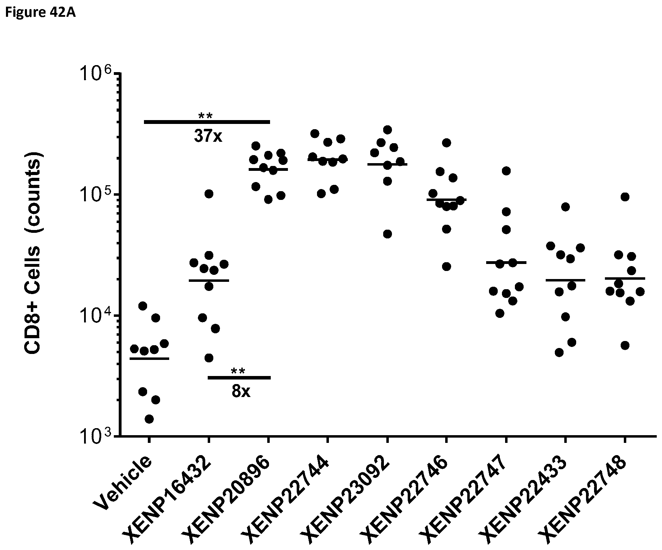

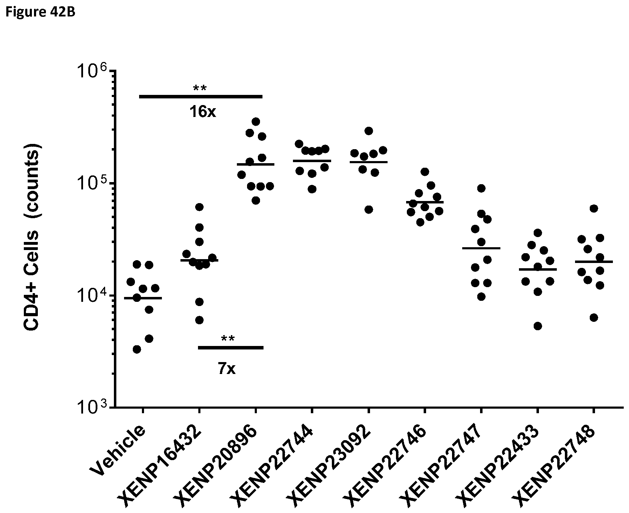

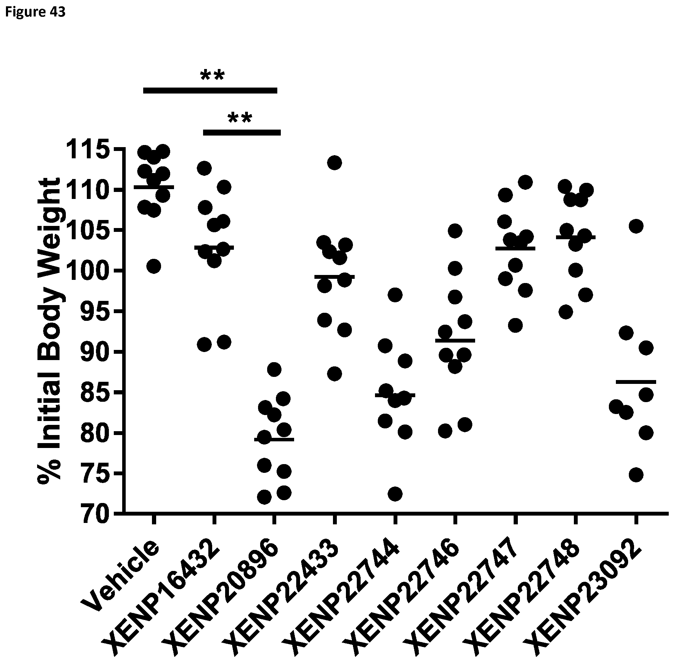

In a first study, 10 million human PBMCs were engrafted into NSG mice via IV-OSP on Day 0 followed by dosing with the indicated test articles on Day 1. IFNγ levels and human CD45+, CD8+ T cell and CD4+ T cell counts were measured on Day 7, 11 and 14. Figures 40A-40B respectively depicts IFNγ levels on Day 7 and 11. Figures 41A-41B respectively depict CD45+ cell counts on Day 11 and 14. Figures 42A-42B respectively depict CD8+ T cell and CD4+ T cell counts on Day 14. Figure 43 depicts the change in body weight in the mice by Day 14 resulting from exacerbation of GVHD due to T cell expansion and IFNγ production.

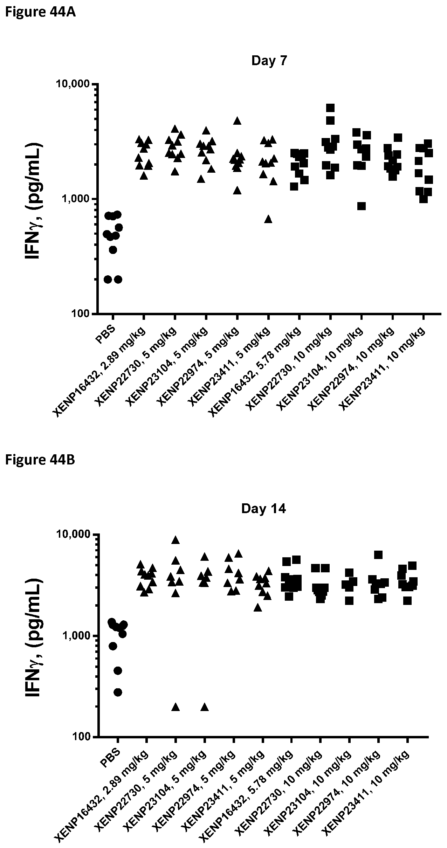

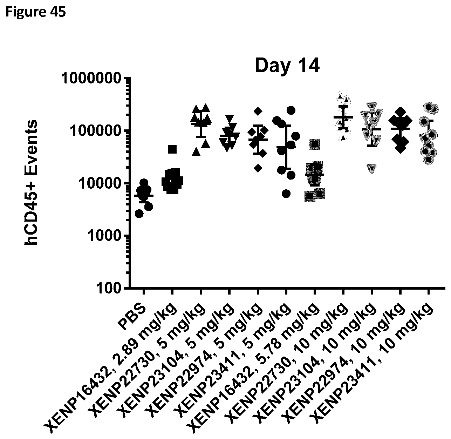

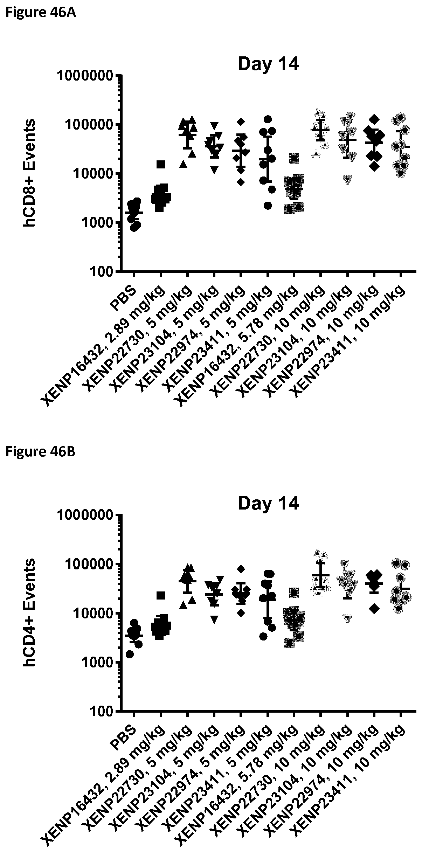

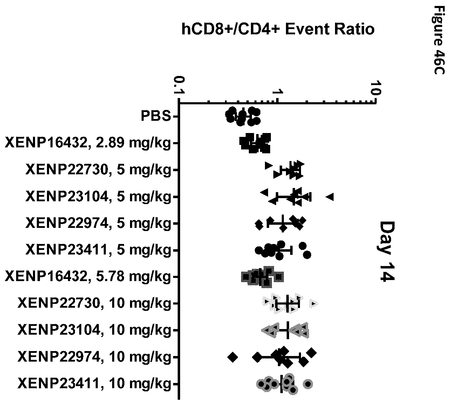

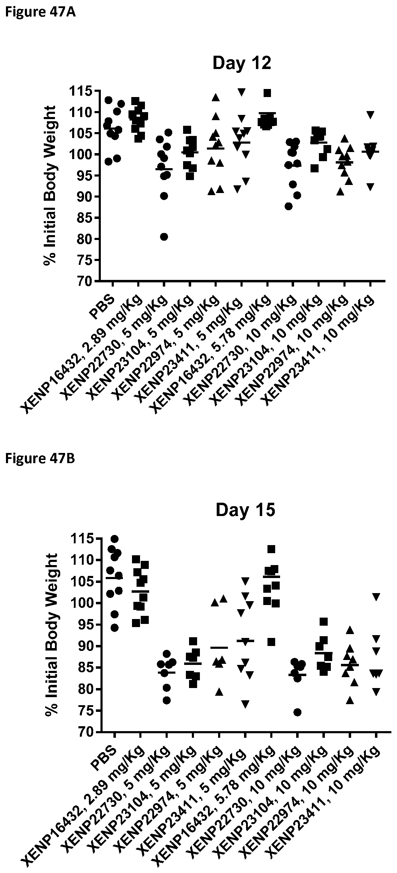

In a further study with additional variant anti-ICOS x anti-PD-1 bottle-openers, 10 million human PBMCs were engrafted into NSG mice via IV-OSP on Day 0 followed by dosing with the indicated test articles at indicated concentrations on Day 1. IFNγ levels and human CD45+, CD8+ T cell and CD4+ T cell counts were measured on Day 7 and 14. Figure 44A-44B respectively depicts IFNγ levels on Day 7 and 14. Figures 45 depicts CD45+ cell count on Day 14. Figure 46A-46C respectively depict CD8+ T cell count, CD4+ T cell counts and CD8+/CD4+ ratio on Day 14. Body weight of mice were also measured on Day 12 and 15 and depicted respectively in Figure 47A-B as percentage of initial body weight.

The Figures show that the anti-ICOS x anti-PD-1 bottle-openers enhance engraftment (as indicated by the proliferation of CD45+ cells, CD8+ T cells and CD4+ T cells and decrease in body weight of mice). The observed activity is correlated to the in vitro potency of each variant. Further, a number of the bottle-openers including XENP20896, XENP22744, XENP23092, XENP22730, XENP23104, XENP22974, and XENP23411 enhance engraftment much more than the control bivalent anti-PD-1 antibody (XENP16432) does.

### 7. 5G: Additional anti-ICOS ABDs work in anti-ICOS x anti-PD-1 bottle opener

Additional anti-ICOS x anti-PD-1 bottle-openers were produced comprising anti-ICOS Fabs based on other anti-ICOS ABDs described in Example 3 as generally described above. Sequences for the additional bottle-openers are depicted in Figure 48A-48D.

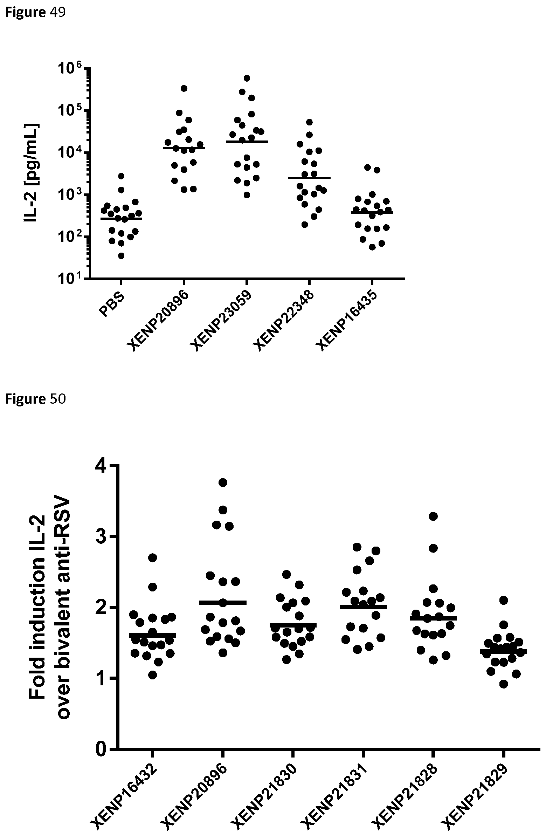

In a first experiment, PBMCs were stimulated with 100 ng/mL SEB for 2 days. Cells were washed twice and restimulated with 100 ng/mL SEB in combination with 20 µg/mL of indicated test articles (PBS as control). 24 hours after treatment, supernatants were assayed for IL-2 concentration as depicted in Figure 49. In a second experiment, PBMCs were stimulated with 10 ng/mL SEB and treated with 20 µg/mL of indicated test articles for 3 days (bivalent anti-PD-1 XENP16432 as control). Supernatants were collected and assays for IL-2. Figure 50 depicts the fold induction in IL-2 over bivalent anti-RSV mAb.

Consistent with the data in Example 5A(a), XENP20896 stimulated secretion of IL-2. Notably, the additional bottle-openers comprising alternative anti-ICOS ABDs were also able to stimulate secretion of IL-2 demonstrating that the enhancement of cytokine secretion by an anti-ICOS x anti-PD-1 bottle-opener is not unique to ABDs derived from the parental anti-ICOS ABD described in Example 4.

### 8. 5H: Clear ICOS signature is exhibited by anti-ICOS x anti-PD-1 bottle-openers

### a. 5H(a): anti-ICOS x anti-PD-1 bispecific antibodies induce AKT phosphorylation

ICOS ligation induces AKT phosphorylation in activated T cells (Fos, C et al. 2008), and as such, AKT phosphorylation would be an indicator of ICOS agonism by anti-ICOS x anti-PD-1 bispecific antibodies of the invention.

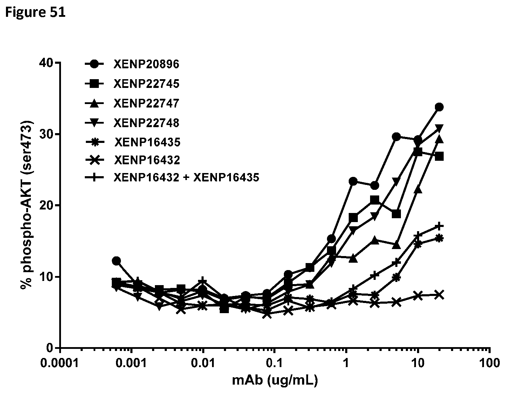

Human PBMCs were stimulated with 100 ng/mL SEB for 2 days. Following stimulation, CD3+ cells were isolated by negative selection using EasySep^{™} Human T Cell Enrichment Kit (STEMCELL Technologies, Vancouver, Canada) and then treated with indicated test articles in combination with plate bound anti-CD3 antibody (OKT3; 500 ng/mL). Cells were lysed 30 minutes after treatment and assayed for total AKT and phosphorylated AKT (Ser473) by a multiplexed phosphoprotein assay on MULTI-SPOT 384-Well Spot plates (Meso Scale Discovery, Rockville, Md.). The data are depicted in Figure 51 as percentage of AKT phosphorylated following treatment.

The data shows no increase in AKT phosphorylation following treatment with negative control bivalent anti-PD-1 mAb (XENP16432). Both bivalent anti-ICOS mAb alone (XENP16435) and XENP16435 in combination with XENP16432 increase AKT phosphorylation demonstrating ICOS ligation and agonism by XENP16435. Surprisingly, despite only monovalent engagement of ICOS,.treatment with the anti-ICOS x anti-PD-1 bispecific antibodies induces more AKT phosphorylation than treatment with XENP16435 alone and XENP16435 in combination with XENP16432. The positive AKT phosphorylation data demonstrate a clear signature of ICOS activity with the bispecific antibodies despite monovalent engagement of ICOS.

### b. 5H(b): Activated T helper cell-associated genes upregulated by anti-ICOS x anti-PD-1 bottle-openers

Guedan et al. (2014) describes gene expression profiles for activated T helper cells (i.e. Th1, Th2, and Th17) and regulatory T cells (Tregs) following activation of ICOS signaling domain-based CAR-Ts. They found that genes related to activated T helper cells such as IL-17A, IL22, IFNγ, TNF, and IL-13 were upregulated, while Treg-related genes such as TGFβ1, SMAD3 and FOXP3 were either unchanged or downregulated.

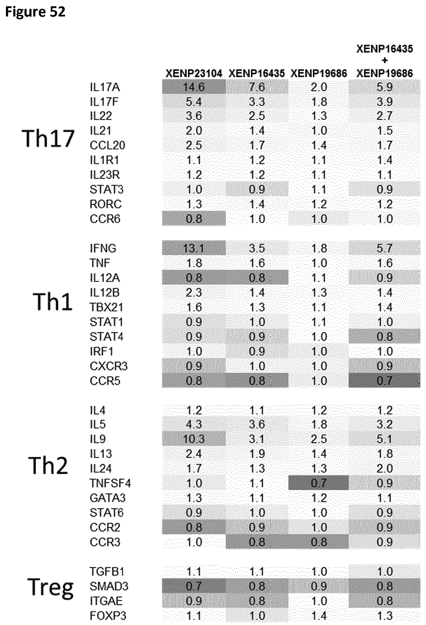

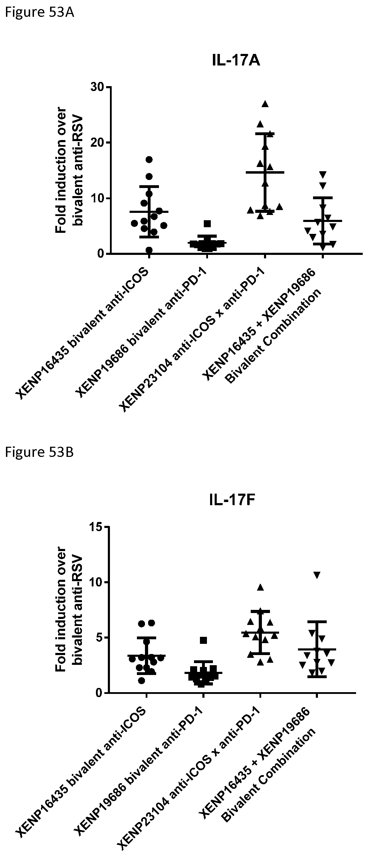

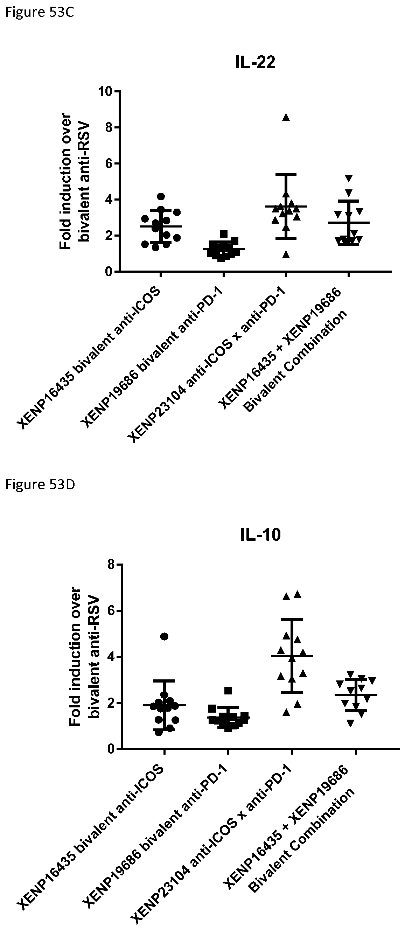

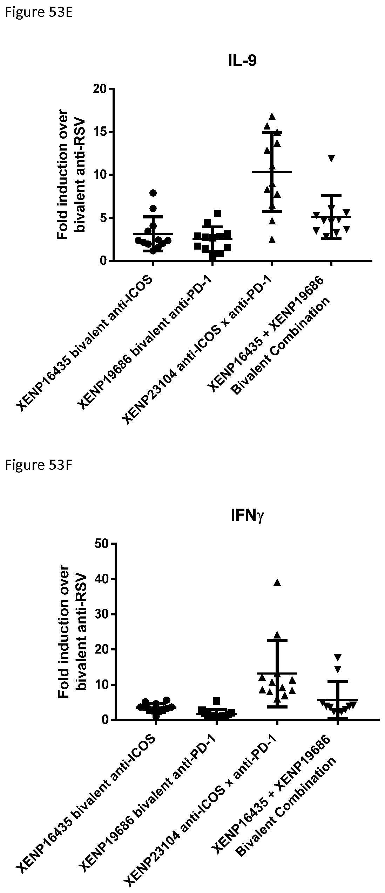

To investigate if engagement of T cells with anti-ICOS x anti-PD-1 bispecific antibodies led to a similar signature, we used Nanostring technology. PBMCs were stimulated with 100 ng/mL SEB for 2 days. Cells were washed 2 times and restimulated with 100 ng/mL SEB and treated with indicated test articles for 24 hours. RNA was extracted from cells and assayed by nCounter^{®} PanCancer Immune Profiling Panel (NanoString Technologies, Seattle, Wash.) which assays 770 target genes covering immune response. Figure 52 depicts mean fold induction in expression of a number of Th-related and Treg-related genes over bivalent anti-RSV antibody. Figures 53A-53F respectively depict fold induction of IL-17A, IL-17F, IL-22, IL-10, IL-9 and IFNγ gene expression by the indicated test articles over induction by bivalent anti-RSV mAb.

As depicted in Figures 52-53 and consistent with the observation by Gueden et al., Th-related genes associated with ICOS signaling such as IL-17A, IL-17F, IL-22, IL-9, and IFNγ are upregulated following treatment with bivalent anti-ICOS mAb and combination of anti-ICOS and anti-PD-1 mAbs. Notably, expression of Th-related genes associated with ICOS signaling are further upregulated following treatment with an anti-ICOS x anti-PD-1 antibody, again indicating a clear ICOS costimulatory signature and dramatic synergy of anti-ICOS and anti-PD-1 engagement by a bispecific antibody.

### 9. 5I: Alternative format anti-ICOS x anti-PD-1 bispecific antibodies

Alternative format costim/checkpoint bispecific antibodies were produced to investigate whether the effect of anti-ICOS x anti-PD-1 bottle-openers was unique to the bottle-opener format or broadly applicable to anti-ICOS x anti-PD-1 bispecific antibodies.

### a. 5I(a): anti-PD-1 x anti-ICOS bottle-opener

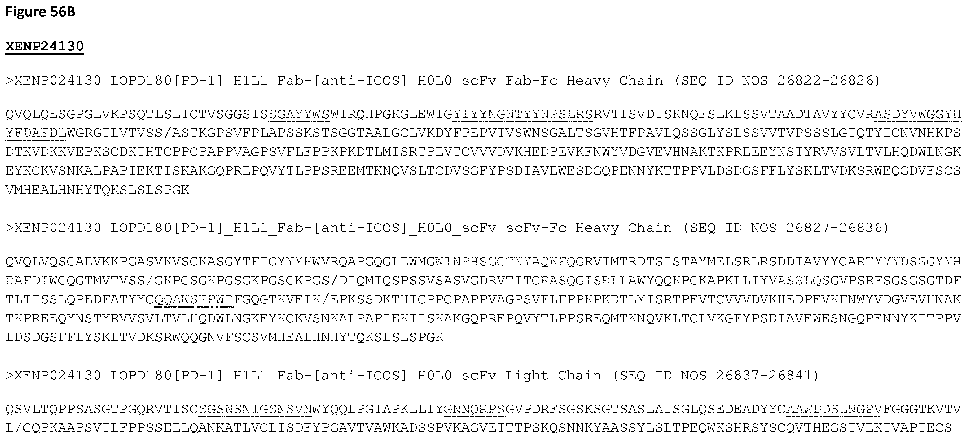

Anti-PD-1 x anti-ICOS bottle-openers with an anti-PD-1 Fab generated using DNA encoding anti-PD-1 mAbs (e.g. XENP16432 and XENP29120) as described in Example 2A and anti-ICOS scFvs as described in Example 4B. Bottle-openers were produced as generally described in Example 5A. Sequences for exemplary anti-PD-1 x anti-ICOS bottle-openers are depicted in Figure 56.

### b. 5I(b): Central-scFv

Schematics for the central-scFv format are depicted as Figure 55A-55B. DNA encoding anti-ICOS Fab-Fc heavy chains was generated using DNA encoding anti-ICOS Fabs described in Example 4A by standard subcloning into the expression vector pTT5. DNA encoding anti-ICOS-anti-PD-1 Fab-scFv-Fc heavy chains was generated using DNA encoding anti-ICOS Fabs described in Example 4A and anti-PD-1 scFv described in Example 2A by a combination of gene synthesis and standard subcloning into the expression vector pTT5. DNA was transfected into HEK293E cells for expression. Sequences for exemplary anti-ICOS x anti-PD-1 central-scFv antibodies are depicted in Figure 57A-57C.

### c. 5I(c): Central-scFv2

A schematic for the central-scFv2 format is depicted as Figure 55C. DNA encoding anti-ICOS-anti-PD-1 Fab-scFv-Fc heavy chains was generated using DNA encoding anti-ICOS Fabs described in Example 4A and anti-PD-1 scFv described in Example 2A by a combination of gene synthesis and standard subcloning into the expression vector pTT5. DNA was transfected into HEK293E cells for expression. Sequences for exemplary anti-ICOS x anti-PD-1 central-scFv2 antibodies are depicted in Figure 58.

### d. 5I(d): Bispecific mAb

A schematic for the bispecific mAb format is depicted as Figure 2K. DNA encoding the anti-ICOS heavy and light chains were generated based on the DNA encoding the anti-ICOS Fabs described in Example, and DNA encoding anti-PD-1 heavy and light chains were generated based on the DNA encoding the anti-PD-1 mAbs described in Example 2A. Heavy chain VH genes were inserted via Gibson assembly into pTT5 encoding the human IgG1 constant region with E233P/L234V/L235A/G236del/S267K substitutions. Light chain VL genes were inserted into pTT5 encoding the human C? constant region.

DNA was transfected into HEK293E cells for expression as 2 separate antibodies which are separately expressed and purified by Protein A affinity (GE Healthcare). These antibodies contain heterodimerization-skewing substitutions in the CH3:CH3 interface. 2-Mercaptoethylamine-HCl (2-MEA) was used to induce controlled reduction of interchain disulfide bonds in the two parental IgGs. 2-MEA was then removed allowing the reoxidation of interchain disulfide bonds to occur enabling recombination of the HC-LC pairs (driven by the heterodimerization mutations). Finally, the trispecific antibodies were purified by cation exchange chromatography. Such methods are common in the art (see, for instance, Labrijn (2013) PNAS 110(13):5145-50 or Strop et al. (2012) J Mol Biol 420(3):204-19). Other methods of design and purification known in the art may be used facilitate bispecific mAb production, for instance, common light chain antibodies (Merchant (1998) Nat Biotechnol 16(7):677-81) or heterodimeric Fab domains (Lewis et al. (2014) Nat Biotechnol 32(2):191-8). Sequence for an exemplary anti-PD-1 x anti-ICOS bispecific mAb is depicted in Figure 59.

### e. 5I(e): DVD-IgG

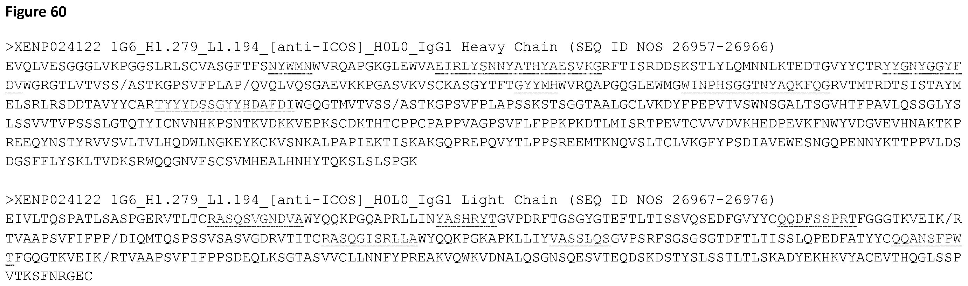

A schematic for the DVD-IgG format is depicted as Figure 55A-55D. DNA encoding anti-PD-1-anti-ICOS VH-VH-CH1-Fc heavy chains and VL-VL-C? light chains was generated using DNA encoding anti-ICOS Fabs described in Example 4A and anti-PD-1 scFv described in Example 2A by a combination of gene synthesis and standard subcloning into the expression vector pTT5. DNA was transfected into HEK293E cells for expression. Sequences for exemplary anti-ICOS x anti-PD-1 DVD-IgGs are depicted in Figure 60.

### f. 5I(f): Trident

A schematic for the Trident format is depicted as Figure 55A-55D. DNA encoding anti-PD-1 VL-VH-Fc heavy chains and VL-VH light chains was generated using DNA encoding anti-ICOS Fabs described in Example 4A and anti-PD-1 scFv described in Example 2A by a combination of gene synthesis and standard subcloning into the expression vector pTT5. DNA was transfected into HEK293E cells for expression. Sequences for exemplary anti-ICOS x anti-PD-1 Tridents are depicted in Figure 61A-61B.

### g. 5I(g): Alternative format anti-ICOS x anti-PD-1 bispecific antibodies enhance cytokine secretion

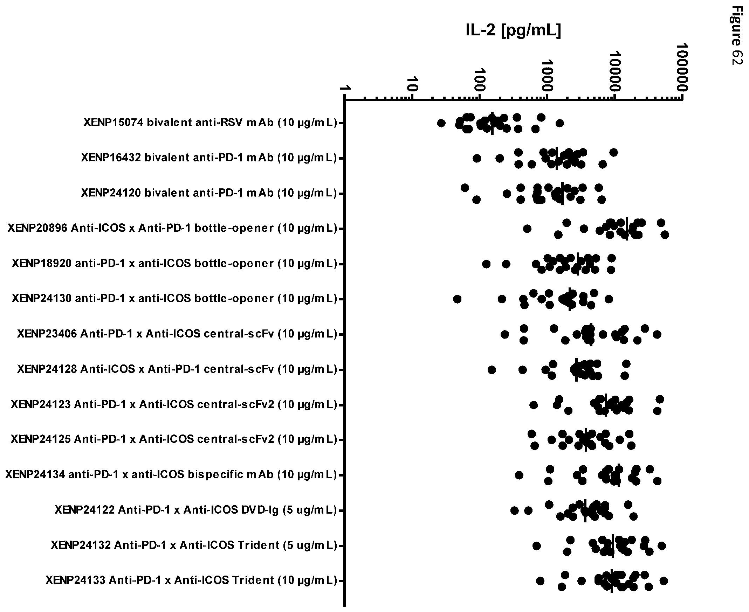

Human PBMCs were stimulated with 200 ng/mL SEB for 2 days. Cells were washed twice then re-stimulated with 100 ng/mL SEB and treated with the indicated concentrations of the indicated test articles. 24 hours after treatment, supernatants were assayed for IL-2. The data are depicted in Figure 62 and show that each of the alternative format bispecific antibodies enhances IL-2 secretion in comparison to bivalent anti-RSV (XENP15074) control. Notably, the majority of these alternative format antibodies enhance IL-2 secretion in comparison to a bivalent anti-PD-1 antibody.

### F. Example 6: Costim/checkpoint bispecific antibodies targeting different immune checkpoint antigens

### 1. 6A: anti-CTLA-4 x anti-ICOS

Anti-CTLA-4 x anti-ICOS bottle-openers were generated with an anti-ICOS Fab derived from XENP16435 and anti-CTLA-4 scFvs derived from ABDs described in Example 2B. Sequence for an exemplary anti-ICOS x anti-CTLA-4 bottle-opener is depicted in Figure 63. Bispecific mAbs were produced as generally described in Example 5I(c).

### 2. 6B: anti-LAG-3 x anti-ICOS

Anti-LAG-3 x anti-ICOS bispecific antibodies were generated with anti-LAG-3 Fabs derived from ABDs described in Example 2C and an anti-ICOS Fab derived from XENP16435. Sequences for exemplary anti-LAG-3 x anti-ICOS bispecific antibodies are depicted in Figure 64A-64B. Bispecific mAbs were produced as generally described in Example 5I(c).

### 3. 6C: anti-TIM-3 x anti-ICOS

Anti-TIM-3 x anti-ICOS bispecific antibodies were generated with anti-TIM-3 Fabs derived from ABDs described in Example 2D and an anti-ICOS Fab derived from XENP16435. Sequence for an exemplary anti-TIM-3 x anti-ICOS bispecific antibody is depicted in Figure 65. Bispecific mAbs were produced as generally described in Example 5I(c).

### 4. 6D: anti-PD-L1 x anti-ICOS

Anti-PD-L1 x anti-ICOS bottle-openers were generated with anti-PD-L1 Fabs derived from ABDs described in Example 2E and anti-ICOS scFvs as described in Example 4B. Sequences for exemplary anti-PD-L1 x anti-ICOS bispecific antibodies are depicted in Figure 66A-66C. The bottle-openers were produced as generally described in Example 5A.

### 5. 6E: Additional costim x checkpoint blockade bispecific antibodies function to enhance cytokine secretion

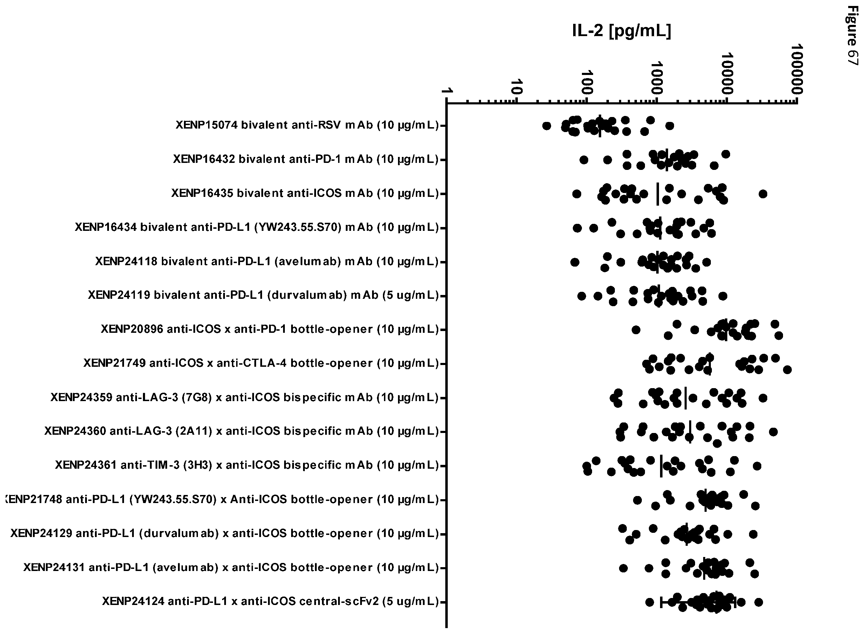

Human PBMCs were stimulated with 200 ng/mL SEB for 2 days. Cells were washed twice then restimulated with 100 ng/mL SEB and treated with the indicated concentrations of the indicated test articles (as described above). 24 hours after treatment, supernatants were assayed for IL-2. The data are depicted in Figure 67 and show that each of the additional costim x checkpoint blockade bispecific antibodies enhance IL-2 secretion in comparison to bivalent anti-RSV (XENP15074) control. Notably, the majority of these alternative checkpoint blockade antibodies (but not all, e.g. TIM-3 blockade) enhance IL-2 secretion in comparison to a bivalent anti-PD-1 antibody (XENP16432).

### G. Example 7: Monovalent ligation of ICOS is superior to bivalent crosslinking

In Example 5A(a), it was surprisingly found that anti-ICOS x anti-PD-1 antibody worked to enhance cytokine secretion despite monovalent binding of ICOS which is contrary to the crosslinking of costimulatory receptors such as ICOS which was thought to be necessary for stimulation of cytokine production. In Example 5H(a), it was surprisingly found that anti-ICOS x anti-PD-1 antibodies enhanced AKT phosphorylation (a signature of ICOS agonism) over bivalent anti-ICOS mAbs. In this section, we further examine this trend in which monovalent binding of ICOS appears to be superior to bivalent binding of ICOS.

### 1. 7A: Production of one-arm anti-ICOS Fab-Fc antibodies

Amino acid sequences for illustrative one-arm anti-ICOS Fab-Fc antibodies are listed in Figure 68A-68G. DNA encoding the three chains needed for antibody expression were generated by gene synthesis and were subcloned using standard molecular biology techniques into the expression vector pTT5. DNA was transfected into HEK293E cells for expression and resulting proteins were purified using standard techniques.

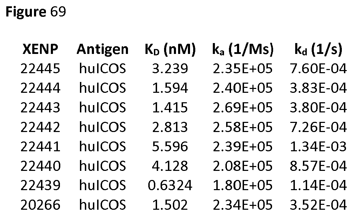

The resultant one-arm anti-ICOS Fab-Fc antibodies were characterized for affinity to human ICOS using Octet. Anti-mouse Fc (AMC) biosensors were used to capture mouse IgG2a Fc fusion of ICOS and dipped into multiple concentrations of the test articles. The resulting equilibrium dissociation constants (KD), association rates (ka), and dissociation rates (kd) are presented in Figures 69. Binding affinities and kinetic rate constants were obtained by analyzing the processed data using a 1:1 binding model using ForteBio Octet Data Analysis software (ForteBio).

### 2. 7B: Monovalent one-arm anti-ICOS Fab-Fc antibodies promote greater AKT activation in PBMCs than bivalent anti-ICOS antibody

PBMCs were stimulated with 100 ng/mL SEB for 2 days. Following stimulation, CD3+ T cells were isolated by negative selection using EasySep^{™} Human T Cell Enrichment Kit (STEMCELL Technologies, Vancouver, Canada) and then treated with indicated test articles in combination with plate bound anti-CD3 antibody (OKT3; 500 ng/mL). Cells were lysed 30 minutes after treatment and assayed for total AKT and phosphorylated AKT (Ser473) by a multiplexed phosphoprotein assay on MULTI-SPOT 384-Well Spot plates (Meso Scale Discovery, Rockville, Md.). The data are depicted in Figure 70 as percentage of AKT phosphorylated following treatment.

Consistent with Example X, the anti-ICOS x anti-PD-1 bispecific antibodies promoted significantly greater AKT activation than bivalent anti-ICOS antibody. Surprisingly, monovalent one-arm anti-ICOS Fab-Fc antibodies enhanced also promoted significantly greater AKT activation than bivalent anti-ICOS antibody to a level comparable to the bispecific antibodies.

### 3. 7C: Monovalent agonism of ICOS works with multiple anti-ICOS ABDs

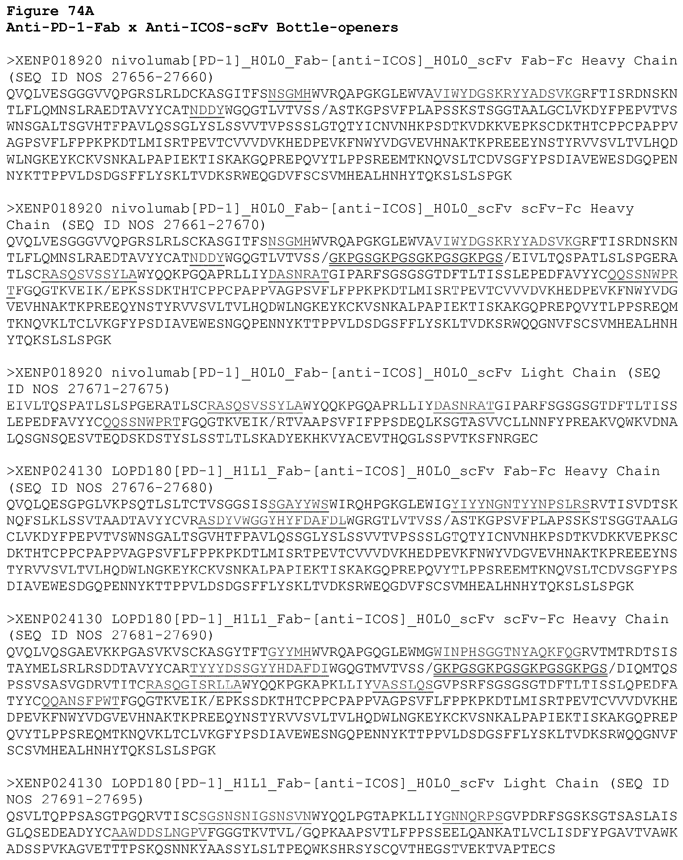

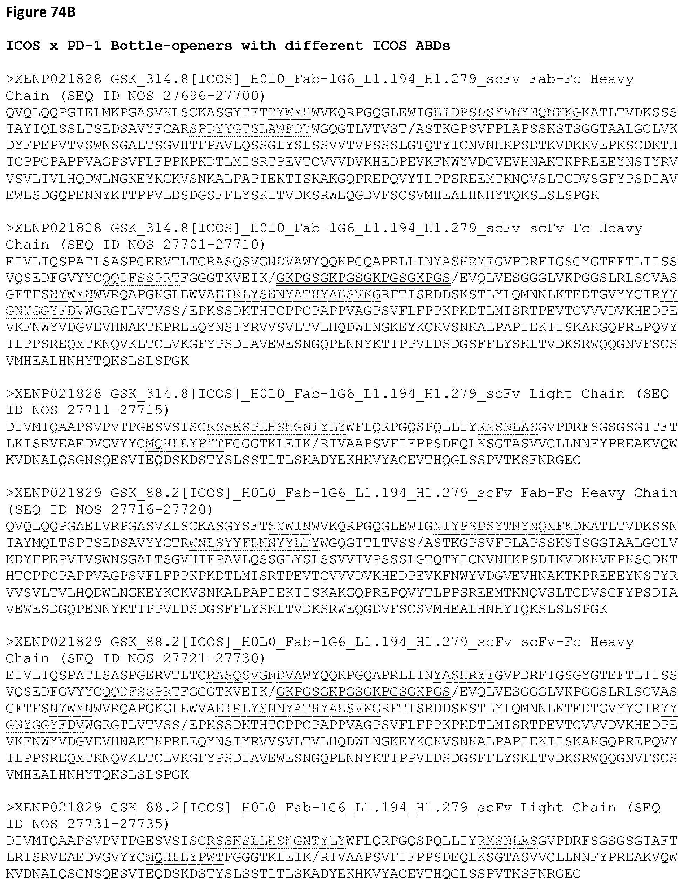

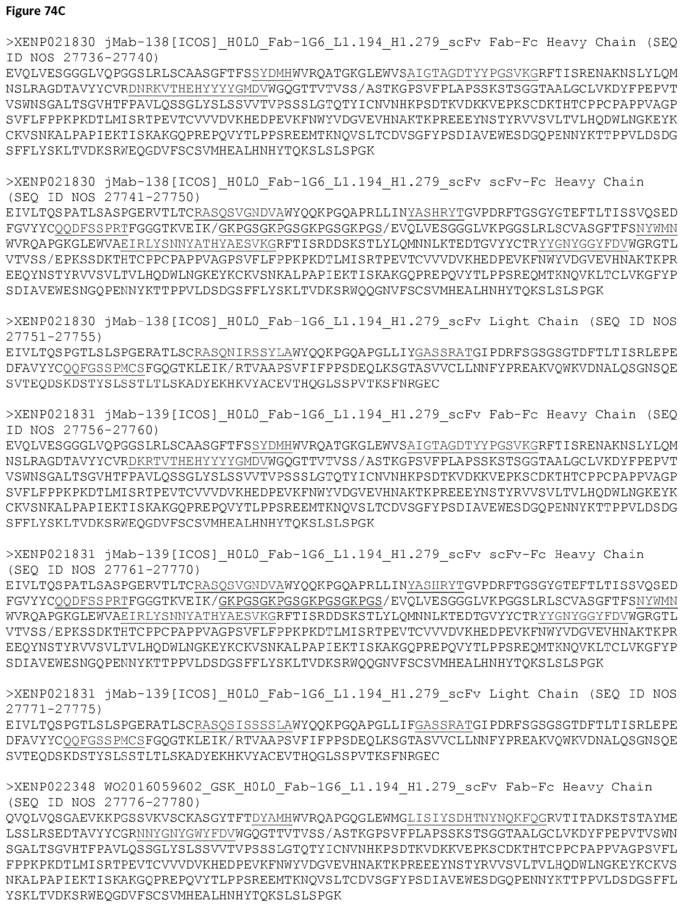

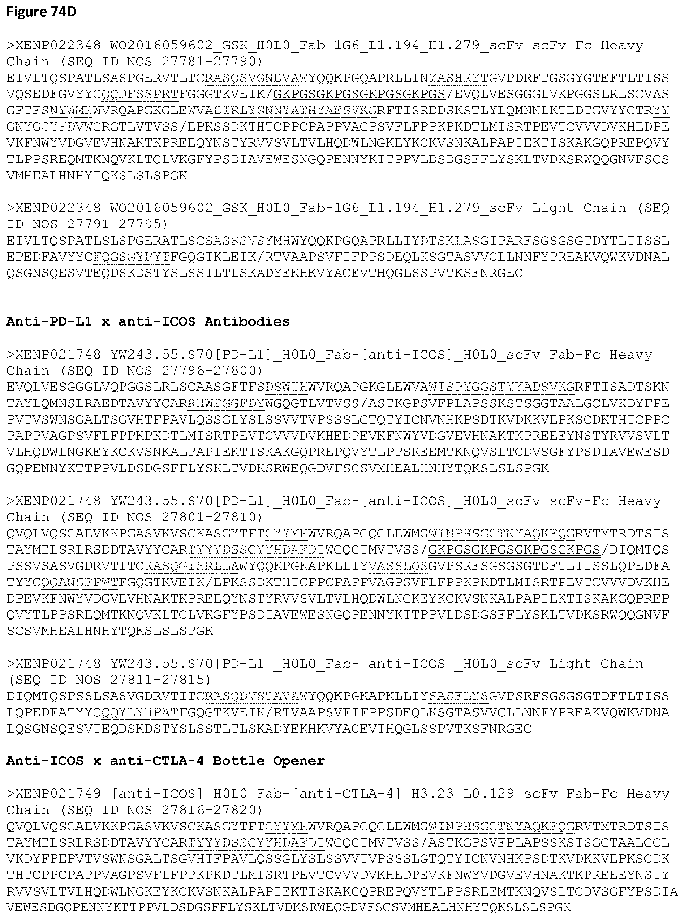

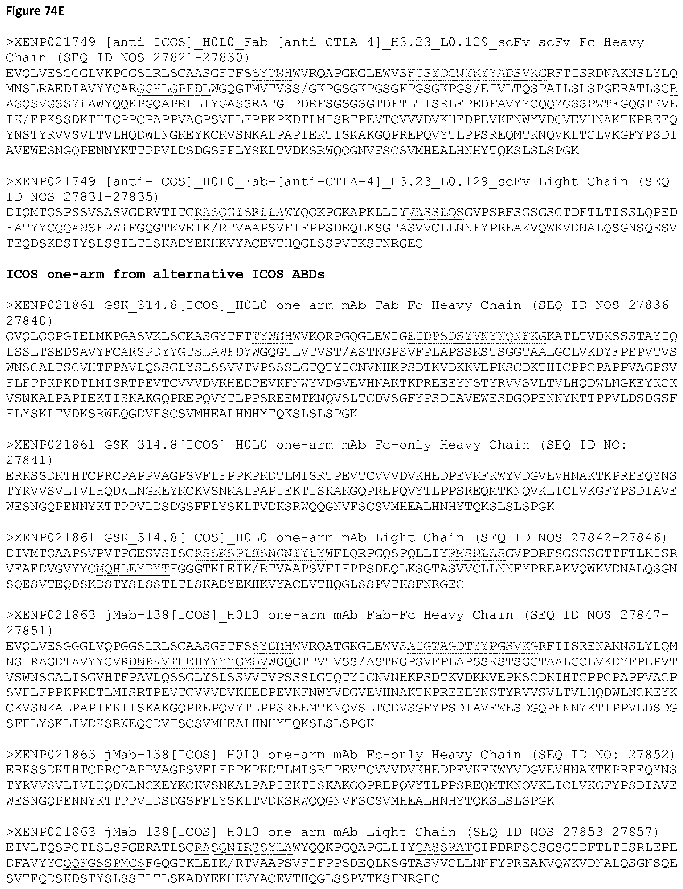

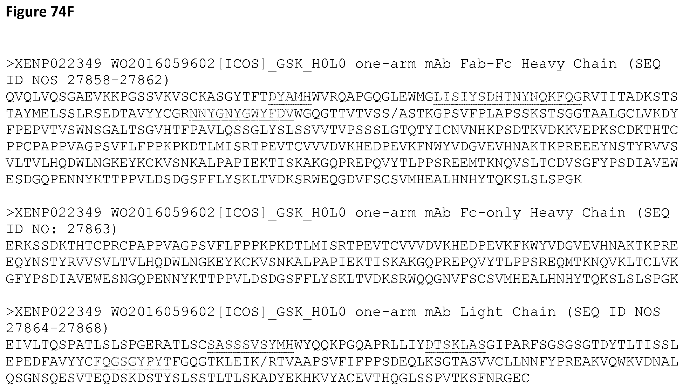

CD3+ T cells were isolated by negative selection using EasySep^{™} Human T Cell Enrichment Kit (STEMCELL Technologies, Vancouver, Canada) and then treated with indicated test articles in combination with plate bound anti-CD3 antibody (OKT3; 500 ng/mL). Cells were lysed 30 minutes after treatment and assayed for total AKT and phosphorylated AKT (Ser473) by a multiplexed phosphoprotein assay on MULTI-SPOT 384-Well Spot plates (Meso Scale Discovery, Rockville, Md.). The data is depicted in Figure 74 and show that monovalent anti-ICOS Fab-Fc antibodies comprising various anti-ICOS ABDs were able to induce AKT phosphorylation.

## Claims

1. A heterodimeric antibody comprising a first monomer, a second monomer, and a light chain, wherein the first monomer consists of amino acid sequence of SEQ ID NO:26362; the second monomer consists of the amino acid sequence of SEQ ID NO:26367; and the light chain consists of the amino acid sequence of SEQ ID NO:26377.

## Patentansprüche

1. Heterodimerer Antikörper, umfassend ein erstes Monomer, ein zweites Monomer und eine leichte Kette, wobei das erste Monomer aus der Aminosäuresequenz gemäß SEQ ID NO: 26362 besteht, das zweite Monomer aus der Aminosäuresequenz gemäß SEQ ID NO: 26367 besteht; und die leichte Kette aus der Aminosäuresequenz gemäß SEQ ID NO: 26377 besteht.

## Revendications

1. - Anticorps hétérodimérique comprenant un premier monomère, un second monomère et une chaîne légère, dans lequel le premier monomère est constitué de la séquence d'acides aminés de SEQ ID NO:26362 ; le second monomère est constitué de la séquence d'acides aminés de SEQ ID NO:26367 ; et la chaîne légère est constituée de la séquence d'acides aminés de SEQ ID NO:26377.
